# EUROPÄISCHE PATENTSCHRIFT

(11) **EP 1 890 695 B1**
(45) Veröffentlichungstag und Bekanntmachung des Hinweises auf die Patenterteilung: **20.02.2013**
(21) Anmeldenummer: 06753682.1
(22) Anmeldetag: 17.05.2006
(51) Int. Cl.: A61K 31/426, A61K 31/427, A61K 31/454, A61K 31/5377, A61K 31/541, A61K 31/55, C07D 277/34, C07D 417/06, C07D 277/42, C07D 277/54, C07D 417/12

(54) **VERWENDUNG VON 2,5-DISUBSTITUIERTEN THIAZOL-4-ON-DERIVATEN IN ARZNEIMITTELN**
USE OF 2,5-DISUBSTITUTED THIAZOL-4-ONE DERIVATIVES IN DRUGS
UTILISATION DE DERIVES DE THIAZOL-4-ONE 2,5-DISUBSTITUES DANS DES MEDICAMENTS

(30) Priorität: 20.05.2005 DE 102005024012
(43) Veröffentlichungstag der Anmeldung: 27.02.2008
(73) Patentinhaber: Grünenthal GmbH, 52078 Aachen (DE)
(72) Erfinder: FRANK, Robert, 52070 Aachen (DE); KLESS, Achim, 52074 Aachen (DE); JOSTOCK, Ruth, 52223 Stolberg (DE)
(74) Vertreter: Brosch, Oliver
(86) Internationale Anmeldenummer: PCT/EP2006/004666
(87) Internationale Veröffentlichungsnummer: WO 2006/122777

(56) Entgegenhaltungen:
- DE-A1- 1 770 583
- SONG YUNTAO ET AL: "Synthesis, structure-activity relationships, and in vivo evaluations of substituted di-tert-butylphenols as a novel class of potent, selective, and orally active cyclooxygenase-2 inhibitors. 1. Thiazolone and oxazolone series" JOURNAL OF MEDICINAL CHEMISTRY, Bd. 42, Nr. 7, 8. April 1999 (1999-04-08), Seiten 1151-1160, XP002400520 ISSN: 0022-2623
- AKERBLOM E B: "Synthesis and structure-activity relationships of a series of antibacterially active 5-(5-nitro-2-furfurylidene)thiazolones, 5-(5-nitro-2-furylpropenylidene) thiazolones, and 6-(5-nitro-2-furyl)-4H- 1,3-thiazinones" JOURNAL OF MEDICINAL CHEMISTRY, AMERICAN CHEMICAL SOCIETY. WASHINGTON, US, Bd. 17, Nr. 6, Juni 1974 (1974-06), Seiten 609-615, XP002272538 ISSN: 0022-2623
- DATABASE CA [Online] CHEMICAL ABSTRACTS SERVICE, COLUMBUS, OHIO, US; 2002, ZIMENKOVSKY, B. S. ET AL: "Study of trends in interaction between structure and biological activity among thiazolidine derivatives" XP002403429 gefunden im STN Database accession no. 2003:170522 & FIZIOLOGICHNO AKTIVNI RECHOVINI , (2), 58-64 CODEN: FARICW, 2002,
- DATABASE REGISTRY STN; RN 314076-45-4 16. Januar 2001 (2001-01-16), XP002403430
- DATABASE REGISTRY STN; RN 302549-02-6 13. November 2000 (2000-11-13), XP002403431
- DATABASE REGISTRY STN; RN 300560-11-6 31. Oktober 2000 (2000-10-31), XP002403432
- DATABASE REGISTRY STN; RN 311799-87-8 28. Dezember 2000 (2000-12-28), XP002403433
- DATABASE REGISTRY STN; RN 463316-89-4 21. Oktober 2002 (2002-10-21), XP002403434
- DATABASE REGISTRY STN; RN 377054-48-3 20. Dezember 2001 (2001-12-20), XP002403435
- DATABASE REGISTRY STN; RN 500107-22-2 20. März 2003 (2003-03-20), XP002403436
- DATABASE REGISTRY STN; RN 300560-36-5 31. Oktober 2000 (2000-10-31), XP002403437
- DATABASE REGISTRY STN; RN 300560-35-4 31. Oktober 2000 (2000-10-31), XP002403438
- DATABASE REGISTRY STN; RN 300560-19-4 31. Oktober 2000 (2000-10-31), XP002403439
- DATABASE REGISTRY STN; RN 300560-18-3 31. Oktober 2000 (2000-10-31), XP002403440
- DATABASE CA [Online] CHEMICAL ABSTRACTS SERVICE, COLUMBUS, OHIO, US; 1967, BESYADETS'KA, O. I.: "Synthesis of 5-substituted 2-piperidino-2-thiazolin-4-ones" XP002403441 gefunden im STN Database accession no. 1967:482152 & FARMATSEVTICHNII ZHURNAL (KIEV) , 22(1), 9-11 CODEN: FRZKAP; ISSN: 0367-3057, 1967,
- HOLZER ET AL: "TRPV1 and the gut: from a tasty receptor for a painful vanilloid to a key player in hyperalgesia" EUROPEAN JOURNAL OF PHARMACOLOGY, AMSTERDAM, NL, Bd. 500, Nr. 1-3, 1. Oktober 2004 (2004-10-01), Seiten 231-241, XP005480419 ISSN: 0014-2999
- SZALLASI ARPAD ET AL: "Vanilloid receptor TRPV1 antagonists as the next generation of painkillers. Are we putting the cart before the horse?" JOURNAL OF MEDICINAL CHEMISTRY, Bd. 47, Nr. 11, 20. Mai 2004 (2004-05-20), Seiten 2717-2723, XP002400521 ISSN: 0022-2623
- APPENDINO G ET AL: "TRPV1 (VANILLOID RECEPTOR, CAPSAICIN RECEPTOR) AGONISTS AND ANTAGONISTS" EXPERT OPINION ON THERAPEUTIC PATENTS, ASHLEY PUBLICATIONS, GB, Bd. 13, Nr. 12, 2003, Seiten 1825-1837, XP002320750 ISSN: 1354-3776
- WINTER J ET AL: "Capsaicin and pain mechanisms.", BRITISH JOURNAL OF ANAESTHESIA AUG 1995 LNKD- PUBMED:7577249, vol. 75, no. 2, August 1995 (1995-08), pages 157-168, ISSN: 0007-0912

## Beschreibung

Die vorliegende Erfindung betrifft 2,5-disubstituierte Thiazol-4-on-Derivate und deren Verwendung zur Herstellung von Arzneimitteln, Verfahren zu ihrer Herstellung und Arzneimittel enthaltend diese Verbindungen.

Die Behandlung von Schmerz, insbesondere von neuropathischem Schmerz, hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufrieden stellende Schmerzbehandlung für den Patienten zu verstehen ist, dokumentiert sich auch in der großen Anzahl von wissenschaftlichen Arbeiten, die auf dem Gebiet der angewandten Analgetik bzw. der Grundiagenforschung zur Nociception in letzter Zeit erschienen sind.

Einen geeigneten Ansatzpunkt zur Behandlung von Schmerz, insbesondere von neuropathischem Schmerz, stellt der Vanilloid-Rezeptor vom Subtyp 1 (VR1/TRPV1) dar, der häufig auch als Capsaicln-Rezeptor bezeichnet wird. Dieser Rezeptor wird u.a. durch Vanilloide wie z.B. Capsaicin, Hitze und Protonen stimuliert und spielt eine zentrale Rolle bei der Schmerzentstehung (P. Holzer, European Journal of Pharmacology (2004), 500, 231-241). Darüber hinaus ist er für eine Vielzahl weiterer physiologischer und pathophysiologischer Prozesse von Bedeutung wie beispielsweise Migräne; Depressionen; neurodegenerativen Erkrankungen; kognitiven Erkrankungen; Angstzuständen; Epilepsie; Husten; Diarrhöe; Pruritus; Störungen des kardiovaskulären Systems; Störungen der Nahrungsaufnahme; Medikamentenabhängigkeit; Medikamentenmißbrauch und insbesondere Haminkontinenz.

Es sind bereits 2,5-disubstituierte Thiazolon-Derivate bekannt, die eine entzündungshemmende Wirkung aufweisen (Y. Song et al., J. Med. Chem. (1999), 42(7), 1151-1160; B. S. Zimenkovsky, Fiziologichno Aktivni Rechovini (2002), (2), 58-64). Über 2,5-disubstituierte Thiazolon-Derivate mit einer antibakteriellen und/oder antimykotischen Wirkung wurde ebenfalls bereits berichtet (E. B. Akerblom, J. Med. Chem. (1974), 17, 609-615; DE 17 70 583 A1). Weitere 2,5-disubstituierte Thiazolon-Derivate sind kommerziell erhältlich (Anbieter: z.B. Otava, Interchim, Ambinter und ChemDiv; Database Registry STN, RN: 314076-45-4, 302549-02-6, 300660-11-6, 311799-87-8. 463316-89-4, 377054-48-3, 500107-22-2. 300560-36-5, 300560-35-4, 300560-19-4, 300560-18-3). Die Synthese 5-substituierter 2-Piperidino-2-thiazolin-4-on-Derivate ist aus B. S. Zimenkovsky, Fiziologichno Aktivni Rechovini (2002), (2), 58-64 bekannt.

Kürzlich wurde über Verbindungen mit einer Affinität zum VR1 (VRPV1)-Rezeptor berichtet, die eine analgetische Wirksamkeit aufweisen (A. Szallasi et al., J. Med. Chem. (2004), 47(11), 2717-2723; G. Appendino et al., Expert Opin. Ther. Patents (2003), 13(12), 1825-1837). Diese Verbindungen welsen jedoch keine Thiazol-Substruktur auf.

Eine Aufgabe der vorliegenden Erfindung bestand daher darin, neue Verbindungen zur Verfügung zu stellen, die sich Insbesondere als pharmakologische Wirkstoffe in Arzneimitteln eignen, vorzugsweise in Arzneimitteln zur Behandlung von Störungen oder Krankheiten, die zumindest teilweise durch Vanilloid-Rezeptoren 1 (VR1/TRPV1-Rezeptoren) vermittelt werden.

Überraschenderweise wurde nun gefunden, dass 2,5-disubstituierte Thiazol-4-on-Derivate der nachstehend angegebenen allgemeinen Formel I sich zur Bekämpfung von Schmerzen eignen und eine ausgezeichnete Affinität zum Vanilloid-Rezeptor vom Subtyp 1 (VR1/TRPV1-Rezeptor) aufweisen und sich daher insbesondere zur Prophylaxe und/oder Behandlung von Störungen oder Krankheiten eignen, die zumindest teilweise durch Vanilloid-Rezeptoren 1 (VR1/TRPV1) vermittelt werden.

Ein Gegenstand der vorliegenden Erfindung ist daher die Verwendung wenigstens eines 2,5-disubstituierten Thiazol-4-on-Derivats der allgemeinen Formel I, 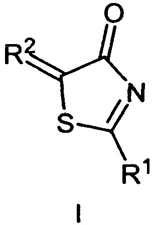 worin
- R¹: für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
für einen unsubstituierten oder wenigstens einfach substituierten Aryl-Rest, der mit einem unsubstituierten oder wenigstens einfach substituierten mono-oder polyzyklischen Ringsystem kondensiert sein kann;
für eine -NR³R⁴-Gruppe;
für eine -NR⁵-C(=O)-R⁶-Gruppe
oder für eine -NR⁷-C(=O)-NR⁸R⁹ Gruppe steht;
- R²: für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
für CH-U-X;
für CH-CH₂-V-Y, CH-CH(CH₃)-V-Y, CH-CHCI-V-Y, CH-CHBr-V-Y, CH-CHF-V-Y oder CH-CH(OH)-V-Y;
oder für CH-CH=C(CH₃)-W-Z, CH-CH=CH-W-Z, CH-C(CH₃)=CH-W-Z, CH-C(Phenyl)=CH-W-Z, CH-CBr=CH-W-Z, CH-CCl=CH-W-Z, CH-CF=CH-W-Z, CH-C(OH)=CH-W-Z, CH-CH₂-CH₂-W-Z, CH-CH₂-CH(CH₃)-W-Z oder CH-CH(CH₃)-CH₂-W-Z steht;
wobei U, V und W jeweils nicht vorhanden sein können oder jeweils unabhängig voneinander für einen Rest ausgewählt aus der Gruppe bestehend aus O, S, N(H), N(CH₃), N(C₂H₅) und N(CH(CH₃)₂] stehen;
- R³: für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopentyl Cyclohexyl, Cycloheptyl, Cyclopentenyl, Cyclohexenyl. Cycloheptenyl, Imidazolidinyl, Tetrahydrofuranyl, Tetrahydrothiophenyl, Pyrrolidinyl, Piperidinyl, Morpholinyl, Thiomorpholinyl und Azepanyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl substituiert sein kann; wobei jeweils der zyklische Teil der Reste -NH-Phenyl, -NH-Pyrldlnyl, -N(CH₃)-Phenyl, -N(C₂H₅)-Phenyl, -N[CH(CH₃)₂]-Phenyl, -N(CH₃)-Pyridinyl, -N(C₂H₅)-Pyridinyl und - N[CH(CH₃)₂]-Pyridinyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃. Phenyl und -O-Benzyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ und -NH-C(CH₃)₃ substituiert sein kann; steht;
- R⁴: für einen Wasserstoff-Rest
oder für einen linearen oder verzweigten, gesättigten oder ungesättigten, unsubstituierten oder wenigstens einfach substituierten, ggf. wenigstens ein Heteroatom als Kettenglied aufweisenden aliphatischen Rest steht;
- R⁵, R⁷ und R⁸,: unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
- R⁶ und R⁸,: unabhängig voneinander, jeweils
für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, stehen;
- X: für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹⁰ substituiert sein kann, steht;
- Y: für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹¹ substituiert sein kann, steht;
- Z: für einen linearen oder verzweigten, gesättigten oder ungesättigten, unsubstituierten oder wenigstens einfach substituierten aliphatischen Rest;
oder für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹². substituiert sein kann, steht;
- R¹⁰, R¹¹ und R¹²,: unabhängig voneinander, jeweils
für einen Rest ausgewählt aus der Gruppe bestehend aus F, Cl, Br, I, -CN, - CF₃, SF₅, -OH, -O-C₁₋₁₀-Alkyl, -O-C₂₋₁₀-Alkenyl, -NH₂, -O-CF₃, -S-CF₃, - SH, -S-C₁₋₅-Alkyl, -C₁₋₁₀-Alkyl. -C(=O)-OH. -C(=O)O-C₁₋₅-Alkyl, -O-C(=O)-C₁₋₅-Alkyl, -NH-C₁₋₅-Alkyl, -N(C₁₋₆-Alkyl)₂, -NH-C(=O)-O-C₁₋₅-Alkyl, -C(=O)-H, - C(=O)-C₁₋₅-Alkyl, -C(=O)-NH₂, -C(=O)-NH-C₁₋₅-Alkyl, -C(=O)-N-(C₁₋₅-Alkyl)₂, - S(=O)₂-C₁₋₅-Alkyl, -S(=O)₂-Phenyl, -O-S(=O)₂-Phenyl, -NH-S(=O)₂-C₁₋₅-Alkyl, - S(=O)₂-NH-C₁₋₆-Allkyl, Cyclohexyl, Cyclopentyl, Pyridinyl, Pyridazinyl, -(CH₂)-Benzo[b]furanyl, -O-Phenyl, -O-Benzyl, Phenyl und Benzyl stehen; wobei jeweils der zyklische Teil der Reste Pyridinyl, Cyclopentyl, Cyclohexyl, Pyridezinyl, -S(=O)₂-Phenyl, -O-S(=O)₂-Phenyl, -O-Phenyl, -O-Benzyl, Phenyl, -(CH₂)-Benzo[b]furanyl und Benzyl substituiert sein kann;
für eine -C(=O)-NR¹³-(CH₂)ₘ-NR¹⁴-R¹⁵-Gruppe mit m gleich 0, 1, 2, 3, 4 oder 5;
oder für eine -C(=O)-R¹⁶-Gruppe stehen:
- R¹³ und R¹⁴,: unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
- R¹⁵: für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, steht;
und
- R¹⁶: für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann, steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate;
zur Herstellung eines Arzneimittels zur Prophylaxe und/oder Behandlung von Schmerz.

Aliphatische Reste umfassen im Sinne dieser Erfindung azyklische gesättigte oder ungesättigte Kohlenwasserstoffreste, die verzweigt oder geradkettig sowie unsubstituiert oder einfach substituiert oder mehrfach gleich oder verschieden substituiert sein können, bevorzugt mit 1 bis 20 (d.h. 1, 2, 3, 4, 5, 6, 7, 8, 9, 10, 11, 12, 13, 14, 15, 16, 17, 18, 19 oder 20), besonders bevorzugt 1 bis 12 (d.h. 1, 2, 3, 4, 5, 6, 7, 8, 9, 10, 11 oder 12), ganz besonders bevorzugt 1 bis 6 (d.h. 1, 2, 3, 4, 5 oder 6) Kohlenstoffatomen, d.h. C₁₋₂₀-, C₁₋₁₂-, C₁₋₆-Alkyle, C₂₋₂₀-, C₂₋₁₂-, C₂₋₆-Alkenyle und C₂₋₂₀-, C₂₋₁₂-, C₂₋₆-Alkinyle. Dabei weisen Alkenyle mindestens eine C-C-Doppelbindung und Alkinyle mindestens eine C-C-Dreifachbindung auf. Vorteilhaft werden aliphatische Reste ausgewählt aus der Gruppe, die Methyl, Ethyl, n-Propyl, Isopropyl, n-Butyl, Isobutyl, sec-Butyl, tert-Butyl, n-Pentyl, Isopentyl, neo-Pentyl, n-Hexyl, 2-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosanyl, Ethenyl (Vinyl), Ethinyl, Propenyl (-CH₂CH=CH₂, -CH=CH-CH₃, -C(=CH₂)-CH₃), 2-Methyl-propenyl, Propinyl (-CH₂-C≡CH, -CC-CH₃), Butenyl, Butinyl, Pentenyl, Pentinyl, Hexenyl, Hexinyl, Octenyl und Octinyl umfaßt.

Die vorstehend genannten aliphatischen Reste können bevorzugt 1, 2 oder 3 Heteroatome ausgewählt aus der Gruppe umfassend Sauerstoff, Schwefel und Stickstoff, d. h. -N(H)- und -N(C₁₋₆-Alkyl), aufweisen.

Beispielhaft für aliphatische Reste, die 1, 2 oder 3 Heteroatome aufweisen, seien -(CH₂)-(CH₂)-O-CH₃, -(CH₂)-(CH₂)-(CH₂)-O-CH₃, -(CH₂)-(CH₂)-(CH₂)-N(C₂H₅)-(C₂H₅), -(CH₂)-(CH₂)-S-CH₃, -(CH₂)-(CH₂)-(CH₂)-S-CH₃, -(CH₂)-(CH₂)-(CH₂)-N(CH₃)-(CH₃) und -(CH₂)-O-CH₃ genannt.

Im Zusammenhang mit aliphatischen Resten versteht man unter dem Begriff "substituiert" - soweit der Ausdruck nicht an anderer Stelle der Beschreibung bzw. in den Ansprüchen definiert ist - im Sinne dieser Erfindung die einfache oder mehrfache Substitution, bevorzugt ein-, zwei-, drei-, vier-; fünf-, sechs-, sieben-, acht- oder neunfache Substitution, von einem oder mehreren Wasserstoffatomen durch beispielsweise F, Cl, Br, I, -CN, -NO₂, -OH, -SH und -NH₂, wobei die mehrfache Substitution entweder an verschiedenen oder an gleichen Atomen mehrfach, z.B. zwei- oder dreifach, erfolgt, beispielsweise dreifach am gleichen C-Atom wie im Falle von -CF₃ oder -CH₂CF₃ oder an verschiedenen Stellen wie im Falle von -CH(OH)-CH=CCl-CH₂Cl. Die Mehrfachsubstitution kann mit dem gleichen oder mit verschiedenen Substituenten erfolgen. Besonders bevorzugte substituierte aliphatische Reste sind -CF₃, -C₂F₅, -CH₂F, -CHF₂, -CF₂-CF₂-CF₃, -CH₂-Cl, -CH₂-Br, -CH₂-CH₂-Cl, -CH₂-CH₂-Br, -CH₂-CH₂-CH₂-Br und -CH₂-CH₂-CH₂-Cl.

Cycloaliphatische Reste im Sinne dieser Erfindung sind zyklische gesättigte oder ungesättigte Kohlenwasserstoffreste mit bevorzugt 3, 4, 5, 6, 7, 8, 9, 10, 11, 12, 13, 14, 15 oder 16, besonders bevorzugt 3, 4, 5, 6, 7 oder 8 Kohlenstoffatomen, wobei jeder Rest unsubstituiert oder einfach substituiert oder mehrfach gleich oder verschieden substituiert sein kann. Cycloaliphatische Reste weisen bevorzugt 1, 2, 3, 4 oder 5 Heteroatome unabhängig voneinander ausgewählt aus der Gruppe bestehend aus Sauerstoff, Stickstoff (NH) und Schwefel auf.

Beispielhaft für cycloaliphatischen Rest, die mit einen mono- oder polyzyklischen Ringsystem kondensiert sein können, seien Cyclopropyl, Cyclobutyl, Cyclopentyl, Cyclohexyl, Cyclooctyl, Cyclononyl, Cyclodecyl, Cycloundecyl, Cyclododecyl, Cycloheptyl, Cyclopentenyl, Cyclohexenyl, Cycloheptenyl, [6,6]-Dimethyl-[3.1.1]-bicycloheptyl, Adamantyl, Imidazolidinyl, Tetrahydrofuranyl, Tetrahydrothiophenyl, Pyrrolidinyl, Piperidinyl, Morpholinyl, Piperazinyl, Thiomorpholinyl, Tetrahydropyranyl, Azepanyl, Diazepanyl, Dithiolanyl, Indanyl, Indenyl, (1,4)-Benzodioxanyl, (1,2,3,4)-Tetrahydronaphthyl, (1,2,3,4)-Tetrahydrochinolinyl, (1,2,3,4)-Tetrahydroisochinolinyl, (1,2,3,4)-Tetrahydrochinazolinyl, (1,3,4,5)-Tetrahydropyrido[4,3-b]indolyl, (3,4)-Dihydro-1H-isochinolinyl, (1,3,4,9)-Tetrahydro-[b]-carbolinyl, Imidazolidinyl, [3,4]-Dihydro-2H-1,4-benzoxazinyl, (2,3)-Dihydro-1H-isoindolyl, (2,3)-Dihydro-indolyl und (1,3)-Thiazolidinyl genannt.

Unter einem mono- oder polyzyklischen Ringsystem werden im Sinne der vorliegenden Erfindung mono- oder polyzyklische Kohlenwasserstoffreste verstanden, die gesättigt oder ungesättigt sein und ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind. Ein solches mono- bzw. polyzyklisches Ringsystem kann beispielsweise mit einem Aryl-Rest oder einem Heteroaryl-Rest kondensiert (anneliert) sein.

Sofern ein polyzyklisches Ringsystem wie beispielsweise ein bizyklisches Ringsystem vorliegt, können die verschiedenen Ringe, jeweils unabhängig voneinander, einen unterschiedlichen Sättigungsgrad aufweisen, d.h. gesättigt oder ungesättigt sein. Bevorzugt ist ein polyzyklisches Ringsystem ein bizyklisches Ringsystem.

Beispielhaft für Aryl-Reste, die mit einem mono- bzw. polyzyklischen Ringsystem kondensiert sind, seien [1,3]-Benzodioxolyl, [1,4]-Benzodioxanyl, [1,2,3,4]-Tetrahydronaphthyl, [1,2,3,4]-Tetrahydrochinolinyl, [1,2,3,4]-Tetrahydrochinazolinyl und [3,4]-Dihydro-2H-1,4-benzoxazinyl genannt.

Im Zusammenhang mit cycloaliphatischen Resten und mono- oder polyzyklischen Ringsystemen versteht man unter dem Begriff "substituiert" - soweit der Ausdruck nicht an anderer Stelle der Beschreibung bzw. in den Ansprüchen definiert ist - im Sinne dieser Erfindung die einfache oder mehrfache Substitution, bevorzugt ein-, zwei-, drei-, vier-, fünf-, sechs-, sieben-, acht- oder neunfache Substitution, von einem oder mehreren Wasserstoffatomen durch beispielsweise Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, - SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, - NH-C₁₋₅-Alkyl, -N(C₁₋₅-Alkyl)₂, -NH-Phenyl, -NH-Pyridinyl, -N(C₁₋₅-Alkyl)-Phenyl, - N(C₁₋₅-Alkyl)-Pyridinyl, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl, wobei jeweils der zyklische Teil der Reste -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, -S(=O)₂-Phenyl, -O-Phenyl, -O-Benzyl, Phenyl, -(CH₂)-Benzo[b]furanyl und Benzyl bevorzugt mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann.

Unter dem Ausdruck Aryl-Rest ist für die Zwecke der vorliegenden Erfindung ein Rest zu verstehen, der bevorzugt aus der Gruppe, die Phenyl, Naphthyl, Phenanthrenyl und Anthracenyl umfaßt, ausgewählt ist und unsubstituiert oder einfach oder mehrfach gleich oder verschieden substituiert ist. Besonders bevorzugt ist Aryl ein unsubstituiertes oder einfach substituiertes oder mehrfach, beispielsweise zwei-, drei- vier oder fünffach, gleich oder verschieden substituiertes Phenyl, 1-Naphthyl oder 2-Naphthyl.

Heteroaryl-Reste im Sinne der vorliegenden Erfindung sind solche Heterozyklen, die heteroaromatisch sind. Heteroaryl-Reste sind bevorzugt 5- bis 14-gliedrig, d. h. 5-, 6- , 7-, 8-, 9-, 10-, 11-, 12-, 13- oder 14-gliedrig und weisen bevorzugt 1, 2, 3, 4 oder 5 Heteroatome unabhängig voneinander ausgewählt aus der Gruppe umfassend Sauerstoff, Stickstoff und Schwefel auf. Jeder Heteroaryl-Rest kann unsubstituiert oder einfach substituiert oder mehrfach, bevorzugt zwei-, drei-, vier- oder fünffach, gleich oder verschieden substituiert vorliegen.

Beispielhaft für Heteroaryl-Rest im Sinne der vorliegenden Erfindung seien Thiophenyl, Furanyl, Pyrrolyl, Pyrazolyl, Pyranyl, Pyridinyl, Imidazolyl, Indolyl, Isoindolyl, Benzo[b]furanyl, Benzo[b]thiophenyl, Thiazolyl, Oxazolyl, Isoxazolyl, Pyridazinyl, Pyrazinyl, Pyrimidinyl, Indazolyl, Chinazolinyl, Chinolinyl, Isochinolinyl, Benzimidazolinyl, Benzoxazolyl, Benzisoxazolyl, Benzothiazolyl, Benzo[2,1,3]thiadiazolyl, [1,2,3]-Benzothiadiazolyl, [2,1,3]-Benzoxadiazolyl und [1,2,3]-Benzoxadiazolyl genannt.

In Bezug auf Aryl- und Heteroaryl-Reste versteht man im Sinne dieser Erfindung unter "substituiert" die ein- oder mehrfache, bevorzugt die ein-, zwei-, drei-, vier- oder fünffache, Substitution eines oder mehrerer Wasserstoffatome des Ringsystems durch geeignete Substituenten. Soweit die Bedeutung dieser geeigneten Substituenten im Zusammenhang mit Aryl- oder Heteroaryl-Resten nicht an anderer Stelle der Beschreibung oder in den Ansprüchen definiert ist, sind geeignete Substituenten F, Cl, Br, I, -CN, -CF₃, -SF₅, -OH, -O-C₁₋₁₀-Alkyl, -O-C₂₋₁₀-Alkenyl, - NH₂, -NO₂, -O-CF₃, -S-CF₃, -SH, -S-C₁₋₅-Alkyl, -C₁₋₁₀-Alkyl, -C(=O)-OH, -C(=O)-O-C₁-₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, - C(=O)-H, -C(=OFC₁₋₅-Alkyl, -C(=O)-NH₂, -C(=O)-NH-C₁₋₅-Alkyl, -C(=O)-N-(C₁₋₅-Alkyl)₂, -S(=O)₂-C₁₋₅-Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl; wobei jeweils der zyklische Teil der Reste Pyridinyl, Cyclopentyl, Cyclohexyl, Pyridazinyl, -S(=O)₂-Phenyl, -O-S(=O)₂-Phenyl, -O-Phenyl, -O-Benzyl, Phenyl, -(CH₂)-Benzo[b]furanyl und Benzyl bevorzugt mit 1, 2, 3, 4 oder 5 Substituenten unabhängig voneinander ausgewählt aus der Gruppe bestehend aus F, Cl, Br, -OH, -CF₃, -SF₅, -CN, -NO₂, - C₁₋₅-Alkyl, -O-C₁₋₅-Alkyl, -O-CF₃, -S-CF₃, Phenyl und -O-Benzyl substituiert sein kann. Die Mehrfachsubstitution erfolgt dabei mit dem gleichen oder mit unterschiedlichen Substituenten.

Die vorstehend genannten linearen oder verzweigten Alkylen-, Alkenylen- oder Alkinylen-Gruppen weisen bevorzugt 1 bis 5 Kohlenstoffatome auf, d.h. es handelt sich um C₁₋₅-Alkylen, C₂₋₅-Alkenylen oder C₂₋₅-Alkinylen-Gruppen, die jeweils unsubstituiert oder bevorzugt mit 1, 2, 3, 4 oder 5 Substituenten unabhängig voneinander ausgewählt aus der Gruppe bestehend aus F, Cl, Br, -OH, -SH, -NH₂, - CN, -NO₂ und Phenyl substituiert sein können, wobei der Phenyl-Rest bevorzugt mit 1, 2, 3, 4 oder 5 Substituenten unabhängig voneinander ausgewählt aus der Gruppe bestehend aus Methyl, Ethyl, n-Propyl, Isopropyl, n-Butyl, Isobutyl, sec-Butyl, tert-Butyl, n-Pentyl, Isopentyl und neo-Pentyl substituiert sein kann.

Besonders bevorzugt können Alkylen-Gruppen ausgewählt werden aus der Gruppe bestehend aus -(CH₂)-, -(CH₂)₂-, -C(H)(CH₃)-, -C(CH₃)₂-, -(CH₂)₃-, -(CH₂)₄-, -(CH₂)₅-, - C(H)(CH₃)-(CH₂)-, -C(H)(C₂H₅)-(CH₂)-, -C(Phenyl)₂- und -C(H)(Phenyl)-.

Besonders bevorzugt können Alkenylen-Gruppen ausgewählt werden aus der Gruppe bestehend aus -CH=CH-, -C(CH₃)=CH-, -C(C₂H₅)=CH-, -CH=C(CH₃)-, - CH=C(C₂H₅)-, -CH=C(Phenyl)-. -CH=C(p-Tolyl), -C(Phenyl)=CH- und -C(p-Tolyl)=CH-.

Besonders bevorzugt als Alkinylen-Gruppe ist eine -C≡C-Gruppe.

Besonders bevorzugt ist die Verwendung wenigstens eines 2,5-disubstituierten Thiazol-4-on-Derivats der vorstehend angegebenen allgemeinen Formel I, worin
- R¹: für einen der folgenden Reste steht 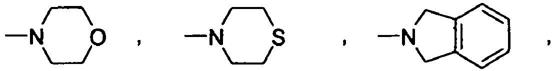 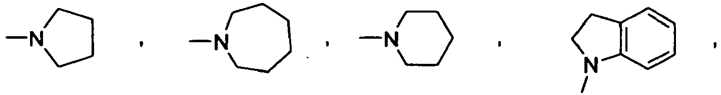 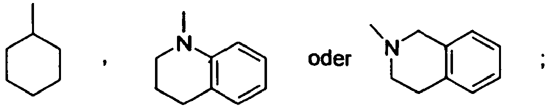 wobei der Rest mit 1, 2 oder 3 Substituenten unabhängig voneinander ausgewählt aus der Gruppe bestehend aus -OH, -O-CH₃, -O-C₂H₅, Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, - NH-Pyridinyl, -N(CH₃)-Phenyl, -N(C₂H₅)-Phenyl, -N[CH(CH₃)₂]-Phenyl, - N(CH₃)-Pyridinyl, -N(C₂H₅)-Pyridinyl, -N[CH(CH₃)₂]-Pyridinyl und Phenyl substituiert sein kann; wobei jeweils der zyklische Teil des Phenyl-Restes mit 1, 2 oder 3 Substituenten unabhängig voneinander ausgewählt aus der Gruppe bestehend aus F, Cl, Br und -CF₃ substituiert sein kann;
für einen der folgenden Reste steht 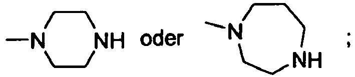 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus -OH, -O-CH₃ und -O-C₂H₅ substituiert sein kann;
für eine NR³R⁴-Gruppe steht
oder für eine NR⁷-C(=O)-NR⁸R⁹ Gruppe steht;
- R²: für einen 1,3-Dihydro-indol-2-onyl- oder einen 1,3-Dihydro-indol-2-thionyl-Rest steht;
für CH-X steht;
für CH-CH₂-Y, CH-CH(CH₃)-Y, CH-CHCl-Y, CH-CHBr-Y, CH-CHF-Y oder CH-CH(OH)-Y steht;
oder für CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(Phenyl)=CH-Z, CH-CBr=CH-Z, CH-CCI=CH-Z, CH-CF=CH-Z, CH-C(OH)=CH-Z, CH-CH₂-CH₂-Z, CH-CH₂-CH(CH₃)-Z oder CH-CH(CH₃)-CH₂-Z steht;
- R³: für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopentyl Cyclohexyl, Cycloheptyl, Cyclopentenyl, Cyclohexenyl und Cycloheptenyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, - NH-Pyridinyl, -N(CH₃)-Phenyl, -N(C₂H₅)-Phenyl, -N[CH(CH₃)₂]-Phenyl, - N(CH₃)-Pyridinyl, -N(C₂H₅)-Pyridinyl, -N[CH(CH₃)₂]-Pyridinyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
- R⁴: für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
- R⁷ und R³,: unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
- R⁹: für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CF₃, -S-CF₃, Methyl, Ethyl, n-Propyl, Isopropyl, n-Butyl, sec-Butyl, Isobutyl und tert-Butyl substituiert sein kann;
- X: für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht; der ggf. mit 1, 2, 3, 4 oder 5 gleichen oder verschiedenen Substituenten R¹⁰ substituiert sein kann;
- Y: für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht, der ggf. mit 1, 2, 3, 4 oder 5 gleichen oder verschiedenen Substituenten R¹¹ substituiert sein kann;
- Z: für einen Rest ausgewählt aus der Gruppe bestehend aus Methyl, Ethyl, n-Propyl, isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht;
oder für einen Rest ausgewählt aus der Gruppe bestehend aus Phenyl, Naphthyl, Phenanthrenyl. (1,3)-Benzodioxolyl, (1,4)-Benzodioxanyl. Thiophenyl, Furanyl, Pyrrolyl, Pyrazolyl, Pyrazinyl, Pyridinyl, Pyridazinyl, Pyrimidinyl, Chinolinyl und isochinolinyl steht; der ggf. mit 1, 2, 3, 4 oder 5 gleichen oder verschiedenen Substituenten R¹² substituiert sein kann;
- R¹⁰: für einen Rest ausgewählt aus der Gruppe bestehend aus F, Cl, Br, I, -CN, - CF₃, -SF₅, -OH, -O-CH₃, -OC₂H₅, -O-CH₂-CH₂-CH₃. -O-CH₂-CH₂-CH₂-CH₃, - O-C(CH₃)₃, -O-CH₂-CH₂-H₂-CH₂-CH₃, -O-CH₂-CH₂-C(CH₃)₃, -O-CH=CH₂, -0-CH₂-CH=CH₂, -NH₂, -O-CF₃, -S-CF₃, -SH, -S-CH₃, -S-C₂H₆, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl, n-Octyl, - C(=O)OH. -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-(₄H₅, -NH-C(=O)-CH₃, -NH-C(O)-C₂H₅, -NH-C(=O)-C(CH₃)₃, -O-S(=O)₂-Phenyl, -O-Phenyl, -O-Benzyl, Phenyl und Benzyl, steht;
für eine -C(=O)-NR¹³-(CH₂)ₘ-NR¹⁴-R¹⁵-Gruppe mit m gleich 0, 1, 2 oder 3 steht;
oder für eine -C(=O)-R¹⁶-Gruppe steht;
- R¹¹: für einen Rest ausgewählt aus der Gruppe bestehend aus F, Cl, Br, -SF₅, Methyl, Ethyl, n-Propyl, Isopropyl, n-Butyl, sec-Butyl, tert-Butyl, n-Pentyl, n-Hexyl, n-Heptyl und n-Octyl steht;
- R¹²: für einen Rest ausgewählt aus der Gruppe bestehend aus F, Cl, Br, -SF₅, - CF₃, -O-CH₃, -O-C₂H₅, -O-CH₂-CH₂-CH₃, -O-CH₂-CH₂-CH₂-CH₃, -O-C(CH₃)₃, - O-CH₂-CH₂-CH₂-CH₂-CH₃, -O-CH₂-CH₂-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
- R¹³ und R¹⁴: jeweils für einen Wasserstoff-Rest stehen;
- R¹⁵: für einen Rest ausgewählt aus der Gruppe bestehend aus Phenyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
und
- R¹⁶: für einen Rest ausgewählt aus der Gruppe bestehend aus Thiomorpholinyl, Azepanyl, Morpholinyl, (2,3)-Dihydro-1 H-isoindolyl, (2,3)-Dihydro-indolyl, Piperidinyl und Pyrrolidinyl steht; wobei der Rest mit 1, 2 oder 3 Substituenten unabhängig voneinander ausgewählt aus der Gruppe bestehend aus -OH, Methyl, Ethyl, n-Propyl, Isopropyl, n-Butyl, sec-Butyl, Isobutyl, tert-Butyl, Benzyl und Phenyl substituiert sein kann; wobei jeweils der zyklische Teil der Reste Benzyl und Phenyl mit 1, 2, 3, 4 oder 5 Substituenten unabhängig voneinander ausgewählt aus der Gruppe bestehend aus F, Cl, Br und -CF₃ substituiert sein kann;
oder für einen der folgenden Reste steht 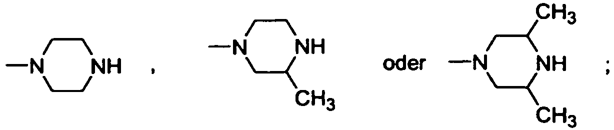 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt ist die Verwendung wenigstens eines 2,5-disubstituierten Thiazol-4-on-Derivats der vorstehend angegebenen allgemeinen Formel I, worin
- R¹: für einen der folgenden Reste steht 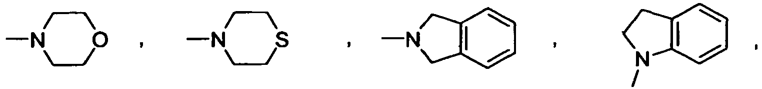 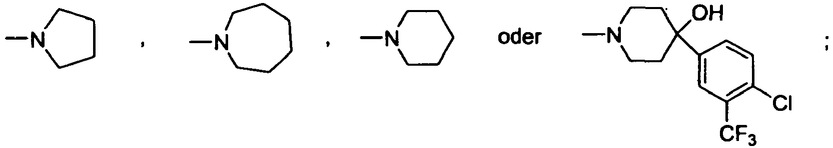 für den folgenden Rest steht 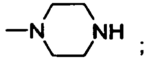 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus -OH, -O-CH₃ und -O-C₂H₅ substituiert sein kann;
für eine NR³R⁴-Gruppe steht
oder für eine NR⁷-C(=O)-NR⁸R⁹ Gruppe steht;
- R²: für einen 1,3-Dihydro-indol-2-onyl- oder einen 1,3-Dihydro-indol-2-thionyl-Rest steht;
für CH-X steht;
für CH-CH₂-Y, CH-CH(CH₃)-Y oder CH-CH(OH)-Y steht;
oder für CH-CH=C(CH₃)-Z, CH-CH=CH-Z, CH-CH=CH-S-Z, CH-C(CH₃)=CH-Z, CH-C(Phenyl)=CH-Z, CH-CBr=CH-Z, CH-CCI=CH-Z, CH-CF=CH-Z, CH-CH₂-CH₂-Z, CH-CH₂-CH(CH₃)-Z oder CH-CH(CH₃)-CH₂-Z steht;
- R³: für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
oder für einen Rest ausgewählt aus der Gruppe bestehend aus Phenyl, Naphthyl, Benzyl, (1,3)-Benzodioxolyl und (1,4)-Benzodioxanyl steht; wobei der Rest jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
- R⁴: für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
- R⁷ und R⁸: jeweils für einen Wasserstoff-Rest stehen;
- R⁹: für einen Phenyl-Rest steht, der mit 1, 2, 3, 4 oder 5 Substituenten unabhängig voneinander ausgewählt aus der Gruppe bestehend aus F, Cl, Br und -CF₃ substituiert sein kann;
- X: für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyridinyl, Pyridazinyl, Pyrazinyl, Pyrimidinyl, Chinolinyl und Isochinolinyl steht, der mit 1, 2, 3, 4 oder 5 gleichen oder verschiedenen Substituenten R¹⁰ substituiert sein kann;
- Y: für einen Phenyl- oder Naphthyl-Rest steht, der mit 1, 2, 3 4 oder 5 gleichen oder verschiedenen Substituenten R¹¹ substituiert sein kann;
- Z: für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl und n-Octyl steht;
oder für einen Phenyl- oder Naphthyl-Rest steht, der mit 1, 2, 3, 4 oder 5 gleichen oder verschiedenen Substituenten R¹² substituiert sein kann;
- R¹⁰: für einen Rest ausgewählt aus der Gruppe bestehend aus F, Cl. Br, I, -CF₃, - SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, NH₂, -O-CF₃, -S-CF₃, Methyl, Ethyl, n-Propyl, Isopropyl, n-Butyl, sec-Butyl, tert-Butyl, n-Pentyl, n-Hexyl, n-Heptyl, n-Octyl, -C(=O)-OH, - C(=O)-O-CH₃, -C(=O)-O-C₂H₅, -C(=O)-O-C(CH₃)₃, -N(CH₃)₂, -N(C₂H₅)₂, -NH-CH₃, -NH-C₂H₅, -NH-C(=O)-CH₃, -O-S(=O)₂-Phenyl, -O-Phenyl, -O-Benzyl und Phenyl steht;
für eine -C(=O)-NR¹³-(CH₂)ₘ-NR¹⁴-R¹⁵-Gruppe mit m gleich 0, 1, 2 oder 3 steht;
oder für eine -C(=O)-R¹⁶-Gruppe steht;
- R¹¹: für einen Rest ausgewählt aus der Gruppe bestehend aus F, Cl, Br, I, -CN, - CF₃, -SF₅, -OH, -O-CH₃, -O-C₂H₅, -O-CH₂-CH₂-CH₃, -O-CH₂-CH₂-CH₂-CH₃, - O-C(CH₃)₃, -O-CH₂-CH₂-CH₂-CH₂-CH₃, -O-CH₂-CH₂-C(CH₃)₃, -O-CH=CH₂, -0-CH₂-CH=CH₂, -NH₂, -O-CF₃, -S-CF₃, -SH. -S-CH₃, -S-C₄H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
- R¹²: für einen Rest ausgewählt aus der Gruppe bestehend aus F, Cl, Br, I, -CN, - CF₃, -SF₅, -OH, -O-CH₃, -O-C₂H₅, -O-CH₂-CH₂-CH₃, -O-CH₂-CH₂-CH₂-CH₃, - O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
- R¹³ und R¹⁴: jeweils für einen Wasserstoff-Rest stehen;
- R¹⁵: für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CF₃, -S-CF₃, Methyl, Ethyl, n-Propyl, Isopropyl, n-Butyl, sec-Butyl, Isobutyl und tert-Butyl substituiert sein kann;
und
- R¹⁶: für einen der folgenden Reste steht 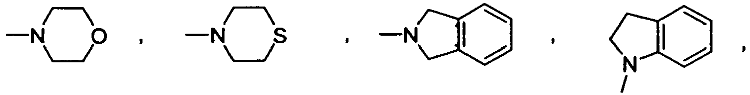 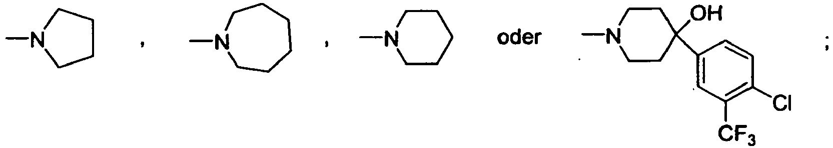 oder für einen der folgenden Reste steht 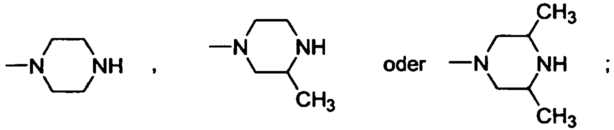 wobei das Wasserstoffatom der -NH-Gruppe durch einen Substituenten ausgewählt aus der Gruppe bestehend aus Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt ist die Verwendung wenigstens eines 2,5-disubstituierten Thiazol-4-on-Derivats der vorstehend angegebenen allgemeinen Formel I ausgewählt aus der Gruppe bestehend aus

| | |
|---|---|
| 1 | 5-[3-(4-Methoxy-phenyl)-but-2-enyliden]-2-morpholin-4-yl-thiazol-4-on |
| 2 | 2-Morpholin-4-yl-5-phenethyliden-thiazol-4-on Hydrochlorid |
| 3 | 2-Morpholin-4-yl-5-(2-o-tolyl-ethyliden)-thiazol-4-on Hydrochlorid |
| 4 | 2-Morpholin-4-yl-5-(3-phenyl-allyliden)-thiazol-4-on |
| 5 | 2-Morpholin-4-yl-5-(3-phenyl-butyliden)-thiazol-4-on |
| 6 | 2-Morpholin-4-yl-5-(3-phenyl-propyliden)-thiazol-4-on |
| 7 | 5-[3-(4-Fluor-phenyl)-allyliden]-2-morpholin-4-yl-thiazol-4-on |
| 8 | N-[4-(2-Morpholin-4-yl-4-oxo-4H-thiazol-5-ylidenmethyl)-phenyl]-acetamid |
| 9 | 5-Benzo[1,3]dioxol-5-ylmethylen-2-morpholin-4-yl-thiazol-4-on |
| 10 | 2-Morpholin-4-yl-5-(3-m-tolyl-allyliden)-thiazol-4-on |
| 11 | 5-(3-lodo-4,5-dimethoxy-benzyliden)-2-morpholin-4-yl-thiazol-4-on |
| 12 | 2-Morpholin-4-yl-5-(3-phenylsulfanyl-allyliden)-thiazol-4-on |
| 13 | 5-(3-Benzyloxy-4-methoxy-benzyliden)-2-morpholin-4-yl-thiazol-4-on |
| 14 | 5-(4-Methyl-benzyliden)-2-thiomorpholin-4-yl-thiazol-4-on Hydrobromid |
| 15 | N-[4-(2-Morpholin-4-yl-4-oxo-4H-thiazol-5-ylidenmethyl)-phenyl]-amin |
| 16 | 5-(4-Butoxy-benzyliden)-2-morpholin-4-yl-thiazol-4-on |
| 17 | 2-Morpholin-4-yl-5-(4-phenoxy-benzyliden)-thiazol-4-on |
| 18 | 2-Morpholin-4-yl-5-(3-phenoxy-benzyliden)-thiazol-4-on |
| 19 | 2-Morpholin-4-yl-5-(3-p-tolyl-allyliden)-thiazol-4-on |
| 20 | 5-(3-Benzyloxy-benzyliden)-2-morpholin-4-yl-thiazol-4-on |
| 21 | 5-(4-Benzyloxy-benzyliden)-2-morpholin-4-yl-thiazol-4-on |
| 22 | 2-Thiomorpholin-4-yl-5-(2-trifluormethyl-benzyliden)-thiazol-4-on |
| 23 | 5-(4-Brom-benzyliden)-2-thiomorpholin-4-yl-thiazol-4-on |
| 24 | 2-Thiomorpholin-4-yl-5-(4-trifluormethoxy-benzyliden)-thiazol-4-on |
| 25 | 5-(3-Phenyl-allyliden)-2-thiomorpholin-4-yl-thiazol-4-on |
| 26 | 2-Morpholin-4-yl-5-octyliden-thiazol-4-on |
| 27 | 5-(4-Benzyloxy-3-methoxy-benzyliden)-2-morpholin-4-yl-thiazol-4-on |
| 28 | 5-(3,4-Bis-benzyloxy-benzyliden)-2-morpholin-4-yl-thiazol-4-on |
| 29 | 5-Butyliden-2-morpholin-4-yl-thiazol-4-on |
| 30 | 2-[4-(3-Chlor-pyridin-2-yl)-piperazin-1-yl]-5-(4-methyl-benzyliden)-thiazol-4-on |
| 31 | 2-[4-(3-Chlor-pyridin-2-yl)-piperazin-1-yl]-5-(3-phenyl-allyliden)-thiazol-4-on |
| 32 | 5-Hexyliden-2-morpholin-4-yl-thiazol-4-on |
| 33 | 2-Morpholin-4-yl-5-(3-p-tolyl-but-2-enyliden)-thiazol-4-on |
| 34 | 5-[3-(4-tert-Butyl-phenyl)-but-2-enyliden]-2-morpholin-4-yl-thiazol-4-on |
| 35 | 5-(4-Methoxy-naphthalen-1-ylmethylen)-2-morpholin-4-yl-thiazol-4-on |
| 36 | 2-Morpholin-4-yl-5-(4-trifluormethoxy-benzyliden)-thiazol-4-on |
| 37 | 2-[4-(3-Methoxy-phenyl)-piperazin-1-yl]-5-(3-phenyl-allyliden)-thiazol-4-on |
| 38 | 5-[3-(4-tert-Butyl-phenyl)-allyliden]-2-[4-(3-methoxy-phenyl)-piperazin-1-yl]-thiazol-4-on |
| 39 | 5-(4-Hydroxy-3-methoxy-benzyliden)-2-thiomorpholin-4-yl-thiazol-4-on |
| 40 | 5-[3-(4-Hydroxy-3-methoxy-phenyl)-allyliden]-2-thiomorpholin-4-yl-thiazol-4-on |
| 41 | 5-Naphthalen-1-ylmethylen-2-thiomorpholin-4-yl-thiazol-4-on |
| 42 | 5-Benzyliden-2-thiomorpholin-4-yl-thiazol-4-on |
| 43 | 2-Morpholin-4-yl-5-[3-(4-trifluormethyl-phenyl)-but-2-enyliden]-thiazol-4-on |
| 44 | 2-Morpholin-4-yl-5-[3-(3-trifluormethyl-phenyl)-but-2-enyliden]-thiazol-4-on |
| 45 | 5-(3-Methyl-butyliden)-2-morpholin-4-yl-thiazol-4-on |
| 46 | 2-[4-(3-Chlor-pyridin-2-yl)-piperazin-1-yl]-5-(2-trifluormethyl-benzyliden)- |
| | thiazol-4-on |
| 47 | 5-(4-Brom-benzyliden)-2-[4-(3-chlor-pyridin-2-yl)-piperazin-1-yl]-thiazol-4-on |
| 48 | 5-Benzyliden-2-[4-(3-chlor-pyridin-2-yl)-piperazin-1-yl]-thiazol-4-on |
| 49 | 2-[4-(3-Chlor-pyridin-2-yl)-piperazin-1-yl]-5-naphthalen-2-ylmethylen-thiazol-4-on |
| 50 | 5-(2,3-Diphenyl-allyliden)-2-morpholin-4-yl-thiazol-4-on |
| 51 | 2-Morpholin-4-yl-5-phenanthren-9-ylmethylen-thiazol-4-on |
| 52 | 2-Morpholin-4-yl-5-chinolin-3-ylmethylen-thiazol-4-on |
| 53 | 2-(4-Ethyl-piperazin-1-yl)-5-(4-methyl-benzyliden)-thiazol-4-on Hydrobromid |
| 54 | 5-Naphthalen-2-ylmethylen-2-thiomorpholin-4-yl-thiazol-4-on |
| 55 | 2-[4-(4-Methoxy-phenyl)-piperazin-1-yl]-5-(3-phenyl-allyliden)-thiazol-4-on |
| 56 | 2-[4-(2-Chlor-phenyl)-piperazin-1-yl]-5-(3-phenyl-allyliden)-thiazol-4-on |
| 57 | 5-(3-Phenyl-allyliden)-2-(4-phenyl-piperazin-1-yl)-thiazol-4-on |
| 58 | 5-Decyliden-2-morpholin-4-yl-thiazol-4-on |
| 59 | 5-[3-(4-tert-Butyl-phenyl)-allyliden]-2-morpholin-4-yl-thiazol-4-on |
| 60 | 2-(2-Morpholin-4-yl-4-oxo-4H-thiazol-5-ylidenmethyl)-benzoesäure Triethylammoniumsalz |
| 61 | 5-(2-Brom-3-phenyl-allyliden)-2-[4-(3-chlor-pyridin-2-yl)-piperazin-1-yl]-thiazol-4-on |
| 62 | 5-[3-(4-tert-Butyl-phenyl)-allyliden]-2-[4-(3-chlor-pyridin-2-yl)-piperazin-1-yl]-thiazol-4-on |
| 63 | 2-(4-Benzyl-piperazin-1-yl)-5-(4-methyl-benzyliden)-thiazol-4-on |
| 64 | 2-(4-Phenyl-piperazin-1-yl)-5-chinolin-3-ylmethylen-thiazol-4-on |
| 65 | 2-[4-(3-Chlor-pyridin-2-yl)-piperazin-1-yl]-5-(2-methyl-3-phenyl-allyliden)-thiazol-4-on |
| 66 | 2-[4-(3-Chlor-pyridin-2-yl)-piperazin-1-yl]-5-naphthalen-1-ylmethylen-thiazol-4-on |
| 67 | 5-(2-Chlor-3-phenyl-allyliden)-2-[4-(3-chlor-pyridin-2-yl)-piperazin-1-yl]-thiazol-4-on |
| 68 | 5-(4-Hydroxy-3-methoxy-benzyliden)-2-(4-phenyl-piperazin-1-yl)-thiazol-4-on |
| 69 | 5-Phenanthren-9-ylmethylen-2-(4-phenyl-piperazin-1-yl)-thiazol-4-on |
| 70 | 5-(6-Methoxy-naphthalen-2-ylmethylen)-2-(4-phenyl-piperazin-1-yl)-thiazol-4-on |
| 71 | 5-Naphthalen-1-ylmethylen-2-(4-phenyl-piperazin-1-yl)-thiazol-4-on |
| 72 | 5-(4-Brom-benzyliden)-2-(4-phenyl-piperazin-1-yl)-thiazol-4-on |
| 73 | 5-(4-Methyl-benzyliden)-2-(4-phenyl-piperazin-1-yl)-thiazol-4-on |
| 74 | 2-(4-Phenyl-piperazin-1-yl)-5-(2-trifluormethyl-benzyliden)-thiazol-4-on |
| 75 | 3-(4-Oxo-2-thiomorpholin-4-yl-4H-thiazol-5-yliden)-1,3-dihydro-indol-2-on |
| 76 | 3-(2-Morpholin-4-yl-4-oxo-4H-thiazol-5-yliden)-1,3-dihydro-indol-2-on |
| 77 | 2-[4-(2-Fluor-phenyl)-piperazin-1-yl]-5-(4-methyl-benzyliden)-thiazol-4-on |
| 78 | 5-(4-tert-Butyl-benzyliden)-2-[4-(2-fluor-phenyl)-piperazin-1-yl]-thiazol-4-on |
| 79 | 2-[4-(2-Fluor-phenyl)-piperazin-1-yl]-5-(3-phenyl-allyliden)-thiazol-4-on |
| 80 | 5-[3-(4-tert-Butyl-phenyl)-allyliden]-2-[4-(2-fluor-phenyl)-piperazin-1-yl]-thiazol-4-on |
| 81 | 2-[4-(2-Fluor-phenyl)-piperazin-1-yl]-5-(4-trifluormethyl-benzyliden)-thiazol-4-on |
| 82 | 2-[4-(2-Fluor-phenyl)-piperazin-1-yl]-5-(4-trifluormethoxy-benzyliden)-thiazol-4-on |
| 83 | 5-(2-Chlor-3-phenyl-allyliden)-2-[4-(2-fluor-phenyl)-piperazin-1-yl]-thiazol-4-on |
| 84 | 2-[4-(2-Fluor-phenyl)-piperazin-1-yl]-5-naphthalen-1-ylmethylen-thiazol-4-on |
| 85 | 5-(2-Chlor-3-phenyl-allyliden)-2-thiomorpholin-4-yl-thiazol-4-on |
| 86 | 3-(4-Oxo-2-thiomorpholin-4-yl-4H-thiazol-5-ylidenmethyl)-benzoesäure |
| 87 | 4-(4-Oxo-2-thiomorpholin-4-yl-4H-thiazol-5-ylidenmethyl)-benzoesäure |
| 88 | 5-{3-[4-(3-Chlor-pyridin-2-yl)-piperazin-1-carbonyl]-benzyliden}-2-thiomorpholin-4-yl-thiazol-4-on |
| 89 | 5-{4-[4-(3-Chlor-pyridin-2-yl)-piperazin-1-carbonyl]-benzyliden}-2- |
| 90 | thiomorpholin-4-yl-thiazol-4-on 2-[4-(3-Chlor-pyridin-2-yl)-piperazin-1-yl]-5-chinolin-3-ylmethylen-thiazol-4-on |
| 91 | 2-[4-(3-Chlor-pyridin-2-yl)-piperazin-1-yl]-5-(4-trifluormethyl-benzyliden)-thiazol-4-on |
| 92 | 2-(2,3-Dihydro-indol-1-yl)-5-(4-methyl-benzyliden)-thiazol-4-on |
| 93 | 2-(2,3-Dihydro-indol-1-yl)-5-(4-trifluormethyl-benzyliden)-thiazol-4-on |
| 94 | 5-[4-(Morpholin-4-carbonyl)-benzyliden]-2-thiomorpholin-4-yl-thiazol-4-on |
| 95 | 2-[4-(3-Chlor-pyridin-2-yl)-piperazin-1-yl]-5-(4-hydroxy-3-methoxy-benzyliden)-thiazol-4-on |
| 96 | 1-[4-Oxo-5-(4-trifluormethyl-benzyliden)-4,5-dihydro-thiazol-2-yl]-3-(2-trifluormethyl-phenyl)-harnstoff |
| 97 | 2-(4-Benzyl-piperazin-1-yl)-5-chinolin-3-ylmethylen-thiazol-4-on |
| 98 | 2-(4-Benzyl-piperazin-1-yl)-5-naphthalen-2-ylmethylen-thiazol-4-on |
| 99 | 2-(4-Benzyl-piperazin-1-yl)-5-(3-phenoxy-benzyliden)-thiazol-4-on |
| 100 | 2-(4-Benzyl-piperazin-1-yl)-5-(4-trifluormethyl-benzyliden)-thiazol-4-on |
| 101 | 1-[5-(4-Methyl-benzyliden)-4-oxo-4,5-dihydro-thiazol-2-yl]-3-(2-trifluormethyl-phenyl)-harnstoff |
| 102 | 2-(4-Benzyl-piperazin-1-yl)-5-naphthalen-1-ylmethylen-thiazol-4-on |
| 103 | 2-(4-Benzyl-piperazin-1-yl)-5-(6-methoxy-naphthalen-2-ylmethylen)-thiazol-4-on |
| 104 | 2-(4-Benzyl-piperazin-1-yl)-5-(4-tert-butyl-benzyliden)-thiazol-4-on |
| 105 | 1-[4-Oxo-5-(3-phenyl-allyliden)-4,5-dihydro-thiazol-2-yl]-3-(2-trifluormethyl-phenyl)-harnstoff |
| 106 | 1-[4-Oxo-5-(4-trifluormethyl-benzyliden)-4,5-dihydro-thiazol-2-yl]-3-(4-trifluormethyl-phenyl)-harnstoff |
| 107 | 1-[5-(4-Methyl-benzyliden)-4-oxo-4,5-dihydro-thiazol-2-yl]-3-(4-trifluormethyl-phenyl)-harnstoff |
| 108 | 5-{3-[4-(6-Chlor-pyridin-2-yl)-piperazin-1-carbonyl]-benzyliden}-2-thiomorpholin-4-yl-thiazol-4-on |
| 109 | 2-(4-Benzyl-piperazin-1-yl)-5-(4-trifluormethoxy-benzyliden)-thiazol-4-on |
| 110 | 5-{3-[4-(3-Chlor-pyridin-2-yl)-3-methyl-piperazin-1-carbonyl]-benzyliden}-2-thiomorpholin-4-yl-thiazol-4-on |
| 111 | N-[3-(3-Chlor-5-trifluormethyl-pyridin-2-ylamino)-propyl]-3-(4-oxo-2-thiomorpholin-4-yl-4H-thiazol-5-ylidenmethyl)-benzamid |
| 112 | 3-(4-Oxo-2-thiomorpholin-4-yl-4H-thiazol-5-ylidenmethyl)-benzoesäure-N'-(2-chlor-4-trifluormethyl-phenyl)-hydrazid |
| 113 | 5-{3-[4-(3-Chlor-5-trifluormethyl-pyridin-2-yl)-piperazin-1-carbonyl]-benzyliden}-2-thiomorpholin-4-yl-thiazol-4-on |
| 114 | 3-(4-Oxo-2-thiomorpholin-4-yl-4H-thiazol-5-ylidenmethyl)-benzoesäure-N'-(3-chlor-5-trifluormethyl-pyridin-2-yl)-hydrazid |
| 115 | 5-{3-[4-(4-Chlor-[1,2,5]thiadiazol-3-yl)-piperazin-1-carbonyl]-benzyliden}-2-thiomorpholin-4-yl-thiazol-4-on |
| 116 | 3-(4-Oxo-2-thiomorpholin-4-yl-4H-thiazol-5-ylidenmethyl)-benzoesäure-N'-(2-chlor-5-trifluormethyl-phenyl)-hydrazid |
| 117 | 2-[4-(4-Chlor-[1,2,5]thiadiazol-3-yl)-piperazin-1-yl]-5-(4-methyl-benzyliden)-thiazol-4-on |
| 118 | 2-[4-(4-Chlor-[1,2,5]thiadiazol-3-yl)-piperazin-1-yl]-5-(4-trifluormethyl-benzyliden)-thiazol-4-on |
| 119 | 2-[4-(4-Chlor-[1,2,5]thiadiazol-3-yl)-piperazin-1-yl]-5-(4-trifluormethoxy-benzyliden)-thiazol-4-on |
| 120 | 5-(4-tert-Butyl-benzyliden)-2-[4-(4-chlor-[1,2,5]thiadiazol-3-yl)-piperazin-1-yl]-thiazol-4-on |
| 121 | 2-[4-(4-Chlor-[1,2,5]thiadiazol-3-yl)-piperazin-1-yl]-5-(3-phenyl-allyliden)-thiazol-4-on |
| 122 | 5-[3-(4-tert-Butyl-phenyl)-allyliden]-2-[4-(4-chlor-[1,2,5]thiadiazol-3-yl)-piperazin-1-yl]-thiazol-4-on |
| 123 | 2-[4-(4-Chlor-[1,2,5]thiadiazol-3-yl)-piperazin-1-yl]-5-naphthalen-1-ylmethylen-thiazol-4-on |
| 124 | 2-[4-(4-Chlor-[1,2,5]thiadiazol-3-yl)-piperazin-1-yl]-5-(3-phenoxy-benzyliden)-thiazol-4-on |
| 125 | 2-[4-(4-Chlor-[1,2,5]thiadiazol-3-yl)-piperazin-1-yl]-5-(4-phenoxy-benzyliden)-thiazol-4-on |
| 126 | 5-(3-Benzyloxy-benzyliden)-2-[4-(4-chlor-[1,2,5]thiadiazol-3-yl)-piperazin-1-yl]-thiazol-4-on |
| 127 | 2-[4-(4-Chlor-[1,2,5]thiadiazol-3-yl)-piperazin-1-yl]-5-(4-hydroxy-3-methoxy-benzyliden)-thiazol-4-on |
| 128 | 2-[4-(4-Chlor-[1,2,5]thiadiazol-3-yl)-piperazin-1-yl]-5-furan-2-ylmethylen-thiazol-4-on |
| 129 | 2-[4-(4-Chlor-[1,2,5]thiadiazol-3-yl)-piperazin-1-yl]-5-naphthalen-2-ylmethylen-thiazol-4-on |
| 130 | 3-[2-(4-Benzyl-piperazin-1-yl)-4-oxo-4H-thiazol-5-ylidenmethyl]-benzoesäure |
| 131 | 5-Naphthalen-1-ylmethylen-2-piperazin-1-yl-thiazol-4-on |
| 132 | 2-Piperazin-1-yl-5-(4-trifluormethyl-benzyliden)-thiazol-4-on |
| 133 | 5-(4-Methyl-benzyliden)-2-piperazin-1-yl-thiazol-4-on |
| 134 | 5-(4-Hydroxy-3-methoxy-benzyliden)-2-piperazin-1-yl-thiazol-4-on |
| 135 | 5-(4-Isopropyl-benzyliden)-2-morpholin-4-yl-thiazol-4-on |
| 136 | 5-(4-Isopropyl-benzyliden)-2-thiomorpholin-4-yl-thiazol-4-on |
| 137 | 2-[4-(2-Fluor-phenyl)-piperazin-1-yl]-5-(4-isopropyl-benzyliden)-thiazol-4-on |
| 138 | 5-(4-Pentafluorsulfanyl)-benzyliden)-2-thiomorpholin-4-yl-thiazol-4-on |
| 139 | 2-[4-(3-Chlor-pyridin-2-yl)-piperazin-1-yl]-5-(4-isopropyl-benzyliden)-thiazol-4-on |
| 140 | 2-(4-Benzyl-piperazin-1-yl)-5-(4-butyl-benzyliden)-thiazol-4-on Hydrochlorid |
| 141 | 2-(4-Benzyl-piperazin-1-yl)-5-(4-pentyl-benzyliden)-thiazol-4-on Hydrochlorid |
| 142 | 2-(4-Benzyl-piperazin-1-yl)-5-(4-octyl-benzyliden)-thiazol-4-on |
| 143 | 2-[4-(6-Chlor-pyridin-2-yl)-piperazin-1-yl]-5-(4-methyl-benzyliden)-thiazol-4-on |
| 144 | 5-(4-tert-Butyl-benzyliden)-2-[4-(6-chlor-pyridin-2-yl)-piperazin-1-yl]-thiazol-4-on |
| 145 | 2-[4-(6-Chlor-pyridin-2-yl)-piperazin-1-yl]-5-(4-trifluormethyl-benzyliden)-thiazol-4-on |
| 146 | 2-[4-(6-Chlor-pyridin-2-yl)-piperazin-1-yl]-5-(3-phenyl-allyliden)-thiazol-4-on |
| 147 | 5-[3-(4-tert-Butyl-phenyl)-allyliden]-2-[4-(6-chlor-pyridin-2-yl)-piperazin-1-yl]-thiazol-4-on |
| 148 | 2-[4-(6-Chlor-pyridin-2-yl)-piperazin-1-yl]-5-(4-hydroxy-3-methoxy-benzyliden)-thiazol-4-on |
| 149 | 2-[4-(6-Chlor-pyridin-2-yl)-piperazin-1-yl]-5-(4-isopropyl-benzyliden)-thiazol-4-on |
| 150 | 2-[4-(6-Chlor-pyridin-2-yl)-piperazin-1-yl]-5-(4-trifluormethoxy-benzyliden)-thiazol-4-on |
| 151 | 2-[4-(6-Chlor-pyrid in-2-yl)-piperazin-1-yl]-5-naphthalen-2-ylmethylen-thiazol-4-on |
| 152 | 5-(4-Methyl-benzyliden)-2-[4-(6-methyl-pyridazin-3-yl)-piperazin-1-yl]-thiazol-4-on |
| 153 | 5-(4-Isopropyl-benzyliden)-2-[4-(6-methyl-pyridazin-3-yl)-piperazin-1-yl]-thiazol-4-on |
| 154 | 5-(4-tert-Butyl-benzyliden)-2-[4-(6-methyl-pyridazin-3-yl)-piperazin-1-yl]-thiazol-4-on |
| 155 | 5-[3-(4-tert-Butyl-phenyl)-allyliden]-2-[4-(6-methyl-pyridazin-3-yl)-piperazin-1-yl]-thiazol-4-on |
| 156 | 2-[4-(6-Methyl-pyridazin-3-yl)-piperazin-1-yl]-5-(4-trifluormethoxy-benzyliden)-thiazol-4-on |
| 157 | 5-(4-Hydroxy-3-methoxy-benzyliden)-2-[4-(6-methyl-pyridazin-3-yl)-piperazin-1-yl]-thiazol-4-on |
| 158 | 2-(4-Benzyl-piperazin-1-yl)-5-(4-pentafluorsulfanyl-benzyliden)-thiazol-4-on |
| 159 | 2-(4-Benzyl-piperazin-1-yl)-5-{3-[4-(4-chlor-[1,2,5]thiadiazol-3-yl)-piperazin-1-carbonyl]-benzyliden}-thiazol-4-on |
| 160 | 2-[4-(6-Methyl-pyridazin-3-yl)-piperazin-1-yl]-5-(4-trifluormethyl-benzyliden)-thiazol-4-on |
| 161 | 2-[4-(6-Methyl-pyridazin-3-yl)-piperazin-1-yl]-5-(3-phenyl-allyliden)-thiazol-4-on |
| 162 | 2-[4-(6-Methyl-pyridazin-3-yl)-piperazin-1-yl]-5-naphthalen-2-ylmethylen-thiazol-4-on |
| 163 | 2-[4-(6-Methyl-pyridazin-3-yl)-piperazin-1-yl]-5-naphthalen-1-ylmethylen-thiazol-4-on |
| 164 | 2-(4-Benzyl-piperazin-1-yl)-5-(4-isopropyl-benzyliden)-thiazol-4-on |
| 165 | 3-{2-[4-(4-Chlor-[1,2,5]thiadiazol-3-yl)-piperazin-1-yl]-4-oxo-4H-thiazol-5-ylidenmethyl}-benzoesäure |
| 166 | 5-{3-[4-(4-Chlor-3-trifluormethyl-phenyl)-4-hydroxy-piperidin-1-carbonyl]-benzylidenl-2-thiomorpholin-4-yl-thiazol-4-on |
| 167 | 5-{3-[4-(4-Chlor-[1,2,5]thiadiazol-3-yl)-piperazin-1-carbonyl]-benzyliden}-2-[4-(4-chlor-[1,2,5]thiadiazol-3-yl)-piperazin-1-yl]-thiazol-4-on |
| 168 | 5-{3-[4-(3-Chlor-pyridin-2-yl)-piperazin-1-carbonyl]-benzyliden}-2-[4-(4-chlor-[1,2,5]thiadiazol-3-yl)-piperazin-1-yl]-thiazol-4-on |
| 169 | 5-[3-(4-Benzyl-piperazin-1-carbonyl)-benzyliden]-2-[4-(4-chlor-[1,2,5]thiadiazol-3-yl)-piperazin-1-yl]-thiazol-4-on |
| 170 | 2-[4-(4-Chlor-[1,2,5]thiadiazol-3-yl)-piperazin-1-yl]-5-{3-[4-(6-methoxy-pyridin-2-yl)-piperazin-1-carbonyl]-benzyliden}-thiazol-4-on |
| 171 | 2-[4-(4-Chlor-3-trifluormethyl-phenyl)-4-hydroxy-piperidin-1-yl]-5-(4-methyl-benzyliden)-thiazol-4-on |
| 172 | 2-[4-(4-Chlor-3-trifluormethyl-phenyl)-4-hydroxy-piperidin-1-yl]-5-(4-hydroxy-3-methoxy-benzyliden)-thiazol-4-on |
| 173 | 2-[4-(4-Chlor-3-trifluormethyl-phenyl)-4-hydroxy-piperidin-1-yl]-5-naphthalen-1-ylmethylen-thiazol-4-on |
| 174 | 2-[4-(4-Chlor-3-trifluormethyl-phenyl)-4-hydroxy-piperidin-1-yl]-5-(3-methoxy-benzyliden)-thiazol-4-on Hydrochlorid |
| 175 | 5-Benzyliden-2-morpholin-4-yl-thiazol-4-on Hydrobromid |
| 176 | 5-(4-Methyl-benzyliden)-2-morpholin-4-yl-thiazol-4-on Hydrobromid |
| 177 | 2-Diethylamino-5-(4-methyl-benzyliden)-thiazol-4-on Hydrobromid |
| 178 | 5-(4-Chlor-benzyliden)-2-diethylamino-thiazol-4-on Hydrobromid |
| 179 | 5-(4-Isopropyl-benzyliden)-2-morpholin-4-yl-thiazol-4-on Hydrobromid |
| 180 | 5-Benzyliden-2-diethylamino-thiazol-4-on Hydrobromid |
| 181 | 5-(4-Chlor-benzyliden)-2-morpholin-4-yl-thiazol-4-on Hydrobromid |
| 182 | 5-(4-Chlor-benzyliden)-2-piperidin-1-yl-thiazol-4-on Hydrobromid |
| 183 | 5-(4-Chlor-benzyliden)-2-pyrrolidin-1-yl-thiazol-4-on Hydrobromid |
| 184 | 5-Benzyliden-2-pyrrolidin-1-yl-thiazol-4-on Hydrobromid |
| 185 | 5-Benzyliden-2-pyrrolidin-1-yl-thiazol-4-on |
| 186 | 5-Biphenyl-4-ylmethylen-2-piperidin-1-yl-thiazol-4-on Hydrobromid |
| 187 | 2-Azepan-1-yl-5-biphenyl-4-ylmethylen-thiazol-4-on Hydrobromid |
| 188 | 5-Biphenyl-4-ylmethylen-2-pyrrolidin-1-yl-thiazol-4-on Hydrobromid |
| 189 | 2-Morpholin-4-yl-5-(3-phenyl-allyliden)-thiazol-4-on Hydrobromid |
| 190 | 5-(3-Phenyl-allyliden)-2-thiomorpholin-4-yl-thiazol-4-on Hydrobromid |
| 191 | 5-(3-Phenyl-allyliden)-2-piperidin-1-yl-thiazol-4-on Hydrobromid |
| 192 | 2-Azepan-1-yl-5-(3-phenyl-allyliden)-thiazol-4-on Hydrobromid |
| 193 | 5-(3-Phenyl-allyliden)-2-pyrrolidin-1-yl-thiazol-4-on Hydrobromid |
| 194 | 5-Biphenyl-4-ylmethylen-2-morpholin-4-yl-thiazol-4-on Hydrobromid |
| 195 | 2-Thiomorpholin-4-yl-5-(3-trifluormethyl-benzyliden)-thiazol-4-on Hydrobromid |
| 196 | 5-(4-Chlor-benzyliden)-2-thiomorpholin-4-yl-thiazol-4-on |
| 197 | 5-(4-tert-Butyl-benzyliden)-2-morpholin-4-yl-thiazol-4-on |
| 198 | 5-(4-tert-Butyl-benzyliden)-2-thiomorpholin-4-yl-thiazol-4-on Hydrobromid |
| 199 | 5-(4-tert-Butyl-benzyliden)-2-pyrrolidin-1-yl-thiazol-4-on Hydrobromid |
| 200 | 2-Azepan-1-yl-5-(4-tert-butyl-benzyliden)-thiazol-4-on Hydrobromid |
| 201 | 5-(4-tert-Butyl-benzyliden)-2-piperidin-1-yl-thiazol-4-on Hydrobromid |
| 202 | 2-Thiomorpholin-4-yl-5-(4-trifluormethyl-benzyliden)-thiazol-4-on |
| 203 | 5-(4-tert-Butyl-benzyliden)-2-diethylamino-thiazol-4-on Hydrobromid |
| 204 | 2-Morpholin-4-yl-5-(4-trifluormethyl-benzyliden)-thiazol-4-on |
| 205 | 2-Morpholin-4-yl-5-(3-trifluormethyl-benzyliden)-thiazol-4-on Hydrobromid |
| 206 | 2-Morpholin-4-yl-5-(3-trifluormethyl-benzyliden)-thiazol-4-on |
| 207 | 5-Biphenyl-4-ylmethylen-2-diethylamino-thiazol-4-on |
| 208 | 2-Morpholin-4-yl-5-thiophen-2-ylmethylen-thiazol-4-on |
| 209 | 2-Morpholin-4-yl-5-naphthalen-1-ylmethylen-thiazol-4-on |
| 210 | 2-Morpholin-4-yl-5-pyridin-2-ylmethylen-thiazol-4-on |
| 211 | 2-Morpholin-4-yl-5-(4-trifluormethyl-benzyliden)-thiazol-4-on |
| 212 | 2-Morpholin-4-yl-5-pyridin-3-ylmethylen-thiazol-4-on |
| 213 | 2-Morpholin-4-yl-5-pyridin-4-ylmethylen-thiazol-4-on |
| 214 | 2-Diethylamino-5-(3-trifluormethyl-benzyliden)-thiazol-4-on |
| 215 | 5-(4-Methyl-benzyliden)-2-pyrrolidin-1-yl-thiazol-4-on |
| 216 | 5-(4-tert-Butyl-benzyliden)-2-(2-methoxy-ethylamino)-thiazol-4-on |
| 217 | 2-Morpholin-4-yl-5-(4-octyl-benzyliden)-thiazol-4-on Hydrobromid |
| 218 | 5-(4-Methyl-benzyliden)-2-morpholin-4-yl-thiazol-4-on |
| 219 | 5-(3,4-Dimethoxy-benzyliden)-2-morpholin-4-yl-thiazol-4-on |
| 220 | 5-(2-Hydroxy-benzyliden)-2-morpholin-4-yl-thiazol-4-on |
| 221 | 5-(2-Hydroxy-3-methoxy-benzyliden)-2-morpholin-4-yl-thiazol-4-on |
| 222 | 5-(4-tert-Butyl-benzyliden)-2-morpholin-4-yl-thiazol-4-on |
| 223 | 5-(4-Diethylamino-benzyliden)-2-morpholin-4-yl-thiazol-4-on |
| 224 | 5-(4-Hydroxy-3-methoxy-benzyliden)-2-morpholin-4-yl-thiazol-4-on |
| 225 | 4-(2-Morpholin-4-yl-4-oxo-4H-thiazol-5-ylidenmethyl)-benzoesäure |
| 226 | 2-Morpholin-4-yl-5-(2,3,4-trimethoxy-benzyliden)-thiazol-4-on |
| 227 | 2-Morpholin-4-yl-5-(3,4,5-trimethoxy-benzyliden)-thiazol-4-on |
| 228 | 5-(4-Methoxy-benzyliden)-2-morpholin-4-yl-thiazol-4-on |
| 229 | 5-(4-Hydroxy-benzyliden)-2-morpholin-4-yl-thiazol-4-on |
| 230 | 5-(3-Ethoxy-4-hydroxy-benzyliden)-2-morpholin-4-yl-thiazol-4-on |
| 231 | 5-(3-Hydroxy-benzyliden)-2-morpholin-4-yl-thiazol-4-on |
| 232 | 5-(4-Brom-benzyliden)-2-morpholin-4-yl-thiazol-4-on |
| 233 | 2-Morpholin-4-yl-5-(4-vinyloxy-benzyliden)-thiazol-4-on |
| 234 | Benzensulfonsäure-4-(2-morpholin-4-yl-4-oxo-4H-thiazol-5-ylidenmethyl)-phenylester |
| 235 | 5-(4-Dimethylamino-benzyliden)-2-morpholin-4-yl-thiazol-4-on |
| 236 | 4-(2-Morpholin-4-yl-4-oxo-4H-thiazol-5-ylidenmethyl)-benzoesäuremethylester |
| 237 | 5-(3-Hydroxy-4-methoxy-benzyliden)-2-morpholin-4-yl-thiazol-4-on |
| 238 | 5-[4-(3,3-Dimethyl-butoxy)-3-methoxy-benzyliden]-2-morpholin-4-yl-thiazol-4-on |
| 239 | 5-(2-Methoxy-benzyliden)-2-morpholin-4-yl-thiazol-4-on |
| 240 | 5-(4-Hydroxy-benzyliden)-2-morpholin-4-yl-thiazol-4-on |
| 241 | 2-[(Benzo[1,3]dioxol-5-ylmethyl)-amino]-5-[3-(4-tert-butyl-phenyl)-allyliden]-thiazol-4-on |
| 242 | 2-[(Benzo[1,3]dioxol-5-ylmethyl)-amino]-5-(4-trifluormethyl-benzyliden)-thiazol-4-on |
| 243 | 2-[(Benzo[1,3]dioxol-5-ylmethyl)-amino]-5-(4-trifluormethoxy-benzyliden)-thiazol-4-on |
| 244 | 2-[(Benzo[1,3]dioxol-5-ylmethyl)-amino]-5-naphthalen-1-ylmethylen-thiazol-4-on |
| 245 | 2-[(Benzo[1,3]dioxol-5-ylmethyl)-amino]-5-(2-chlor-3-phenyl-allyliden)-thiazol-4-on |
| 246 | 2-(4-Methyl-benzylamino)-5-naphthalen-1-ylmethylen-thiazol-4-on |
| 247 | 2-(4-Methyl-benzylamino)-5-(3-phenoxy-benzyliden)-thiazol-4-on |
| 248 | 2-(4-Methyl-benzylamino)-5-(4-phenoxy-benzyliden)-thiazol-4-on |
| 249 | 5-(2-Chlor-3-phenyl-allyliden)-2-(4-methyl-benzylamino)-thiazol-4-on |
| 250 | 2-(4-Methyl-benzylamino)-5-naphthalen-2-ylmethylen-thiazol-4-on |
| 251 | 2-(3-Methoxy-benzylamino)-5-(4-methyl-benzyliden)-thiazol-4-on |
| 252 | 5-(4-tert-Butyl-benzyliden)-2-(3-methoxy-benzylamino)-thiazol-4-on |
| 253 | 2-(3-Methoxy-benzylamino)-5-(4-trifluormethyl-benzyliden)-thiazol-4-on |
| 254 | 2-(3-Methoxy-benzylamino)-5-(4-trifluormethoxy-benzyliden)-thiazol-4-on |
| 255 | 2-(3-Methoxy-benzylamino)-5-(3-phenyl-allyliden)-thiazol-4-on |
| 256 | 5-Naphthalen-1-ylmethylen-2-(4-trifluormethyl-benzylamino)-thiazol-4-on |
| 257 | 5-(3-Phenoxy-benzyliden)-2-(4-trifluormethyl-benzylamino)-thiazol-4-on |
| 258 | 5-(4-Phenoxy-benzyliden)-2-(4-trifluormethyl-benzylamino)-thiazol-4-on |
| 259 | 5-(2-Chloro-3-phenyl-allyliden)-2-(4-trifluormethyl-benzylamino)-thiazol-4-on |
| 260 | 5-(3-Benzyloxy-benzyliden)-2-(4-trifluormethyl-benzylamino)-thiazol-4-on |
| 261 | 5-Naphthalen-2-ylmethylen-2-(4-trifluormethyl-benzylamino)-thiazol-4-on |
| 262 | 2-(4-Hydroxy-3-methoxy-phenyl)-5-(4-methyl-benzyliden)-thiazol-4-on und |
| 263 | 2-(4-tert-Butyl-phenyl-amino)-5-(4-methyl-benzyliden)-thiazol-4-on |

jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, vorzugsweise ggf. ausgewählt aus der Gruppe bestehend aus Hydrobromiden, Hydrochloriden und Triethylammoniumsalzen, oder jeweils in Form entsprechender Solvate.

Des weiteren kann die Verwendung von erfindungsgemäßen Verbindungen bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist die Verwendung wenigstens eines erfindungsgemäßen 2,5-disubstituierten Thiazol-4-on-Derivats der vorstehend angegebenen allgemeinen Formel I, jeweils ggf. in Form eines seiner reiner Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Vanilloid-Rezeptor 1-(VR1 /TRPV1)-Regulation, vorzugsweise zur Vanilloid-Rezeptor 1-(VR1/TRPV1)-Hemmung oder zur Vanilloid-Rezeptor 1-(VR1/TRPV1)-Stimutation.

Bevorzugt ist die Verwendung wenigstens eines 2,5-disubstituierten Thiazol-4-on-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Prophylaxe und/oder Behandlung von Störungen oder Krankheiten, die zumindest teilweise durch Vanilloid-Rezeptoren 1 vermittelt werden.

Besonders bevorzugt ist die Verwendung wenigstens eines substituierten 2,5-disubstituierten Thiazol-4-on-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

Ganz besonders bevorzugt ist die Verwendung wenigstens eines substituierten 2,5-Thiazol-4-on-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; Angstzuständen; kognitiven Dysfunktionen, vorzugsweise Gedächtnisstörungen; kognitive Mangelzustände (attention deficit syndrom, ADS); Epilepsie; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden.

Noch weiter bevorzugt ist die Verwendung wenigstens eines 2,5-disubstituierten Thiazol-4-on-Derivats der vorstehend angegebenen allgemeinen Formel I, jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. eines oder mehrerer pharmazeutisch verträglicher Hilfsstoffe zur Herstellung eines Arzneimittels zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Ein weiterer Gegenstand der vorliegenden Erfindung sind 2,5-disubstituierte Thiazol-4-on-Derivate der allgemeinen Formel Ia, 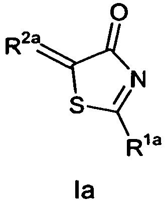 worin
- R^{1a}: für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
für eine NR^{3a}R^{4a}-Gruppe;
für eine NR^{5a}-C(=O)-R^{6a}-Gruppe
oder für eine NR^{7a}-C(=O)-NR^{8a}R^{9a} Gruppe steht;
- R^{2a}: für CH-CH=C(CH₃)-W^{a}-Z^{a}, CH-CH=CH-W^{a}-Z^{a}, CH-C(CH₃)=CH-W^{a}-Z^{a}, CH-C(Phenyl)=CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} oder CH-CH(CH₃)-CH₂-W^{a}-Z^{a} steht;
wobei W^{a} jeweils nicht vorhanden sein kann oder jeweils für einen Rest ausgewählt aus der Gruppe bestehend aus O, S, N(H), N(CH₃), N(C₂H₅) und N[CH(CH₃)₂] steht;
- R^{3a}: für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
oder für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert und/oder über eine lineare oder verzweigte, unsubstituierte oder wenigstens einfach substituierte Alkylen-, Alkenylen- oder Alkinylen-Gruppe gebunden sein kann, steht;
- R^{4a}: für einen Wasserstoff-Rest
oder für einen linearen oder verzweigten, gesättigten oder ungesättigten, unsubstituierten oder wenigstens einfach substituierten, ggf. wenigstens ein Heteroatom als Kettenglied aufweisenden aliphatischen Rest steht;
- R^{5a}, R^{7a} und R^{9a},: unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
- R^{6a} und R^{8a},: unabhängig voneinander, jeweils
für einen unsubstituierten oder wenigstens einfach substituierten Aryl- oder Heteroaryl-Rest, der mit einem unsubstituierten oder wenigstens einfach substituierten mono-oder polyzyklischen Ringsystem kondensiert sein kann, stehen;
- **Z^{a}**: für einen linearen oder verzweigten, gesättigten oder ungesättigten, unsubstituierten oder wenigstens einfach substituierten aliphatischen Rest;
für einen Aryl-Rest steht, der mit einem unsubstituierten oder wenigstens einfach substituierten mono- oder polyzyklischen Ringsystem kondensiert ist und/oder mit gleichen oder verschieden Substituenten R^{12a} substituiert ist:
oder für einen Heteroaryl-Rest, der mit einem unsubstituierten oder wenigstens einfach substituierten mono- oder polyzyklischen Ringsystem kondensiert ist und/oder mit gleichen oder verschiedenen Substituenten R^{12a} substituiert ist, steht:
- R^{12a}: für einen Rest ausgewählt aus der Gruppe bestehend aus F, Cl. Br, I, -CN, - CF₃, -SF₆, -OH, -O-C₁₋₁₀-Alkyl, -O-C₂₋₁₀-Alkenyl, -NH₂, -O-CF₃, -S-CF₃, -SH, - S-C₁₋₅-Alkyl, -C₁₋₁₀-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₃Alkyl, -C(=O)-N-(C₁₋₅-Alkyl)₂, -S(=O)₂-C₁₋₅-Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl steht; wobei jeweils der zyklische Teil der Reste Pyridinyl, Cyclopentyl, Cyclohexyl, Pyridazinyl, - S(=O)₂-Phenyl, -O-S(=O)₂-Phenyl, -O-Phenyl, -O-Benzyl, Phenyl, -(CH₂)-Benzo[b]furanyl und Benzyl substituiert sein kann;
für eine -C(=O)-NR^{13a}-(CH₂)ₘ-NR^{14a}-R^{15a}-Gruppe mit m^{a} gleich 0, 1, 2, 3, 4 oder 5;
oder für eine -C(=O)-R^{16a}-Gruppe steht;
- R^{13a} und R^{14a},: unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
- R^{15a}: für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, steht;
und
- R^{16a}: für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann, steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Besonders bevorzugt sind 2,5-disubstituierte Thiazol-4-on-Derivate der vorstehend angegebenen allgemeinen Formel Ia, worin
- R^{1a}: für einen der folgenden Reste steht 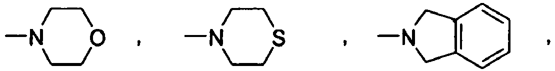 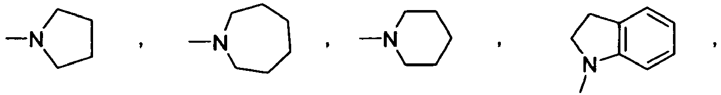 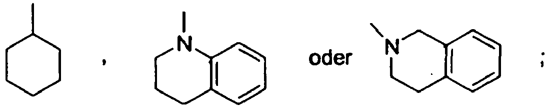 wobei der Rest mit 1, 2 oder 3 Substituenten unabhängig voneinander ausgewählt aus der Gruppe bestehend aus -OH, -O-CH₃, -O-C₂H₅, Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, - NH-Pyridinyl, -N(CH₃)-Phenyl, -N(C₂H₅)-Phenyl, -N[CH(CH₃)₂]-Phenyl, - N(CH₃)-Pyridinyl, -N(C₂H₅)-Pyridinyl, -N[CH(CH₃)₂]-Pyridinyl und Phenyl substituiert sein kann; wobei jeweils der zyklische Teil des Phenyl-Restes mit 1, 2 oder 3 Substituenten unabhängig voneinander ausgewählt aus der Gruppe bestehend aus F, Cl, Br und -CF₃ substituiert sein kann;
für einen der folgenden Reste steht 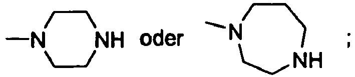 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für eine NR^{3a}R^{4a}-Gruppe steht
oder für eine NR^{7a}-C(=O)-NR^{8a}R^{9a} Gruppe steht;
- R^{2a}: für CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(Phenyl)=CH-Z^{a}, CH-CBr=CH-Z^{a}, CH-CCl=CH-Z^{a}, CH-CF=CH-Z^{a}, CH-C(OH)=CH-Z^{a}, CH-CH₂-CH₂-Z^{a}, CH-CH₂-CH(CH₃)-Z^{a} oder CH-CH(CH₃)-CH₂-Z^{a} steht;
- R^{3a}: für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopentyl Cyclohexyl, Cycloheptyl, Cyclopentenyl, Cyclohexenyl und Cycloheptenyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, - NH-Pyridinyl, -N(CH₃)-Phenyl, -N(C₂H₅)-Phenyl, -N[CH(CH₃)₂]-Phenyl, - N(CH₃)-Pyridinyl, -N(C₂H₅)-Pyridinyl, -N[CH(CH₃)₂]-Pyridinyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
- R^{4a}: für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
- R^{7a} und R^{8a},: unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
- R^{9a}: für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CF₃, -S-CF₃, Methyl, Ethyl, n-Propyl, Isopropyl, n-Butyl, sec-Butyl, Isobutyl und tert-Butyl substituiert sein kann;
- Z^{a}: für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht;
für einen Rest ausgewählt aus der Gruppe bestehend aus Phenyl, Naphthyl, Phenanthrenyl, (1,3)-Benzodioxolyl und (1,4)-Benzodioxanyl steht; der mit 1, 2, 3, 4 oder 5 gleichen oder verschiedenen Substituenten R^{12a} substituiert ist;
oder für einen Rest ausgewählt aus der Gruppe bestehend Thiophenyl, Furanyl, Pyrrolyl, Pyrazolyl, Pyrazinyl, Pyridinyl, Pyridazinyl, Pyrimidinyl, Chinolinyl und Isochinolinyl steht; der mit 1, 2, 3, 4 oder 5 gleichen oder verschiedenen Subsbtuenten R^{12a} substituiert ist;
- R^{12a}: für einen Rest ausgewählt aus der Gruppe bestehend aus F, Cl, Br, -SF₅, - CF₃, -O-CH₃, -O-C₂H₅, -O-CH₂-CH₂-CH₃, -O-CH₂-CH₂-CH₂-CH₃, -O-C(CH₃)_{3,} - O-CH₂-CH₂-CH₂-CH₂-CH₃, -O-CH₂-CH₂-C(CH₃)₃, Methyl, Ethyl, n-Propyl. Isopropyl, n-Butyl, sec-Butyl, tert-Butyl, n-Pentyl, n-Hexyl, n-Heptyl und n-Octyl steht;
Jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Ganz besonders bevorzugt sind 2,5-disubstituierte Thiazol-4-on-Derivate der vorstehend angegebene allgemeinen Formel Ia, worin
- R^{1a}: für einen der folgenden Reste steht 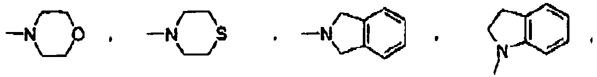 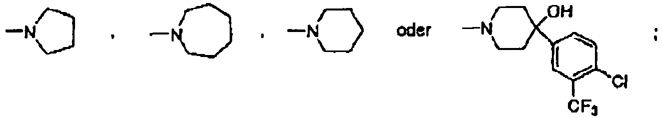 für den folgenden Rest steht 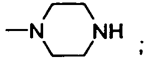 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für eine NR^{3a}R^{4a}-Gruppe steht
oder für eine NR^{7a}-C(=O)-NR^{8a}R^{9a} Gruppe steht;
- R^{2a}: für CH-CH=C(CH₃)-Z^{a}, CH-CH=CH-Z^{a}, CH-CH=CH-S-Z^{a}, CH-C(CH₃)=CH-Z^{a}, CH-C(Phenyl)=CH-Z^{a}, CH-CBr=CH-Z^{a}, CH-CCl=CH-Z^{a}, CH-CF=CH-Z^{a}, CH-CH₂-CH₂-Z^{a}, CH-CH₂-CH(CH₃)-Z^{a} oder CH-CH(CH₃)-CH₂-Z^{a} steht;
- R^{3a}: für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
oder für einen Rest ausgewählt aus der Gruppe bestehend aus Phenyl, Naphthyl, Benzyl, (1,3)-Benzodioxolyl und (1,4)-Benzodioxanyl steht; wobei der Rest jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
- R^{4a}: für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
- R^{7a} und R^{8a}: jeweils für einen Wasserstoff-Rest stehen;
- R^{9a}: für einen Phenyl-Rest steht, der mit 1, 2, 3, 4 oder 5 Substituenten unabhängig voneinander ausgewählt aus der Gruppe bestehend aus F, Cl, Br und -CF₃ substituiert sein kann;
- Z^{a}: für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl und n-Octyl steht;
oder für einen Phenyl- oder Naphthyl-Rest steht, der mit 1, 2, 3, 4 oder 5 gleichen oder verschiedenen Substituenten R^{12a} substituiert ist;
- R^{12a}: für einen Rest ausgewählt aus der Gruppe bestehend aus F, Cl, Br, I, -CN, - CF₃, -SF₅, -OH, -O-CH₃, -O-C₂H₅, -O-CH₂-CH₂-CH₃, -O-CH₂-CH₂-CH₂-CH₃, - O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

Noch weiter bevorzugt sind 2,5-disubstituierte Thiazol-4-on-Derivate der vorstehend angegebenen allgemeinen Formel Ia ausgewählt aus der Gruppe bestehend aus
1. 5-[3-(4-Methoxy-phenyl)-but-2-enyliden]-2-morpholin-4-yl-thiazol-4-on
2. 5-[3-(4-Fluor-phenyl)-allyliden]-2-morpholin-4-yl-thiazol-4-on
3. 2-Morpholin-4-yl-5-(3-m-tolyl-allyliden)-thiazol-4-on
4. 2-Morpholin-4-yl-5-(3-p-tolyl-allyliden)-thiazol-4-on
5. 2-Morpholin-4-yl-5-octyliden-thiazol-4-on
6. 5-Butyliden-2-morpholin-4-yl-thiazol-4-on
7. 5-Hexyliden-2-morpholin-4-yl-thiazol-4-on
8. 2-Morpholin-4-yl-5-(3-p-tolyl-but-2-enyliden)-thiazol-4-on
9. 5-[3-(4-tert-Butyl-phenyl)-but-2-enyliden]-2-morpholin-4-yl-thiazol-4-on
10. 5-[3-(4-tert-Butyl-phenyl)-allyliden]-2-[4-(3-methoxy-phenyl)-piperazin-1-yl]-thiazol-4-on
11. 5-[3-(4-Hydroxy-3-methoxy-phenyl)-allyliden]-2-thiomorpholin-4-yl-thiazol-4-on
12. 2-Morpholin-4-yl-5-[3-(4-trifluormethyl-phenyl)-but-2-enyliden]-thiazol-4-on
13. 2-Morpholin-4-yl-5-[3-(3-trifluormethyl-phenyl)-but-2-enyliden]-thiazol-4-on
14. 5-Decyliden-2-morpholin-4-yl-thiazol-4-on
15. 5-[3-(4-tert-Butyl-phenyl)-allyliden]-2-morpholin-4-yl-thiazol-4-on
16. 5-[3-(4-tert-Butyl-phenyl)-allyliden]-2-[4-(3-Chlor-pyridin-2-yl)-piperazin-1-yl]-thiazol-4-on
17. 5-[3-(4-tert-Butyl-phenyl)-allyliden]-2-[4-(2-fluor-phenyl)-piperazin-1-yl]-thiazol-4-on
18. 5-[3-(4-tert-Butyl-phenyl)-allyliden]-2-[4-(4-chlor-[1,2,5]thiadiazol-3-yl)-piperazin-1-yl]-thiazol-4-on
19. 5-[3-(4-tert-Butyl-phenyl)-allyliden]-2-[4-(6-chlor-pyridin-2-yl)-piperazin-1-yl]-thiazol-4-on
20. 5-[3-(4-tert-Butyl-phenyl)-allyliden]-2-[4-(6-methyl-pyridazin-3-yl)-piperazin-1-yl]-thiaxol-4-on
21. 2-[(Benzo[1,3]dioxol-5-ylmethyl)-amino]-6-[3-(4-tert-butyl-phenyl)-allyliden]-thiazol-4-on
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, vorzugsweise ihrer Hydrochloride oder Hydrobromide, oder jeweils in Form entsprechender Solvate.

Ferner können erfindungsgemäße Verbindungen bevorzugt sein, die im FLIPR-Assay in einer Konzentration von 10 µM eine Hemmung des Ca²⁺-Ionen-Einstroms in Dorsalwurzelganglien von Ratten von wenigstens 10 %, bevorzugt von wenigstens 30 %, besonders bevorzugt von wenigstens 50 %, ganz besonders bevorzugt von wenigstens 70 %, noch weiter bevorzugt von wenigstens 90 %, im Vergleich zur maximal erreichbaren Hemmung des Ca²⁺-Ionen-Einstroms mit Capsaicin in einer Konzentration von 10 µM aufweisen.

Dabei wird Im FLIPR-Assay der Ca²⁺-Einstrom mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert, wie untenstehend beschrieben.

Ein weiterer Gegenstand der vorliegenden Erfindung ist ein Verfahren zur Herstellung von erfindungsgemäßen Verbindungen der vorstehend angegebenen allgemeinen Formel I Gemäß dem 2-Amino-thiazol-4-on in Essigsäure in Gegenwart wenigstens eines Salzes ausgewählt aus der Gruppe bestehend aus Natriumacetat und Kaliumacetat oder in einem Reaktionsmedium ausgewählt aus der Gruppe bestehend aus Methanol, Ethanol, Isopropanol und n-Butanol in Gegenwart wenigstens einer organischen Base ausgewählt aus der Gruppe bestehend aus Triethylamin, Pyridin, Diisopropylamin und N-Methylmorpholin mit wenigstens einer Verbindung der allgemeinen Formel R-C(=O)-H, worin R für -CH=C(CH₃)-W^{a}-Z^{a}, - CH=CH-W^{a}-Z^{a}, -C(CH₃)=CH-W^{a}-Z^{a}, -C(Phenyl)=CH-W^{a}-Z^{a}, -CBr=CH-W^{a}-Z^{a}, - CCl=CH-W^{a}-Z^{a}, -CF=CH-W^{a}-Z^{a}, -C(OH)=CH-W^{a}-Z^{a}, -CH₂-CH₂-W^{a}-Z^{a}, -CH₂-CH(CH₃)-W^{a}-Z^{a} oder -CH(CH₃)-CH₂-W^{a}-Z^{a} steht, wobei W^{a} und Z^{a} die vorstehend genannte Bedeutung haben, zu wenigstens einer Verbindung der allgemeinen Formel IIa, 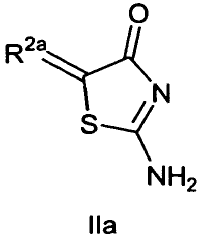 worin R^{2a} die vorstehend genannte Bedeutung hat, umgesetzt wird und diese ggf. gereinigt und/oder isoliert wird
und wenigstens eine Verbindung der allgemeinen Formel IIa in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R^{3a} oder der allgemeinen Formel LG-R^{7a} oder der allgemeinen Formel LG-R^{5a}, worin LG jeweils für eine Abgangsgruppe, bevorzugt für ein Halogen-Atom, besonders bevorzugt für ein Chloratom steht und R^{3a}, R^{5a} und R^{7a} jeweils die vorstehend genannte Bedeutung haben, zu wenigstens einer Verbindung der allgemeinen Formel Ia, worin R^{2a} die vorstehend genannte Bedeutung hat und R^{1a} für eine NHR^{3a}-, NHR^{5a}- oder NHR^{7a}-Gruppe steht, umgesetzt wird und diese ggf. gereinigt und/oder isoliert wird,
und ggf. wenigstens eine Verbindung der allgemeinen Formel lia, worin R^{2a} die vorstehend genannte Bedeutung hat und R^{1a} für eine NHR^{7a}-Gruppe steht, in einem Reaktionsmedium mit wenigstens einem Isocyanat der allgemeinen Formel R^{8a}-N=C=O, worin R^{8a} die vorstehend genannte Bedeutung hat, ggf. in Gegenwart wenigstens einer Base, bevorzugt in Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel Ia, worin R^{2a} die vorstehend genannte Bedeutung hat und R^{1a} für eine NR^{7a}-C(=O)-NHR^{8a}-Gruppe steht, umgesetzt wird und diese ggf. gereinigt und/oder isoliert wird;
und ggf. wenigstens eine Verbindung der allgemeinen Formel Ia, worin R^{2a} die vorstehend genannte Bedeutung hat und R^{1a} für eine NR^{7a}-C(=O)-NHR^{8a}-Gruppe steht, in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R^{9a}, worin LG für eine Abgangsgruppe, bevorzugt für ein Halogen-Atom, besonders bevorzugt für ein Chloratom steht und R^{9a} die vorstehend genannte Bedeutung hat, zu wenigstens einer Verbindung der allgemeinen Formel Ia, worin R^{2a} die vorstehend genannte Bedeutung hat und R^{1a} für eine NR^{7a}-C(=O)-NR^{8a}R⁹-Gruppe steht, umgesetzt wird und diese ggf. gereinigt und/oder isoliert wird;
oder
ggf. wenigstens eine Verbindung der allgemeinen Formel IIa, worin R^{2a} die vorstehend genannte Bedeutung hat und R^{1a} für eine NHR^{5a}-Gruppe steht, in einem Reaktionsmedium ggf. in Gegenwart wenigstens einer Base mit wenigstens einer Verbindung der allgemeinen Formel R^{6a}-C(=O)-LG, worin R^{6a} die vorstehend genannte Bedeutung hat und LG für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel R^{6a}-C(=O)-OH, worin R^{5a} die vorstehend genannte Bedeutung hat, zu wenigstens einer Verbindung der allgemeinen Formel la umgesetzt wird, worin R^{2a} die vorstehend genannte Bedeutung hat und R^{1a} für eine NR^{5a}-C(=O)-R^{6a}-Gruppe steht, und diese ggf. gereinigt und/oder isoliert wird
oder
ggf. wenigstens eine Verbindung der allgemeinen Formel Ia, worin R^{2a} die vorstehend genannte Bedeutung hat und R^{1a} für eine NHR^{3a}-Gruppe steht, in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R^{4a}, worin LG für eine Abgangsgruppe, bevorzugt für ein Halogen-Atom, besonders bevorzugt für ein Chloratom steht und R^{4a} die vorstehend genannte Bedeutung hat, zu wenigstens einer Verbindung der allgemeinen Formel Ia, worin R^{2a} die vorstehend genannte Bedeutung hat und R^{1a} für eine NR^{3a}R^{4a}-Gruppe steht, umgesetzt wird und diese ggf. gereinigt und/oder isoliert wird.

Die Umsetzung von Verbindungen der allgemeinen Formel IIa, worin R^{1a} für eine - NHR^{5a}-Gruppe steht, mit Verbindungen der allgemeinen Formel R^{6a}-C(=O)-OH erfolgt vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart einer organischen Base, vorzugsweise ausgewählt aus der Gruppe bestehend aus Triethylamin, 4,4-Dimethylaminopyridin, Pyridin und Diisopropylethylamin, oder einer anorganischen Base, bei Temperaturen von vorzugsweise -70°C bis 100°C.

Die Umsetzung von Verbindungen der allgemeinen Formel IIa, worin R^{1a} für eine - NHR^{5a}-Gruppe steht, mit Verbindungen der allgemeinen Formel R^{6a}-C(=O)-OH erfolgt vorzugsweise in einem Reaktionsmedium ausgewählt aus der Gruppe bestehend aus Diethylether, Tetrahydrofuran, Acetonitril, Methanol, Ethanol, Dimethylformamid, Dichlormethan und entsprechenden Mischungen, ggf. in Gegenwart wenigstens eines Kupplungsreagenzes, vorzugsweise ausgewählt aus der Gruppe bestehend aus 1-Benzotriazolyloxy-tris-(dimethylamino)-phosphonium hexafluorophosphat (BOP), Dicyclohexylcarbodiimid (DCC), N'-(3-Dimethylaminopropyl)-N-ethylcarbodiimid (EDCI), N-[(Dimethyamino)-1H-1, 2, 3-triazolo[4, 5-b]pyridino-1-ylmethylen]-N-methylmethanaminium hexafluorophosphat N-oxid (HATU), O-(Benzotriazol-1-yl)-N,N,N',N'-tetramethyluroniom hexafluorophosphat (HBTU), O-(Benzotriazol-1-yl)-N,N,N',N'-tetramethyluroniom tetrafluoroborat (TBTU), 1-Hydroxybenzotriazol (HOBt) und 1-Hydroxy-7-azabenzotriazol (HOAt), ggf. in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, 4-Methylmorpholin, Pyridin, N,N-Dimethylaminopyridin und Diisopropylethylamin vorzugsweise bei Temperaturen von -70°C bis 100°C.

Ebenfalls ein Gegenstand der vorliegenden Erfindung ist ein Verfahren zur Herstellung von 2,5-disubstiuierten Thiazol-4-on-Derivaten der allgemeinen Formel la gemäß dem wenigstens eine Verbindung der allgemeinen Formel R^{1a}-CN, worin R^{1a} die vorstehend genannte Bedeutung mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, in einem Reaktionsmedium ausgewählt aus der Gruppe bestehend aus Methanol, Ethanol, Isopropanol und n-Butanol, in Gegenwart wenigstens einer organischen Base ausgewählt aus der Gruppe bestehend aus Triethylamin, Pyridin, Diisopropylamin und N-Methylmorpholin oder in Pyridin als Reaktionsmedium mit Thioglykolsäure zu wenigstens einer Verbindung der allgemeinen Formel IIIa, 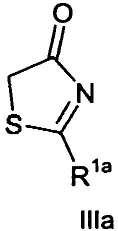 worin R^{1a} die vorstehend genannte Bedeutung mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, umgesetzt wird und diese ggf. gereinigt und/oder isoliert wird;
oder wenigstens einer Verbindung der allgemeinen Formel H₂N-C(=S)-NHR^{1a}, worin R^{1a} die vorstehend genannte Bedeutung mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, in einem Reaktionsmedium ausgewählt aus der Gruppe bestehend aus Methanol, Ethanol, Isopropanol und n-Butanol, ggf. in Gegenwart wenigstens einer organischen Base ausgewählt aus der Gruppe bestehend aus Triethylamin, Pyridin, Diisopropylamin und N-Methylmorpholin, mit Chloressigsäure zu wenigstens einer Verbindung der allgemeinen Formel IIIa, worin R^{1a} die vorstehend genannte Bedeutung mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, umgesetzt wird und diese ggf. gereinigt und/oder isoliert wird;
und wenigstens eine Verbindung der allgemeinen Formel IIIa in Essigsäure in Gegenwart wenigstens eines Salzes ausgewählt aus der Gruppe bestehend aus Natriumacetat und Kaliumacetat oder in einem Reaktionsmedium ausgewählt aus der Gruppe bestehend aus Methanol, Ethanol, Isopropanol und n-Butanol in Gegenwart wenigstens einer organischen Base ausgewählt aus der Gruppe bestehend aus Triethylamin, Pyridin, Diisopropylamin und N-Methylmorpholin mit wenigstens einer Verbindung der allgemeinen Formel R-C(=O)-H, worin R für-CH=C(CH₃)-W^{a}-Z^{a}, -CH=CH-W^{a}-Z^{a}, -C(CH₃)=CH-W^{a}-Z^{a}, -C(Phenyl)=CH-W^{a}-Z^{a}, - CBr=CH-W^{a}-Z^{a}, -CCl=CH-W^{a}-Z^{a}, -CF=CH-W^{a}-Z^{a}, -C(OH)=CH-W^{a}-Z^{a}, -CH₂-CH₂-W^{a}-Z^{a}, -CH₂-CH(CH₃)-W^{a}-Z^{a} oder -CH(CH₃)-CH₂-W^{a}-Z^{a} steht, worin W^{a} und Z^{a} die vorstehend genannte Bedeutung haben, zu wenigstens einer Verbindung der allgemeinen Formel Ia, worin R^{1a} die vorstehend genannte Bedeutung mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat und R^{2a} die vorstehend genannte Bedeutung hat.

Die Verbindungen der vorstehend angegebenen allgemeinen Formeln R^{1a}-CN, R-C(=O)-H, R^{3a}-LG, R^{4a}-LG, R^{5a}-LG, R^{7a}-LG, R^{9a}-LG, H₂N-C(=S)-NHR^{1a}, R^{6a}-C(=O)-LG, R^{6a}-C(=O)-OH und R^{8a}-N=C=O sind jeweils am Markt käuflich erhältlich und können auch nach üblichen, dem Fachmann bekannten Verfahren hergestellt werden.

Bevorzugt lassen sich Verbindungen der vorstehend angegebenen allgemeinen Formel H₂N-C(=S)-NHR^{1a} durch Umsetzung von 1,1'-Thiocarbonyldiimidazol mit Verbindungen der allgemeinen Formel R^{1a}NH₂ in Tetrahydrofuran erhalten.

Die vorstehend beschriebenen Umsetzungen können jeweils unter den üblichen dem Fachmann geläufigen Bedingungen, beispielsweise in Hinblick auf Druck oder Reihenfolge der Zugabe der Komponenten durchgeführt werden. Ggf. kann die unter den jeweiligen Bedingungen optimale Verfahrensführung vom Fachmann durch einfache Vorversuche ermittelt werden. Die nach den vorstehend beschriebenen Umsetzungen erhaltenen Zwischen- und Endprodukte können jeweils, falls gewünscht und/oder erforderlich, nach üblichen, dem Fachmann bekannten Methoden gereinigt und/oder isoliert werden. Geeignete Reinigungsverfahren sind beispielsweise Extraktionsverfahren und chromatographische Verfahren wie Säulenchromatographie oder präparative Chromatographie. Sämtliche der vorstehend genannten Verfahrensschritte sowie jeweils auch die Reinigung und/oder Isolierung von Zwischen- oder Endprodukten können teilweise oder vollständig unter einer Inertgasatmosphäre, vorzugsweise unter Stickstoffatmosphäre, durchgeführt werden.

Die erfindungsgemäßen 2,5-disubstiuierten Thiazol-4-on-Derivate der vorstehend genannten allgemeinen Formeln I und Ia, im Folgenden nur als 2,5-disubstituierte Thiazol-4-on-Derivate der allgemeinen Formel I bezeichnet, sowie entsprechende Stereoisomere können sowohl in Form ihrer freien Basen, ihrer freien Säuren wie auch in Form entsprechender Salze, insbesondere physiologisch verträglicher Salze, isoliert werden. Die freien Basen der jeweiligen erfindungsgemäßen 2,5-disubstituierten Thiazol-4-on-Derivate der vorstehend genannten allgemeinen Formel I sowie entsprechender Stereoisomere können beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Zitronensäure, Glutaminsäure oder Asparaginsäure, in die entsprechenden Salze, vorzugsweise physiologisch verträglichen Salze, überführt werden. Die freien Basen der jeweiligen 2,5-disubstituierten Thiazol-4-on-Derivate der vorstehend genannten allgemeinen Formel I und entsprechender Stereoisomere können ebenfalls mit der freien Säure oder einem Salz eines Zuckerersatzstoffes, wie z.B. Saccharin, Cyclamat oder Acesulfam, in die entsprechenden physiologisch verträglichen Salze überführt werden.
Entsprechend können die freien Säuren der 2,5-disubstituierten Thiazol-4-on-Derivate der vorstehend genannten allgemeinen Formel I und entsprechender Stereoisomere durch Umsetzung mit einer geeigneten Base in die entsprechenden physiologisch verträglichen Salze überführt werden. Beispielhaft seien die Alkalimetallsalze, Erdalkalimetallsalze oder Ammoniumsalze [NHₓR₄₋ₓ]⁺, worin x = 0, 1, 2, 3 oder 4 ist und R für einen linearen oder verzweigten C₁₋₄-Alkyl-Rest steht, genannt.

Die erfindungsgemäßen 2,5-disubstituierten Thiazol-4-on-Derivate der vorstehend genannten allgemeinen Formel I und entsprechender Stereoisomere können ggf., ebenso wie die entsprechenden Säuren, die entsprechenden Basen oder Salze dieser Verbindungen, nach üblichem, dem Fachmann bekannten Methoden auch in Form ihrer Solvate, vorzugsweise in Form ihrer Hydrate, erhalten werden.

Sofern die erfindungsgemäßen 2,5-disubstituierten Thiazol-4-on-Derivate der vorstehend genannten allgemeinen Formel I nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler stationärer Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen 2,5-disubstituierten Thiazol-4-on-Derivate der vorstehend genannten allgemeinen Formel I und entsprechende Stereoisomere sowie jeweils die entsprechenden Säuren, Basen, Salze und Solvate sind toxikologisch unbedenklich und eignen sich daher als pharmazeutische Wirkstoffe in Arzneimitteln.

Ein weiterer Gegenstand der vorliegenden Erfindung ist daher ein Arzneimittel enthaltend wenigstens ein erfindungsgemäßes 2,5-disubstituiertes Thiazol-4-on-Derivat der vorstehend angegebenen allgemeinen Formel I, jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form eines entsprechenden Salzes, oder jeweils in Form eines entsprechenden Solvates, sowie ggf. einen oder mehrere pharmazeutisch verträgliche Hilfsstoffe.

Diese erfindungsgemäßen Arzneimittel eignen sich insbesondere zur Vanilloid-Rezeptor 1-(VR1/TRPV1)-Regulation, insbesondere zur Vanilloid-Rezeptor 1-(VR1/TRPV1)-Hemmung oder zur Vanilloid-Rezeptor 1-Stimulation.

Ebenfalls bevorzugt eignen sich die erfindungsgemäßen Arzneimittel zur Prophylaxe und/oder Behandlung von Störungen oder Krankheiten, die zumindest teilweise durch Vanilloid-Rezeptoren 1 vermittelt werden.

Bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Migräne; Depressionen; Harninkontinenz; Husten; neurodegenerativen Erkrankungen, vorzugsweise ausgewählt aus der Gruppe bestehend aus Morbus Parkinson, Morbus Huntington, Morbus Alzheimer und multipler Sklerose; Störungen der Nahrungsmittelaufnahme, vorzugsweise ausgewählt aus der Gruppe bestehend aus Bulimie, Anorexie, Fettsucht und Kachexie; Angstzuständen; kognitiven Dysfunktionen, vorzugsweise Gedächtnisstörungen; kognitive Mangelzustände (attention deficit syndrom, ADS); Epilepsie; Diarrhoe und Pruritus; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden; zur Regulation der Nahrungsaufnahme; zur Modulation der Bewegungsaktivität; zur Regulation des kardiovaskulären Systems; zur Lokalanästhesie; zur Vigilanzsteigerung; zur Libidosteigerung; zur Diurese und/oder zur Antinatriurese.

Besonders bevorzugt eignet sich das erfindungsgemäße Arzneimittel daher zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz; zur Prophylaxe und/oder Behandlung von einer oder mehreren Erkrankungen ausgewählt aus der Gruppe bestehend aus Migräne; Depressionen; neurodegenerativen Erkrankungen, vorzugsweise ausgewählt aus der Gruppe bestehend aus Morbus Parkinson, Morbus Huntington, Morbus Alzheimer und multipler Sklerose; Angstzuständen; kognitiven Dysfunktionen, vorzugsweise Gedächtnisstörungen; kognitive Mangelzustände (attention deficit syndrom, ADS); Epilepsie; zur Prophylaxe und/oder Behandlung von Alkohol- und/oder Drogen- und/oder Medikamentenmißbrauch und Alkohol- und/oder Drogen- und/oder Medikamentenabhängigkeit, vorzugsweise zur Prophylaxe und/oder Verminderung von Entzugserscheinungen bei Alkohol- und/oder Drogen- und/oder Medikamentenabhängigkeit; zur Prophylaxe und/oder Verminderung einer Toleranzentwicklung gegenüber Medikamenten, insbesondere Medikamenten auf Basis von Opioiden.

Ganz besonders bevorzugt eignet sich das erfindungsgemäße Arzneimittel zur Behandlung und/oder Prophylaxe von Schmerz, vorzugsweise von Schmerz ausgewählt aus der Gruppe bestehend aus akutem Schmerz, chronischem Schmerz, visceralem Schmerz und neuropathischem Schmerz.

Das erfindungsgemäße Arzneimittel eignet sich zur Verabreichung an Erwachsene und Kinder einschließlich Kleinkindern und Säuglingen. Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem 2,5-disubstituierten Thiazol-4-on-Derivat der vorstehend angegebenen allgemeinen Formel I, ggf. in Form eines seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, oder ggf. in Form eines entsprechenden Salzes oder jeweils in Form eines entsprechendes Solvates, enthält das erfindungsgemäße Arzneimittel üblicherweise weitere physiologisch verträgliche pharmazeutische Hilfsstoffe, die beispielsweise ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden erfindungsgemäßen 2,5-disubstituierten Thiazol-4-on-Derivate in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können das jeweilige erfindungsgemäße 2,5-disubstituierte Thiazol-4-on-Derivat auch verzögert freisetzen.

Die Herstellung der erfindungsgemäßen Arzneimittel erfolgt mit Hilfe von üblichen, aus dem Stande der Technik bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind. Die entsprechende Beschreibung wird hiermit als Referenz eingeführt und gilt als Teil der Offenbarung.

Die an den Patienten zu verabreichende Menge der jeweiligen erfindungsgemäßen 2,5-disubstituierten Thiazol-4-on-Derivate der vorstehend angegebenen allgemeinen Formel I kann variieren und ist beispielsweise abhängig vom Gewicht oder Alter des Patienten sowie von der Applikationsart, der Indikation und dem Schweregrad der Erkrankung. Üblicherweise werden 0,001 bis 100 mg/kg, vorzugsweise 0,05 bis 75 mg/kg, besonders bevorzugt 0,05 bis 50 mg/kg, Körpergewicht des Patienten wenigstens einer solchen erfindungsgemäßen Verbindung appliziert.

### Pharmakologische Methoden:

### I. Funktionelle Untersuchung am Vanilloid-Rezeptor 1 (VRI/TRPV1-Rezeptor)

Die agonistische bzw. antagonistische Wirkung der zu untersuchenden Substanzen am Vanilloid-Rezeptor 1 (VR1/TRPV1) der Spezies Ratte kann mit folgendem Assay bestimmt werden. Gemäß diesem Assay wird der Ca²⁺-Einstrom durch den Rezeptorkanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### Methode:

Komplett-Medium: 50 mL HAMS F12 Nutrient Mixture (Gibco Invitrogen GmbH, Karlsruhe, Deutschland) mit
10 Vol-% FCS (fetal calf serum, Gibco Invitrogen GmbH, Karlsruhe, Deutschland, hitzeinaktiviert);
2mM L-Glutamin (Sigma, München, Deutschland);
1 Gew-% AA-Lösung (Antibiotika/Antimykotika-Lösung, PAA, Pasching, Österreich) und 25 ng/ml Medium NGF (2.5 S, Gibco Invitrogen GmbH, Karlsruhe, Deutschland)

Zellkultur-Platte: Poly-D-Lysin-beschichtete, schwarze 96-Loch-Platten mit klarem Boden (96 well black/clear plate, BD Biosciences, Heidelberg, Deutschland) werden zusätzlich mit Laminin (Gibco Invitrogen GmbH, Karlsruhe, Deutschland) beschichtet, indem Laminin auf eine Konzentration 100 µg/mL mit PBS (Ca-Mg-free PBS, Gibco Invitrogen GmbH, Karlsruhe, Deutschland) verdünnt wird. Es werden Aliquots mit einer Konzentration von 100 µg/mL an Laminin entnommen und bei -20 °C gelagert. Die Aliquots werden mit PBS im Verhältnis 1:10 auf 10 µg/mL Laminin verdünnt und jeweils 50 µL der Lösung in eine Vertiefung der Zellkultur-Platte pipettiert. Die Zellkultur-Platten werden mindestens zwei Stunden bei 37 °C inkubiert, die überstehende Lösung abgesaugt und die Vertiefungen werden jeweils zweimal mit PBS gewaschen. Die beschichteten Zellkultur-Platten werden mit überstehendem PBS aufbewahrt und dieses erst direkt vor der Aufgabe der Zellen entfernt.

### Präparation der Zellen:

Enthaupteten Ratten wird die Wirbelsäule entnommen und diese direkt in kalten, d. h. in einem Eisbad befindlichen, HBSS-Puffer (Hank's buffered saline solution, Gibco Invitrogen GmbH, Karlsruhe, Deutschland) versetzt mit 1 Vol-% (Volumenprozent) einer AA-Lösung (Antibiotika/Antimykotika-Lösung, PAA, Pasching, Österreich) gelegt. Die Wirbelsäule wird längs durchtrennt und zusammen mit Fascien dem Wirbelkanal entnommen. Anschließend werden die Dorsalwurzelganglien (DRGs; dorsal root ganglia) entnommen und wiederum in kaltem HBSS-Puffer versetzt mit 1 Vol-% einer AA-Lösung aufbewahrt. Die vollständig von Blutresten und Spinalnerven befreiten DRGs werden jeweils in 500 µL kalte Collagenase Typ 2 (PAA, Pasching, Österreich) überführt und 35 Minuten bei 37 °C inkubiert. Nach Zugabe von 2.5 Vol-% Trypsin (PAA, Pasching, Österreich) wird weitere 10 Minuten bei 37 °C inkubiert. Nach der vollständigen Inkubation wird die Enzymlösung vorsichtig ab pipettiert und die zurückgebliebenen DRGs werden jeweils mit 500 µL Komplett-Medium versetzt. Die DRGs werden jeweils mehrfach suspendiert, mittels einer Spritze durch Kanülen Nr. 1, Nr. 12 und Nr. 16 gezogen und in 50 mL Falcon-Röhrchen überführt und dieses mit Komplett-Medium auf 15 mL aufgefüllt. Der Inhalt jedes Falcon-Röhrchen wird jeweils durch einen 70 µm Falcon-Filtereinsatz filtriert und 10 Minuten bei 1200 Umdrehungen und Raumtemperatur zentrifugiert. Das resultierende Pellet wird jeweils in 250 µL Komplett-Medium aufgenommen und die Zellzahl ermittelt.

Die Anzahl der Zellen in der Suspension wird auf 3 mal 10⁵ pro mL eingestellt und jeweils 150 µL dieser Suspension in eine Vertiefung der wie vorstehend beschrieben beschichteten Zellkultur-Platten gegeben. Im Brutschrank werden die Platten bei 37 °C, 5 Vol-% CO₂ und 95 % relativer Luftfeuchtigkeit zwei bis drei Tage stehen gelassen.

Anschließend werden die Zellen mit 2 µM Fluo-4 und 0,01 Vol-% Pluronic F127 (Molecular Probes Europe BV, Leiden Niederlande) in HBSS-Puffer (Hank's buffered saline solution, Gibco Invitrogen GmbH, Karlsruhe, Deutschland) für 30 min bei 37 °C beladen, 3 x mit HBSS-Puffer gewaschen und nach einer weiteren Inkubation von 15 Minuten bei Raumtemperatur zur Ca²⁺-Messung im FLIPR-Assay eingesetzt. Die Ca²⁺-abhängige Fluoreszenz wird dabei vor und nach Zugabe von Substanzen gemessen (λex = 488 nm, λem = 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Verbindungen (10 µM) auf die Zellen pipettiert und der Ca²⁺-Einstrom mit der Kontrolle (Capsaicin 10 µM) verglichen. Daraus ergibt sich die Angabe in % Aktivierung bezogen auf das Ca²⁺-Signal nach Zugabe von 10 µM Capsaicin (CP). Nach 5 Minuten Inkubation werden 100 nM Capsaicin appliziert und ebenfalls der Einstrom von Ca²⁺ ermittelt.

Desensitisierende Agonisten und Antagonisten führen zu einer Unterdrückung des Ca²⁺-Einstroms. Es werden % Inhibierung im Vergleich zu der maximal erreichbaren Inhibierung mit 10 µM Capsaicin berechnet.

Es werden Dreifach-Bestimmungen (n=3) durchgeführt und diese in mindestens 3 unabhängigen Experimenten wiederholt (N=4).

### II. Funktionelle Untersuchungen am Vanilloid Rezeptor (VR1)

Die agonistische bzw. antagonistische Wirkung der zu untersuchenden Substanzen auf Vanilloid Rezeptor (VR1) kann auch mit dem folgenden Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes, Europe BV, Leiden, Niederlande) im Fluorescent Imaging Plate Reader (FLIPR, Molecular Devices, Sunnyvale, USA) quantifiziert.

### Methode:

Chinese Hamster Ovary Zellen (CHO K1-Zellen, European Collection of Cell Cultures (ECACC) Großbritannien) werden stabil mit dem VR1-Gen transfiziert. Für funktionelle Untersuchungen werden diese Zellen auf Poly-D-Lysin-beschichtete, schwarze 96-Loch-Platten mit klarem Boden (BD Biosciences, Heidelberg, Deutschland) in einer Dichte von 25.000 Zellen/Loch ausplattiert. Über Nacht werden die Zellen bei 37 °C und 5 % CO₂ in einem Kulturmedium (Nutrient Mixture Ham's F12, 10 Vol-% FCS (Fetal calf serum), 18 µg/ml L-Prolin) inkubiert. Am folgenden Tag werden die Zellen mit Fluo-4 (Fluo-4 2 µM, Pluronic F127 0,01 Vol-%, Molecular Probes in HBSS (Hank's buffered saline solution), Gibco Invitrogen GmbH, Karlsruhe, Deutschland) für 30 Minuten bei 37 °C inkubiert. Anschließend werden die Platten 3 mal mit HBSS-Puffer gewaschen und nach einer weiteren Inkubation von 15 Minuten bei Raumtemperatur zur Ca²⁺⁻-Messung im FLIPR eingesetzt. Die Ca²⁺-abhängige Fluoreszenz wird dabei vor und nach Zugabe der zu untersuchenden Substanzen gemessen (Wellenlänge λₑₓ=488 nm, λₑₘ= 540 nm). Die Quantifizierung erfolgt durch die Messung der höchsten Fluoreszenzintensität (FC, Fluorescence Counts) über die Zeit.

### FLIPR-Assay:

Das FLIPR-Protokoll besteht aus 2 Substanzzugaben. Zunächst werden die zu testenden Substanzen (10µM) auf die Zellen pipettiert und der Ca²⁺-Einstrom mit der Kontrolle (Capsaicin 10 µM) verglichen (% Aktivierung bezogen auf das Ca²⁺⁻-Signal nach Zugabe von 10 µM Capsaicin). Nach 5 Minuten Inkubation werden 100 nM Capsaicin appliziert und ebenfalls der Einstrom von Ca²⁺ ermittelt.

### III. Formalin-Test an der Maus

Die Untersuchung zur Bestimmung der antinociceptiven Wirkung der erfindungsgemäßen 2,5-disubstituierten Thiazo-4-on-Derivate wird im Formalin-Test an männlichen Mäusen (NMRI, 20 bis 30 g Körpergewicht, Iffa, Credo, Belgien) durchgeführt.

Im Formalin-Test werden gemäß D. Dubuisson et al., Pain 1977, 4, 161-174 die erste (frühe) Phase (0 bis 15 Minuten nach der Formalin-Injektion) und die zweite (späte) Phase (15 bis 60 Minuten nach der Formalin-Injektion) unterschieden. Die frühe Phase stellt als direkte Reaktion auf die Formalin-Injektion ein Modell für Akutschmerz dar, während die späte Phase als Modell für persistierenden (chronischen) Schmerz angesehen wird (T. J. Coderre et al., Pain 1993, 52, 259-285). Die entsprechenden Literaturbeschreibungen werden hiermit als Referenz eingeführt und gelten als Teil der Offenbarung.

Die erfindungsgemäßen 2,5-disubstituierten Thiazol-4-on-Derivate werden in der zweiten Phase des Formalin-Tests untersucht, um Aussagen über Substanzwirkungen auf chronisch/entzündlichen Schmerz zu erhalten.

In Abhängigkeit von der Applikationsart der erfindungsgemäßen Verbindungen wird der Applikationszeitpunkt der erfindungsgemäßen 2,5-disubstituierten Thiazol-4-on-Derivate vor der Formalin-Injektion gewählt. Die intravenöse Applikation von 10 mg/kg Körpergewicht der Testsubstanzen erfolgt 5 Minuten vor der Formalin-Injektion. Diese erfolgt durch eine einmalige subkutane Formalin-Injektion (20 µL, 1 %-ige wäßrige Lösung) in die dorsale Seite der rechten Hinterpfote, so dass bei freibeweglichen Versuchstieren eine nociceptive Reaktion induziert wird, die sich in deutlichem Lecken und Beißen der betreffenden Pfote äußert.

Anschließend wird für einen Untersuchungszeitraum von drei Minuten in der zweiten (späten) Phase des Formalin-Tests (21 bis 24 Minuten nach der Formalin-Injektion) das nociceptive Verhalten durch Beobachtung der Tiere kontinuierlich erfasst. Die Quantifizierung des Schmerzverhaltens erfolgt durch Summation der Sekunden, in denen die Tiere in dem Untersuchungszeitraum ein Lecken und Beißen der betroffenen Pfote zeigen.

Der Vergleich erfolgt jeweils mit Kontrolltieren, die anstelle der erfindungsgemäßen Verbindungen Vehikel (0.9%-ige wäßrige Natriumchlorid-Lösung) vor Formalinapplikation erhalten. Basierend auf der Quantifizierung des Schmerzverhaltens wird die Substanzwirkung im Formalin-Test als Änderung gegen die entsprechende Kontrolle in Prozent ermittelt.

Nach Injektion von Substanzen, die im Formalin-Test antinociceptiv wirksam sind, werden die beschriebenen Verhaltensweisen der Tiere, d. h. Lecken und Beißen, reduziert bzw. aufgehoben.

### IV. Untersuchung auf analgetische Wirksamkeit im Writhing-Test

Die Untersuchung der erfindungsgemäßen Verbindungen der allgemeinen Formel I auf analgetische Wirksamkeit wurde im Phenylchinon-induzierten Writhing an der Maus, modifiziert nach I.C. Hendershot und J. Forsaith (1959) J. Pharmacol. Exp. Ther. 125, 237-240 durchgeführt. Die entsprechende Literaturbeschreibung wird hiermit als Referenz eingeführt und gilt als Teil der Offenbarung.

Dazu wurden männliche NMRI-Mäuse mit einem Gewicht von 25 bis 30 g verwendet. Gruppen von 10 Tieren pro Verbindungsdosis erhielten 10 Minuten nach intravenöser Gabe der zu untersuchenden Verbindungen 0,3 ml/Maus einer 0,02%igen wäßrigen Lösung von Phenylchinon (Phenylbenzochinon, Fa. Sigma, Deisenhofen, Deutschland; Herstellung der Lösung unter Zusatz von 5 Gew.-% Ethanol und Aufbewahrung im Wasserbad bei 45°C) intraperitoneal appliziert. Die Tiere wurden einzeln in Beobachtungskäfige gesetzt. Mit Hilfe eines Drucktastenzählers wurde die Anzahl der schmerzinduzierten Streckbewegungen (sogenannte Writhingreaktionen = Durchdrücken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten hatten. Alle Verbindungen wurden in der Standarddosierung von 10 mg/kg getestet.

Im Folgenden wird die Erfindung mit Hilfe einiger Beispiele erläutert. Diese Erläuterungen sind lediglich beispielhaft und schränken den allgemeinen Erfindungsgedanken nicht ein.

### Beispiele:

Die Ausbeuten der hergestellten Verbindungen sind nicht optimiert.

Alle Temperaturen sind unkorrigiert.

### Abkürzungen:

| | |
|---|---|
| aq. | wässrig |
| DCM | Dichlormethan |
| DMF | Dimethylformamid |
| EtOAc | Ethylacetat |
| EtOH | Ethanol |
| ges. | gesättigt |
| h | Stunde |
| HOBT | 1-Hydroxybenzotriazol |
| MeOH | Methanol |
| RT | Raumtemperatur |
| TBTU | O-(Benzotriazol-1-yl)-N,N,N',N'-tetramethyluroniom tetrafluoroborat |

Die eingesetzten Chemikalien und Lösungsmittel wurden kommerziell bei den herkömmlichen Anbietern (Acros, Avocado, Aldrich, Bachem, Fluka, Lancaster, Maybridge, Merck, Sigma, TCI, etc.) bezogen oder nach den für den Fachmann bekannten Methoden synthetisiert.

Als stationäre Phase für die Säulenchromathographie wurde Kieselgel 60 (0.040 - 0.063 mm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt. Die Mischungsverhältnisse von Lösungsmitteln, Laufmitteln oder für chromatographische Untersuchungen sind stets in Volumen/Volumen angegeben.
Die Analytik erfolgte durch Massenspektroskopie und NMR.

### Synthese von 2-Phenyl-substituierten Thiazol-4-on-Derivaten

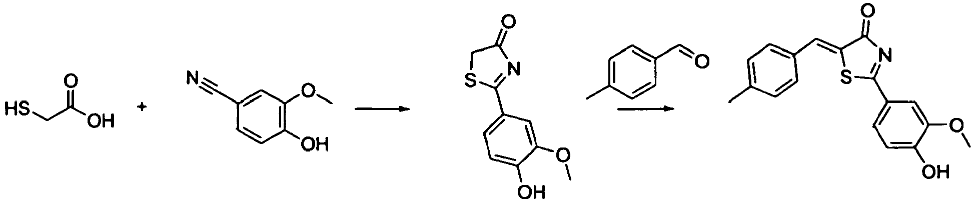

### 1a. Synthese von 2-(4-Hydroxy-3-methoxy-phenyl)-thiazol-4-on

4-Hydroxy-3-methoxy-benzonitril (25 g, 167.7 mmol) wurde in EtOH (355 ml) gelöst und mit Thioglykolsäure (18.8 g, 167.7 mmol) und Triethylamin (12.2 g, 120.4 mmol) versetzt. Das Lösungsmittel wurde im Vakuum bis auf 150 ml reduziert und der ausgefallene Niederschlag wurde abgesaugt, mit wenig EtOH und Diisopropylether gewaschen und getrocknet.
Die Mutterlauge wurde weiter bis auf 75 ml reduziert und abgekühlt.
Der erneut ausgefallene Niederschlag wurde abgesaugt, mit wenig EtOH und Diisopropylether gewaschen und getrocknet.
Die kombinierten Niederschläge wurden erneut in EtOH (50 ml) zum Rückfluß erhitzt.
Der ausgefallene Niederschlag wurde abgesaugt, mit wenig EtOH und Diisopropylether gewaschen und getrocknet. Das gewünschte Produkt wurde in einer Ausbeute von 37 % der Theorie (13.9 g) erhalten.

### 1b. Synthese von Beispielverbindung 262:

### 2-(4-Hydroxy-3-methoxy-phenyl)-5-(4-methyl-benzyliden)-thiazol-4-on

2-(4-Hydroxy-3-methoxy-phenyl)-thiazol-4-on (1.34 mmol, 300 mg) und 4-Methylbenzaldehyd (194 mg, 1.61 mmol) wurden in Essigsäure (7 g, 117.5 mmol) gelöst und mit Natriumacetat (408 mg, 4.972 mmol) versetzt. Die Reaktionsmischung wurde über Nacht zum Rückfluß erhitzt, mit Wasser versetzt und der entstandene Niederschlag wurde mit Butanol und Diisopropylether gewaschen und getrocknet. Das gewünschte Produkt wurde als Feststoff (323 mg, 74 % der Theorie) erhalten.

### 2. Synthese von 2-Morpholinyl-substituierten Thiazol-4-on-Derivaten

### 2a. Synthese von 2-Morpholin-4-yl-thiazol-4-on

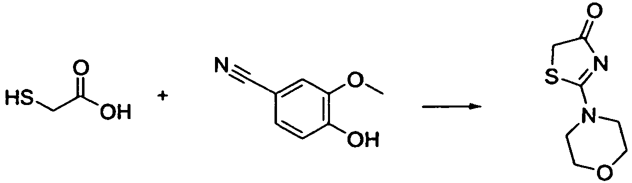

Cyanomorpholin (15.3 g, 136.3 mmol) und Thioglykolsäure (12.5 g, 136.3 mmol) wurden mit Pyridin (12.2 ml) in ein Reaktionsgefäß gegeben. Das Reaktionsgefäß wurde in einem Pyrrexrohr auf 100 °C erhitzt. Nach einer Stunde wurde die Reaktion beendet und die Mischung abgekühlt.
Der entstandene Niederschlag wurde abgesaugt, mit wenig EtOH und Diisopropylether gewaschen und getrocknet. Das gewünschte Produkt wurde als weißer Feststoff (21.5 g, 85 % der Theorie) erhalten.

### 2b. Synthese von Beispielverbindung 58:

### 5-Decyliden-2-morpholin-4-yl-thiazol-4-on

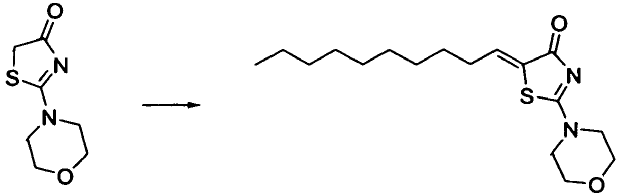

2-Morpholin-4-yl-thiazol-4-on (500 mg, 2.7 mmol) und n-Decanal (503 mg, 3.22 mol) wurden in EtOH (10 ml) gelöst und mit Triethylamin (815 mg) versetzt. Die Reaktionsmischung wurde 48 h zum Rückfluß erhitzt. Der ausgefallene Niederschlag wurde abgesaugt und getrocknet. Zur weiteren Aufreinigung wurde der Niederschlag in wenig Butanol (3 ml) erhitzt. Der wiederum ausgefallene Niederschlag wurde abgesaugt und getrocknet. Das gewünschte Produkt wurde als Feststoff (82 mg, 10 % der Theorie) erhalten.

### 2c. Synthese von Beispielverbindung 17:

### 2-Morpholin-4-yl-5-(4-phenoxy-benzyliden)-thiazol-4-on

2-Morpholin-4-yl-thiazol-4-on (1 g, 5.4 mmol) und 3-Phenoxybenzaldehyd (1.27 g, 6.43 mmol) wurden in Essigsäure (28 g) gelöst und mit Natriumacetat (1.63 g, 19.9 mmol) versetzt. Die Reaktionsmischung wurde über Nacht zum Rückfluß erhitzt, 24 h bei RT gerührt und der entstandene Niederschlag wurde mit Butanol und Diisopropylether gewaschen und getrocknet. Das gewünschte Produkt wurde als Feststoff (1.37 g, 70 % der Theorie) erhalten.

### 2d. Synthese von Beispielverbindung 19:

### 2-Morpholin-4-yl-5-(3-p-tolyl-allyliden)-thiazol-4-on

2-Morpholin-4-yl-thiazol-4-on (530 mg, 2.85 mmol) und 3-p-Tolyl-propenal (500 mg, 3.42 mmol) wurden in Essigsäure (15 g) gelöst und mit Natriumacetat (865 mg, 10.5 mmol) versetzt. Die Reaktionsmischung wurde 5 h zum Rückfluß erhitzt und 48 h bei RT gerührt. Das Lösungsmittel wurde im Vakuum entfernt und der Rückstand in Wasser und EtOAc aufgenommen. Die organische Phase wurde über Magnesiumsulfat getrocknet, das Lösungsmittel im Vakuum entfernt und der Rückstand getrocknet. Der Rückstand wurde in Butanol (20 ml) erhitzt und der ausgefallene Niederschlag wurde mit wenig Diisopropylether gewaschen und getrocknet. Das gewünschte Produkt wurde als Feststoff (224 mg, 21 % der Theorie) erhalten.

### 2e. Synthese von Beispielverbindung 50:

### 5-(2,3-Diphenyl-allyliden)-2-morpholin-4-yl-thiazol-4-on

2-Morpholin-4-yl-thiazol-4-on (500 mg, 2.7 mmol) und Diphenylacrolein (670 mg, 3.22 mol) wurden in EtOH (10 ml) gelöst und mit Triethylamin (815 mg) versetzt. Die Reaktionsmischung wurde 48 h zum Rückfluß erhitzt. Der ausgefallene Niederschlag wurde abgesaugt und getrocknet. Das gewünschte Produkt wurde als Feststoff (862 mg, 85 % der Theorie) erhalten.

### 3. Synthese von 2-Thiomorpholinyl-substituierten Thiazol-4-on-Derivaten

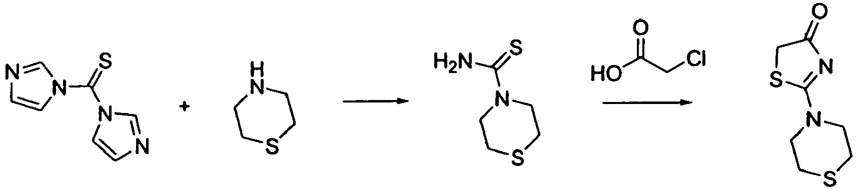

### 3a. Synthese von Thiomorpholin-4-thiocarbonsäureamid

1,1'-Thiocarbonyldiimidazol (45.4 g, 254 mmol) wurde in THF (605 g) suspendiert. Thiomorpholin (25 g, 242.6 mmol) wurde in wenig THF gelöst und zu der Suspension gegeben. Die Reaktionsmischung wurde 1 h bei RT gerührt. Es wurde 7 N Ammoniak-Lösung in MeOH (346 g) dazu gegeben und die Reaktionsmischung über Nacht bei 40 °C gerührt. Das Lösungsmittel wurde im Vakuum entfernt, der Rückstand mit wenig kaltem Butanol und Ether gewaschen und getrocknet. Das gewünschte Produkt wurde in einer Menge von 31.5 g erhalten.

### 3b. Synthese von 2-Thiomorpholin-4-yl-thiazol-4-on

Thiomorpholin-4-thiocarbonsäureamid (38 g, 234 mmol) wurde mit Chloressigsäure (23 g, 243 mmol) in Pyridin (87 g) suspendiert. Die Suspension wurde 45 min in einem Mikrowellengefäß erhitzt und über Nacht stehen gelassen. Der ausgefallene Niederschlag wurde abgesaugt, mit wenig Butanol und Ether gewaschen und getrocknet. Das gewünschte Produkt wurde als Feststoff erhalten (21.7 g, 46 % der Theorie).

### 3c. Synthese von Beispielverbindung 85:

### 5-(2-Chlor-3-phenyl-allyliden)-2-thlomorpholin-4-yl-thiazol-4-on

2-Thiomorpholin-4-yl-thiazol-4-on (800 mg, 3.96 mmol) und alpha-Chlorzimtaldehyd (789 mg, 4.752 mmol) wurden in Essigsäure (21 g) gelöst und mit Natriumacetat (1.2 g, 14.6 mmol) versetzt. Die Reaktionsmischung wurde 4 h zum Rückfluß erhitzt und in Wasser (100 ml) gegeben. Die wässrige Phase wurde mit DCM extrahiert und das Lösungsmittel wurde im Vakuum entfernt. Die wässrige Phase wurde über Nacht stehen gelassen. Es bildete sich eine zweite Phase, die im Vakuum eingeengt wurde. Der Rückstand wurde in Butanol (1 ml) erhitzt und der ausgefallene Niederschlag wurde mit wenig Diisopropylether gewaschen und getrocknet. Das gewünschte Produkt wurde als Feststoff (138 mg, 10 % der Theorie) erhalten.

### 3d. Synthese von Beispielverbindung 86:

### 3-(4-Oxo-2-thiomorpholin-4-yl-4H-thiazol-5-ylidenmethyl)-benzoesäure

2-Thiomorpholin-4-yl-thiazol-4-on (5 g, 25 mmol) und 3-Carboxybenzaldehyd (4.45 g, 29.7 mmol) wurden in Essigsäure (130 g) gelöst und mit Natriumacetat (7.5 g, 91.5 mmol) versetzt. Die Reaktionsmischung wurde über Nacht zum Rückfluß erhitzt. Nach Abkühlen entstand ein Niederschlag, der abgesaugt, mit Wasser, Butanol und Diisopropylether gewaschen und getrocknet wurde. Das gewünschte Produkt wurde als Feststoff (4.91 g, 60 % der Theorie) erhalten.

### 3e. Synthese von 1-(6-Chlor-pyridin-2-yl)-piperazin

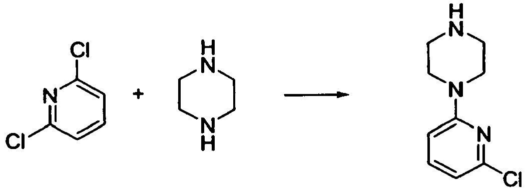

Piperazin (22.4 g, 260.2 mmol) wurde in DMSO (462 g) gelöst und mit 2,6-Dichlorpyridin (35 g, 236 mmol) versetzt. Das Reaktionsgemisch wurde über Nacht bei 110 °C gerührt. Das Reaktionsgemisch wurde portionsweise mit ges. aq. Natriumhydrogencarbonat-Lösung (insgesamt 250 ml) versetzt und der entstandene Niederschlag wurde abgesaugt. Das Filtrat wurde mehrfach mit EtOAc extrahiert und die vereinigten organischen Phasen wurden getrocknet und das Lösungsmittel im Vakuum entfernt. Der Rückstand wurde bei 2,5⁻¹ mbar destilliert. Das Produkt wurde bei 120 °C Kopftemperatur und 165 °C Sumpftemperatur erhalten. Ausbeute: 17,7 g (38 % der Theorie)

### 3f. Synthese von Beispielverbindung 108:

### 5-{3-[4-(6-Chlor-pyridin-2-yl)-piperazin-1-carbonyl]-benzyliden}-2-thiomorpholin-4-yl-thiazol-4-on

3-(4-Oxo-2-thiomorpholin-4-yl-4H-thiazol-5-ylidenmethyl)-benzoesäure (500 mg, 1.5 mmol), 1-(6-Chlor-pyridin-2-yl)-piperazin (295 mg, 1.5 mmol), TBTU (480 mg, 1.5 mmol), HOBT (201 mg, 1.5 mmol) und Diisopropylethylamin (643 mg, 4.5 mmol) wurden in THF (10 ml) gelöst und über Nacht bei RT gerührt. Der entstandene Niederschlag wurde abgesaugt, mit EtOAc und Ether gewaschen und getrocknet. Ausbeute: 579 mg (75 % der Theorie)

### 3g. Synthese von Beispielverbindung 112: 3-(4-Oxo-2-thiomorpholin-4-yl-4H-thiazol-5-ylidenmethyl)-benzoesäure-N'-(2-chlor-4-trifluormethyl-phenyl)-hydrazid

3-(4-Oxo-2-thiomorpholin-4-yl-4H-thiazol-5-ylidenmethyl)-benzoesäure (500 mg, 1.5 mmol), 1-(2-Chlor-4-(trifluormethyl)-phenyl)-hydrazin (315 mg, 1.5 mmol), TBTU (480 mg, 1.5 mmol), HOBT (201 mg, 1.5 mmol) und Diisopropylethylamin (643 mg, 4.5 mmol) wurden in THF (10 ml) gelöst und 48 h bei RT gerührt. Der entstandene Niederschlag wurde abgesaugt, mit EtOAc und Ether gewaschen und getrocknet. Ausbeute: 305 mg (39 % der Theorie)

### 4. Synthese von 2-Piperazinyl-substituierten Thiazol-4-on-Derivaten

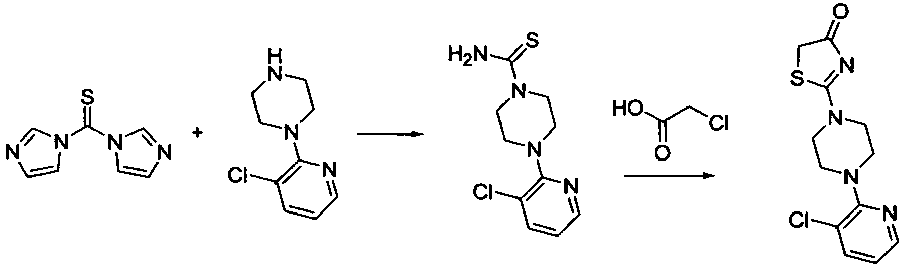

### 4a. Synthese von 1-(3-Chlor-pyridin-2-yl)-piperazin

Piperazin (15 g, 174 mmol) wurde in DMSO (309 g) gelöst und mit 2,3-Dichlorpyridin (23.4 g, 158 mmol) versetzt. Das Reaktionsgemisch wurde 60 h bei 110 °C gerührt. Das Reaktionsgemisch wurde portionsweise mit ges. aq. Natriumhydrogencarbonat-Lösung (insgesamt 250 ml) versetzt und der entstandene Niederschlag wurde abgesaugt. Das Filtrat wurde mehrfach mit EtOAc (jeweils 150 ml) extrahiert und die vereinigten organischen Phasen wurden getrocknet und das Lösungsmittel im Vakuum entfernt. Der Rückstand wurde bei 2,1⁻¹ mbar destilliert. Das Produkt wurde bei 95 °C Kopftemperatur und 155 °C Sumpftemperatur erhalten. Ausbeute: 9.6 g (28 % der Theorie)

### 4b. Synthese von 4-(3-Chlor-pyridin-2-yl)-piperazin-1-thiocarbonsäureamid

1,1'-Thiocarbonyldiimidazol (3.8 g, 21.2 mmol) wurde in THF (23.4 g) suspendiert. 1-(3-Chlor-pyridin-2-yl)-piperazin (4 g, 20.2 mmol) wurde in wenig THF gelöst und zu der Suspension gegeben. Die Reaktionsmischung wurde 1 h bei RT gerührt. Es wurde 7 N Ammoniak-Lösung in MeOH (28.9 g) dazu gegeben und die Reaktionsmischung über Nacht bei 40 °C gerührt. Das Lösungsmittel wurde im Vakuum entfernt, der Rückstand mit wenig kaltem Butanol und Ether gewaschen und getrocknet. Das gewünschte Produkt wurde in einer Menge von 4.1 g (78 % der Theorie) erhalten.

### 4c. Synthese von 2-[4-(3-Chlor-pyridin-2-yl)-piperazin-1-yl]-thiazol-4-on

4-(3-Ch)or-pyridin-2-yl)-piperazin-1-thiocarbonsäureamid (8.1 g, 31.9 mmol) wurde mit Chloressigsäure (3.1 g, 33.1 mmol) in EtOH(7 g) suspendiert. Die Suspension wurde 60 min in einem Mikrowellengefäß auf 85 °C erhitzt und über Nacht stehen gelassen. Der ausgefallene Niederschlag wurde abgesaugt, mit wenig Butanol und Ether gewaschen und getrocknet. Der Niederschlag wurde in wenig Butanol (10 ml) erhitzt und der zurückbleibende Feststoff wurde abgesaugt. Das gewünschte Produkt wurde als Feststoff erhalten (2.34 g, 25 % der Theorie).

### 4d. Synthese von Beispielverbindung 95:

### 2-[4-(3-Chlor-pyridin-2-yl)-piperazin-1-yl]-5-(4-hydroxy-3-methoxy-benzyliden)-thiazol-4-on

2-[4-(3-Chlor-pyridin-2-yl)-piperazin-1-yl]-thiazol-4-on (500 mg, 1.7 mmol) und Vanillin (308 mg, 2.02 mmol) wurden in Essigsäure (9 g) gelöst und mit Natriumacetat (514 mg, 6.3 mmol) versetzt. Die Reaktionsmischung wurde über Nacht zum Rückfluß erhitzt. Nach Abkühlen entstand ein Niederschlag, der abgesaugt, mit Diisopropylether gewaschen und getrocknet wurde. Der Niederschlag wurde in wenig Butanol (8 ml) erhitzt und heiß filtriert. Der Niederschlag wurde verworfen und das Filtrat wurde stehen gelassen. Der entstandene Niederschlag wurde abgesaugt und getrocknet. Das gewünschte Produkt wurde als Feststoff (157 mg, 22 % der Theorie) erhalten.

### 4e. Synthese von Beispielverbindung 49:

### 2-[4-(3-Chlor-pyridin-2-yl)-piperazin-1-yl]-5-naphthalen-2-ylmethylen-thiazol-4-on

2-[4-(3-Chlor-pyridin-2-yl)-piperazin-1-yl]-thiazol-4-on (500 mg, 1.7 mmol) und Naphthalin-2-carbaldehyd (263 mg, 1.7 mmol) wurden in EtOH (10 ml) gelöst und mit Triethylamin (512 mg, 5.1 mmol) versetzt. Die Reaktionsmischung wurde 72 h zum Rückfluß erhitzt. Nach Abkühlen entstand ein Niederschlag, der abgesaugt, mit Butanol und Diisopropylether gewaschen und getrocknet wurde. Das gewünschte Produkt wurde als Feststoff (295 mg, 40 % der Theorie) erhalten.

### 4f. Synthese von Beispielverbindung 90:

### 2-[4-(3-Chlor-pyridin-2-yl)-piperazin-1-yl]-5-chinolin-3-ylmethylen-thiazol-4-on

2-[4-(3-Chlor-pyridin-2-yl)-piperazin-1-yl]-thiazol-4-on (500 mg, 1.7 mmol) und 3-Chinolin-carboxaldehyd (318 mg, 2.02 mmol) wurden in Essigsäure (9 g) gelöst und mit Natriumacetat (514 mg, 6.3 mmol) versetzt. Die Reaktionsmischung wurde über Nacht zum Rückfluß erhitzt. Nach Abkühlen wurde mit Wasser (100 ml) versetzt. Der dabei entstandene Niederschlag wurde abgesaugt und in wenig Butanol (4 ml) erhitzt. Der entstandene Niederschlag wurde abgesaugt, mit wenig Butanol und Ether gewaschen und getrocknet. Das gewünschte Produkt wurde als Feststoff (367 mg, 50 % der Theorie) erhalten.

### 5. Synthese von 2-(4-Benzyl-piperazinyl)-substituierten Thiazol-4-on-Derivaten

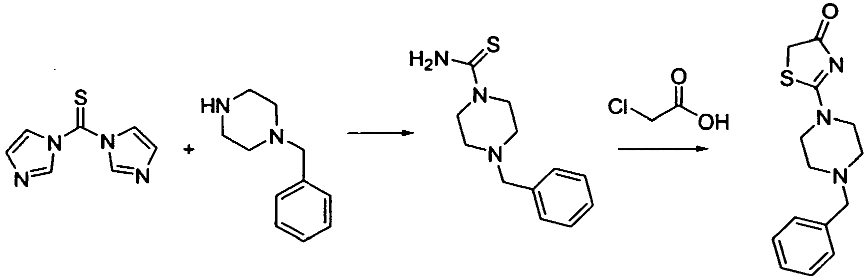

### 5a. Synthese von 4-Benzyl-piperazin-1-thiocarbonsäureamid

1,1'-Thiocarbonyldiimidazol (18 g, 101.2 mmol) wurde in THF (112 g) suspendiert. 1-Benzyl-piperazin (17 g, 96.5 mmol) wurde in wenig THF gelöst und zu der Suspension gegeben. Die Reaktionsmischung wurde 1 h bei RT gerührt. Es wurde 7 N Ammoniak-Lösung in MeOH (137 g) dazu gegeben und die Reaktionsmischung über Nacht bei 40 °C gerührt. Das Lösungsmittel wurde im Vakuum entfernt, der Rückstand in wenig Butanol (25 ml) erhitzt. Nach dem Abkühlen wurde ein Niederschlag erhalten, der mit wenig kaltem Butanol und Ether gewaschen und anschließend getrocknet wurde. Das gewünschte Produkt wurde in einer Menge von 14.1 g erhalten.

### 5b. Synthese von 2-(4-Benzyl-piperazin-1-yl)-thiazol-4-on

4-Benzyl-piperazin-1-thiocarbonsäureamid (14 g, 59.5 mmol) wurde mit Chloressigsäure (5.8 g, 61.9 mmol) in Pyridin (55 g) suspendiert. Die Suspension wurde 45 min in einem Mikrowellengefäß auf 120 °C erhitzt. Die Reaktionsmischung wurde im Vakuum vollständig eingeengt und der Rückstand in MeOH (150 ml) erhitzt. Der ausgefallene Niederschlag wurde abgesaugt, mit Ether gewaschen und getrocknet. Das gewünschte Produkt wurde als Feststoff erhalten (8.9 g, 55 % der Theorie).

### 5c. Synthese von Beispielverbindung 104:

### 2-(4-Benzyl-piperazin-1-yl)-5-(4-tert-butyl-benzyliden)-thiazol-4-on

2-(4-Benzyl-piperazin-1-yl)-thiazol-4-on (500 mg, 1.8 mmol) und 4-tert-Butylbenzaldehyd (353 mg, 2.18 mmol) wurden in Essigsäure (9.5 g) gelöst und mit Natriumacetat (552 mg, 6.7 mmol) versetzt. Die Reaktionsmischung wurde über Nacht zum Rückfluß erhitzt. Nach Abkühlen wurde mit Wasser (50 ml) versetzt. Der dabei entstandene Niederschlag wurde abgesaugt und in wenig Butanol (1 ml) erhitzt. Der entstandene Niederschlag wurde abgesaugt, mit wenig Butanol und Ether gewaschen und getrocknet. Das gewünschte Produkt wurde als Feststoff (245 mg, 32 % der Theorie) erhalten.

### 5d. Synthese von Beispielverbindung 158:

### 2-(4-Benzyl-piperazin-1-yl)-5-(4-pentafluorsulfanyl-benzyliden)-thiazol-4-on

2-(4-Benzyl-piperazin-1-yl)-thiazol-4-on (350 mg, 1.3 mmol) und 4-(Pentafluorsulfanyl)-benzaldehyd (353 mg, 1.52 mmol) wurden in Essigsäure (6.7 g) gelöst und mit Natriumacetat (387 mg, 4.7 mmol) versetzt. Die Reaktionsmischung wurde über Nacht zum Rückfluß erhitzt. Der entstandene Niederschlag wurde abgesaugt, mit wenig Butanol und Ether gewaschen und getrocknet. Das gewünschte Produkt wurde als Feststoff (108 mg) erhalten.

### 6. Synthese von 2-Amino-Phenyl-substituierten Thiazol-4-on-Derivaten

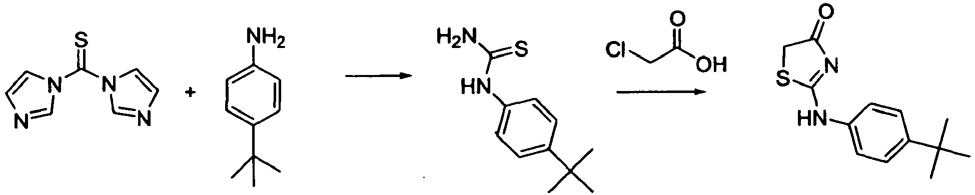

### 6a. Synthese von (4-tert-Butyl-phenyl)-thioharnstoff

1,1'-Thiocarbonyldiimidazol (12.5 g, 70.4 mmol) wurde in THF (167 g) suspendiert. 4-tert-Butylanilin (10 g, 67 mmol) wurde in wenig THF gelöst und zu der Suspension gegeben. Die Reaktionsmischung wurde 1 h bei RT gerührt. Es wurde 7 N Ammoniak-Lösung in MeOH (95 g) dazu gegeben und die Reaktionsmischung 48 h bei 40 °C gerührt. Das Lösungsmittel wurde im Vakuum entfernt und der Rückstand in wenig Butanol (25 ml) erhitzt. Nach dem Abkühlen wurde ein Niederschlag erhalten, der mit wenig kaltem Butanol und Ether gewaschen und anschließend getrocknet wurde. Das gewünschte Produkt wurde in einer Menge von 14 g erhalten.

### 6b. Synthese von 2-(4-tert-Butyl-phenylamino)-thiazol-4-on

(4-tert-Butyl-phenyl)-thioharnstoff (14 g, 68.7 mmol) wurde mit Chloressigsäure (6.7 g, 71.49 mmol) in EtOH (15 g) suspendiert. Die Suspension wurde 60 min in einem Mikrowellengefäß auf 85 °C erhitzt. Der ausgefallene Niederschlag wurde abgesaugt, mit wenig Butanol und Diisopropylether gewaschen und getrocknet. Das gewünschte Produkt wurde als Feststoff erhalten (4.9 g).

### 6c. Synthese von Beispielverbindung 263:

### 2-(4-tert-Butyl-phenyl-amino)-5-(4-methyl-benzyliden)-thiazol-4-on

2-(4-tert-Butyl-phenylamino)-thiazol-4-on (550 mg, 2.2 mmol) und 4-Methylbenzaldehyd (319 mg, 1.02 mmol) wurden in Essigsäure (11.6 g) gelöst und mit Natriumacetat (673 mg, 8.2 mmol) versetzt. Die Reaktionsmischung wurde 5 h zum Rückfluß erhitzt und 72 h bei RT gerührt. Der entstandene Niederschlag wurde abgesaugt, mit wenig Wasser und Hexan gewaschen und getrocknet. Das gewünschte Produkt wurde als Feststoff (525 mg, 67 % der Theorie) erhalten.

### 7. Synthese von 2-Amino-Benzyl-substituierten Thiazol-4-on-Derivaten

### 7a. Synthese von Benzo[1.3]dioxol-5-ylmethyl-thioharnstoff

1,1'-Thiocarbonyldiimidazol (12.3 g, 69.5 mmol) wurde in THF (167 g) suspendiert. 3,4-Methylendioxybenzylamin (10 g, 66.2 mmol) wurde in wenig THF gelöst und zu der Suspension gegeben. Die Reaktionsmischung wurde 1 h bei RT gerührt. Es wurde 7 N Ammoniak-Lösung in MeOH (94.5 g) dazu gegeben und die Reaktionsmischung 24 h bei 40 °C gerührt. Das Lösungsmittel wurde im Vakuum entfernt und der Rückstand in wenig Butanol (15 ml) erhitzt. Nach dem Abkühlen wurde ein Niederschlag erhalten, der mit wenig kaltem Butanol und Ether gewaschen und anschließend getrocknet wurde. Das gewünschte Produkt wurde in einer Menge von 11.1 g (80 % der Theorie) erhalten.

### 7b. Synthese von 2-[(Benzo[1,3]dioxol-5-ylmethyl)-amino]-thiazol-4-on

Benzo[1.3]dioxol-5-ylmethyl-thioharnstoff (11 g, 52.4 mmol) wurde mit Chloressigsäure (5.1 g, 54.4 mol) in Pyridin (20 g) suspendiert. Die Suspension wurde 60 min in einem Mikrowellengefäß auf 110 °C erhitzt. Die Reaktionsmischung wurde im Vakuum eingeengt und der Rückstand mit wenig Butanol (15 ml) in der Hitze gelöst. Nach dem Abkühlen entstand ein Niederschlag, der abgesaugt, mit wenig Butanol und Diisopropylether gewaschen und getrocknet wurde. Das gewünschte Produkt wurde als Feststoff erhalten (4.0 g, 30 % der Theorie).

### 7c. Synthese von Beispielverbindung 243:

### 2-[(Benzo[1,3]dioxol-5-ylmethyl)-amino]-5-(4-trifluormethoxy-benzyliden)-thiazol-4-on

2-[(Benzo[1,3]dioxol-5-ylmethyl)-amino]-thiazol-4-on (350 mg, 1.4 mmol) und 4-(Trifluormethoxy)-benzaldehyd (319 mg, 1.68 mmol) wurden in Essigsäure (7.3 g) gelöst und mit Natriumacetat (425 mg, 5.2 mmol) versetzt. Die Reaktionsmischung wurde 14 h zum Rückfluß erhitzt. Die Reaktionsmischung wurde mit Wasser versetzt (30 ml), der entstandene Niederschlag abgesaugt, mit wenig Wasser und Hexan gewaschen und getrocknet. Das gewünschte Produkt wurde als Feststoff (460 mg, 78 % der Theorie) erhalten.

### 8. Synthese von 2-Harnstoff-substituierten Thiazol-4-on-Derivaten

### 8a. Synthese von 2-Amino-thiazol-4-on

Thioharnstoff (30 g, 395 mmol) wurde mit Chloressigsäure (38.7 g, 410 mol) in Pyridin (146 g) suspendiert. Die Suspension wurde 45 min in einem Mikrowellengefäß erhitzt. Die Reaktionsmischung wurde im Vakuum eingeengt und der Rückstand in EtOH (250 ml) in der Hitze gelöst. Nach dem Abkühlen entstand ein Niederschlag, der abgesaugt, mit Ether gewaschen und getrocknet wurde. Das gewünschte Produkt wurde als Feststoff erhalten (26.9 g).

### 8b. Synthese von 2-Amino-5-(4-trifluormethyl-benzyliden)-4-thiazol-4-on

2-Amino-thiazol-4-on (6 g, 51.7 mmol) und 4-(Trifluormethyl)-benzaldehyd (10.8 g, 62 mmol) wurden in Essigsäure (271 g) gelöst und mit Natriumacetat (15.7 g, 191 mmol) versetzt. Die Reaktionsmischung wurde 5 h zum Rückfluß erhitzt. Die Reaktionsmischung wurde mit Wasser versetzt (30 ml), der entstandene Niederschlag abgesaugt, mit EtOH und Ether gewaschen und getrocknet. Das gewünschte Produkt wurde als Feststoff (8.8 g, 63 % der Theorie) erhalten.

### 8c. Synthese von Beispielverbindung 106:

### 1-[4-Oxo-5-(4-trifluormethyl-benzyliden)-4,5-dihydro-thiazol-2-yl]-3-(4-trifluormethyl-phenyl)-harnstoff

2-Amino-5-(4-trifluormethyl-benzyliden)-4-thiazol-4-on (436 mg, 1.6 mmol) wurde in DMSO (28 g) gelöst und mit Triethylamin (179 mg 1.76 mmol) und 1-Isocyanato-4-trifluormethyl-benzol (436 mg, 1.6 mmol) versetzt. Das Reaktionsgemisch wurde über Nacht bei RT gerührt und in Eiswasser (50 ml) gegeben. Der entstandene Niederschlag wurde abgesaugt und in wenig Aceton bis zum Rückfluß erhitzt. Es wurde wenig Wasser hinzu gegeben und die Reaktionsmischung stehen gelassen. Der entstandene Niederschlag wurde abgesaugt, mit Wasser gewaschen und getrocknet. Das gewünschte Produkt wurde als weißer Feststoff erhalten (553 mg, 75 % der Theorie).

Die folgenden erfindungsgemäßen 2,5-disubstituierten Thiazol-4-on-Derivate wurden wie unter 1. bis 8. beschrieben hergestellt.

| | |
|---|---|
| 1 | 5-[3-(4-Methoxy-phenyl)-but-2-enyliden]-2-morpholin-4-yl-thiazol-4-on |
| 2 | 2-Morpholin-4-yl-5-phenethyliden-thiazol-4-on Hydrochlorid |
| 3 | 2-Morpholin-4-yl-5-(2-o-tolyl-ethyliden)-thiazol-4-on Hydrochlorid |
| 4 | 2-Morpholin-4-yl-5-(3-phenyl-allyliden)-thiazol-4-on |
| 5 | 2-Morpholin-4-yl-5-(3-phenyl-butyliden)-thiazol-4-on |
| 6 | 2-Morpholin-4-yl-5-(3-phenyl-propyliden)-thiazol-4-on |
| 7 | 5-[3-(4-Fluor-phenyl)-allyliden]-2-morpholin-4-yl-thiazol-4-on |
| 8 | N-[4-(2-Morpholin-4-yl-4-oxo-4H-thiazol-5-ylidenmethyl)-phenyl]-acetamid |
| 9 | 5-Benzo[1,3]dioxol-5-ylmethylen-2-morpholin-4-yl-thiazol-4-on |
| 10 | 2-Morpholin-4-yl-5-(3-m-tolyl-allyliden)-thiazol-4-on |
| 11 | 5-(3-Iodo-4,5-dimethoxy-benzyliden)-2-morpholin-4-yl-thiazol-4-on |
| 12 | 2-Morpholin-4-yl-5-(3-phenylsulfanyl-allyliden)-thiazol-4-on |
| 13 | 5-(3-Benzyloxy-4-methoxy-benzyliden)-2-morpholin-4-yl-thiazol-4-on |
| 14 | 5-(4-Methyl-benzyliden)-2-thiomorpholin-4-yl-thiazol-4-on Hydrobromid |
| 15 | N-[4-(2-Morpholin-4-yl-4-oxo-4H-thiazol-5-ylidenmethyl)-phenyl]-amin |
| 16 | 5-(4-Butoxy-benzyliden)-2-morpholin-4-yl-thiazol-4-on |
| 17 | 2-Morpholin-4-yl-5-(4-phenoxy-benzyliden)-thiazol-4-on |
| 18 | 2-Morpholin-4-yl-5-(3-phenoxy-benzyliden)-thiazol-4-on |
| 19 | 2-Morpholin-4-yl-5-(3-p-tolyl-allyliden)-thiazol-4-on |
| 20 | 5-(3-Benzyloxy-benzyliden)-2-morpholin-4-yl-thiazol-4-on |
| 21 | 5-(4-Benzyloxy-benzyliden)-2-morpholin-4-yl-thiazol-4-on |
| 22 | 2-Thiomorpholin-4-yl-5-(2-trifluormethyl-benzyliden)-thiazol-4-on |
| 23 | 5-(4-Brom-benzyliden)-2-thiomorpholin-4-yl-thiazol-4-on |
| 24 | 2-Thiomorpholin-4-yl-5-(4-trifluormethoxy-benzyliden)-thiazol-4-on |
| 25 | 5-(3-Phenyl-allyliden)-2-thiomorpholin-4-yl-thiazol-4-on |
| 26 | 2-Morpholin-4-yl-5-octyliden-thiazol-4-on |
| 27 | 5-(4-Benzyloxy-3-methoxy-benzylidenl-2-morpholin-4-yl-thiazol-4-on |
| 28 | 5-(3,4-Bis-benzyloxy-benzyliden)-2-morpholin-4-yl-thiazol-4-on |
| 29 | 5-Butyliden-2-morpholin-4-yl-thiazol-4-on |
| 30 | 2-[4-(3-Chlor-pyridin-2-yl)-piperazin-1-yl]-5-(4-methyl-benzyliden)-thiazol-4-on |
| 31 | 2-[4-(3-Chlor-pyridin-2-yl)-piperazin-1-yl]-5-(3-phenyl-allyliden)-thiazol-4-on |
| 32 | 5-Hexyliden-2-morpholin-4-yl-thiazol-4-on |
| 33 | 2-Morpholin-4-yl-5-(3-p-tolyl-but-2-enyliden)-thiazol-4-on |
| 34 | 5-[3-(4-tert-Butyl-phenyl)-but-2-enyliden]-2-morpholin-4-yl-thiazol-4-on |
| 35 | 5-(4-Methoxy-naphthalen-1-ylmethylen)-2-morpholin-4-yl-thiazol-4-on |
| 36 | 2-Morpholin-4-yl-5-(4-trifluormethoxy-benzyliden)-thiazol-4-on |
| 37 | 2-[4-(3-Methoxy-phenyl)-piperazin-1-yl]-5-(3-phenyl-allyliden)-thiazol-4-on |
| 38 | 5-[3-(4-tert-Butyl-phenyl)-allyliden]-2-[4-(3-methoxy-phenyl)-piperazin-1-yl]-thiazol-4-on |
| 39 | 5-(4-Hydroxy-3-methoxy-benzyliden)-2-thiomorpholin-4-yl-thiazol-4-on |
| 40 | 5-[3-(4-Hydroxy-3-methoxy-phenyl)-allyliden]-2-thiomorpholin-4-yl-thiazol-4-on |
| 41 | 5-Naphthalen-1-ylmethylen-2-thiomorpholin-4-yl-thiazol-4-on |
| 42 | 5-Benzyliden-2-thiomorpholin-4-yl-thiazol-4-on |
| 43 | 2-Morpholin-4-yl-5-[3-(4-trifluormethyl-phenyl)-but-2-enyliden]-thiazol-4-on |
| 44 | 2-Morpholin-4-yl-5-[3-(3-trifluormethyl-phenyl)-but-2-enyliden]-thiazol-4-on |
| 45 | 5-(3-Methyl-butyliden)-2-morpholin-4-yl-thiazol-4-on |
| 46 | 2-[4-(3-Chlor-pyridin-2-yl)-piperazin-1-yl]-5-(2-trifluormethyl-benzyliden)-thiazol-4-on |
| 47 | 5-(4-Brom-benzyliden)-2-[4-(3-chlor-pyridin-2-yl)-piperazin-1-yl]-thiazol-4-on |
| 48 | 5-Benzyliden-2-[4-(3-chlor-pyridin-2-yl)-piperazin-1-yl]-thiazol-4-on |
| 49 | 2-[4-(3-Chlor-pyridin-2-yl)-piperazin-1-yl]-5-naphthalen-2-ylmethylen-thiazol-4-on |
| 50 | 5-(2,3-Diphenyl-allyliden)-2-morpholin-4-yl-thiazol-4-on |
| 51 | 2-Morpholin-4-yl-5-phenanthren-9-ylmethylen-thiazol-4-on |
| 52 | 2-Morpholin-4-yl-5-chinolin-3-ylmethylen-thiazol-4-on |
| 53 | 2-(4-Ethyl-piperazin-1-yl)-5-(4-methyl-benzyliden)-thiazol-4-on Hydrobromid |
| 54 | 5-Naphthalen-2-ylmethylen-2-thiomorpholin-4-yl-thiazol-4-on |
| 55 | 2-[4-(4-Methoxy-phenyl)-piperazin-1-yl]-5-(3-phenyl-allyliden)-thiazol-4-on |
| 56 | 2-[4-(2-Chlor-phenyl)-piperazin-1-yl]-5-(3-phenyl-allyliden)-thiazol-4-on |
| 57 | 5-(3-Phenyl-allyliden)-2-(4-phenyl-piperazin-1-yl)-thiazol-4-on |
| 58 | 5-Decyliden-2-morpholin-4-yl-thiazol-4-on |
| 59 | 5-[3-(4-tert-Butyl-phenyl)-allyliden]-2-morpholin-4-yl-thiazol-4-on |
| 60 | 2-(2-Morpholin-4-yl-4-oxo-4H-thiazol-5-ylidenmethyl)-benzoesäure Triethylammoniumsalz |
| 61 | 5-(2-Brom-3-phenyl-allyliden)-2-[4-(3-chlor-pyridin-2-yl)-piperazin-1-yl]-thiazol-4-on |
| 62 | 5-[3-(4-tert-Butyl-phenyl)-allyliden]-2-[4-(3-chlor-pyridin-2-yl)-piperazin-1-yl]-thiazol-4-on |
| 63 | 2-(4-Benzyl-piperazin-1-yl)-5-(4-methyl-benzylidenythiazol-4-on |
| 64 | 2-(4-Phenyl-piperazin-1-yl)-5-chinolin-3-ylmethylen-thiazol-4-on |
| 65 | 2-[4-(3-Chlor-pyridin-2-yl)-piperazin-1-yl]-5-(2-methyl-3-phenyl-allyliden)-thiazol-4-on |
| 66 | 2-[4-(3-Chlor-pyridin-2-yl)-piperazin-1-yl]-5-naphthalen-1-ylmethylen-thiazol-4-on |
| 67 | 5-(2-Chlor-3-phenyl-allyliden)-2-[4-(3-chlor-pyridin-2-yl)-piperazin-1-yl]-thiazol-4-on |
| 68 | 5-(4-Hydroxy-3-methoxy-benzyliden)-2-(4-phenyl-piperazin-1-yl)-thiazol-4-on |
| 69 | 5-Phenanthren-9-ylmethylen-2-(4-phenyl-piperazin-1-yl)-thiazol-4-on |
| 70 | 5-(6-Methoxy-naphthalen-2-ylmethylen)-2-(4-phenyl-piperazin-1-yl)-thiazol-4-on |
| 71 | 5-Naphthalen-1-ylmethylen-2-(4-phenyl-piperazin-1-yl)-thiazol-4-on |
| 72 | 5-(4-Brom-benzyliden)-2-(4-phenyl-piperazin-1-yl)-thiazol-4-on |
| 73 | 5-(4-Methyl-benzyliden)-2-(4-phenyl-piperazin-1-yl)-thiazol-4-on |
| 74 | 2-(4-Phenyl-piperazin-1-yl)-5-(2-trifluormethyl-benzyliden)-thiazol-4-on |
| 75 | 3-(4-Oxo-2-thiomorpholin-4-yl-4H-thiazol-5-yliden)-1,3-dihydro-indol-2-on |
| 76 | 3-(2-Morpholin-4-yl-4-oxo-4H-thiazol-5-yliden)-1,3-dihydro-indol-2-on |
| 77 | 2-[4-(2-Fluor-phenyl)-piperazin-1-yl]-5-(4-methyl-benzyliden)-thiazol-4-on |
| 78 | 5-(4-tert-Butyl-benzyliden)-2-(4-(2-fluor-phenylypiperazin-1-yl]-thiazol-4-on |
| 79 | 2-[4-(2-Fluor-phenyl)-piperazin-1-yl]-5-(3-phenyl-allyliden)-thiazol-4-on |
| 80 | 5-[3-(4-tert-Butyl-phenyl)-allyliden]-2-[4-(2-fluor-phenyl)-piperazin-1-yl]-thiazol-4-on |
| 81 | 2-[4-(2-Fluor-phenyl)-piperazin-1-yl]-5-(4-trifluormethyl-benzyliden)-thiazol-4-on |
| 82 | 2-[4-(2-Fluor-phenyl)piperazin-1-yl]-5-(4-trifluormethoxy-benzyliden)-thiazol-4-on |
| 83 | 5-(2-Chlor-3-phenyl-allyliden)-2-[4-(2-fluor-phenyl)-piperazin-1-yl]-thiazol-4-on |
| 84 | 2-[4-(2-Fluor-phenyl)-piperazin-1-yl]-5-naphthalen-1-ylmethylen-thiazol-4-on |
| 85 | 5-(2-Chlor-3-phenyl-allyliden)-2-thiomorpholin-4-yl-thiazol-4-on |
| 86 | 3-(4-Oxo-2-thiomorpholin-4-yl-4H-thiazol-5-ylidenmethyl)-benzoesäure |
| 87 | 4-(4-Oxo-2-thiomorpholin-4-yl-4H-thiazol-5-ylidenmethyl)-benzoesäure |
| 88 | 5-{3-[4-(3-Chlor-pyridin-2-yl)-piperazin-1-carbonyl]-benzyliden}-2-thiomorpholin-4-yl-thiazol-4-on |
| 89 | 5-{4-[4-(3-Chlor-pyridin-2-yl)-piperazin-1-carbonyl]-benzyliden}-2-thiomorpholin-4-yl-thiazol-4-on |
| 90 | 2-[4-(3-Chlor-pyridin-2-yl)-piperazin-1-yl]-5-chinolin-3-ylmethylen-thiazol-4-on |
| 91 | 2-[4-(3-Chlor-pyridin-2-yl)-piperazin-1-yl]-5-(4-trifluormethyl-benzyliden)-thiazol-4-on |
| 92 | 2-(2,3-Dihydro-indol-1-yl)-5-(4-methyl-benzyliden)-thiazol-4-on |
| 93 | 2-(2,3-Dihydro-indol-1-yl)-5-(4-trifluormethyl-benzyliden)-thiazol-4-on |
| 94 | 5-[4-(Morpholin-4-carbonyl)-benzyliden]-2-thiomorpholin-4-yl-thiazol-4-on |
| 95 | 2-[4-(3-Chlor-pyridin-2-yl)-piperazin-1-yl]-5-(4-hydroxy-3-methoxy-benzyliden)-thiazol-4-on |
| 96 | 1-[4-Oxo-5-(4-trifluormethyl-benzyliden)-4,5-dihydro-thiazol-2-yl]-3-(2-trifluormethyl-phenyl)-harnstoff |
| 97 | 2-(4-Benzyl-piperazin-1-yl)-5-chinolin-3-ylmethylen-thiazol-4-on |
| 98 | 2-(4-Benzyl-piperazin-1-yl)-5-naphthalen-2-ylmethylen-thiazol-4-on |
| 99 | 2-(4-Benzyl-piperazin-1-yl)-5-(3-phenoxy-benzyliden)-thiazol-4-on |
| 100 | 2-(4-Benzyl-piperazin-1-yl)-5-(4-trifluormethyl-benzyliden)-thiazol-4-on |
| 101 | 1-[5-(4-Methyl-benzyliden)-4-oxo-4,5-dihydro-thiazol-2-yl]-3-(2-trifluormethyl-phenyl)-harnstoff |
| 102 | 2-(4-Benzyl-piperazin-1-yl)-5-naphthalen-1-ylmethylen-thiazol-4-on |
| 103 | 2-(4-Benzyl-piperazin-1-yl)-5-(6-methoxy-naphthalen-2-ylmethylen)-thiazol-4-on |
| 104 | 2-(4-Benzyl-piperazin-1-yl)-5-(4-tert-butyl-benzyliden)-thiazol-4-on |
| 105 | 1-[4-Oxo-5-(3-phenyl-allyliden)-4,5-dihydro-thiazol-2-yl]-3-(2-trifluormethyl-phenyl)-harnstoff |
| 107 | 1-[4-Oxo-5-(4-trifluormethyl-benzyliden)-4,5-dihydro-thiazol-2-yl]-3-(4-trifluormethyl-phenyl)-harnstoff |
| 107 | 1-[5-(4-Methyl-benzyliden)-4-oxo-4,5-dihydro-thiazol-2-yl]-3-(4-trifluormethyl-phenyl)-harnstoff |
| 108 | 5-{3-[4-(6-Chlor-pyridin-2-yl)-piperazin-1-carbonyl]-benzyliden}-2-thiomorpholin-4-yl-thiazol-4-on |
| 109 | 2-(4-Benzyl-piperazin-1-yl)-5-(4-trifluormethoxy-benzyliden)-thiazol-4-on |
| 110 | 5-{3-[4-(3-Chlor-pyridin-2-yl)-3-methyl-piperazin-1-carbonyl]-benzyliden}-2-thiomorpholin-4-yl-thiazol-4-on |
| 111 | N-[3-(3-Chlor-5-trifluormethyl-pyridin-2-ylamino)-propyl]-3-(4-oxo-2-thiomorpholin-4-yl-4H-thiazol-5-ylidenmethyl)-benzamid |
| 112 | 3-(4-Oxo-2-thiomorpholin-4-yl-4H-thiazol-5-ylidenmethyl)-benzoesäure-N'-(2-chlor-4-trifluormethyl-phenyl)-hydrazid |
| 113 | 5-{3-[4-(3-Chlor-5-trifluormethyl-pyridin-2-yl)-piperazin-1-carbonyl]-benzyliden}-2-thiomorpholin-4-yl-thiazol-4-on |
| 114 | 3-(4-Oxo-2-thiomorpholin-4-yl-4H-thiazol-5-ylidenmethyl)-benzoesäure-N'-(3-chlor-5-trifluormethyl-pyridin-2-yl)-hydrazid |
| 115 | 5-{3-[4-(4-Chlor-[1,2,5]thiadiazol-3-yl)-piperazin-1-carbonyl]-benzyliden}-2-thiomorpholin-4-yl-thiazol-4-on |
| 116 | 3-(4-Oxo-2-thiomorpholin-4-yl-4H-thiazol-5-ylidenmethyl)-benzoesäure-N'-(2-chlor-5-trifluormethyl-phenyl)-hydrazid |
| 117 | 2-[4-(4-Chlor-[1,2,5]thiadiazol-3-yl)-piperazin-1-yl]-5-(4-methyl-benzyliden)-thiazol-4-on |
| 118 | 2-(4-(4-Chlor-[1,2,5]thiadiazol-3-yl)-piperazin-1-yl]-5-(4-trifluormethyl-benzyliden)-thiazol-4-on |
| 119 | 2-[4-(4-Chlor-[1,2,5]thiadiazol-3-yl)-piperazin-1-yl]-5-(4-trifluormethoxy-benzyliden)-thiazol-4-on |
| 120 | 5-(4-tert-Butyl-benzyliden)-2-[4-(4-chlor-[1,2,5]thiadiazol-3-yl)-piperazin-1-yl]-thiazol-4-on |
| 121 | 2-[4-(4-Chlor-[1,2,5]thiadiazol-3-yl)-piperazin-1-yl]-5-(3-phenyl-allyliden)-thiazol-4-on |
| 122 | 5-[3-(4-tert-Butyl-phenyl)-allyliden]-2-[4-(4-chlor-[1,2,5]thiadiazol-3-yl)-piperazin-1-yl]-thiazol-4-on |
| 123 | 2-[4-(4-Chlor-[1,2,5]thiadiazol-3-yl)-piperazin-1-yl]-5-naphthalen-1-ylmethylen-thiazol-4-on |
| 124 | 2-[4-(4-Chlor-[1,2,5]thiadiazol-3-yl)-piperazin-1-yl]-5-(3-phenoxy-benzyliden)-thiazol-4-on |
| 125 | 2-[4-(4-Chlor-[1,2,5]thiadiazol-3-yl)-piperazin-1-yl]-5-(4-phenoxy-benzyliden)-thiazol-4-on |
| 126 | 5-(3-Benzyloxy-benzyliden)-2-[4-(4-chlor-[1,2,5]thiadiazol-3-yl)-piperazin-1-yl]-thiazol-4-on |
| 127 | 2-[4-(4-Chlor-[1,2,5]thiadiazol-3-yl)-piperazin-1-yl]-5-(4-hydroxy-3-methoxy-benzyliden)-thiazol-4-on |
| 128 | 2-[4-(4-Chlor-[1,2,5]thiadiazol-3-yl)-piperazin-1-yl]-5-furan-2-ylmethylen-thiazol-4-on |
| 129 | 2-[4-(4-Chlor-[1,2,5]thiadiazol-3-yl)-piperazin-1-yl]-5-naphthalen-2-ylmethylen-thiazolin-4-on |
| 130 | 3-[2-(4-Benzyl-piperazin-1-yl)-4-oxo-4H-thiazol-5-ylidenmethyl]-benzoesäure |
| 131 | 5-Naphthalen-1-ylmethylen-2-piperazin-1-yl-thiazol-4-on |
| 132 | 2-Piperazin-1-yl-5-(4-trifluormethyl-benzyliden)-thiazol-4-on |
| 133 | 5-(4-Methyl-benzyliden)-2-piperazin-1-yl-thiazol-4-on |
| 134 | 5-(4-Hydroxy-3-methoxy-benzyliden)-2-piperazin-1-yl-thiazol-4-on |
| 135 | 5-(4-Isopropyl-benzyliden)-2-morpholin-4-yl-thiazol-4-on |
| 136 | 5-(4-Isopropyl-benzyliden)-2-thiomorpholin-4-yl-thiazol-4-on |
| 137 | 2-[4-(2-Fluor-phenyl)-piperazin-1-yl]-5-(4-isopropyl-benzyliden)-thiazol-4-on |
| 138 | 5-(4-Pentafluorsulfanyl)-benzyliden)-2-thiomorpholin-4-yl-thiazol-4-on |
| 139 | 2-[4-(3-Chlor-pyridin-2-yl)-piperazin-1-yl]-5-(4-isopropyl-benzyliden)-thiazol-4-on |
| 140 | 2-(4-Benzyl-piperazin-1-yl)-5-(4-butyl-benzyliden)-thiazol-4-on Hydrochlorid |
| 141 | 2-(4-Benzyl-piperazin-1-yl)-5-(4-pentyl-benzyliden)-thiazol-4-on Hydrochlorid |
| 142 | 2-(4-Benzyl-piperazin-1-yl)-5-(4-octyl-benzyliden)-thiazol-4-on |
| 143 | 2-[4-(6-Chlor-pyridin-2-yl)-piperazin-1-yl]-5-(4-methyl-benzyliden)-thiazol-4-on |
| 144 | 5-(4-tert-Butyl-benzyliden)-2-[4-(6-chlor-pyridin-2-yl)-piperazin-1-yl]-thiazol-4-on |
| 145 | 2-[4-(6-Chlor-pyridin-2-yl)-piperazin-1-yl)-5-(4-trifluormethyl-benzyliden)-thiazol-4-on |
| 146 | 2-[4-(6-Chlor-pyridin-2-yl)-piperazin-1-yl]-5-(3-phenyl-allyliden)-thiazol-4-on |
| 147 | 5-[3-(4-tert-Butyl-phenyl)-allyliden]-2-[4-(6-chlor-pyridin-2-yl)-piperazin-1-yl]-thiazol-4-on |
| 148 | 2-[4-(6-Chlor-pyridin-2-yl)-piperazin-1-yl]-5-(4-hydroxy-3-methoxy-benzyliden)-thiazol-4-on |
| 149 | 2-[4-(6-Chlor-pyridin-2-yl)-piperazin-1-yl]-5-(4-isopropyl-benzyliden)-thiazol-4-on |
| 150 | 2-[4-(6-Chlor-pyridin-2-yl)-piperazin-1-yl]-5-(4-trifluormethoxy-benzyliden)-thiazol-4-on |
| 151 | 2-[4-(6-Chlor-pyridin-2-yl)-piperazin-1-yl]-5-naphthalen-2-ylmethylen-thiazol-4-on |
| 152 | 5-(4-Methyl-benzyliden)-2-[4-(6-methyl-pyridazin-3-yl)-piperazin-1-yl]-thiazol-4-on |
| 153 | 5-(4-Isopropyl-benzyliden)-2-[4-(6-methyl-pyridazin-3-yl)-piperazin-1-yl]-thiazol-4-on |
| 154 | 5-(4-tert-Butyl-benzyliden)-2-[4-(6-methyl-pyridazin-3-yl)-piperazin-1-yl]-thiazol-4-on |
| 155 | 5-[3-(4-tert-Butyl-phenyl)-allyliden]-2-[4-(6-methyl-pyridazin-3-yl)-piperazin-1-yl]-thiazol-4-on |
| 156 | 2-[4-(6-Methyl-pyridazin-3-yl)-piperazin-1-yl]-5-(4-trifluormethoxy-benzyliden)-thiazol-4-on |
| 157 | 5-(4-Hydroxy-3-methoxy-benzyliden)-2-[4-(6-methyl-pyridazin-3-yl)-piperazin-1-yl]-thiazol-4-on |
| 158 | 2-(4-Benzyl-piperazin-1-yl)-5-(4-pentafluorsulfanyl-benzyliden)-thiazol-4-on |
| 159 | 2-(4-Benzyl-piperazin-1-yl)-5-{3-[4-(4-chlor-[1,2,5]thiadiazol-3-yl)-piperazin-1-carbonyl]-benzyliden}-thiazol-4-on |
| 160 | 2-[4-(6-Methyl-pyridazin-3-yl)piperazin-1-yl]-5-(4-trifluormethyl-benzyliden)-thiazol-4-on |
| 161 | 2-[4-(6-Methyl-pyridazin-3-yl)piperazin-1-yl]-5-(3-phenyl-allyliden)-thiazol-4-on |
| 162 | 2-[4-(6-Methyl-pyridazin-3-yl)-piperazin-1-yl]-5-naphthalen-2-ylmethylen-thiazol-4-on |
| 163 | 2-[4-(6-Methyl-pyridazin-3-yl)-piperazin-1-yl]-5-naphthalen-1-ylmethylen-thiazol-4-on |
| 164 | 2-(4-Benzyl-piperazin-1-yl)-5-(4-isopropyl-benzyliden)-thiazol-4-on Hydrochlorid |
| 165 | 3-{2-[4-(4-Chlor-[1,2,5]thiadiazol-3-yl)-piperazin-1-yl]-4-oxo-4H-thiazol-5-ylidenmethyl}-benzoesäure |
| 166 | 5-{3-[4-(4-Chlor-3-trifluormethyl-phenyl)-4-hydroxy-piperidin-1-carbonyl]-benzyliden}-2-thiomorpholin-4-yl-thiazol-4-on |
| 167 | 5-{3-[4-(4-Chlor-[1,2,5]thiadiazol-3-yl)-piperazin-1-carbonyl]-benzyliden}-2-[4-(4-chlor-[1,2,5]thiadiazol-3-yl)-piperazin-1-yl]-thiazol-4-on |
| 168 | 5-{3-[4-(3-Chlor-pyridin-2-yl)-piperazin-1-carbonyl]-benzyliden}-2-[4-(4-chlor-[1,2,5]thiadiazol-3-yl)-piperazin-1-yl]-thiazol-4-on |
| 169 | 5-[3-(4-Benzyl-piperazin-1-carbonyl)-benzyliden]-2-[4-(4-chlor-[1,2,5]thiadiazol-3-yl)-piperazin-1-yl]-thiazol-4-on |
| 170 | 2-[4-(4-Chlor-[1,2,5]thiadiazol-3-yl]-piperazin-1-yl]-5-{3-[4-(6-methoxy-pyridin-2-yl)-piperazin-1-carbonyl]-benzyliden}-thiazol-4-on |
| 171 | 2-[4-(4-Chlor-3-trifluormethyl-phenyl)-4-hydroxy-piperidin-1-yl]-5-(4-methyl-benzyliden)-thiazol-4-on |
| 172 | 2-[4-(4-Chlor-3-trifluormethyl-phenyl)-4-hydroxy-piperidin-1-yl]-5-(4-hydroxy-3-methoxy-benzyliden)-thiazol-4-on |
| 173 | 2-[4-(4-Chlor-3-trifluormethyl-phenyl)-4-hydroxy-piperidin-1-yl]-5-naphthalen-1-ylmethylen-thiazol-4-on |
| 174 | 2-[4-(4-Chlor-3-trifluormethyl-phenyl)-4-hydroxy-piperidin-1-yl]-5-(3-methoxy-benzyliden)-thiazol-4-on |
| 175 | 5-Benzyliden-2-morpholin-4-yl-thiazol-4-on Hydrobromid |
| 176 | 5-(4-Methyl-benzyliden)-2-morpholin-4-yl-thiazol-4-on Hydrobromid |
| 177 | 2-Diethylamino-5-(4-methyl-benzyliden)-thiazol-4-on Hydrobromid |
| 178 | 5-(4-Chlor-benzyliden)-2-diethylamino-thiazol-4-on Hydrobromid |
| 179 | 5-(4-Isopropyl-benzyliden)-2-morpholin-4-yl-thiazol-4-on Hydrobromid |
| 180 | 5-Benzyliden-2-diethylamino-thiazol-4-on Hydrobromid |
| 181 | 5-(4-Chlor-benzyliden)-2-morpholin-4-yl-thiazol-4-on Hydrobromid |
| 182 | 5-(4-Chlor-benzyliden)-2-piperidin-1-yl-thiazol-4-on Hydrobromid |
| 183 | 5-(4-Chlor-benzyliden)-2-pyrrolidin-1-yl-thiazol-4-on Hydrobromid |
| 184 | 5-Benzyliden-2-pyrrolidin-1-yl-thiazol-4-on Hydrobromid |
| 185 | 5-Benzyliden-2-pyrrolidin-1-yl-thiazol-4-on |
| 186 | 5-Biphenyl-4-ylmethylen-2-piperidin-1-yl-thiazol-4-on Hydrobromid |
| 187 | 2-Azepan-1-yl-5-biphenyl-4-ylmethylen-thiazol-4-on Hydrobromid |
| 188 | 5-Biphenyl-4-ylmethylen-2-pyrrolidin-1-yl-thiazol-4-on Hydrobromid |
| 189 | 2-Morpholin-4-yl-5-(3-phenyl-allyliden)-thiazol-4-on Hydrobromid |
| 190 | 5-(3-Phenyl-allyliden)-2-thiomorpholin-4-yl-thiazol-4-on Hydrobromid |
| 191 | 5-(3-Phenyl-allyliden)-2-piperidin-1-yl-thiazol-4-on Hydrobromid |
| 192 | 2-Azepan-1-yl-5-(3-phenyl-allyliden)-thiazol-4-on Hydrobromid |
| 193 | 5-(3-Phenyl-allyliden)-2-pyrrolidin-1-yl-thiazol-4-on Hydrobromid |
| 194 | 5-Biphenyl-4-ylmethylen-2-morpholin-4-yl-thiazol-4-on Hydrobromid |
| 195 | 2-Thiomorpholin-4-yl-5-(3-trifluormethyl-benzyliden)-thiazol-4-on Hydrobromid |
| 196 | 5-(4-Chlor-benzyliden)-2-thiomorpholin-4-yl-thiazol-4-on |
| 197 | 5-(4-tert-Butyl-benzyliden)-2-morpholin-4-yl-thiazol-4-on |
| 198 | 5-(4-tert-Butyl-benzyliden)-2-thiomorpholin-4-yl-thiazol-4-on Hydrobromid |
| 199 | 5-(4-tert-Butyl-benzyliden)-2-pyrrolidin-1-yl-thiazol-4-on Hydrobromid |
| 200 | 2-Azepan-1-yl-5-(4-tert-butyl-benzyliden)-thiazol-4-on Hydrobromid |
| 201 | 5-(4-tert-Butyl-benzyliden)-2-piperidin-1-yl-thiazol-4-on Hydrobromid |
| 202 | 2-Thiomorpholin-4-yl-5-(4-trifluormethyl-benzyliden)-thiazol-4-on |
| 203 | 5-(4-tert-Butyl-benzyliden)-2-diethylamino-thiazol-4-on Hydrobromid |
| 204 | 2-Morpholin-4-yl-5-(4-trifluormethyl-benzyliden)-thiazol-4-on |
| 205 | 2-Morpholin-4-yl-5-(3-trifluormethyl-benzyliden)-thiazol-4-on Hydrobromid |
| 206 | 2-Morpholin-4-yl-5-(3-trifluormethyl-benzyliden)-thiazol-4-on |
| 207 | 5-Biphenyl-4-ylmethylen-2-diethylamino-thiazol-4-on |
| 208 | 2-Morpholin-4-yl-5-thiophen-2-ylmethylen-thiazol-4-on |
| 209 | 2-Morpholin-4-yl-5-naphthalen-1-ylmethylen-thiazol-4-on |
| 210 | 2-Morpholin-4-yl-5-pyridin-2-ylmethylen-thiazol-4-on |
| 211 | 2-Morpholin-4-yl-5-(4-trifluormethyl-benzyliden)-thiazol-4-on |
| 212 | 2-Morpholin-4-yl-5-pyridin-3-ylmethylen-thiazol-4-on |
| 213 | 2-Morpholin-4-yl-5-pyridin-4-ylmethylen-thiazol-4-on |
| 214 | 2-Diethylamino-5-(3-trifluormethyl-benzyliden)-thiazol-4-on |
| 215 | 5-(4-Methyl-benzyliden)-2-pyrrolidin-1-yl-thiazol-4-on |
| 216 | 5-(4-tert-Butyl-benzyliden)-2-(2-methoxy-ethylamino)-thiazol-4-on Hydrobromid |
| 217 | 2-Morpholin-4-yl-5-(4-octyl-benzyliden)-thiazol-4-on |
| 218 | 5-(4-Methyl-benzyliden)-2-morpholin-4-yl-thiazol-4-on |
| 219 | 5-(3,4-Dimethoxy-benzyliden)-2-morpholin-4-yl-thiazol-4-on |
| 220 | 5-(2-Hydroxy-benzyliden)-2-morpholin-4-yl-thiazol-4-on |
| 221 | 5-(2-Hydroxy-3-methoxy-benzyliden)-2-morpholin-4-yl-thiazol-4-on |
| 222 | 5-(4-tert-Butyl-benzyliden)-2-morpholin-4-yl-thiazol-4-on |
| 223 | 5-(4-Diethylamino-benzyliden)-2-morpholin-4-yl-thiazol-4-on |
| 224 | 5-(4-Hydroxy-3-methoxy-benzyliden)-2-morpholin-4-yl-thiazol-4-on |
| 225 | 4-(2-Morpholin-4-yl-4-oxo-4H-thiazol-5-ylidenmethyl)-benzoesäure |
| 226 | 2-Morpholin-4-yl-5-(2,3,4-trimethoxy-benzyliden)-thiazol-4-on |
| 227 | 2-Morpholin-4-yl-5-(3,4,5-trimethoxy-benzyliden)-thiazol-4-on |
| 228 | 5-(4-Methoxy-benzyliden)-2-morpholin-4-yl-thiazol-4-on |
| 229 | 5-(4-Hydroxy-benzylidenl-2-morpholin-4-yl-thiazol-4-on |
| 230 | 5-(3-Ethoxy-4-hydroxy-benzyliden)-2-morpholin-4-yl-thiazol-4-on |
| 231 | 5-(3-Hydroxy-benzyliden)-2-morpholin-4-yl-thiazol-4-on |
| 232 | 5-(4-Brom-benzyliden)-2-morpholin-4-yl-thiazol-4-on |
| 233 | 2-Morpholin-4-yl-5-(4-vinyloxy-benzyliden)-thiazol-4-on |
| 234 | Benzensulfonsäure-4-(2-morpholin-4-yl-4-oxo-4H-thiazol-5-ylidenmethyl)-phenylester |
| 235 | 5-(4-Dimethylamino-benzyliden)-2-morpholin-4-yl-thiazol-4-on |
| 236 | 4-(2-Morpholin-4-yl-4-oxo-4H-thiazol-5-ylidenmethyl)-benzoesäuremethylester |
| 237 | 5-(3-Hydroxy-4-methoxy-benzyliden)-2-morpholin-4-yl-thiazol-4-on |
| 238 | 5-[4-(3,3-Dimethyl-butoxy)-3-methoxy-benzyliden]-2-morpholin-4-yl-thiazol-4-on |
| 239 | 5-(2-Methoxy-benzyliden)-2-morpholin-4-yl-thiazol-4-on |
| 240 | 5-(4-Hydroxy-benzyliden)-2-morpholin-4-yl-thiazol-4-on |
| 241 | 2-[(Benzo[1,3]dioxol-5-ylmethyl)-amino]-5-[3-(4-tert-butyl-phenyl)-allyliden]-thiazol-4-on |
| 242 | 2-[(Benzo[1,3]dioxol-5-ylmethyl)-amino]-5-(4-trifluormethyl-benzyliden)-thiazol-4-on |
| 243 | 2-[(Benzo[1,3]dioxol-5-ylmethyl)amino]-5-(4-trifluormethoxy-benzyliden)-thiazol-4-on |
| 244 | 2-[(Benzo[1,3]dioxol-5-ylmethyl)-amino]-5-naphthalen-1-ylmethylen-thiazol-4-on |
| 245 | 2-[(Benzo[1,3]dioxol-5-ylmethyl)-amino]-5-(2-chlor-3-phenyl-allyliden)-thiazol-4-on |
| 246 | 2-(4-Methyl-benzylamino)-5-naphthalen-1-ylmethylen-thiazol-4-on |
| 247 | 2-(4-Methyl-benzylamino)-5-(3-phenoxy-benzyliden)-thiazol-4-on |
| 248 | 2-(4-Methyl-benzylamino)-5-(4-phenoxy-benzylideny)-thiazol-4-on |
| 249 | 5-(2-Chlor-3-phenyl-allyliden)-2-(4-methyl-benzylamino)-thiazol-4-on |
| 250 | 2-(4-Methyl-benzylamino)-5-naphthalen-2-ylmethylen-thiazol-4-on |
| 251 | 2-(3-Methoxy-benzylamino)-5-(4-methyl-benzyliden)-thiazol-4-on |
| 252 | 5-(4-tert-Butyl-benzyliden)-2-(3-methoxy-benzylamino)-thiazol-4-on |
| 253 | 2-(3-Methoxy-benzylamino)-5-(4-trifluormethyl-benzyliden)-thiazol-4-on |
| 254 | 2-(3-Methoxy-benzylamino)-5-(4-trifluormethoxy-benzyliden)-thiazol-4-on |
| 255 | 2-(3-Methoxy-benzylamino)-5-(3-phenyl-allyliden)-thiazol-4-on |
| 256 | 5-Naphthalen-1-ylmethylen-2-(4-trifluormethyl-benzylamino)-thiazol-4-on |
| 257 | 5-(3-Phenoxy-benzyliden)-2-(4-trifluormethyl-benzylamino)-thiazol-4-on |
| 258 | 5-(4-Phenoxy-benzyliden)-2-(4-trifluormethyl-benzylamino)-thiazol-4-on |
| 259 | 5-(2-Chloro-3-phenyl-allyliden)-2-(4-trifluormethyl-benzylamino)-thiazol-4-on |
| 260 | 5-(3-Benzyloxy-benzyliden)-2-(4-trifluormethyl-benzylamino)-thiazol-4-on |
| 261 | 5-Naphthalen-2-ylmethylen-2-(4-trifluormethyl-benzylamino)-thiazol-4-on |

### Pharmakologische Daten

Die Affinität der erfindungsgemäßen 2,5-disubstituierten Thiazol-4-on-Derivate für den Vanilloid-Rezeptor 1 (VR1/TRPV1-Rezeptor) wurde wie vorstehend beschrieben bestimmt.

| **Verbindung gemäß Beispiel** | **VR1 (Ratte) (% Stimulation im Vgl. zu 10 µM CP)** | **VR1 (Ratte) (% Hemmung im Vgl. zu 10 µM CP)** | **VR1 (Mensch) (% Stimulation im Vgl. zu 10 µM CP)** | **VR1 (Mensch) (% Hemmung im Vgl. zu 10 µM CP)** | **EC₅₀ VR1 (Ratte)** | **EC₅₀ VR1 (Mensch)** | **IC₅₀ VR1 (Ratte)** | **IC₅₀ VR1 (Mensch)** |
|---|---|---|---|---|---|---|---|---|
| **12** | 53,99 | 68,39 | 55,40 | 54,33 | | | | |
| **13** | 42,13 | 46,23 | 6,15 | 11,13 | | | | |
| **14** | 107,63 | 82,14 | 106,96 | 89,44 | 0,95 | 5,17 | | |
| **17** | 119,55 | 91,14 | 45,99 | 19,65 | 6,36 | 17,78 | | |
| **18** | 144,22 | 88,01 | 139,33 | 79,17 | 1,295 | 3,645 | | |
| **19** | 11,71 | 31,38 | 8,82 | 10,83 | 7,755 | 13,02 | | |
| **21** | 55,77 | 50,12 | 36,52 | 28,51 | 6,595 | 8,51 | | |
| **22** | 116,13 | 88,08 | 63,95 | 80,79 | 8,485 | 9,06 | | |
| **24** | 110,20 | 91,35 | 61,33 | 61,63 | 11,875 | > 25 | | |
| **25** | 80,77 | 87,21 | 29,07 | 37,65 | | | | |
| **30** | 0,92 | 97,01 | 0,06 | 73,26 | | | > 50 | >100 |
| **33** | 49,60 | 64,80 | 25,74 | 14,65 | | | | |
| **34** | 100,54 | 87,96 | 110,57 | 89,29 | | | | |
| **36** | 67,65 | 100,59 | 47,43 | 68,80 | | | | |
| **39** | 31,17 | 50,97 | 18,89 | 23,67 | | | | |
| **41** | 86,95 | 90,93 | 0,47 | 22,60 | 5,82 | 10,56 | | |
| **42** | 98,33 | 93,08 | 49,72 | 50,18 | | | | |
| **44** | 39,61 | 64,34 | 10,95 | 34,31 | | | | |
| **49** | 0,40 | 78,48 | 0,00 | 34,15 | | | > 50 | > 50 |
| **50** | 7,19 | 21,27 | 0,00 | 51,62 | | | | |
| **54** | 96,08 | 87,88 | 79,60 | 81,73 | 2,5 | > 25 | | |
| **63** | 0,34 | 70,90 | 0,00 | 52,80 | | | 3,04 | 9,5 |
| **68** | 73,37 | 11,64 | 54,28 | 39,34 | | | | |
| **85** | 54,91 | 77,11 | 82,46 | 97,47 | | | | |
| **88** | 72,85 | 90,08 | 61,42 | 71,14 | | | | |
| **96** | 28,48 | 26,98 | 5,03 | 54,65 | | | | |
| **110** | 49,33 | 50,28 | 1,02 | 28,75 | | | | |
| **113** | 135,99 | 97,39 | 90,31 | 97,83 | | | | |
| **115** | 128,30 | 95,77 | 68,25 | 66,21 | | | | |
| **135** | 79,68 | 87,60 | 85,02 | 84,29 | | | | |
| **136** | 83,98 | 96,93 | 114,42 | 86,67 | | | | |
| **154** | 3,99 | 54,11 | 6,56 | 34,23 | | | | |
| **161** | 2,50 | 17,01 | 2,43 | 47,22 | | | | |
| **176** | 44,68 | 97,55 | 14,52 | 35,32 | | | | |
| **179** | 41,89 | 38,36 | 5,04 | 30,72 | | | | |
| **189** | 6,64 | 5,61 | 11,15 | 8,14 | >25 | > 50 | | |
| **190** | 51,35 | 83,13 | 34,09 | 32,35 | 14,27 | > 25 | | |
| **191** | -0,17 | 17,65 | 15,81 | 8,04 | > 25 | > 50 | | |
| **192** | 1,34 | 2,18 | 16,72 | 10,31 | > 50 | > 50 | | |
| **195** | 94,27 | 96,09 | 80,31 | 90,32 | 2,2 | 5,9 | | |
| **197** | 63,86 | 94,35 | 48,05 | 72,42 | | | | |
| **198** | 60,71 | 97,57 | 54,31 | 76,67 | 1,12 | 1,02 | | |
| **199** | 42,91 | 69,55 | 9,64 | 4,43 | | | | |
| **200** | 31,45 | 46,20 | 17,52 | 2,12 | > 25 | >25 | | |
| **205** | 79,60 | 96,40 | 68,23 | 102,91 | | | | |
| **206** | 76,51 | 102,30 | 77,26 | 95,06 | | | | |
| **209** | 39,99 | 34,68 | 9,44 | 5,47 | 14,83 | 28,4 | | |
| **210** | 2,22 | 23,01 | 1,83 | 3,03 | | | | |
| **214** | 13,70 | 50,09 | 6,08 | 56,74 | | | | |
| **215** | 2,14 | 4,47 | 3,64 | 49,80 | | | | |
| **216** | 1,24 | 22,13 | 2,08 | 51,07 | 1,5 | 5,4 | | |
| **218** | 55,76 | 78,83 | 17,91 | 12,88 | | | | |
| **222** | 102,46 | 98,64 | 41,92 | 79,67 | | | | |
| **223** | 101,77 | 9,30 | 70,53 | 57,72 | | | | |
| **228** | 16,03 | 4,84 | 12,18 | 0,61 | 14,3 | 26,5 | | |
| **232** | 80,44 | 96,18 | 29,19 | 43,80 | | | | |
| **234** | 57,90 | 98,08 | 46,10 | 27,80 | | | | |
| **235** | 123,39 | 13,66 | 73,87 | 44,50 | | | | |

## Patentansprüche

1. Verwendung wenigstens eines 2,5-disubstituierten Thiazol-4-on-Derivats der allgemeinen Formel I, 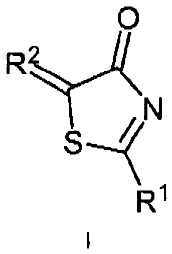 worin
R¹ für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
für einen unsubstituierten oder wenigstens einfach substituierten Aryl-Rest, der mit einem unsubstituierten oder wenigstens einfach substituierten mono- oder polyzyklischen Ringsystem kondensiert sein kann;
für eine MR³R⁴-Gruppe;
für eine NR⁶-C(=O)-R⁶-Gruppe
oder für eine NR⁷-C(=O)-NR⁸R⁹ Gruppe steht;
R² für einen unsubstituierten oder wenigstens einfach substituierten,
ungesättigten oder gesättigten, ggf. wenigstens ein Heteroatom als Ringglied aufweisenden cycloallphatlschen Rest, der mit einem unsubstituierten oder wenigstens einfach substituierten mono- oder polyzyklischen Ringsystem kondensiert sein kann;
für CH-U-X;
für CH-CH₂-V-Y, CH-CH(CH₃)-V-Y, CH-CHCl-V-Y, CH-CHBr-V-Y, CH-CHF-V-Y oder CH-CH(OH)-V-Y;
oder für CH-CH=C(CH₃)-W-Z, CH-CH=CH-W-Z, CH-C(CH₃)=CH-W-Z, CH-C(Phenyl)=CH-W-Z, CH-CBr=CH-W-Z. CH-CCl=CH-W-Z, CH-CF=CH-W-Z, CH-C(OH)=CH-W-Z, CH-CH₂-CH₂-W-Z, CH-CH₂-CH(CH₃)-W-Z oder CH-CH(CH₃)-CH₂-W-Z steht;
wobei U, V und W jeweils nicht vorhanden sein können oder jeweils unabhängig voneinander für einen Rest ausgewählt aus der Gruppe bestehend aus O, S, N(H), N(CH₃), N(C₂H₅) und N[CH(CH₃)₂] stehen;
R³ für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopentyl Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Thiomorpholinyl und Azepanyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, fsobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl. -N(C₂H₅)-Pyridinyl, -N[CH(CH₃)₂]-Pyridinyl substituiert sein kann; wobei jeweils der zyklische Teil der Reste -NH-Phenyl, -NH-Pyridinyl, -N(CH₃)-Phenyl, -N(C₂H₆)-Phenyl, -N[CH(CH₃)₂]-Phenyl, -N(CH₃)-Pyridinyl, -N(C₂H₅)-Pyridinyl und -N[CH(CH₃)₂]-Pyridinyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl und (1,4)-Benzodioxanyi steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, I, -SF₅, -OH, -O-CH₃, -O-C₂H₆, -NH₂, -NO₂, -CF₃, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=OFO-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-CH(CH₃)₂ und -NH-C(CH₃)₃ substituiert sein kann;
R⁴ für einen Wasserstoff-Rest
oder für einen linearen oder verzweigten, gesättigten oder ungesättigten, unsubstituierten oder wenigstens einfach substituierten, ggf. wenigstens ein Heteroatom als Kettenglied aufweisenden aliphatischen Rest steht;
R⁵, R⁷ und R⁹, unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
R⁶ und R⁸, unabhängig voneinander, jeweils
für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, stehen;
X für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹⁰ substituiert sein kann, steht:
Y für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹¹ substituiert sein kann, steht:
Z für einen linearen oder verzweigten, gesättigten oder ungesättigten, unsubstituierten oder wenigstens einfach substituierten aliphatischen Rest;
oder für einen Aryl- oder Heteroaryl-Rest, der mit einem unsubstituierten oder wenigstens einfach substituierten mono- oder polyzyklischen Ringsystem kondensiert und/oder mit gleichen oder verschiedenen Substituenten R¹² substituiert sein kann, steht;
R¹⁰, R¹¹ und R¹², unabhängig voneinander, jeweils
für einen Rest ausgewählt aus der Gruppe bestehend aus F, Cl, Br, I, - CN, -CF₃, -SF₅, -OH, -O-C₁₋₁₀-Alkyl, -O-C₂₋₁₀-Alkenyl, -NH₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, - O-C(=O)-C₁₋₅-Alkyl, -NH-C₁₋₅Alkyl, -N(C₁₋₅-Alkyl)₂. -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-C₁₋₅Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, - O-Benzyl, Phenyl und Benzyl stehen; wobei jeweils der zyklische Teil der Reste Pyridinyl, Cyclopentyl, Cyclohexyl, Pyridazinyl, -S(=O)₂-Phenyl, -O-S(=O)₂-Phenyl, -O-Phenyl, -O-Benzyl, Phenyl, -(CH₂)-Benzo[b]furanyl und Benzyl substituiert sein kann;
für eine -C(=O)-NR¹³-(CH₂)ₘ-NR¹⁴-R¹⁵-Gruppe mit m gleich 0, 1, 2, 3, 4 oder 5;
oder für eine -C(=O)-R¹⁶-Gruppe stehen;
R¹³ und R¹⁴, unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
R¹⁵ für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, steht;
und
R¹⁶ für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann, steht;
jeweils ggf. in Form eines ihrer reinen Stereoisomere, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate;
zur Herstellung eines Arzneimittels zur Prophylaxe und/oder Behandlung von Schmerz.

2. Verwendung einer Verbindung gemäß Anspruch 1, **dadurch gekennzeichnet, dass**
R¹ für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann;
für einen ggf. substituierten 6- oder 10-gliedrigen Aryl-Rest, der mit einem gesättigten oder ungesättigten, ggf. substituierten mono- oder polyzyklischen Ringsystem kondensiert sein kann;
für eine NR³R⁴-Gruppe;
für eine NR⁵-C(=O)-R⁶-Gruppe
oder für eine NR⁷-C(=O)-NR⁸R⁹ Gruppe steht;
R² für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann;
für CH-U-X;
für CH-CH₂-V-Y, CH-CH(CH₃)-V-Y, CH-CHCl-V-Y, CH-CHBr-V-Y, CH-CHF-V-Y oder CH-CH(OH)-V-Y;
oder für CH-CH=C(CH₃)-W-Z, CH-CH=CH-W-Z, CH-C(CH₃)=CH-W-Z, CH-C(Phenyl)=CH-W-Z, CH-CBr=CH-W-Z, CH-CCl=CH-W-Z, CH-CF=CH-W-Z, CH-C(OH)=CH-W-Z, CH-CH₂-CH₂-W-Z, CH-CH₂-CH(CH₃)-W-Z oder CH-CH(CH₃)-CH₂-W-Z steht;
wobei U, V und W jeweils nicht vorhanden sein können oder jeweils unabhängig voneinander für einen Rest ausgewählt aus der Gruppe bestehend aus O, S, N(H), N(CH₃), N(C₂H₅) und N[CH(CH₃)₂] stehen;
R³ für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopentyl Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Thiomorpholinyl und Azepanyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl substituiert sein kann; wobei jeweils der zyklische Teil der Reste -NH-Phenyl, -NH-Pyridinyl, -N(CH₃)-Phenyl, -N(C₂H₅)-Phenyl, -N[CH(CH₃)₂]-Phenyl, -N(CH₃)-Pyridlnyl, -N(C₂H₅)-Pyridinyl und -N[CH(CH₃)₂]-Pyridinyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder-(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, Methyl, Ethyl, n-Propyl, Isopropyl, n-Butyl, sec-Butyl, Isobuty, tert-Butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-CH(CH₃)₂ und -NH-C(CH₃)₃ substituiert sein kann.;
R⁴ für einen Wasserstoff-Rest
oder für einen linearen oder verzweigten, gesättigten oder ungesättigten, ggf. substituierten, ggf. 1, 2 oder 3 Heteroatome unabhängig voneinander ausgewählt aus der Gruppe bestehend aus Sauerstoff, Stickstoff (NH) und Schwefel aufweisenden C₁₋₁₀ aliphatischen Rest steht;
R⁵, R⁷ und R⁸, unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
R⁶ und R⁹, unabhängig voneinander, jeweils
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
X für einen 5- bis 14-gliedrigen Aryl- oder Heteroaryl-Rest, der mit einem gesättigten oder ungesättigten, ggf. substituierten mono- oder polyzyklischen Ringsystem kondensiert und/oder ggf. mit 1, 2, 3, 4 oder 5 gleichen oder verschiedenen Substituenten R¹⁰ substituiert sein kann, steht;
Y oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder ggf. mit 1, 2, 3, 4 oder 5 gleichen oder verschiedenen Substituenten R¹¹ substituiert sein kann, steht;
Z für einen linearen oder verzweigten, gesättigten oder ungesättigten, ggf. substituierten C₁₋₂₀ aliphatischen Rest;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder ggf. mit 1, 2, 3, 4 oder 5 gleichen oder verschiedenen Substituenten R¹² substituiert sein kann, steht;
R¹⁰, R¹¹ und R¹², unabhängig voneinander, jeweils
für einen Rest ausgewählt aus der Gruppe bestehend aus F, Cl, Br, I, - CN, -CF₃, -SF₅, -OH, -O-C₁₋₁₀-Alkyl, -O-C₂₋₁₀-Alkenyl, -NH₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, - O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-C₁₋₅-Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, - O-Benzyl, Phenyl und Benzyl stehen; wobei jeweils der zyklische Teil der Reste Pyridinyl, Cyclopentyl, Cyclohexyl, Pyridazinyl, -S(=O)₂-Phenyl, -O-S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann;
für eine -C(=O)-NR¹³-(CH₂)ₘ-NR¹⁴-R¹⁵-Gruppe mit m gleich 0, 1, 2, 3, 4 oder 5;
oder für eine -C(=O)-R¹⁶ -Gruppe stehen;
R¹³ und R¹⁴, unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
R¹⁵ für einen 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
und
R¹⁶ für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann, steht;
wobei
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl. Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅₋Alkyl, -O-C(=O)-C₁₋₅-Alkyl, -NH-C₁₋₅-Alkyl, -N(C₁₋₅-Alkyl)₂, - NH-Phenyl, -NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅Alkyl, Cyclohexyl, Cyclopentyl, Pyridinyl, [1.2.5]-Thiadiazolyl, Pyridazinyl, -(CH₂)-Benzo[b]furanyl, -O-Phenyl, -O-Benzyl, Phenyl und Benzyl substituiert sein können, wobei jeweils der zyklische Teil der Reste -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglled(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(-O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
die vorstehend genannten C₁₋₁₀ aliphatischen Reste oder C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -SH und -NH₂ substituiert sein können;
die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl. Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, O-C(=O)-C₁₋₅-Alkyl, -NH-C₁₋₅-Alkyl, -N(C₁₋₅-Alkyl)₂, -NH-C(=O)-O-C₁₋₅-Alkyl, - NH-C(=O)-C₁₋₅-Alkyl, -C(=O)-H, -C(=O)-C₁₋₅-Alkyl, -C(=O)-NH₂, -C(=O)-NH-C_{1- 5}-Alkyl, -C(=O)-N-(C₁₋₅-Alkyl)₂, -S(=O)₂-C₁₋₅-Alkyl, -S(=O)₂-Phenyl, -NH-S(=O)₂-C₁₋₆-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₆, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

3. Verwendung einer Verbindung gemäß Anspruch 1 oder 2, **dadurch gekennzeichnet, dass**
R¹ für einen Rest ausgewählt aus der Gruppe bestehend aus Cyclopentyl Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Thiomorpholinyl, Azepanyl, (2,3)-Dihydro-1H-isoindolyl, (2,3)-Dihydro-indolyl, (2,3,4,9)-Tetrahydro-1H-β-carbolinyl, (2,3,4,9)-Tetrahydro-1H-pyrido[2,3-b]indofyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl und [3,4]-Dihydro-2H-1,4-benzoxazinyl steht; wobei der Rest jeweils ggf. mit 1, 2, 3, 4 oder 5 Substituenten unabhängig voneinander ausgewählt aus der Gruppe bestehend aus Oxo (-O), Thioxo (=S), F, Cl, Br, I, -SF₅, -OH, - O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -O-C(=O)-CH₃, -O-C(=O)-C₂H₅, -O-C(-O)-C(CH₃)₃, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, - N(CH₃)-Phenyl, -N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl, -NH-C(=O)-O-CH₃, -NH-C(=O)-O-C₂H₅, -NH-C(=O)-O-C(CH₃)₃, -C(=O)-CH₃, -C(=O)-C₂H₅, - C(=O)-NH-CH₃, -C(=O)-NH-C₂H₅, -C(=O)-N-(CH₃)₂, -C(=O)-N-(C₂H₅)₂, Pyridinyl, Pyridazinyl und Phenyl substituiert sein kann; wobei jeweils der zyklische Teil der Reste -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl, - N(C₂H₅)-Pyridinyl, -N[CH(CH₃)₂]-Pyridinyl, Pyridinyl, Pyridazinyl und Phenyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für einen Piperazinyl-Rest steht, der mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -C(=O)C₁₋₅-Alkyl, - C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, Pyridinyl, Pyridazinyl, [1,2,5]-Thiadiazolyl, Benzyl und Phenyl substituiert sein kann; wobei jeweils der zyklische Teil der Reste Pyridinyl, Pyridazinyl, [1,2,5]-Thiadiazolyl, Benzyl und Phenyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₇H_{5.} -O-CF₃, -S-CF₃, Phenyl und -O-Benzyl substituiert sein kann;
für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus F, Cl, Br, I, -SF₅, -OH, -O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, - C(=O)-O-C(CH₃)₃, -O-C(=O)-CH₃, -O-C(=O)-C₂H₅, -O-C(=O)-C(CH₃)₃, - N(CH₃)₂, -N(C₂H₆)₂, -NH-CH₃, -NH-C₂H₅, -NH-CH(CH₃)₂ und -NH-C(CH₃)₃ substituiert sein kann;
für eine NR³R⁴-Gruppe steht;
für eine NR⁵-C(=O)-R⁶-Gruppe steht
oder für eine NR⁷-C(=O)-NR⁸R⁹ Gruppe steht.

4. Verwendung einer Verbindung gemäß einem oder mehreren der Ansprüche 1 bis 3, **dadurch gekennzeichnet, dass**
R² für einen 1,3-Dihydro-indol-2-onyl- oder einen 1,3-Dihydro-indol-2-thionyl-Rest steht;
für CH-X steht;
für CH-CH₂-Y, CH-CH(CH₃)-Y, CH-CHCl-Y, CH-CHBr-Y, CH-CHF-Y oder CH-CH(OH)-Y steht;
oder für CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(Phenyl)=CH-Z, CH-CBr=CH-Z, CH-CCI=CH-Z, CH-CF=CH-Z, CH-C(OH)=CH-Z, CH-CH₂-CH₂-Z, CH-CH₂-CH(CH₃)-Z oder CH-CH(CH₃)-CH₂-Z steht.

5. Verwendung einer Verbindung gemäß einem oder mehreren der Ansprüche 1 bis 4, **dadurch gekennzeichnet, dass**
R⁴ für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, - CN, -NO₂, -OH, -SH und -NH₂ substituiert sein kann.

6. Verwendung einer Verbindung gemäß einem oder mehreren der Ansprüche 1 bis 5, **dadurch gekennzeichnet, dass**
R⁵, R⁷ und R⁸, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

7. Verwendung einer Verbindung gemäß einem oder mehreren der Ansprüche 1 bis 6, **dadurch gekennzeichnet, dass**
R⁶ und R⁹, unabhängig voneinander, jeweils
für einen Rest ausgewählt aus der Gruppe bestehend aus Phenyl, Naphthyl, Thiophenyl, Furanyl, Pyridlnyl, Thiazolyl und Oxazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(-O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, - C(=O)-C₂H₅, -C(=O)-C(CH₃)₃ substituiert sein kann.

8. Verwendung einer Verbindung gemäß einem oder mehreren der Ansprüche 1 bis 7, **dadurch gekennzeichnet, dass**
X für einen Rest ausgewählt aus der Gruppe bestehend aus Phenyl, Naphthyl, Phenanthrenyl, (1,3)-Benzodioxoiyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht, der ggf. mit 1, 2, 3, 4 oder 5 gleichen oder verschiedenen Substituenten R¹⁰ substituiert sein kann.

9. Verwendung einer Verbindung gemäß einem oder mehreren der Ansprüche 1 bis 8, **dadurch gekennzeichnet, dass**
Y für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht, der ggf. mit 1, 2, 3, 4 oder 5 gleichen oder verschiedenen Substituenten R¹¹ substituiert sein kann.

10. Verwendung einer Verbindung gemäß einem oder mehreren der Ansprüche 1 bis 9, **dadurch gekennzeichnet, dass**
Z für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
oder für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht, der ggf. mit 1, 2, 3, 4 oder 5 gleichen oder verschiedenen Substituenten R¹² substituiert sein kann.

11. Verwendung einer Verbindung gemäß einem oder mehreren der Ansprüche 1 bis 10, **dadurch gekennzeichnet, dass**
R¹⁰, R¹¹ und R¹², unabhängig voneinander, jeweils
für einen Rest ausgewählt aus der Gruppe bestehend aus F, Cl, Br, I, - CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₆, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, - C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-Phenyl, -O-S(=O)₂-Phenyl, Cyclohexyl, Cyclopentyl, Pyridinyl, Pyridazinyl, -(CH₂)-Benzo[b]furanyl, -O-Phenyl, -O-Benzyl, Phenyl und Benzyl stehen; wobei jeweils der zyklische Teil der Reste Pyridinyl, Cyclopentyl, Cyclohexyl, Pyridazinyl, -S(=O)₂-Phenyl, -O-S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂ Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für eine -C(=O)-NR¹³-(CH₂)ₘ-NR¹⁴-R¹⁵-Gruppe mit m gleich 0, 1, 2 oder 3 stehen;
oder für eine -C(=O)-R¹⁶-Gruppe stehen;
wobei
R¹³ und R¹⁴, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
R¹⁵ für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, Methyl, Ethyl, n-Propyl, Isopropyl, n-Butyl, sec-Butyl, Isobutyl, tert-Buty, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, - C(=O)-C₂H₆, -C(=O)C(CH₃)₃ substituiert sein kann;
und
R¹⁶ für einen Rest ausgewählt aus der Gruppe bestehend aus Piperazinyl, Thlomorpholinyl, Azepanyl, Morpholinyl, (2,3)-Dihydro-1 H-isoindolyl, (2,3)-Dihydro-indolyl, Piperidinyl und Pyrrolidinyl steht; wobei der Rest mit 1, 2 oder 3 Substituenten unabhängig voneinander ausgewählt aus der Gruppe bestehend aus -OH, Methyl, Ethyl, n-Propyl, Isopropyl, n-Butyl, sec-Butyl, Isobutyl, tert-Butyl, -C(-O)-O-CH₃, -C(=O)-O-C₂H₅, - C(=O)-O-C(CH₃)₃, -C(=O)-C₁₋₅-Alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, Pyridinyl, Pyridazinyl, [1,2,5]-Thiadiazolyl, Benzyl und Phenyl substituiert sein kann; wobei jeweils der zyklische Teil der Reste Pyridinyl, Pyridazinyl, [1,2,5]-Thiadiazolyl, Benzyl und Phenyl mit 1, 2, 3, 4 oder 5 Substituenten unabhängig voneinander ausgewählt aus der Gruppe bestehend aus F, Cl, Br, -OH, -CF₃, -SF₃, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

12. Verwendung einer Verbindung gemäß einem oder mehreren der Ansprüche 1 bis 11, **dadurch gekennzeichnet, dass**
R¹ für einen der folgenden Reste steht 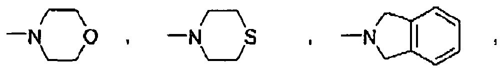 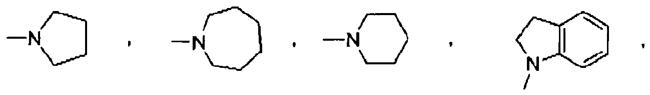 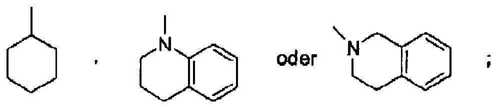 wobei der Rest mit 1, 2 oder 3 Substituenten unabhängig voneinander ausgewählt aus der Gruppe bestehend aus -OH, -O-CH₃, -O-C₂H₅, Methyl, Ethyl, n-Propyl, Isopropyl, n-Butyl, sec-Butyl, Isobutyl, tert-Butyl, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₆)-Phenyl, - N[CH(CH₃)₂]-Phenyl, -N(CH₃)-Pyridinyl, -N(C₂H₅)-Pyridinyl, - N[CH(CH₃)₂]-Pyridinyl und Phenyl substituiert sein kann; wobei jeweils der zyklische Teil des Phenyl-Restes mit 1, 2 oder 3 Substituenten unabhängig voneinander ausgewählt aus der Gruppe bestehend aus F, Cl, Br und -CF₃ substituiert sein kann;
für einen der folgenden Reste steht 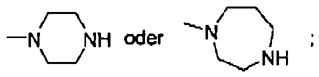 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus -OH, -O-CH₃ und -O-C₂H₅ substituiert sein kann;
für eine NR³R⁴-Gruppe steht
oder für eine NR⁷-C(=O)-NR⁸R⁹ Gruppe steht;
R² für einen 1,3-Dihydro-indol-2-onyl- oder einen 1,3-Dihydro-indol-2-thionyl-Rest steht;
für CH-X steht;
für CH-CH₂-Y, CH-CH(Ch₃)-Y, CH-CHCl-Y, CH-CHBr-Y, CH-CHF-Y oder CH-CH(OH)-Y steht;
oder für CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(Phenyl)=CH-Z, CH-CBr=CH-Z, CH-CCI=CH-Z, CH-CF=CH-Z, CH-C(OH)-CH-Z, CH-CH₂-CH₂-Z, CH-CH₂-CH(CH₃)-Z oder CH-CH(CH₃)-CH₂-Z steht;
R³ für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-GH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopentyl Cyclohexyl, Cycloheptyl, Cyclopentenyl, Cyclohexenyl und Cycloheptenyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, - N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder- (CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
R⁴ für einen Wasserstoff-Rest
oder für einen Rest ausgewählt aus der Gruppe bestehend aus Methyl, Ethyl, n-Prcpyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
R⁷ und R⁸, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
R⁹ für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CF₃, -S-CF₃, Methyl, Ethyl, n-Propyl, Isopropyl, n-Butyl, sec-Butyl, Isobutyl und tert-Butyl substituiert sein kann;
X für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht; der ggf. mit 1, 2, 3, 4 oder 5 gleichen oder verschiedenen Substituenten R¹⁰ substituiert sein kann;
Y für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht, der ggf. mit 1, 2, 3, 4 oder 5 gleichen oder verschiedenen Substituenten R¹¹ substituiert sein kann;
Z für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht;
oder für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyrrolyl, Pyrazolyl, Pyrazinyl, Pyridinyl, Pyridazinyl, Pyrimidinyl, Chinolinyl und Isochinolinyl steht; der ggf. mit 1, 2, 3, 4 oder 5 gleichen oder verschiedenen Substituenten R¹² substituiert sein kann;
R¹⁰ für einen Rest ausgewählt aus der Gruppe bestehend aus F, Cl, Br, I, - CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, - SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl, n-Octyl, -C(=O)-OH, -C(=O)-O-CH₃, - C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-CH₃, -NH-C(=O)-C₂H₅, -NH-C(=O)-C(CH₃)₃, -O-S(=O)₂-Phenyl, -O-Phenyl, -O-Benzyl, Phenyl und Benzyl steht;
für eine -C(-O)-NR¹³-(CH₂)ₘ-NR¹⁴-R¹⁵-Gruppe mit m gleich 0, 1, 2 oder 3 steht;
oder für eine -C(=O)-R¹⁶-Gruppe steht;
R¹¹ für einen Rest ausgewählt aus der Gruppe bestehend aus F, Cl, Br, - SF₅. Methyl, Ethyl, n-Propyl, Isopropyl, butyl, sec-Butyl, tert-Butyl, n-Pentyl, n-Hexyl, n-Heptyl und n-Octyl steht;
R¹² für einen Rest ausgewählt aus der Gruppe bestehend aus F, Cl, Br, - SF₆, -CF₃, -O-CH₃, -O-C₂H₅, -O-CH₂-CH₂-CH₃, -O-CH₂-CH₂-CH₂-CH₃, - O-C(CH₃)₃, -O-CH₂-CH₂-CH₂-CH₂-CH₃, -O-CH₂-CH₂-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
R¹³ und R¹⁴ jeweils für einen Wasserstoff-Rest stehen;
R¹⁵ für einen Rest ausgewählt aus der Gruppe bestehend aus Phenyl und Pyridinyl steht, wobei der Rest ggf. jeweils mit 1, 2, 3, 4 oder 5 Substituenten unabhängig voneinander ausgewählt aus der Gruppe bestehend aus F, Cl, Br, -CF₃, -O-CF₃, -S-CF₃, Methyl, Ethyl, n-Propyl, Isopropyl, n-Butyl, sec-Butyl, Isobutyl und tert-Butyl substituiert sein kann;
und
R¹⁶ für einen Rest ausgewählt aus der Gruppe bestehend aus Thiomorpholinyl, Azepanyl, Morpholinyl, (2,3)-Dihydro-1H-isoindolyl, (2,3)-Dihydro-indolyl, Piperidinyl und Pyrrolidinyl steht; wobei der Rest mit 1, 2 oder 3 Substituenten unabhängig voneinander ausgewählt aus der Gruppe bestehend aus -OH, Methyl, Ethyl, n-Propyl, Isopropyl, n-Butyl, sec-Butyl, Isobutyl, tert-Butyl, Benzyl und Phenyl substituiert sein kann; wobei jeweils der zyklische Teil der Reste Benzyl und Phenyl mit 1, 2, 3, 4 oder 5 Substituenten unabhängig voneinander ausgewählt aus der Gruppe bestehend aus F, Cl, Br und -CF₃ substituiert sein kann;
oder für einen der folgenden Reste steht 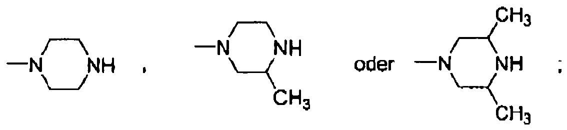 wobei das Wasserstoffatom der-NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
jeweils ggf. In Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

13. Verwendung einer Verbindung gemäß einem oder mehreren der Ansprüche 1 bis 12, **dadurch gekennzeichnet, dass**
R¹ für einen der folgenden Reste steht 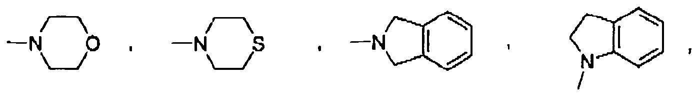 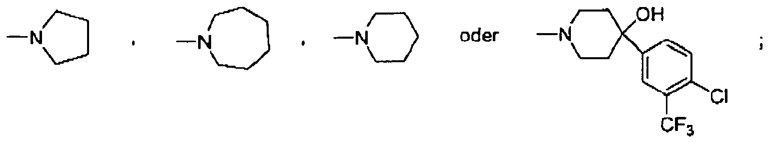 für den folgenden Rest steht 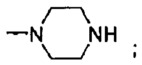 wobei das Wasserstoffatom der -NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅. -O-CF₃ und -S-CF₃ substituiert sein kann;
für einen Rest ausgewählt aus der Gruppe bestehend aus Phenyl und Naphthyl steht, wobei der Rest jeweils ggf. mit 1, 2, 3, 4 oder 5 Substituenten unabhängig voneinander ausgewählt aus der Gruppe bestehend aus -OH, -O-CH₃ und -O-C₂H₅ substituiert sein kann;
für eine NR³R⁴-Gruppe steht
oder für eine NR⁷-C(=O)-NR⁸R⁹ Gruppe steht;
R² für einen 1,3-Dihydro-indol-2-onyl- oder einen 1,3-Dihydro-indol-2-thionyl-Rest steht;
für CH-X steht;
für CH-CH₂-Y, CH-CH(CH₃)-Y oder CH-CH(OH)-Y steht;
oder für CH-CH=C(CH₃)-Z, CH-CH=CH-Z, CH-CH=CH-S-Z, CH-C(CH₃)=CH-Z, CH-C(Phenyl)=CH-Z, CH-CBr=CH-Z. CH-CCl=CH-Z, CH-CF=CH-Z, CH-CH₂-CH₂-Z, CH-CH₂-CH(CH₃)-Z oder CH-CH(CH₃)-CH₂-Z steht;
R³ für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1 )-Dimethyl-propyl, n-Pentyl, sec-Pentyl, n-Hexyl und n-Heptyl steht;
oder für einen Rest ausgewählt aus der Gruppe bestehend aus Phenyl, Naphthyl, Benzyl, (1,3)-Benzodioxolyl und (1,4)-Benzodioxanyl steht; wobei der Rest jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
R⁴ für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
R⁷ und R⁸ jeweils für einen Wasserstoff-Rest stehen;
R⁹ für einen Phenyl-Rest steht, der mit 1, 2, 3, 4 oder 5 Substituenten unabhängig voneinander ausgewählt aus der Gruppe bestehend aus F, Cl, Br und -CF₃ substituiert sein kann;
X für einen Rest ausgewählt aus der Gruppe bestehend aus Phenyl, Naphthyl, Phenanthrenyl, (1,3)-Benzodioxolyl, (1,4)-Benzodioxanyl, Thiophenyl, Furanyl, Pyridinyl, Pyridazinyl, Pyrazinyl, Pyrimidinyl, Chinolinyl und Isochinolinyl steht, der mit 1, 2, 3, 4 oder 5 gleichen oder verschiedenen Substituenten R¹⁰ substituiert sein kann;
Y für einen Phenyl- oder Naphthyl-Rest steht, der mit 1, 2, 3 4 oder 5 gleichen oder verschiedenen Substituenten R¹¹ substituiert sein kann;
Z für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl und n-Octyl steht;
oder für einen Phenyl- oder Naphthyl-Rest steht, der mit 1, 2, 3, 4 oder 5 gleichen oder verschiedenen Substituenten R¹² substituiert sein kann;
R¹⁰ für einen Rest ausgewählt aus der Gruppe bestehend aus F, Cl, Br, I, - CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH-CH₂, -NH₂, -O-CF₃, -S-CF₃, Methyl, Ethyl, n-Propyl, Isopropyl, n-Butyl, sec-Butyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -N(CH₃)₂, -N(C₂H₅)₂, -NH-CH₃, -NH-C₂H₅, -NH-C(=O)-CH₃, -O-S(=O)₂-Phenyl, -O-Phenyl, -O-Benzyl und Phenyl steht;
für eine -C(=4)-NR¹³-(CH₂)ₘ-NR¹⁴-R¹⁴-Gruppe mit m gleich 0, 1, 2 oder 3 steht;
oder für eine -C(=O)-R¹⁶-Gruppe steht;
R¹¹ für einen Rest ausgewählt aus der Gruppe bestehend aus F, Cl, Br, I, - CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, - SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
R¹² für einen Rest ausgewählt aus der Gruppe bestehend aus F, Cl, Br, I, - CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
R¹³ und R¹⁴ jeweils für einen Wasserstoff-Rest stehen;
R¹⁶ für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CF₃, -S-CF₃, Methyl, Ethyl, n-Propyl, Isopropyl, n-Butyl, sec-Butyl, Isobutyl und tert-Butyl substituiert sein kann;
und
R¹⁶ für einen der folgenden Reste steht 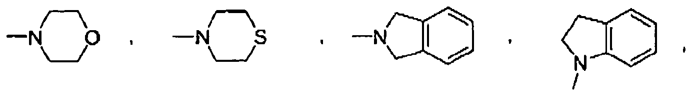 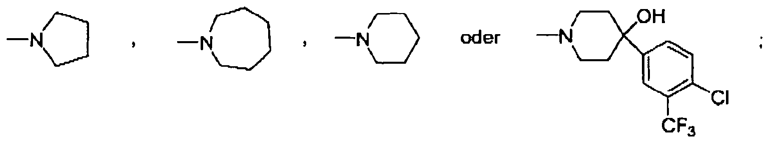 oder für einen der folgenden Reste steht 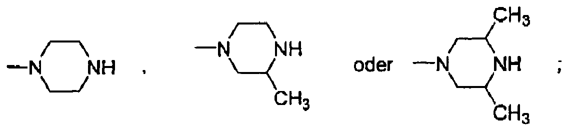 wobei das Wasserstoffatom der-NH-Gruppe durch einen Substituenten ausgewählt aus der Gruppe bestehend aus Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

14. Verwendung einer Verbindung gemäß einem oder mehreren der Ansprüche 1 bis 13 ausgewählt aus der Gruppe bestehend aus
| | |
|---|---|
| 1 | 5-[3-(4-Methoxy-phenyl)-but-2-enyliden]-2-morpholin-4-yl-thiazol-4-on |
| 2 | 2-Morpholin-4-yl-5-phenethyliden-thiazol-4-on |
| 3 | 2-Morpholin-4-yl-5-(2-o-tolyl-ethyliden)-thiazol-on |
| 4 | 2-Morpholin-4-yl-5-(3-phenyl-allyliden)-thiazol-4-on |
| 5 | 2-Morpholin-4-yl-5-(3-phenyl-butyliden)-thiazol-4-on |
| 6 | 2-Morpholin-4-yl-5-(3-phenyl-propyliden)-thiazol-4-on |
| 7 | 5-[3-(4-Fluor-phenyl)-allyliden]-2-morpholin-4-yl-thiazol-4-on |
| 8 | N-[4-(2-Morpholin-4-yl-4-oxo-4H-thiazol-5-ylidenmethyl)-phenyl]-acetamid |
| 9 | 5-Benzo[1,3]dioxol-5-ylmethylen-2-morpholin-4-yl-thiazol-4-on |
| 10 | 2-Morpholin-4-yl-5-(3-m-tolyl-allyliden)-thiazol-4-on |
| 11 | 5-(3-Iodo-4,5-dimethoxy-benzyliden)-2-morpholino-4-yl-thiazol-4-on |
| 12 | 2-Morpholin-4-yl-5-(3-phenylsulfanyl-allyliden)-thiazol-4-on |
| 13 | 5-(3-Benzyloxy-4-methoxy-benzyliden)-2-morpholin-4-yl-thiazol-4-on |
| 14 | 5-(4-Methylbenzyliden)-2-thiomorpholin-4-yl-thiazol-4-on |
| 15 | N-[4-(2-Morpholin-4-yl-4-oxo-4H-thiazol-5-ylidenmethyl)-phenyl]-amin |
| 16 | 5-(4-Butoxy-benzyliden)-2-morpholin-4-yl-thiazol-4-on |
| 17 | 2-Morpholin-4-yl-5-(4-phenoxy-benzyliden)-thiazol-4-on |
| 18 | 2-Morpholin-4-yl-5-(3-phenoxy-benzyliden)-thiazol-4-on |
| 19 | 2-Morpholin-4-yl-5-(3-p-tolyl-allyliden)-thiazol-4-on |
| 20 | 5-(3-Benzyloxy-benzyliden)-2-morpholin-4-yl-thiazol-4-on |
| 21 | 5-(4-Benzyloxy-benzyliden)-2-morpholin-4-yl-thiazol-4-on |
| 22 | 2-Thiomorpholin-4-yl-5-(2-trifluormethyl-benzyliden)-thiazol-4-on |
| 23 | 5-(4-Brom-benzyliden)-2-thiomorpholin-4-yl-thiazol-4-on |
| 24 | 2-Thiomorpholin-4-yl-5-(4-trifluormethoxy-benzyliden)-thiazol-4-on |
| 25 | 5-(3-Phenyl-allyliden)-2-thiomorpholin-4-yl-thiazol-4-on |
| 26 | 2-Morpholin-4-yl-5-octyliden-thiazol-4-on |
| 27 | 5-(4-Benzyloxy-3-methoxy-benzyliden)-2-morpholin-4-yl-thiazol-4-on |
| 28 | 5-(3,4-Bis-benzyloxy-benzyliden)-2-morpholin-4-yl-thiazol-4-on |
| 29 | 5-Butyliden-2-morpholin-4-yl-thiazol-4-on |
| 30 | 2-[4-(3-Chlor-pyridin-2-yl)-piperazin-1-yl]-5-(4-methyl-benzyliden)-thiazol-4-on |
| 31 | 2-[4-(3-Chlor-pyridin-2-yl)-piperazin-1-yl]-5-(3-phenyl-allyliden)-thiazol-4-on |
| 32 | 5-Hexyliden-2-morpholin-4-yl-thiazol-4-on |
| 33 | 2-Morpholin-4-yl-5-(3-p-tolyl-but-2-enyliden)-thiazol-4-on |
| 34 | 5-[3-(4-tert-Butyl-phenyl)-but-2-enyliden]-2-morpholin-4-yl-thiazol-4-on |
| 35 | 5-(4-Methoxy-naphthalen-1-ylmethylen)-2-morpholin-4-yl-thiazol-4-on |
| 36 | 2-Morpholin-4-yl-5-(4-trifluorrnethoxy-benzyliden)-thiazol-4-on |
| 37 | 2-[4-(3-Methoxy-phenyl)-piperazin-1-yl]-5-(3-phenyl-allyliden)-thiazol-4-on |
| 38 | 5-[3-(4-tert-Butyl-phenyl)-allyliden]-2-[4-(3-methoxy-phenyl)-piperazin-1-yl]-thiazol-4-on |
| 39 | 5-(4-Hydroxy-3-methoxy-benzyliden)-2-thiomorpholin-4-yl-thiazol-4-on |
| 40 | 5-[3-(4-Hydroxy-3-methoxy-phenyl)-allyliden]-2-thiomorpholin-4-yl-thiazol-4-on |
| 41 | 5-Naphthalen-1-ylmethylen-2-thiomorpholin-4-yl-thiazol-4-on |
| 42 | 5-Benzyliden-2-thiomorpholin-4-yl-thiazol-4-on |
| 43 | 2-Morpholin-4-yl-5-[3-(4-trifluoromethyl-phenyl)-but-2-enyliden]-thiazol-4-on |
| 44 | 2-Morpholin-4-yl-5-[3-(3-trifluormethyl-phenyl)-but-2-enyliden]-thiazol-4-on |
| 45 | 5-(3-Methyl-butyliden)-2-morpholin-4-yl-thiazol-4-on |
| 46 | 2-[4-(3-Chlor-pyridin-2-yl)-piperazin-1-yl]-5-(2-trifluormethyl-benzyliden)-thiazol-4-on |
| 47 | 5-(4-Brom-benzyliden)-2-[4-(3-chlor-pyridin-2-yl)-piperazin-1-yl]-thiazol-4-on |
| 48 | 5-Benzyliden-2-[4-(3-chlor-pyridin-2-yl)-piperazin-1-yl]-thiazol-4-on |
| 49 | 2-[4-(3-Chlor-pyridin-2-yl)-piperazin-1-yl]-5-naphthalen-2-ylmethylen-thiazol-4-on |
| 50 | 5-(2,3-Diphenyl-allyliden)-2-morpholin-4-yl-thiazol-4-on |
| 51 | 2-Morpholin-4-yl-5-phenanthren-9-ylmethylen-thiazol-4-on |
| 52 | 2-Morpholin-4-yl-5-chinolin-3-ylmethylen-thiazol-4-on |
| 53 | 2-(4-Ethyl-piperazin-1-yl)-5-(4-methyl-benzyliden)-thiazol-4-on |
| 54 | 5-Naphthalen-2-ylmethylen-2-thiomorpholin-4-yl-thiazol-4-on |
| 55 | 2-[4-(4-Methoxy-phenyl)-piperazin-1-yl]-5-(3-phenyl-allyliden)-thiazol-4-on |
| 56 | 2-[4-(2-Chlor-phenyl)-piperazin-1-yl]-5-(3-phenyl-allyliden)-thiazol-4-on |
| 57 | 5-(3-Phenyl-allyliden)-2-(4-phenyl-piperazin-1-yl)-thiazol-4-on |
| 58 | 5-Decyliden-2-morpholin-4-yl-thiazol-4-on |
| 59 | 5-[3-(4-tert-Butyl-phenyl)-allyliden]-2-morpholin-4-yl-thiazol-4-on |
| 60 | 2-(2-Morpholin-4-yl-4-oxo-4H-thiazol-5-ylidenmethyl)-benzoesäure |
| 61 | 5-(2-Brom-3-phenyl-allyliden)-2-[4-(3-chlor-pyridin-2-yl)-piperazin-1-yl]-thiazol-4-on |
| 62 | 5-[3-(4-tert-Butyl-phenyl)-allyliden]-2-[4-(3-chlor-pyridin-2-yl)-piperazin-1-yl]-thiazol-4-on |
| 63 | 2-(4-Benzyl-piperazin-1-yl)-5-(4-methyl-benzyliden)-thiazol-4-on |
| 64 | 2-(4-Phenyl-piperazin-1-yl)-5-chinolin-3-ylmethylen-thiazol-4-on |
| 65 | 2-[4-(3-Chlor-pyridin-2-yl)-piperazin-1-yl]-5-(2-methyl-3-phenyl-allyliden)-thiazol-4-on |
| 66 | 2-[4-(3-Chlor-pyridin-2-yl]-5-piperazin-1-yl]-5-naphthalen-1-ylmethylen-thiazol-4-on |
| 67 | 5-(2-Chlor-3-phenyl-allyliden)-2-[4-(3-chlor-pyridin-2-yl)-piperazin-1-yl]-thiazol-4-on |
| 68 | 5-(4-Hydroxy-3-methoxy-benzyliden)-2-(4-phenyl-piperazin-1-yl)-thiazol-4-on |
| 69 | 5-Phenanthren-9-ylmethylen-2-(4-phenyl-piperazin-1-yl)-thiazol-4-on |
| 70 | 5-(6-Methoxy-naphthalen-2-ylmethylen)-2-(4-phenyl-piperazin-1-yl)-thiazol-4-on |
| 71 | 5-Naphthalen-1-ylmethylen-2-(4-phenyl-piperazin-1-yl)-thiazol-4-on |
| 72 | 5-(4-Brom-benzyliden)-2-(4-phenyl-piperazin-1-yl)-thiazol-4-on |
| 73 | 5-(4-Methyl-benzyliden)-2-(4-phenyl-piperazin-1-yl)-thiazol-4-on |
| 74 | 2-(4-Phenyl-piperazin-1-yl)-5-(2-trifluormethyl-benzyliden)-thiazol-4-on |
| 75 | 3-(4-Oxo-2-thiomorpholin-4-yl-4H-thiazol-5-yliden)-1,3-dihydro-indol-2-on |
| 76 | 3-(2-Morpholin-4-yl-4-oxo-4H-thiazol-5-yliden)-1,3-dihydro-indol-2-on |
| 77 | 2-[4-(2-Fluor-phenyl)-piperazin-1-yl]-5-(4-methyl-benzyliden)-thiazol-4-on |
| 78 | 5-(4-tert-Butyl-benzyliden)-2-[4-(2-fluor-phenyl)-piperazin-1-yl]-thiazol-4-on |
| 79 | 2-[4-(2-Fluor-phenyl)-piperazin-1-yl]-5-(3-phenyl-allyliden)-thiazol-4-on |
| 80 | 5-[3-(4-tert-Butyl-phenyl)-allyliden]-2-[4-(2-fluor-phenyl)-piperazin-1-yl]-thiazol-4-on |
| 81 | 2-[4-(2-Fluor-phenyl)-piperazin-1-yl]-5-(4-trifluormethyl-benzyliden)-thiazol-4-on |
| 82 | 2-[4-(2-Fluor-phenyl)-piperazin-1-yl]-5-(4-trifluormethoxy-benzyliden)-thiazol-4-on |
| 83 | 5-(2-Chlor-3-phenyl-allyliden-2-[4-(2-fluor-phenyl)-piperazin-1-yl]-thiazol-4-on |
| 84 | 2-[4-(2-Fluor-phenyl)-piperazin-1-yl]-5-naphthalen-1-ylmethylen-thiazol-4-on |
| 85 | 5-(2-Chlor-3-phenyl-allyliden)-2-thiomorpholin-4-yl-thiazol-4-on |
| 86 | 3-(4-Oxo-2-thiomorpholin-4-yl-4H-thiazol-5-ylidenmethyl)-benzoesäure |
| 87 | 4-(4-Oxo-2-thiomorpholin-4-yl)-4H-thiazol-5-ylidenmethyl)-benzoesäure |
| 88 | 5-{3-[4-(3-Chlor-pyridin-2-yl)-piperazin-1-carbonyl]-benzyliden}-2-thiomorpholin-4-yl-thiazol-4-on |
| 89 | 5-{4-[4-(3-Chlor-pyridin-2-yl)-piperazin-1-carbonyl]-benzyliden}-2-thiomorpholin-4-yl-thiazol-4-on |
| 90 | 2-[4-(3-Chlor-pyridin-2-yl)-piperazin-1-yl]-5-chinolin-3-ylmethylen-thiazol-4-on |
| 91 | 2-[4-(3-Chlor-pyridin-2-yl)-piperazin-1-yl]-5-(4-trifluormethyl-benzyliden)-thiazol-4-on |
| 92 | 2-(2,3-Dihydro-indol-1-yl)-5-(4-methyl-benzyliden)-thiazol-4-on |
| 93 | 2-(2,3-Dihydro-indol-1-yl)-5-(4-trifluormethyl-benzyliden)-thiazol-4-on |
| 94 | 5-[4-(Morpholin-4-carbonyl)-benzyliden]-2-thiomorpholin-4-yl-thiazol-4-on |
| 95 | 2-[4-(3-Chlor-pyridin-2-yl)-piperazin-1-yl]-5-(4-hydroxy-3-methoxy-benzyliden)-thiazol-4-on |
| 96 | 1-[4-Oxo-5-(4-trifluormethyl-benzyliden)-4,5-dihydro-thiazol-2-yl]-3-(2-trifluormethyl-phenyl)-harnstoff |
| 97 | 2-(4-Benzyl-piperazin-1-yl)-5-chinolin-3-ylmethylen-thiazol-4-on |
| 98 | 2-(4-Benzyl-piperazin-1-yl)-5-naphthalen-2-ylmethylen-thiazol-4-on |
| 99 | 2-(4-Benzyl-piperazin-1-yl)-5-(3-phenoxy-benzyliden)-thiazol-4-on |
| 100 | 2-(4-Benzyl-piperazin-1-yl)-5-(4-trifluormethyl-benzyliden)-thiazol-4-on |
| 101 | 1-[5-(4-Methyl-benzyliden)-4-oxo-4,5-dihydro-thiazol-2-yl]-3-(2-trifluormethyl-phenyl)-harnstoff |
| 102 | 2-(4-Benzyl-piperazin-1-yl)-5-naphthalen-1-ylmethylen-thiazol-4-on |
| 103 | 2-(4-Benzyl-piperazin-1-yl)-5-(6-methoxy-naphthalen-2-ylmethylen)-thiazol-4-on |
| 104 | 2-(4-Benzyl-piperazin-1-yl)-5-(4-tert-butyl-benzyliden)-thiazol-4-on |
| 105 | 1-[4-Oxo-5-(3-phenyl-allyliden)-4,5-dihydro-thiazol-2-yl]-3-(2-trifluormethyl-phenyl)-harnstoff |
| 106 | 1-[4-Oxo-5-(4-trifluormethyl-benzyliden)-4,5-dihydro-thiazol-2-yl]-3-(4-trifluormethyl-phenyl)-harnstoff |
| 107 | 1-[5-(4-Methyl-benzyliden)-4-oxo-4,5-dihydro-thiazol-2-yl]-3-(4-trifluormethyl-phenyl)-harnstoff |
| 108 | 5-{3-[4-(6-Chlor-pyridin-2-yl)-piperazin-1-carbonyl]-benzyliden}-2-thiomorpholin-4-yl-thiazol-4-on |
| 109 | 2-(4-Benzyl-piperazin-1-yl)-5-(4-trifluormethoxy-benzyliden)-thiazol-4-on |
| 110 | 5-{3[4-(3-Chlor-pyridin-2-yl)-3-methyl-piperazin-1-carbonyl]-benzyliden}-2-thiomorpholin-4-yl-thiazol-4-on |
| 111 | N-[3-(3-Chlor-5-trifluormethyl-pyridin-2-ylamino)-propyl]-3-(4-oxo-2-thiomorpholin-4-yl-4H-thiazol-5-ylidenmethyl)-benzamid |
| 112 | 3-(4-Oxo-2-thiomorpholin-4-yl-4H-thiazol-5-ylidenmethyl)-benzoesäure-N'-(2-chlor-4-trifluormethyl-phenyl)-hydrazid |
| 113 | 5-{3-[4-(3-Chlor-5-trifluormethyl-pyridin-2-yl)-piperazin-1-carbonyl]-benzyliden}-2-thiomorpholin-4-yl-thiazol-4-on |
| 114 | 3-(4-Oxo-2-thiomorpholin-4-yl-4H-thiazol-5-ylidenmethyl)-benzoesäure-N'-(3-chlor-5-trifluormethyl-pyridin-2-yl-hydrazid |
| 115 | 5-{3-[4-(4-Chlor-[1,2,5]thiadiazol-3-yl)-piperazin-1-carbonyl]-benzyliden}-2-thiomorpholin-4-yl-thiazol-4-on |
| 116 | 3-(4-Oxo-2-thiomorpholin-4-yl-4H-thiazol-5-ylidenmethyl)-benzoesäure-N'-(2-chlor-5-trifluormethyl-phenyl)-hydrazid |
| 117 | 2-[4-(4-Chlor-[1,2,5]thiadiazol-3-yl)-piperazin-1-yl]-5-(4-methyl-benzyliden)-thiazol-4-on |
| 118 | 2-[4-(4-Chlor-[1,2,5]thiadiazol-3-yl)-piperazin-1-yl]-5-(4-trifluormethyl-benzyliden)-thiazol-4-on |
| 119 | 2-[4-(4-Chlor-[1,2,5]thiadiazol-3-yl)-piperazin-1-yl]-5-(4-trifluomethoxy-benzyliden)-thiazol-4-on |
| 120 | 5-(4-tert-Butyl-benzyliden)-2-[4-(4-chlor-[1,2,5]thiadiazol-3-yl)-piperazin-1-yl]-thiazol-4-on |
| 121 | 2-[4-(4-Chlor-[1,2,5]thiadiazol-3-yl)-piperazin-1-yl]-5-(3-phenyl-allyliden)-thiazol-4-on |
| 122 | 5-[3-(4-tert-Butyl-phenyl)-allyliden]-2-[4-(4-chlor-[1,2,5]thiadiazol-3-yl)-piperazin-1-y1]-thiazol-4-on |
| 123 | 2-[4-(4-Chlor-[1,2,5]thiadiazol-3-yl)-piperazin-1-yl]-5-naphthalen-1-ylmethylen-thiazol-4-on |
| 124 | 2-[4-(4-Chlor-[1,2,5]thiadiazol-3-yl)-piperazin-1-yl]-5-(3-phenoxy-benzyliden)-thiazol-4-on |
| 125 | 2-[4-(4-Chlor-[1,2,5]thiadiazol-3-yl)-piperazin-1-yl]-5-(4-phenoxy-benzyliden)-thiazol-4-on |
| 126 | 5-(3-Benzyloxy-benzyliden)-2-[4-(4-chlor-[1,2,5]thiadiazol-3-yl)-piperazin-1-yl]-thiazol-4-on |
| 127 | 2-[4-(4-Chlor-[1,2,5]thiadiazol-3-yl)-piperazin-1-yl]-5-(4-hydroxy-3-methoxy-benzyliden)-thiazol-4-on |
| 128 | 2-[4-(4-Chlor-[1,2,5]thiadiazol-3-yl)-piperazin-1-yl]-5-furan-2-ylmethylen-thiazol-4-on |
| 129 | 2-[4-(4-Chlor-[1,2,5]thiadiazol-3-yl)-piperazin-1-yl]-5-naphthalen-2-ylmethylen-thiazol-4-on |
| 130 | 3-[2-(4-Benzyl-piperazin-1-yl)-4-oxo-4H-thiazol-5-ylidenmethyl]-benzoesäure |
| 131 | 5-Naphthalen-1-ylmethylen-2-piperazin-1-yl-thiazol-4-on |
| 132 | 2-Piperazin-1-yl-5-(4-trifluormethyl-benzyliden)-thiazol-4-on |
| 133 | 6-(4-Methyl-benzyliden)-2-piperazin-1-yl-thiazol-4-on |
| 134 | 5-(4-Hydroxy-3-methoxy-benzyliden)-2-piperazin-1-yl-thiazol-4-on |
| 135 | 5-(4-Isopropyl-benzyliden)-2-morpholin-4-yl-thiazol-4-on |
| 136 | 5-(4-Isopropyl-benzyliden)-2-thiomorpholin-4-yl-thiazol-4-on |
| 137 | 2-[4-(2-Fluor-phenyl)-piperazin-1-yl]-5-(4-isopropyl-benzyliden)-thiazol-4-on |
| 138 | 5-(4-Pentafluorsulfanyl)-benzyliden)-2-thiomorpholin-4-yl-thiazol-4-on |
| 139 | 2-[4-(3-Chlor-pyridin-2-yl)-piperazin-1-yl]-5-(4-isopropyl-benzyliden)-thiazol-4-on |
| 140 | 2-(4-Benzyl-piperazin-1-yl)-5-(4-butyl-benzyliden)-thiazol-4-on |
| 141 | 2-(4-Benzyl-piperazin-1-yl)-5-(4-pentyl-benzyliden)-thiazol-4-on |
| 142 | 2-(4-Benzyl-piperazin-1-yl)-5-(4-octyl-benzyliden)-thiazol-4-on |
| 143 | 2-[4-(6-Chlor-pyridin-2-yl)-piperazin-1-yl]-5-(4-methyl-benzyliden)-thiazol-4-on |
| 144 | 5-(4-tert-Butyl-benzyliden)-2-[4-(6-chlor-pyridin-2-yl)-piperazin-1-yl]-thiazol-4-on |
| 145 | 2-[4-(6-Chlor-pyridin-2-yl)-piperazin-1-yl]-5-(4-trifluormethyl-benzyliden)-thiazol-4-on |
| 146 | 2-[4-(6-Chlor-pyridin-2-yl)-piperazin-1-yl]-5-(3-phenyl-allyliden)-thiazol-4-on |
| 147 | 5-[3-(4-tert-Butyl-phenyl)-allyliden]-2-[4-(6-chlor-pyridin-2-yl)-piperazin-1-yl]-thiazol-4-on |
| 148 | 2-[4-(6-Chlor-pyridin-2-yl)-piperazin-1-yl]-5-(4-hydroxy-3-methoxy-benzyliden)-thiazol-4-on |
| 149 | 2-[4-(6-Chlor-pyridin-2-yl)-piperazin-1-yl]-5-(4-isopropyl-benzyliden)-thiazol-4-on |
| 150 | 2-[4-(6-Chlor-pyridin-2-yl)-piperazin-1-yl]-5-(4-trifluormethoxy-benzyliden)-thiazol-4-on |
| 151 | 2-[4-(6-Chlor-pyridin-2-yl)-piperazin-1-yl]-5-naphthalen-2-ylmethylen-thiazol-4-on |
| 152 | 5-(4-Methyl-benzyliden)-2-[4-(6-methyl-pyridazin-3-yl)-piperazin-1-yl]-thiazol-4-on |
| 153 | 5-(4-Isopropyl-benzyliden)-2-[4-(6-methyl-pyridazin-3-yl)-piperazin-1-yl]-thiazol-4-on |
| 154 | 5-(4-tert-Butyl-benzyliden)-2-[4-(6-methyl-pyridazin-3-yl)-piperazin-1-yl]-thiazol-4-on |
| 155 | 5-[3-(4-tert-Butyl-phenyl)-allyliden]-2-[4-(6-methyl-pyridazin-3-yl)-piperazin-1-yl]-thiazol-4-on |
| 156 | 2-[4-(6-Methyl-pyridazin-3-yl)-piperazin-1-yl]-5-(4-trifluormethoxy-benzyliden)-thiazol-4-on |
| 157 | 5-(4-Hydroxy-3-methoxy-benzyliden)-2-[4-(6-methyl-pyridazin-3-yl)-piperazin-1-yl]-thiazol-4-on |
| 158 | 2-(4-Benzyl-piperazin-1-yl)-5-(4-pentafluorsulfanyl-benzyliden)-thiazol-4-on |
| 159 | 2-(4-Benzyl-piperazin-1-yl)-5-{3-[4-(4-chlor-[1,2,5]thiadiazol-3-yl)-piperazin-1-carbonyl]-benzyliden}-thiazol-4-on |
| 160 | 2-[4-(6-Methyl-pyridazin-3-yl)-piperazin-1-yl]-5-(4-trifluormethyl-benzyliden)-thiazol-4-on |
| 161 | 2-[4-(6-Methyl-pyridazin-3-yl)-piperazin-1-yl]-5-(3-phenyl-allyliden)-thiazol-4-on |
| 162 | 2-[4-(6-Methyl-pyridazin-3-yl)-piperazin-1-yl]-5-naphthalen-2-ylmethylen-thiazol-4-on |
| 163 | 2-[4-(6-Methyl-pyridazin-3-yl)-piperazin-1-yl]-5-naphthalen-1-ylmethylen-thiazol-4-on |
| 164 | 2-(4-Benzyl-piperazin-1-yl)-5-(4-isopropyl-benzyliden)-thiazol-4-on |
| 165 | 3-{2-[4-(4-Chlor-[1,2,5]thiadiazol-3-yl)-piperazin-1-yl]-4-oxo-4H-thiazol-5-ylidenmethyl}-benzoesäure |
| 166 | 5-{3-[4-(4-Chlor-3-trifluormethyl-phenyl)-4-hydroxy-piperidin-1-carbonyl]-benzyliden}-2-thiomorpholin-4-yl-thiazol-4-on |
| 167 | 5-{3-[4-(4-Chlor-[1,2,5]thiadiazol-3-yl)-piperazin-1-carbonyl]-benzyliden}-2-[4-(4-chlor-[1,2,5]thiadiazol-3-yl)-piperazin-1-yl]-thiazol-4-on |
| 168 | 5-{3-[4-(3-Chlor-pyridin-2-yl)-piperazin-1-carbonyl]-benzyliden}-2-[4-(4-chlor-[1,2,5]thiadiazol-3-yl)-piperazin-1-yl]-thiazol-4-on |
| 169 | 5-[3-(4-Benzyl-piperazin-1-carbonyl)-benzyliden]-2-[4-(4-chlor-[1,2,5]thiadiazol-3-yl)-piperazin-1-yl]-thiazol-4-on |
| 170 | 2-[4-(4-Chlor-[1,2,5]thiadiazol-3-yl)-piperazin-1-yl]-5-{3-[4-(6-methoxy-pyridin-2-yl)piperazin-1-carbonyl]-benzyliden}-thiazol-4-on |
| 171 | 2-[4-(4-Chlor-3-trifluormethyl-phenyl)-4-hydroxy-piperidin-1-yl]-5-(4-methyl-benzyliden)-thiazol-4-on |
| 172 | 2-[4-(4-Chlor-3-trifluormethyl-phenyl)-4-hydroxy-piperidin-1-yl]-5-(4-hydroxy-3-methoxy-benzyliden)-thiazol-4-on |
| 173 | 2-[4-(4-Chlor-3-trifluormethyl-phenyl)-4-hydroxy-piperidin-1-yl]-5-naphthalen-1-ylmethylen-thiazol-4-on |
| 174 | 2-[4-(4-Chlor-3-trifluormethyl-phenyl)-4-hydroxy-piperidin-1-yl]-5-(3-methoxy-benzyliden)-thiazol-4-on |
| 175 | 5-Benzyliden-2-morpholin-4-yl-thiazol-4-on |
| 176 | 5-(4-Methyl-benzyliden)-2-morpholin-4-yl-thiazol-4-on |
| 177 | 2-Diethylamino-5-(4-methyl-benzyliden)-thiazol-4-on |
| 178 | 5-(4-Chlor-benzyliden)-2-diethylamino-thiazol-4-on |
| 179 | 5-(4-Isopropyl-benzyliden)-2-morpholin-4-yl-thiazol-4-on |
| 180 | 5-Benzyliden-2-diethylamino-thiazol-4-on |
| 181 | 5-(4-Chlor-benzyliden)-2-morpholin-4-yl-thiazol-4-on |
| 182 | 5-(4-Chlor-benzyliden)-2-piperidin-1-yl-thiazol-4-on |
| 183 | 5-(4-Chlor-benzyliden)-2-pyrrolidin-1-yl-thiazol-4-on |
| 184 | 5-Benzyliden-2-pyrrolidin-1-yl-thiazol-4-on |
| 185 | 5-Benzyliden-2-pyrrolidin-1-yl-thiazol-4-on |
| 186 | 5-Biphenyl-4-ylmethylen-2-piperidin-1-yl-thiazol-4-on |
| 187 | 2-Azepan-1-yl-5-biphenyl-4-ylmethylen-thiazol-4-on |
| 188 | 5-Biphenyl-4-ylmethylen-2-pyrrolidin-1-yl-thiazol-4-on |
| 189 | 2-Morpholin-4-yl-5-(3-phenyl-allyliden)-thiazol-4-on |
| 190 | 5-(3-Phenyl-allyliden)-2-thiomorpholin-4-yl-thiazol-4-on |
| 191 | 5-(3-Phenyl-allyliden)-2-piperidin-1-yl-thiazol-4-on |
| 192 | 2-Azepan-1-yl-5-(3-phenyl-allyliden)-thiazol-4-on |
| 193 | 5-(3-Phenyl-allyliden)-2-pyrrolidin-1-yl-thiazol-4-on |
| 189 | 5-Biphenyl-4-ylmethylen-2-morpholin-4-yl-thiazol-4-on |
| 190 | 2-Thiomorpholin-4-yl-5-(3-trifluormethyl-benzyliden)-thiazol-4-on |
| 191 | 5-(4-Chlor-benzyliden)-2-thiomorpholin-4-yl-thiazol-4-on |
| 192 | 5-(4-tert-Butyl-benzyliden)-2-morpholin-4-yl-thiazol-4-on |
| 193 | 5-(4-tert-Butyl-benzyliden)-2-thiomorpholin-4-yl-thiazol-4-on |
| 194 | 5-(4-tert-Butyl-benzyliden)-2-pyrrolidin-1-yl-thiazol-4-on |
| 200. | 2-Azepan-1-yl-5-(4-tert-butyl-benzyliden)-thiazol-4-on |
| 201. | 5-(4-tert-Butyl-benzyliden)-2-piperidin-1-yl-thiazol-4-on |
| 202. | 2-Thiomorpholin-4-yl-5-(4-trifluormethyl-benzyliden)-thiazol-4-on |
| 203. | 5-(4-tert-Butyl-benzyliden)-2-diethylamino-thiazol-4-on |
| 204. | 2-Morpholin-4-yl-5-(4-trifluormethyl-benzyliden)-thiazol-4-on |
| 205. | 2-Morpholin-4-yl-5-(3-trifluormethyl-benzyliden)-thiazol-4-on |
| 206. | 2-Morpholin-4-yl-5-(3-trifluormethyl-benzyliden)-thiazol-4-on |
| 207. | 5-Biphenyl-4-ylmethylen-2-diethylamino-thiazol-4-on |
| 208. | 2-Morpholin-4--yl-5-thiophen-2-ylmethylen-thiazol-4-on |
| 209. | 2-Morpholin-4-yl-5-naphthalen-1-ylmethylen-thiazol-4-on |
| 210. | 2-Morpholin-4-yl-5-pyridin-2-ylmethylen-thiazol-4-on |
| 211. | 2-Morpholin-4-yl-5-(4-trifluormethyl-benzyliden)-thiazol-4-on |
| 212. | 2-Morpholin-4-yl-5-pyridin-3-ylmethylen-thiazol-4-on |
| 213. | 2-Morpholin-4-yl-5-pyridin-4-ylmethylen-thiazol-4-on |
| 214. | 2-Diethylamino-5-(3-trifluormethyl-benzyliden)-thiazol-4-on |
| 215. | 5-(4-Methyl-benzyliden)-2-pyrrolidin-1-yl-thiazol-4-on |
| 216. | 5-(4-tert-Butyl-benzyliden)-2-(2-methoxy-ethylamino)-thiazol-4-on |
| 217. | 2-Morpholin-4-yl-5-(4-octyl-benzyliden)-thiazol-4-on |
| 218. | 5-(4-Methyl-benzyliden)-2-morpholin-4-yl-thiazol-4-on |
| 219. | 5-(3,4-Dimethoxy-benzyliden)-2-morpholin-4-yl-thiazol-4-on |
| 220. | 5-(2-Hydroxy-benzyliden)-2-morpholin-4-yl-thiazol-4-on |
| 221. | 5-(2-Hydroxy-3-methoxy-benzyliden)-2-morpholin-4-yl-thiazol-4-on |
| 222. | 5-(4-tert-Butyl-benzyliden)-2-morpholin-4-yl-thiazol-4-on |
| 223. | 5-(4-Diethylamino-benzyliden)-2-morpholin-4-yl-thiazol-4-on |
| 224. | 5-(4-Hydroxy-3-methoxy-benzyliden)-2-morpholin-4-yl-thiazol-4-on |
| 225. | 4-(2-Morpholin-4-yl-4-oxo-4H-thiazol-5-ylidenmethyl)-benzoesäure |
| 226. | 2-Morpholin-4-yl-5-(2,3,4-trimethoxy-benzyliden)-thiazol-4-on |
| 227. | 2-Morpholin-4-yl-5-(3,4,5-trimethoxy-benzyliden)-thiazol-4-on |
| 228. | 5-(4-Methoxy-benzyliden)-2-morpholin-4-yl-thiazol-4-on |
| 229. | 5-(4-Hydroxy-benzyliden)-2-morpholin-4-yl-thiazol-4-on |
| 230. | 5-(3-Ethoxy-4-hydroxy-benzyliden)-2-morpholin-4-yl-thiazol-4-on |
| 231. | 5-(3-Hydroxy-benzyliden)-2-morpholin-4-yl-thiazol-4-on |
| 232. | 5-(4-Brom-benzyliden)-2-morpholin-4-yl-thiazol-4-on |
| 233. | 2-Morpholin-4-yl-5-(4-vinyloxy-benzyliden)-thiazol-4-on |
| 234. | Benzensulfonsäure-4-(2-morpholin-4-yl-4-oxo-4H-thiazol-5-ylidenmethyl)-phenylester |
| 235. | 5-(4-Dimethylamino-benzyliden)-2-morpholin-4-yl-thiazol-4-on |
| 236. | 4-(2-Morpholin-4-yl-4-oxo-4H-thiazol-5-ylidenmethyl)-benzoesäuremethylester |
| 237. | 5-(3-Hydroxy-4-methoxy-benzyliden)-2-morpholin-4-yl-thiazol-4-on |
| 238. | 5-[4-(3,3-Dimethyl-butoxy)-3-methoxy-benzyliden]-2-morpholin-4-yl-thiazol-4-on |
| 239. | 5-(2-Methoxy-benzyliden)-2-morpholin-4-yl-thiazol-4-on |
| 240. | 5-(4-Hydroxy-benzyliden)-2-morpholin-4-yl-thiazol-4-on |
| 241. | 2-[(Benzo[1,3]dioxol-5-ylmethyl)-amino]-5-[3-(4-tert-butyl-phenyl)-allyliden]-thiazol-4-on |
| 242 | 2-[(Benzo[1,3]dioxol-5-ylmethyl)-amino]-5-(4-trifluormethyl-benzyliden)-thiazol-4-on |
| 243. | 2-[(Benzo[1,3]dioxol-5-ylmethyl)-amino]-5-(4-trifluormethoxy-benzyliden)-thiazol-4-on |
| 244. | 2-[(Benzo[1,3]dioxol-5-ylmethyl)-amino]-5-naphthalen-1-ylmethylen-thiazol-4-on |
| 245. | 2-[(Benzo[1,3]dioxol-5-ylmethyl)-amino]-5-(2-chlor-3-phenyl-allyliden)-thiazol-4-on |
| 246. | 2-(4-Methyl-benzylamino)-5-naphthalen-1-ylmethylen-thiazol-4-on |
| 247. | 2-(4-Methyl-benzylamino)-5-(3-phenoxy-benzyliden)-thiazol-4-on |
| 248. | 2-(4-Methyl-benzylamino)-5-(4-phenoxy-benzyliden)-thiazol-4-on |
| 249. | 5-(2-Chlor-3-phenyl-allyliden)-2-(4-methyl-benzylamino)-thiazol-4-on |
| 250. | 2-(4-Methyl-benzylamino)-5-naphthalen-2-ylmethylen-thiazol-4-on |
| 251. | 2-(3-Methoxy-benzylamino)-5-(4-methyl-benzyliden)-thiazol-4-on |
| 252. | 5-(4-tert-Butyl-benzyliden)-2-(3-methoxy-benzylamino)-thiazol-4-on |
| 253. | 2-(3-Methoxy-benzylamino)-5-(4-trifluormethyl-benzyliden)-thiazol-4-on |
| 254. | 2-(3-Methoxy-benzylamino)-5-(4-trifluormethoxy-benzyliden)-thiazol-4-on |
| 255. | 2-(3-Methoxy-benzylamino)-5-(3-phenyl-allyliden)-thiazol-4-on |
| 256. | 5-Naphthalen-1-ylmethylen-2-(4-trifluormethyl-benzylamino)-thiazol-4-on |
| 257. | 5-(3-Phenoxy-benzyliden)-2-(4-trifluormethyl-benzylamino)-thiazol-4-on |
| 258. | 5-(4-Phenoxy-benzyliden)-2-(4-trifluormethyl-benzylamino)-thiazol-4-on |
| 259. | 5-(2-Chloro-3-phenyl-allyliden)-2-(4-trifluormethyl-benzylamino)-thiazol-4-on |
| 260. | 5-(3-Benzyloxy-benzyliden)-2-(4-trifluormethyl-benzylamino)-thiazol-4-on |
| 261. | 5-Naphthalen-2-ylmethylen-2-(4-trifluormethyl-benzylamino)-thiazol-4-on |
| 262. | 2-(4-Hydroxy-3-methoxy-phenyl)-5-(4-methyl-benzyliden)-thiazol-4-on und |
| 263. | 2-(4-tert-Butyl-phenyl-amino)-5-(4-methyl-benzyliden)-thiazol-4-on |
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

15. Verwendung wenigstens einer Verbindung gemäß einem oder mehreren der Ansprüche 1 bis 14 zur Herstellung eines Arzneimittels zur Prophylaxe und/oder Behandlung von Schmerz ausgewählt aus der Gruppe bestehend aus akutem Schmerz, chronischem Schmerz, visceralem Schmerz, Gelenkschmerz und neuropathischem Schmerz.

16. Verwendung wenigstens einer Verbindung wie in einem oder mehreren der Ansprüche 1 bis 14 definiert zur Herstellung eines Arzneimittels zur Prophylaxe und/oder Behandlung von einer oder mehreren Erkrankungen ausgewählt aus der Gruppe bestehend aus Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Mangelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

17. 2,5-Disubstituierte Thiazol-4-on-Derivate der allgemeinen Formel Ia, 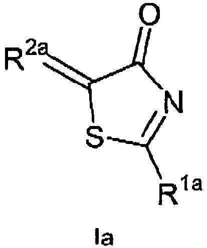 worin
R^{1a} für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann;
für eine NR^{3a}R^{4a}-Gruppe;
für eine NR^{5a}-C(=O)-R^{6a}-Gruppe
oder für eine NR^{7a}-C(=O)-NR^{8a}R^{9a} Gruppe steht;
R^{2a} für CH-CH=C(CH₃)-W^{a}-z^{a}, CH-CH=CH-W^{a}-Z^{a}, CH-C(CH₃)=CH-W^{a}-Z^{a}, CH-C(Phenyl)=CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} oder CH-CH(CH₃)-CH₂-W^{a}-Z^{a} steht
wobei W^{a} jeweils nicht vorhanden sein kann oder jeweils für einen Rest ausgewählt aus der Gruppe bestehend aus O, S, N(H), N(CH₃), N(C₂H₅) und N[CH(CH₃)₂] steht;
R^{3a} für einen linearen oder verzweigten, gesättigten oder ungesättigten, unsubstituierten oder wenigstens einfach substituierten, ggf. wenigstens ein Heteroatom als Kettenglied aufweisenden aliphatischen Rest;
für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklische Ringsystem kondensiert sein kann;
oder für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert und/oder über eine lineare oder verzweigte, unsubstituierte oder wenigstens einfach substituierte Alkylen-, Alkenylen- oder Alkinylen-Gruppe gebunden sein kann, steht;
R^{4a} für einen Wasserstoff-Rest
oder für einen linearen oder verzweigten, gesättigten oder ungesättigten, unsubstituierten oder wenigstens einfach substituierten, ggf. wenigstens ein Heteroatom als Kettenglied aufweisenden aliphatischen Rest steht;
R^{5a}, R^{7a} und R^{9a}, unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
R^{6a} und R^{8a}, unabhängig voneinander, jeweils
für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, stehen;
Z^{a} für einen linearen oder verzweigten, gesättigten oder ungesättigten, unsubstituierten oder wenigstens einfach substituierten aliphatischen Rest;
für einen Aryl-Rest steht, der mit einem unsubstituierten oder wenigstens einfach substituierten mono- oder polyzyklischen Ringsystem kondensiert ist und/oder mit gleichen oder verschieden Substituenten R^{12a} substituiert ist;
oder für einen Heteroaryl-Rest, der mit einem unsubstituierten oder wenigstens einfach substituierten mono- oder polyzyklischen Ringsystem kondensiert ist und/oder mit gleichen oder verschiedenen Substituenten R^{12a} substituiert ist, steht;
R^{12a} für einen Rest ausgewählt aus der Gruppe bestehend aus F, Cl, Br, I, - CN, -CF₃, -SF₅, -OH, -O-C₁₋₁₀-Alkyl, -O-C₂₋₁₀-Alkenyl, -NH₂, -O-CF₃, -S-CF₃, -SH, -S-C₁₋₆-Alkyl, -C₁₋₁₀-Alkyl, -C(=O)-OH, -C(=O)-O-C₁₋₅-Alkyl, - O-C(=O)-C₁₋₅-Alkyl, -NH-C₁₋₆-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-C₁₋₅-Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, - O-Benzyl, Phenyl und Benzyl steht; wobei jeweils der zyklische Teil der Reste Pyridinyl, Cyclopentyl, Cyclohexyl, Pyridazinyl, -S(=O)₂-Phenyl, - O-S(=O)₂-Phenyl, -O-Phenyl, -O-Benzyl, Phenyl, -(CH₂)-Benzo[b]furanyl und Benzyl substituiert sein kann;
für eine -C(=O)-NR^{13a}-(CH₂)ₘ-NR^{14a}-R^{15a}-Gruppe mit m³ gleich 0, 1, 2, 3, 4 oder 5;
oder für eine -C(=O)-R^{16a}-Gruppe steht;
R^{13a} und R^{14a}, unabhängig voneinander, jeweils
für einen Wasserstoff-Rest
oder für einen linearen oder verzweigten, gesättigten oder ungesättigten, unsubstituierten oder wenigstens einfach substituierten aliphatischen Rest stehen;
R^{15a} für einen unsubstituierten oder wenigstens einfach substituierten Aryl- oder Heteroaryl-Rest, der mit einem unsubstituierten oder wenigstens einfach substituierten mono- oder polyzyklischen Ringsystem kondensiert sein kann, steht:
und
R^{16a} für einen unsubstituierten oder wenigstens einfach substituierten, ungesättigten oder gesättigten, ggf. wenigstens ein Heteroatom als Ringglied aufweisenden cycloaliphatischen Rest, der mit einem unsubstituierten oder wenigstens einfach substituierten mono- oder polyzyklischen Ringsystem kondensiert sein kann, steht;
jeweils ggf. In Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

18. Verbindung gemäß Anspruch 17, **dadurch gekennzeichnet, dass**
R^{1a} für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann;
für eine NR^{3a}R^{4a}-Gruppe;
für eine NR^{5a}-C(=O)-R^{6a}-Gruppe
oder für eine NR^{7a}-C(=O)-NR^{8a}R^{9a} Gruppe steht;
R^{2a} für CH-CH=C(CH₃)-W^{a}-Z^{a}, CH-CH=CH-W^{a}-Z^{a}, CH-C(CH₃)=CH-W^{a}-Z^{a}, CH-C(Phenyl)=CH-W^{a}-Z^{a}, CH-CBr=CH-W^{a}-Z^{a}, CH-CCI=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} oder CH-CH(CH₃)-CH₂-W^{a}-Z^{a} steht;
wobei W^{a} jeweils nicht vorhanden sein kann oder jeweils für einen Rest ausgewählt aus der Gruppe bestehend aus O, S, N(H), N(CH₃), N(C₂H₅) und N[CH(CH₃)₂] steht;
R^{3a} für einen linearen oder verzweigten, gesättigten oder ungesättigten, ggf. substituierten, ggf. 1, 2 oder 3 Heteroatome unabhängig voneinander ausgewählt aus der Gruppe bestehend aus Sauerstoff, Stickstoff (NH) und Schwefel aufweisenden C₁₋₁₀ aliphatischen Rest;
für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann;
oder für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert und/oder über eine C₁₋₅-Alkylen-Gruppe gebunden sein kann, steht;
R^{4a} für einen Wasserstoff-Rest
oder für einen linearen oder verzweigten, gesättigten oder ungesättigten, ggf. substituierten, ggf. 1, 2 oder 3 Heteroatome unabhängig voneinander ausgewählt aus der Gruppe bestehend aus Sauerstoff, Stickstoff (NH) und Schwefel aufweisenden C₁₋₁₀ aliphatischen Rest steht;
R^{5a}, R^{7a} und R^{8a}, unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
R^{6a} und R^{9a}, unabhängig voneinander, jeweils
für einen ggf. substituierten 5- bis 14-gliedrigen Aryl- oder Heteroaryl-Rest, der mit einem gesättigten oder ungesättigten, ggf. substituierten mono- oder polyzyklischen Ringsystem kondensiert sein kann, stehen;
Z^{a} für einen linearen oder verzweigten, gesättigten oder ungesättigten, ggf. substituierten C₁₋₂₀ aliphatischen Rest;
für einen substituierten 6- oder 10-gliedrigen Aryl-Rest, der mit einem gesättigten oder ungesättigten, ggf. substituierten mono- oder polyzyklischen Ringsystem kondensiert ist und/oder mit 1, 2, 3, 4 oder 5 gleichen oder verschiedenen Substituenten R^{12a} substituiert ist, steht;
oder für einen ggf. substituierten 5- bis 14-gliedrigen Heteroaryl-Rest, der mit einem gesättigten oder ungesättigten, ggf. substituierten mono-oder polyzyklischen Ringsystem kondensiert ist und/oder mit 1, 2, 3, 4 oder 5 gleichen oder verschiedenen Substituenten R^{12a} substituiert ist, steht;
R^{12a} für einen Rest ausgewählt aus der Gruppe bestehend aus F, Cl, Br, I, - CN, -CF₃, -SF₅, -OH, -O-C₁₋₁₀-Alkyl, -O-C₂₋₁₀-Alkenyl, -NH₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, - O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, - C(=O)-N-(C₁₋₅-Alkyl)₂, -S(=O)₂-C₁₋₅-Alkyl, -S(=O)₂-Phenyl, -O-S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, - O-Benzyl. Phenyl und Benzyl steht; wobei jeweils der zyklische Teil der Reste Pyridinyl, Cyclopentyl, Cyclohexyl, Pyridazinyl, -S(=O)₂-Phenyl, - O-S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, -C₁₋₅-Alkyl, -O-C₁₋₅-Alkyl, -O-CF₃, -S-CF₃, Phenyl und -O-Benzyl substituiert sein kann;
für eine -C(=O)-NR^{13a}-(CH₂)ₘ-NR^{14a}-R^{15a}-Gruppe mit m^{a} gleich 0, 1, 2, 3, 4 oder 5;
oder für eine -C(=O)-R^{16a}-Gruppe steht;
R^{13a} und R^{14a}, unabhängig voneinander, jeweils
für einen Wasserstoff-Rest
oder für einen linearen oder verzweigten, gesättigten oder ungesättigten, ggf. substituierten C₁₋₁₀ aliphatischen Rest stehen;
R^{15a} für einen 5- bis 14-gliedrigen Aryl- oder Heteroaryl-Rest, der mit einem gesättigten oder ungesättigten, ggf. substituierten mono- oder polyzyklischen Ringsystem kondensiert sein kann, steht;
und
R^{16a} für einen ungesättigten oder gesättigten, ggf. substituierten 3-, 4-, 5-, 6-, 7-, 8- oder 9-gliedrigen cycloaliphatischen Rest, der mit einem gesättigten oder ungesättigten, ggf. substituierten mono- oder polyzyklischen Ringsystem kondensiert sein kann, steht;
wobei
die vorstehend genannten cycloaliphatischen Reste jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₅-Alkyl, -C(=O)-OH, -C(=O)-C₁₋₅-Alkyl, -O-C(=O)-C₁₋₅-Alkyl, -NH-C₁₋₅-Alkyl, -N(C₁₋₅-Alkyl)₂, - NH-Phenyl, -NH-Pyridinyl, -N(C₁₋₅-Alkyl)-Phenyl, -N(C₁₋₅-Alkyl)-Pyridinyl, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, [1,2,5]-Thiadiazolyl, Pyridazinyl, -(CH₂)-Benzo[b]furanyl, -O-Phenyl, -O-Benzyl, Phenyl und Benzyl substituiert sein können, wobei jeweils der zyklische Teil der Reste -NH-Phenyl, -NH-Pyridinyl, -N(C₁₋₅-Alkyl)-Phenyl, -N(C₁₋₅-Alkyl)-Pyridinyl, Pyridinyl, Cyclopentyl, [1,2,5]-Thiadiazolyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die vorstehend genannten cycloaliphatischen Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
die Ringe der vorstehend genannten mono- oder polyzyklischen Ringsysteme ggf. jeweils mit 1, 2, 3, 4 oder 5 Substituenten unabhängig voneinander ausgewählt aus der Gruppe bestehend aus Oxo (=O), Thioxo (=S), F, Cl, Br, I, -CN, -CF₃, -SF₅, -OH, -O-C₁₋₅-Alkyl, -NHz, -NO₂, -O-CF₃, -S-CF₃, -SH, -S-C₁₋₅-Alkyl, -C₁₋₅-Alkyl, -C(=O)-OH, -C(=O)-O-C₁₋₅-Alkyl, -O-C(=O)-C₁₋₅-Alkyl, -NH-C₁₋₅-Alkyl, -N(C₁₋₅-Alkyl)₂, -NH-C(=O)-O-C₁₋₅-Alkyl, -C(=O)-H, -C(=O)-C₁₋₅-Alkyl, -C(=O)-NH₂, -C(=O)-NH-C₁₋₅-Alkyl, C(=O)-N-(C₁₋₅-Alkyl)₂, -S(=O)₂-C₁₋₅-Alkyl, -S(=O)₂-Phenyl, -NH-S(=O)₂-C₁₋₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, - S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, - NO₂, -C₁₋₅-Alkyl, -O-C₁₋₅-Alkyl, -O-CF₃, -S-CF₃, Phenyl und -O-Benzyl substituiert sein kann,
und die Ringe der vorstehend genannten mono- oder polyzyklischen Ringsysteme jeweils 5-, 6- oder 7-gliedrig sind und jeweils ggf. 1, 2, 3, 4 oder 5 Heteroatom(e) als Ringglied(er) aufweisen können, die unabhängig voneinander aus der Gruppe bestehend aus Sauerstoff, Stickstoff und Schwefel ausgewählt sind;
die vorstehend genannten C₁₋₁₀ aliphatischen Reste oder C₁₋₂₀ aliphatischen Reste jeweils ggf. mit 1, 2, 3, 4, 5, 6, 7, 8 oder 9 Substituenten unabhängig voneinander ausgewählt aus der Gruppe bestehend aus F, Cl, Br, I, -CN, - NO₂, -OH, -SH und -NH₂ substituiert sein können;
die vorstehend genannten Aryl- oder Heteroaryl-Reste jeweils ggf. mit 1, 2, 3, 4 oder 5 Substituenten unabhängig voneinander ausgewählt aus der Gruppe bestehend aus F, Cl, Br, I, -CN, -CF₃, -SF₅, -OH, -O-C₁₋₅-Alkyl, -NH₂, -NO₂, -O-CF₃, -S-CF₃, -SH, -S-C₁₋₅-Alkyl, -C₁₋₁₀-Alkyl, -C(=O)-OH, -C(=O)-O-C₁₋₅-Alkyl, - O-C(=O)-C₁₋₅-Alkyl, -NH-C₁₋₅-Alkyl, -N(C₁₋₅-Alkyl)₂, -NH-C(=O)-O-C₁₋₅-Alkyl, - NH-C(=O)-C₁₋₅-Alkyl, -C(=O)-H, -C(=O)-C₁₋₅-Alkyl, -C(=O)-NH₂, -C(=O)-NH-C₁₋₅-Alkyl, -C(=O)-N-(C₁₋₅-Alkyl)₂, -S(=O)₂-C₁₋₅-Alkyl, -S(=O)₂-Phenyl, -NH-S(=O)₂-<-₅-Alkyl, -S(=O)₂-NH-C₁₋₅-Alkyl, Cyclohexyl, Cyclopentyl, Pyridinyl, Pyridazinyl, -(CH₂)-Benzo[b]furanyl, -O-Phenyl, -O-Benzyl, Phenyl und Benzyl substituiert sein können, wobei jeweils der zyklische Teil der Reste Pyridinyl, Cyclopentyl, Cyclohexyl, Pyridazinyl, -S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, -C₁₋₅-Alkyl, -O-C₁₋₅-Alkyl, -O-CF₃, -S-CF₃, Phenyl und -O-Benzyl substituiert sein kann,
und die vorstehend genannten Heteroaryl-Reste jeweils ggf. 1, 2, 3, 4 oder 5 Heteroatom(e) unabhängig voneinander ausgewählt aus der Gruppe bestehend aus Sauerstoff, Stickstoff und Schwefel als Ringglied(er) aufweisen können;
und die vorstehend genannten C₁₋₅-Alkylen-Gruppen jeweils ggf. mit 1, 2, 3, 4 oder 5 Substituenten unabhängig voneinander ausgewählt aus der Gruppe bestehend aus F, Cl, Br, -OH, -SH, -NH₂, -CN und NO₂ substituiert sein können;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

19. Verbindung gemäß Anspruch 17 oder 18, **dadurch gekennzeichnet, dass**
R^{1a} für einen Rest ausgewählt aus der Gruppe bestehend aus Cyclopentyl Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Thiomorpholinyl, Azepanyl, (2,3)-Dihydro-1H-isoindolyl, (2,3)-Dihydro-indolyl, (2,3,4,9)-Tetrahydro-1 H-β-carbolinyl, (2,3,4,9)-Tetrahydre-1H-pyrido(2,3-b]indolyl, [1,2,3,4]-Tetrahydronaphthyl, [1,2,3,4]-Tetrahydrochinolinyl, [1,2,3,4]-Tetrahydroisochinolinyl, [1,2,3,4]-Tetrahydrochinazolinyl und [3,4]-Dihydro-2H-1,4-benzoxazinyl steht; wobei der Rest jeweils ggf. mit 1, 2, 3, 4 oder 5 Substituenten unabhängig voneinander ausgewählt aus der Gruppe bestehend aus Oxo (=O), Thioxo (=S), F, Cl, Br, I, -SF₅, -OH, - O-CH₃, -O-C₂H₅, -NH₂, -NO₂, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, - N(CH₃)-Phenyl, -N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl, -NH-C(=O)-O-CH₃, -NH-C(=O)-O-C₂H₅, -NH-C(=O)-O-C(CH₃)₃, -C(=O)-CH₃, -C(=O)-C₂H₅, - C(=O)-NH-CH₃, -C(=O)-NH-C₂H₅, -C(=O)-N-(CH₃)₂, -C(=O)-N-(C₂H₅)₂, Pyridinyl, Pyridazinyl und Phenyl substituiert sein kann; wobei jeweils der zyklische Teil der Reste -NH-Phenyl, -NH-Pyridinyl, -N(CH₃)-Phenyl, -N(C₂H₅)-Phenyl, -N[CH(CH₃)]-Phenyl, -N(CH₃)-Pyridinyl, - N(C₂H₆)-Pyridinyl, -N[CH(CH₃)₂]-Pyridinyl, Pyridinyl, Pyridazinyl und Phenyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -0-Benzyl substituiert sein kann;
für einen Piperazinyl-Rest steht, der mit 1, 2 oder 3 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -C(=O)-C₁₋₅-Alkyl, - C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, Pyridinyl, Pyridazinyl, [1,2,5]-Thiadiazolyl, Benzyl und Phenyl substituiert sein kann; wobei jeweils der zyklische Teil der Reste Pyridinyl, Pyridazinyl, [1,2,5]-Thladiazolyl, Benzyl und Phenyl mit 1, 2, 3, 4 oder 5 Substituenten unabhängig voneinander ausgewählt aus der Gruppe bestehend aus F, Cl, Br, -OH, -CF₃, -SF₅, -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
für eine NR^{3a}R^{4a}-Gruppe steht;
für eine NR^{5a}-C(=O)-R^{6a}-Gruppe steht
oder für eine NR^{7a}-C(=O)-NR^{8a}R^{9a} Gruppe steht.

20. Verbindung gemäß einem oder mehreren der Ansprüche 17 bis 19, **dadurch gekennzeichnet, dass**
R^{2a} für CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(Phenyl)=CH-Z^{a}, CH-CBr=CH-Z^{a}, CH-CCl=CH-Z^{a}, CH-CF=CH-Z^{a}, CH-C(OH)=CH-Z^{a}, CH-CH₂-CH₂-Z^{a}, CH-CH₂-CH(CH₃)-Z^{a} oder CH-CH(CH₃)-CH₂-Z^{a} steht.

21. Verbindung gemäß einem oder mehreren der Ansprüche 17 bis 20, **dadurch gekennzeichnet, dass**
R^{3a} für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann;
für einen Rest ausgewählt aus der Gruppe bestehend aus Cyclopentyl Cyclohexyl, Cycloheptyl, Cyclopentenyl, Cyclohexenyl, Cycloheptenyl, Imidazolidinyl, Tetrahydrofuranyl, Tetrahydrothiophenyl, Pyrrolidinyl, Piperidinyl, Morpholinyl, Thiomorpholinyl und Azepanyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, - N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl substituiert sein kann; wobei jeweils der zyklische Teil der Reste -NH-Phenyl, -NH-Pyridinyl, -N(CH₃)-Phenyl, -N(C₂H₅)-Phenyl, -N[CH(CH₃)₂]-Phenyl, -N(CH₃)-Pyridinyl, -N(C₂H₅)-Pyridinyl und -N[CH(CH₃)₂]-Pyridinyl mit 1, 2, 3, 4 oder 5 Substituenten unabhängig voneinander ausgewählt aus der Gruppe bestehend aus F, Cl, Br, -OH, -CF₃, -SF₅, - CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-CH(CH₃)₂ und -NH-C(CH₃)₃ substituiert sein kann.

22. Verbindung gemäß einem oder mehreren der Ansprüche 17 bis 21, **dadurch gekennzeichnet, dass**
R^{4a} für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht; wobei der Rest ggf. jeweils mit 1, 2, 3, 4 oder 5 Substituenten unabhängig voneinander ausgewählt aus der Gruppe bestehend aus F, Cl, Br, I, - CN, -NO₂, -OH, -SH und -NH₂ substituiert sein kann.

23. Verbindung gemäß einem oder mehreren der Ansprüche 17 bis 22, **dadurch gekennzeichnet, dass**
R^{5a}, R^{7a} und R^{8a}, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen; wobei der Rest ggf. jeweils mit 1, 2, 3, 4 oder 5 Substituenten unabhängig voneinander ausgewählt aus der Gruppe bestehend aus F, Cl, Br, I, -CN, -NO₂, -OH, -SH und -NH₂ substituiert sein kann.

24. Verbindung gemäß einem oder mehreren der Ansprüche 17 bis 23, **dadurch gekennzeichnet, dass**
R^{6a} und R^{9a}, unabhängig voneinander, jeweils
für einen Rest ausgewählt aus der Gruppe bestehend aus Phenyl, Naphthyl, Thiophenyl, Furanyl, Pyridinyl, Thiazolyl und Oxazolyl stehen, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, Methyl, Ethyl, n-Propyl, Isopropyl, n-Butyl, sec-Butyl, Isobutyl, tert-Butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -O(=O)-CH₃, - C(=O)-C₂H₅, -C(=O)-C(CH₃)₃ substituiert sein kann.

25. Verbindung gemäß einem oder mehreren der Ansprüche 17 bis 24, **dadurch gekennzeichnet, dass**
Z^{a} für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht, wobei der Rest ggf. jeweils mit 1, 2, 3, 4 oder 5 Substituenten unabhängig voneinander ausgewählt aus der Gruppe bestehend aus F, Cl, Br, I, -CN, -NO₂. -OH, -SH und -NH₂ substituiert sein kann;
für einen Rest ausgewählt aus der Gruppe bestehend aus Phenyl, Naphthyl, Phenanthrenyl, (1,3)-Benzodioxolyl und (1,4)-Benzodioxanyl, steht, der mit 1, 2, 3, 4 oder 5 gleichen oder verschiedenen Substituenten R^{12a} substituiert ist;
oder für einen Rest ausgewählt aus der Gruppe bestehend aus Thiophenyl, Furanyl, Pyrrolyl, Pyrazolyl, Pyrazinyl, Pyridinyl, Pyridazinyl, Pyrimidinyl, Chinolinyl und Isochinolinyl steht, der mit 1, 2, 3, 4 oder 5 gleichen oder verschiedenen Substituenten R^{12a} substituiert ist.

26. Verbindung gemäß einem oder mehreren der Ansprüche 17 bis 25, **dadurch gekennzeichnet, dass**
R^{12a} für einen Rest ausgewählt aus der Gruppe bestehend aus F, Cl, Br, I, - CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl, n-Octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -NH-C(=O)-CH3, -NH-C(=O)-C₂H₅, -NH-C(=O)-C(CH₃)₃, -C(=O)-H, -C(=O)-CH₃, -C(=O)-C₂H₅, - C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-Phenyl, -O-S(=O)₂-Phenyl, Cyclohexyl, Cyclopentyl, Pyridinyl, Pyridazinyl, -(CH₂)-Benzo[b]furanyl, -O-Phenyl, -O-Benzyl, Phenyl und Benzyl steht; wobei jeweils der zyklische Teil der Reste Pyridinyl, Cyclopentyl, Cyclohexyl, Pyridazinyl, -S(=O)₂-Phenyl, -O-S(=O)₂-Phenyl, -O-Phenyl, -O-Benzyl, Phenyl, -(CH₂)-Benzo[b]furanyl und Benzyl mit 1, 2, 3, 4 oder 5 Substituenten unabhängig voneinander ausgewählt aus der Gruppe bestehend aus F, Cl, Br, -OH, -CF₃. -SF₅, -CN, -NO₂ Methyl, Ethyl, n-Propyl, Isopropyl, n-Butyl, sec-Butyl, Isobutyl, tert-Butyl, -O-CH₃, -0-C₂H₅, -O-CF₃, -S-CF₃, Phenyl und -O-Benzyl substituiert sein kann;
für eine -C(=O)-NR^{13a}-(CH₂)ₘ-NR^{14a}-R^{15a}-Gruppe mit m^{a} gleich 0, 1, 2 oder 3 steht;
oder für eine -C(=O)-R^{16a}-Gruppe steht;
wobei
R^{13a} und R^{14a}, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
R^{15a} für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -OH, -O-CH₃, -O-C₂H₅, -NH₂, -NO₂, -O-CF₃, - S-CF₃, -SH, -S-CH₃, -S-C₂H₅, Methyl, Ethyl, n-Propyl, Isopropyl. n-Butyl, sec-Butyl, Isobuty, tert-Buty, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, - NH-C(=O)-O-C₂H₅, -NH-C(=O)-O-C(CH₃)₃, -C(=O)-H, -C(=O)-CH₃, - C(=O)-C₂H₅, -C(=O)-C(CH₃)₃ substituiert sein kann;
und
R^{16a} für einen Rest ausgewählt aus der Gruppe bestehend aus Piperazinyl, Thiomorpholinyl, Azepanyl, Morpholinyl, (2,3)-Dihydro-1 H-isoindolyl, (2,3)-Dihydro-indolyl, Piperidinyl und Pyrrolidinyl steht; wobei der Rest mit 1, 2 oder 3 Substituenten unabhängig voneinander ausgewählt aus der Gruppe bestehend aus -OH, Methyl, Ethyl, n-Propyl, Isopropyl, n-Butyl, sec-Butyl, Isobutyl, tert-Butyl, -C(=O)-O-CH₃, -C(=O)-O-C₂H₅, - C(=O)-O-C(CH₃)₃, -C(=O)-C₁₋₅-Alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, Pyridinyl, Pyridazinyl, [1,2,5]-Thiadiazolyl, Benzyl und Phenyl substituiert sein kann; wobei jeweils der zyklische Teil der Reste Pyridinyl, Pyridazinyl, [1,2,5]-Thiadiazolyl, Benzyl und Phenyl mit 1, 2, 3, 4 oder 5 Substituenten unabhängig voneinander ausgewählt aus der Gruppe bestehend aus F, Cl, Br, -OH, -CF₃, -SF₅. -CN, -NO₂, Methyl, Ethyl, n-Propyl, Isopropyl, n-Butyl, sec-Butyl, Isobutyl, tert-Butyl, -O-CH₃, -O-C₂H₅, -O-CF₃, -S-CF₃, Phenyl und -O-Benzyl substituiert sein kann.

27. Verbindung gemäß einem oder mehreren der Ansprüche 17 bis 26, **dadurch gekennzeichnet, dass**
R^{1a} für einen der folgenden Reste steht 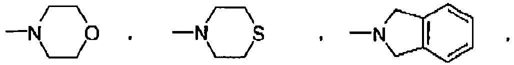 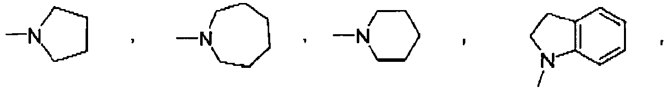 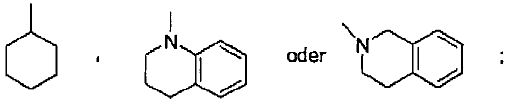 wobei der Rest mit 1, 2 oder 3 Substituenten unabhängig voneinander ausgewählt aus der Gruppe bestehend aus -OH, -O-CH₃, -O-C₂H₅. Methyl, Ethyl, n-Propyl, Isopropyl, n-Butyl, sec-Butyl, Isobutyl, tert-Butyl, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, -N(C₂H₅)-Phenyl, - N[CH(CH₃)₂]-Phenyl, -N(CN₃)-Pyridinyl, -N(C₂H₅)-Pyridinyl, - N[CH(CH₃)₂]-Pyridinyl und Phenyl substituiert sein kann; wobei jeweils der zyklische Teil des Phenyl-Restes mit 1, 2 oder 3 Substituenten unabhängig voneinander ausgewählt aus der Gruppe bestehend aus F, Cl, Br und -CF₃ substituiert sein kann;
für einen der folgenden Reste steht 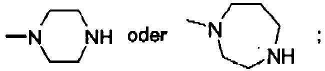 wobei das Wasserstoffatom der-NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl,
[1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für eine NR^{3a}R^{4a}-Gruppe steht
oder für eine NR⁷⁸-C(=O)-NR^{8a}R^{9a} Gruppe steht;
R^{2a} für CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(Phenyl)=CH-Z^{a}, CH-CBr=CH-Z^{a}, CH-CCl=CH-Z^{a}, CH-CF=CH-7^{a}, CH-(OH)=CH-Z^{a}, CH-CH₂-CH₂-Z^{a}, CH-CH₂-CH(CH₃)-Z^{a} oder CH-CH(CH₃)-CH₂-Z^{a} steht;
R^{3a} für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
für einen Rest ausgewählt aus der Gruppe bestehend aus Cyclopentyl Cyclohexyl, Cycloheptyl, Cyclopentenyl, Cyclohexenyl und Cycloheptenyl steht; wobei der Rest jeweils ggf. mit 1, 2, 3, 4 oder 5 Substituenten unabhängig voneinander ausgewählt aus der Gruppe bestehend aus Methyl, Ethyl, n-Propyl, Isopropyl, n-Butyl, sec-Butyl, Isobutyl, tert-Butyl, -N(CH₃)₂, -N(C₂H₅)₂, -NH-CH₃, -NH-C₂H₅, -NH-CH(CH₃)₂, -NH-C(CH₃)₃, -NH-Phenyl, -NH-Pyridinyl, -N(CH₃)-Phenyl, - N(C₂H₅)-Phenyl, -N[CH(CH₃)₂]-Phenyl, -N(CH₃)-Pyridinyl, -N(C₂H₅)-Pyridinyl, -N[CH(CH₃)₂]-Pyridinyl substituiert sein kann;
oder für einen Rest ausgewählt aus der Gruppe bestehend aus Phenyl, Naphthyl, (1,3)-Benzodioxolyl und (1,4)-Benzodioxanyl steht; wobei der Rest über einen -(CH₂)-, -(CH₂)-(CH₂) oder -(CH₂)-(CH₂)-(CH₂)-Gruppe gebunden und/oder jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl substituiert sein kann;
R^{4a} für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Bufyl, (1,1)-Dimethyl-propyl, n-Pentyl, sec-Pentyl, n-Hexyl und n-Heptyl steht;
R^{7a} und R^{8a}, unabhängig voneinander, jeweils
für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl stehen;
R^{9a} für einen Rest ausgewählt aus der Gruppe bestehend aus Phenyl, Naphthyl, Thiophenyl, Furanyl, Pyridinyl, Thiazolyl und Oxazolyl steht, wobei der Rest ggf. jeweils mit 1, 2, 3, 4 oder 5 Substituenten unabhängig voneinander ausgewählt aus der Gruppe bestehend aus F, Cl, Br, I, -CN, -CF₃, -SF₅, -O-CH₃, -O-C₂H₅, -O-CF₃, -S-CF₃, Methyl, Ethyl, n-Propyl, Isopropyl, n-Butyl, sec-Butyl, Isobutyl und tert-Butyl substituiert sein kann;
Z^{a} für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl und n-Eicosanyl steht;
für einen Rest ausgewählt aus der Gruppe bestehend aus Phenyl, Naphthyl, Phenanthrenyl, (1,3)-Benzodioxolyl und (1.4)-Benzodioxanyl steht; der mit 1, 2, 3, 4 oder 5 gleichen oder verschiedenen Substituenten R^{12a} substituiert ist;
oder für einen Rest ausgewählt aus der Gruppe bestehend Thiophenyl, Furanyl, Pyrrolyl, Pyrazolyl, Pyrazinyl, Pyridinyl, Pyridazinyl, Pyrimidinyl, Chinolinyl und Isochinolinyl steht; der mit 1, 2, 3, 4 oder 5 gleichen oder verschiedenen Substituenten R^{12a} substituiert ist;
R^{12a} für einen Rest ausgewählt aus der Gruppe bestehend aus F, CI, Br, - SF₅, -CF₃, -O-CH₃, -O-C₂H₅, -O-CH₂-CH₂-CH₃, -O-CH₂-CH₂-CH₂-CH₃, - O-C(CH₃)₃, -O-CH₂-CH₂-CH₂-CH₂-CH₃, -O-CH₂-CH₂-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

28. Verbindung gemäß einem oder mehreren der Ansprüche 17 bis 27, **dadurch gekennzeichnet, dass**
R^{1a} für einen der folgenden Reste steht 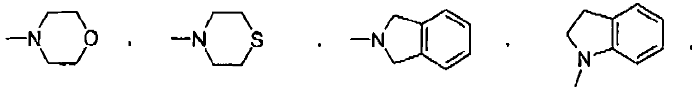 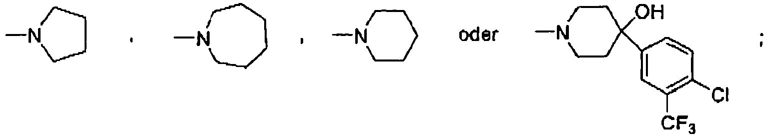 für den folgenden Rest steht 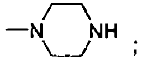 wobei das Wasserstoffatom der-NH-Gruppe durch einen Substituenten ausgewählt aus der Gruppe bestehend aus Methyl, Ethyl, n-Propyl, Isopropyl, n-Butyl, sec-Butyl, Isobutyl, tert-Butyl, Pyridinyl, Pyridazinyl, [1,2,5]-Thiadiazolyl, Benzyl und Phenyl ersetzt sein kann; wobei jeweils der zyklische Teil der Reste Pyridinyl, Pyridazinyl, [1,2,5]-Thiadiazolyl, Benzyl und Phenyl mit 1, 2 oder 3 Substituenten unabhängig voneinander ausgewählt aus der Gruppe bestehend aus F, Cl, Br, -CF₃, Methyl, Ethyl, n-Propyl, Isopropyl, n-Butyl, sec-Butyl, Isobutyl, tert-Butyl, -O-CH₃, -O-C₂H₅, -O-CF₃ und -S-CF₃ substituiert sein kann;
für eine NR^{3a}R^{4a}-Gruppe steht
oder für eine NR^{7a}-C(=O)-NR^{8a}R^{9a} Gruppe steht;
R^{2a} für CH-CH=C(CH₃)-Z^{a}, CH-CH=CH-Z^{a}, CH-CH=CH-S-Z^{a}, CH-C(CH₃)=CH-Z^{a}, CH-C(Phenyl)=CH-Z^{a}, CH-CBr=CH-Z^{a}, CH-CCI=CH-Z^{a}, CH-CF=CH-Z^{a}, CH-CH₂-CH₂-Z^{a}, CH-CH₂-CH(CH₃)-Z^{a} oder CH-CH(CH₃)-CH₂-Z^{a} steht;
R^{3a} für einen Rest ausgewählt aus der Gruppe bestehend aus Methyl, -CH₂-O-CH₃, -CH₂-S-CH₃, Ethyl, -CH₂-CH₂-S-CH₃, -CH₂-CH₂-O-CH₃, -CH₂-CH₂-N(CH₃)-CH₃, n-Propyl, -CH₂-CH₂-CH₂-S-CH₃, -CH₂-CH₂-CH₂-O-CH₃, -CH₂-CH₂-CH₂-N(CH₃)-CH₃, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
oder für einen Rest ausgewählt aus der Gruppe bestehend aus Phenyl, Naphthyl, Benzyl, (1,3)-Benzodioxolyl und (1,4)-Benzodioxanyl steht; wobei der Rest jeweils ggf. mit 1, 2, 3, 4 oder 5 Substituenten unabhängig voneinander ausgewählt aus der Gruppe bestehend aus F, Cl, Br, -CF₃, -O-CF₃, -OCH₃, -OC₂H₅, Methyl, Ethyl, n-Propyl, Isopropyl, n-Butyl und tert-Butyl sein kann;
R^{4a} für einen Wasserstoff-Rest
oder für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl und n-Heptyl steht;
R^{7a} und R^{8a} jeweils für einen Wasserstoff-Rest stehen;
R^{9a} für einen Phenyl-Rest steht, der mit 1, 2, 3, 4 oder 5 Substituenten unabhängig voneinander ausgewählt aus der Gruppe bestehend aus F, Cl, Br und -CF₃ substituiert sein kann;
Z^{a} für einen Rest ausgewählt aus der Gruppe bestehend aus Methyl, Ethyl, n-Propyl, Isopropyl, n-Butyl, sec-Butyl, Isobutyl, tert-Butyl, (1,1)-Dimethyl-propyl, n-Pentyl, sec-Pentyl, n-Hexyl, n-Heptyl und n-Octyl steht;
oder für einen Phenyl- oder Naphthyl-Rest steht, der mit 1, 2, 3, 4 oder 5 gleichen oder verschiedenen Substituenten R^{12a} substituiert ist;
R^{12a} für einen Rest ausgewählt aus der Gruppe bestehend aus F, Cl. Br, I, - CN, -CF₃, -SF₆, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, Methyl, Ethyl, n-Propyl, Isopropyl, n-Butyl, sec-Butyl, tert-Butyl, n-Pentyl, n-Hexyl, n-Heptyl und n-Octyl steht;
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils in Form entsprechender Solvate.

29. Verbindung gemäß einem oder mehreren der Ansprüche 17 bis 28 ausgewählt aus der Gruppe bestehend aus
1. 5-[3-(4-Methoxy-phenyl)-but-2-enyliden]-2-morpholin-4-yl-thiazol-4-on
2. 5-[3-(4-Fluor-phenyl)-allyliden]-2-morpholin-4-yl-thiazol-4-on
3. 2-Morpholin-4-yl-5-(3-m-tolyl-allyliden)-thiazol-4-on
4. 2-Morpholin-4-yl-5-(3-p-tolyl-allyliden)-thiazol-4-on
5. 2-Morphalin-4-yl-5-actyliden-thiazol-4-on
6. 5-Butyliden-2-morpholin-4-yl-thiazol-4-on
7. 5-Hexyliden-2-morpholin-4-yl-thiazol-4-on
8. 2-Morpholin-4-yl-5-(3-p-tolyl-but-2-enyliden)-thiazol-4-on
9. 5-[3-(4-tert-Butyl-phenyl)-but-2-enyliden]-2-morpholin-4-yl-thiazol-4-on
10. 5-[3-(4-tert-Butyl-phenyl)-allyliden]-2-[4-(3-methoxy-phenyl)-piperazin-1-yl]-thiazol-4-on
11. 5-[3-(4-Hydroxy-3-methoxy-phenyl)-allyliden]-2-thiomorpholin-4-yl-thiazol-4-on
12.2-Morpholin-4-yl-5-[3-(4-trifluormethyl-phenyl)-but-2-enyliden]-thiazol-4-on
13. 2-Morpholin-4-yl-5-[3-(3-trifluormethyl-phenyl)-but-2-enyliden]-thiazol-4-on
14. 5-Decyliden-2-morpholin-4-yl-thiazol-4-on
15. 5-[3-(4-tert-Butyl-phenyl)-allyliden]-2-morpholin-4-yl-thiazol-4-on
16. 5-[3-(4-tert-Butyl-phenyl)-allyliden]-2-[4-(3-Chlor-pyridin-2-yl)-piperazin-1-yl]-thiazol-4-on
17. 5-[3-(4-tert-Butyl-phenyl)-allyliden]-2-[4-(2-fluor-phenyl)-piperazin-1-yl]-thiazol-4-on
18. 5-[3-(4-tert-Butyl-phenyl)-allyliden]-2-[4-(4-chlor-[1,2,5]thiadiazol-3-yl)-piperazin-1-yl]-thiazol-4-on
19. 5-[3-(4-tert-Butyl-phenyl)-allyliden]-2-[4-(6-chlor-pyridin-2-yl)-piperazin-1-yl]-thiazol-4-on
20. 5-[3-(4-tert-Butyl-phenyl)-allyliden]-2-[4-(6-methyl-pyridazin-3-yl)-piperazin-1-yl]-thiazol-4-on
21. 2-[(Benzo[1,3]dioxol-5-ylmethyl)-amino]-5-[3-(4-tert-butyl-phenyl)-allyliden]-thiazol-4-on
jeweils ggf. in Form eines ihrer reinen Stereoisomeren, insbesondere Enantiomeren oder Diastereomeren, ihrer Racemate oder in Form einer Mischung von Stereoisomeren, insbesondere der Enantiomeren und/oder Diastereomeren, in einem beliebigen Mischungsverhältnis, oder jeweils in Form entsprechender Salze, oder jeweils In Form entsprechender Solvate.

30. Verfahren zur Herstellung von 2,5-disubstiuierten Thiazol-4-on-Derivaten der allgemeinen Formel Ia gemäß einem oder mehreren der Ansprüche 17 bis 29, **dadurch gekennzeichnet, dass** 2-Amino-thiazol-4-on in Essigsäure in Gegenwart wenigstens eines Salzes ausgewählt aus der Gruppe bestehend aus Natriumacetat und Kaliumacetat oder in einem Reaktionsmedium ausgewählt aus der Gruppe bestehend aus Methanol, Ethanol, Isopropanol und n-Butanol in Gegenwart wenigstens einer organischen Base ausgewählt aus der Gruppe bestehend aus Triethylamin. Pyridin, Diisopropylamin und N-Methylmorpholin mit wenigstens einer Verbindung der allgemeinen Formel R-C(=O)-H, worin R für -CH=C(CH₃)-W^{a}-Z^{a}, -CH=CH-W^{a}-Z^{a}, -C(CH₃)=CH-W^{a}-Z^{a}, -C(Phenyl)=CH-W^{a}-Z^{a}, -CBr=CH-W^{a}-Z^{a}, -CCl=CH-W^{a}-Z^{a}, -CF=CH-W^{a}-Z^{a}, - C(OH)=CH-W^{a}-Z^{a}, -CH₂-CH₂-W^{a}-Z^{a}, -CH₂-CH(CH₃)-W^{a}-Z^{a} oder -CH(CH₃)-CH₂-W^{a}-Z^{a} steht, wobei W^{a} und Z^{a} die Bedeutung gemäß einem oder mehreren der Ansprüche 17 bis 29 haben, zu wenigstens einer Verbindung der allgemeinen Formel IIa, 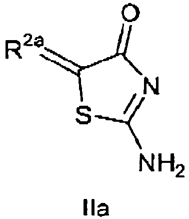 worin R^{2a} die Bedeutung gemäß einem oder mehreren der Ansprüche 17 bis 29 hat, umgesetzt wird und diese ggf. gereinigt und/oder isoliert wird und wenigstens eine Verbindung der allgemeinen Formel IIa in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R^{3a} oder der allgemeinen Formel LG-R^{7a} oder der allgemeinen Formel LG-R^{5a}, worin LG jeweils für eine Abgangsgruppe, bevorzugt für ein Halogen-Atom, besonders bevorzugt für ein Chloratom steht und R^{3a}, R^{5a} und R^{7a} jeweils die Bedeutung gemäß einem oder mehreren der Ansprüche 17 bis 29 haben, zu wenigstens einer Verbindung der allgemeinen Formel Ia, worin R^{2a} die vorstehend genannte Bedeutung hat und R^{1a} für eine NHR^{3a}-, NHR^{5a}- oder NHR^{7a}-Gruppe steht, umgesetzt wird und diese ggf. gereinigt und/oder isoliert wird,
und ggf. wenigstens eine Verbindung der allgemeinen Formel IIa, worin R^{2a} die vorstehend genannte Bedeutung hat und R^{1a} für eine NHR^{7a}-Gruppe steht, in einem Reaktionsmedium mit wenigstens einem Isocyanat der allgemeinen Formel R^{8a}-N=C=O, worin R^{8a} die Bedeutung gemäß einem oder mehreren der Ansprüche 17 bis 29 hat, ggf. in Gegenwart wenigstens einer Base, bevorzugt In Gegenwart wenigstens einer Base ausgewählt aus der Gruppe bestehend aus Triethylamin, 4,4-Dimethylaminopyridin und Diisopropylethylamin, zu wenigstens einer Verbindung der allgemeinen Formel la, worin R^{2a} die vorstehend genannte Bedeutung hat und R^{1a} für eine NR^{7a}-C(=O)-NHR^{8a}-Gruppe steht, umgesetzt wird und diese ggf. gereinigt und/oder isoliert wird;
und ggf. wenigstens eine Verbindung der allgemeinen Formel Ia, worin R^{2a} die vorstehend genannte Bedeutung hat und R^{1a} für eine NR⁷⁸-C(=O)-NHR⁸⁸-Gruppe steht, in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat,
Natriumethanolat und Kaliumethanolat, mit wenigstens einer Verbindung der allgemeinen Formel LG-R^{9a}, worin LG für eine Abgangsgruppe, bevorzugt für ein Halogen-Atom, besonders bevorzugt für ein Chloratom steht und R^{9a} die Bedeutung gemäß einem oder mehreren der Ansprüche 17 bis 29 hat, zu wenigstens einer Verbindung der allgemeinen Formel Ia, worin R^{2a} die vorstehend genannte Bedeutung hat und R^{1a} für eine NR^{7a}-C(=O)-NR^{8a}R^{9a}-Gruppe steht, umgesetzt wird und diese ggf. gereinigt und/oder isoliert wird;
oder
ggf. wenigstens eine Verbindung der allgemeinen Formel IIa, worin R^{2a} die vorstehend genannte Bedeutung hat und R^{1a} für eine NHR^{5a}-Gruppe steht, in einem Reaktionsmedium ggf. in Gegenwart wenigstens einer Base mit wenigstens einer Verbindung der allgemeinen Formel R^{6a}-C(=O)-LG, worin R^{6a} die Bedeutung gemäß einem oder mehreren der Ansprüche 17 bis 29 hat und LG für eine Abgangsgruppe, vorzugsweise für einen Halogen-Rest steht, oder in einem Reaktionsmedium in Gegenwart wenigstens eines Kupplungsreagenzes ggf. in Gegenwart wenigstens einer Base mit einer Verbindung der allgemeinen Formel R^{6a}-C(=O)-OH, worin R^{6a} die Bedeutung gemäß einem oder mehreren der Ansprüche 17 bis 29 hat, zu wenigstens einer Verbindung der allgemeinen Formel Ia umgesetzt wird, worin R^{2a} die vorstehend genannte Bedeutung hat und R^{1a} für eine NR^{5a}-C(=O)-R^{6a}-Gruppe steht, und diese ggf. gereinigt und/oder isoliert wird
oder
ggf. wenigstens eine Verbindung der allgemeinen Formel Ia, worin R^{2a} die vorstehend genannte Bedeutung hat und R^{1a} für eine NHR^{3a}-Gruppe steht, in einem Reaktionsmedium in Gegenwart wenigstens einer Base, bevorzugt in Gegenwart wenigstens eines Metallhydridsalzes oder eines Metallalkoholatsalzes, besonders bevorzugt in Gegenwart eines Metallhydridsalzes oder eines Metallalkoholatsalzes ausgewählt aus der Gruppe bestehend aus Natriumhydrid, Kaliumhydrid, Kalium-tert-butanolat, Natrium-tert-butanolat, Kaliummethanolat, Natriummethanolat, Natriumethanolat und Kaliumethanolat, mit wenigstens einer Verbindung der allgemeinen Formel LG-R^{4a}, worin LG für eine Abgangsgruppe, bevorzugt für ein Halogen-Atom, besonders bevorzugt für ein Chloratom steht und R^{4a} die Bedeutung gemäß einem oder mehreren der Ansprüche 17 bis 29 hat, zu wenigstens einer Verbindung der allgemeinen Formel Ia, worin R^{2a} die vorstehend genannte Bedeutung hat und R^{1a} für eine NR^{3a}R^{4a}-Gruppe steht, umgesetzt wird und diese ggf. gereinigt und/oder isoliert wird.

31. Verfahren zur Herstellung von 2,5-disubstiuierten Thiazol-4-on-Derivaten der allgemeinen Formel Ia gemäß einem oder mehreren der Ansprüche 17 bis 29, **dadurch gekennzeichnet, dass** wenigstens eine Verbindung der allgemeinen Formel R^{1a}-CN, worin R^{1a} die Bedeutung gemäß einem oder mehreren der Ansprüche 17 bis 29 mit Ausnahme einer NR^{3a}R^{4a}-Gruppe, einer NR^{5a}-C(=O)-R^{6a}-Gruppe und einer NR^{7a}-C(=O)-NR^{8a}R^{8a}-Gruppe hat, in einem Reaktionsmedium ausgewählt aus der Gruppe bestehend aus Methanol, Ethanol, Isopropanol und n-Butanol, in Gegenwart wenigstens einer organischen Base ausgewählt aus der Gruppe bestehend aus Triethylamin, Pyridin, Diisopropylamin und N-Methylmorpholin oder in Pyridin als Reaktionsmedium mit Thioglykolsäure zu wenigstens einer Verbindung der allgemeinen Formel IIIa, 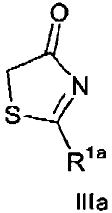 worin R^{1a} die Bedeutung gemäß einem oder mehreren der Ansprüche 17 bis 29 mit Ausnahme einer NR^{3a}R^{4a}-Gruppe, einer NR^{5a}-C(=O)-R^{6a}-Gruppe und einer NR^{7a}-C(=Q)-NR^{8a}R^{9a}-Gruppe hat, umgesetzt wird und diese ggf. gereinigt und/oder isoliert wird;
oder wenigstens einer Verbindung der allgemeinen Formel H₂N-C(=S)-NHR^{1a}, worin R^{1a} die Bedeutung gemäß einem oder mehreren der Ansprüche 17 bis 29 mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, in einem Reaktionsmedium ausgewählt aus der Gruppe bestehend aus Methanol, Ethanol, Isopropanol und n-Butanol, ggf. in Gegenwart wenigstens einer organischen Base ausgewählt aus der Gruppe bestehend aus Triethylamin, Pyridin, Diisopropylamin und N-Methylmorpholin, mit Chloressigsäure zu wenigstens einer Verbindung der allgemeinen Formel IIIa, worin R^{1e} die Bedeutung gemäß einem oder mehreren der Ansprüche 17 bis 29 mit Ausnahme einer NR^{3a}R^{4a}-Gruppe, einer NR^{5a}-C(=O)-R^{6a}-Gruppe und einer NR^{7a}-C(=O)-NR^{8a}R^{9a}-Gruppe hat, umgesetzt wird und diese ggf. gereinigt und/oder isoliert wird;
und wenigstens eine Verbindung der allgemeinen Formel IIIa in Essigsäure in Gegenwart wenigstens eines Salzes ausgewählt aus der Gruppe bestehend aus Natriumacetat und Kaliumacetat oder in einem Reaktionsmedium ausgewählt aus der Gruppe bestehend aus Methanol, Ethanol, Isopropanol und n-Butanol in Gegenwart wenigstens einer organischen Base ausgewählt aus der Gruppe bestehend aus Triethylamin, Pyridin, Diisopropylamin und N-Methylmorpholin mit wenigstens einer Verbindung der allgemeinen Formel R-C(=O)-H. worin R für -CH=C(CH₃)-W^{a}-Z^{a}, -CH=CH-W^{a}-Z^{a}, -C(CH₃)=CH-W^{a}-Z^{a}, -C(Phenyl)=CH-W^{a}-Z^{a}, -CBr=CH-W^{a}-Z^{a}, -CCI=CH-W^{a}-Z^{a}, -CF=CH-W^{a}-Z^{a}, - C(OH)=CH-W^{a}-Z^{a}, -CH₂-CH₂-W^{a}-Z^{a}, -CH₂-CH(CH₃)-W^{a}-Z^{a} oder -CH(CH₃)-CH₂-W^{a}-Z^{a} steht, wobei W^{a} und Z^{a} die Bedeutung gemäß einem oder mehreren der Ansprüche 17 bis 29 haben, zu wenigstens einer Verbindung der allgemeinen Formel Ia, worin R^{1a} die Bedeutung gemäß einem oder mehreren der Ansprüche 17 bis 29 mit Ausnahme einer NR^{3a}R^{4a} -Gruppe, einer NR^{5a}-C(=O)-R^{6a}-Gruppe und einer NR^{7a}-C(=O)-NR^{8a}R^{9a}-Gruppe hat und R^{2a} die Bedeutung gemäß einem oder mehreren der Ansprüche 17 bis 29 hat.

32. Arzneimittel enthaltend wenigstens eine Verbindung gemäß einem oder mehreren der Ansprüche 17 bis 29 und ggf. einen oder mehrere physiologisch verträgliche Hilfsstoffe.

33. Arzneimittel gemäß Anspruch 32 zur Prophylaxe und/oder Behandlung von Schmerz, vorzugsweise von Schmerz ausgewählt aus der Gruppe bestehend aus akutem Schmerz, chronischem Schmerz, visceralem Schmerz, neuropathischem Schmerz und Gelenkschmerz.

34. Arzneimittel gemäß Anspruch 32 zur Prophylaxe und/oder Behandlung von einer oder mehreren Erkrankungen ausgewählt aus der Gruppe bestehend aus Gelenkschmerz; Migräne; Depressionen; Nervenleiden; Nervenverletzungen; neurodegenerativen Erkrankungen, vorzugsweise ausgewählt aus der Gruppe bestehend aus Multipler Sklerose, Morbus Alzheimer, Morbus Parkinson und Morbus Huntington; kognitiven Dysfunktionen, vorzugsweise kognitiven Martgelzuständen, besonders bevorzugt Gedächtnisstörungen; Epilepsie; Atemwegserkrankungen, vorzugsweise ausgewählt aus der Gruppe bestehend aus Asthma und Lungenentzündung; Husten; Harninkontinenz; einer überaktiven Blase (overactive bladder, OAB); Magengeschwüren; Reizdarmsyndrom; Schlaganfällen; Augenreizungen; Hautreizungen; neurotischen Hauterkrankungen; Entzündungskrankheiten, vorzugsweise Entzündungen des Darmes; Diarrhöe; Pruritus; Störungen der Nahrungsaufnahme, vorzugsweise ausgewählt aus der Gruppe bestehend aus Bulimie, Kachexie, Anorexie und Fettleibigkeit; Medikamentenabhängigkeit; Medikamentenmißbrauch; Entzugserscheinungen bei Medikamentenabhängigkeit; Toleranzentwicklung gegenüber Medikamenten, vorzugsweise gegenüber natürlichen oder synthetischen Opioiden; Drogenabhängigkeit; Drogenmißbrauch; Entzugserscheinungen bei Drogenabhängigkeit; Alkoholabhängigkeit; Alkoholmissbrauch und Entzugserscheinungen bei Alkoholabhängigkeit; zur Diurese; zur Antinatriurese; zur Beeinflussung des kardiovaskulären Systems; zur Vigilanzsteigerung; zur Libidosteigerung; zur Modulation der Bewegungsaktivität; zur Anxiolyse; zur Lokalanästhesie und/oder zur Hemmung unerwünschter Nebenwirkungen, vorzugsweise ausgewählt aus der Gruppe bestehend aus Hyperthermie, Bluthochdruck und Verengung der Bronchien, ausgelöst durch die Verabreichung von Vanilloid-Rezeptor 1 (VR1/TRPV1-Rezeptoren)-Agonisten, vorzugsweise ausgewählt aus der Gruppe bestehend aus Capsaicin, Resiniferatoxin, Olvanil, Arvanil, SDZ-249665, SDZ-249482, Nuvanil und Capsavanil (DA-5018).

## Claims

1. The use of at least one 2,5-disubstituted thiazol-4-one derivative of the general formula I, 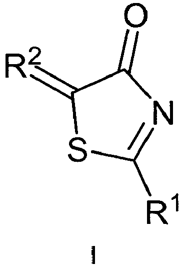 in which
R¹ is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
is an unsubstituted or at least monosubstituted aryl radical which may be fused to an unsubstituted or at least monosubstituted mono- or polycyclic ring system;
is an NR³R⁴ group;
is an NR⁵-C(=O)-R⁶ group;
or is an NR⁷-C(=O)-NR⁸R⁹ group;
R² is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
is CH-U-X;
is CH-CH₂-V-Y, CH-CH(CH₃)-V-Y, CH-CHCI-V-Y, CH-CHBr-V-Y, CH-CHF-V-Y or CH-CH(OH)-V-Y;
or is CH-CH=C(CH₃)-W-Z, CH-CH=CH-W-Z, CH-C(CH₃)=CH-W-Z, CH-C(phenyl)=CH-W-Z, CH-CBr=CH-W-Z, CH-CCI=CH-W-Z, CH-CF=CH-W-Z, CH-C(OH)=CH-W-Z, CH-CH₂-CH₂-W-Z, CH-CH₂-CH(CH₃)-W-Z or CH-CH(CH₃)-CH₂-W-Z;
where U, V and W may each be absent or are each independently a radical selected from the group consisting of O, S, N(H), N(CH₃), N(C₂H₅) and N[CH(CH₃)₂];
R³ is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
is a radical selected from the group consisting of cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, thiomorpholinyl and azepanyl; where the radical may in each case optionally be substituted by 1, 2, 3, 4 or 5 substituents selected independently from the group consisting of methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl; where the cyclic moiety of the -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl and -N[CH(CH₃)₂]-pyridinyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or is a radical selected from the group consisting of phenyl, naphthyl, (1,3)-benzodioxolyl and (1,4)-benzodioxanyl; where the radical may be bonded via a -(CH₂)-, -(CH₂)-(CH₂) or-(CH₂)-(CH₂)-(CH₂) group and/or may in each case optionally be substituted by 1, 2, 3, 4 or 5 substituents selected independently from the group consisting of F, CI, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ and -NH-C(CH₃)₃;
R⁴ is a hydrogen radical
or is a linear or branched, saturated or unsaturated, unsubstituted or at least monosubstituted aliphatic radical optionally having at least one heteroatom as a chain member;
R⁵, R⁷ and R⁹ are each independently
a hydrogen radical
or a linear or branched, saturated or unsaturated, unsubstituted or at least monosubstituted aliphatic radical;
R⁶ and R⁸ are each independently
an unsubstituted or at least monosubstituted aryl or heteroaryl radical which may be fused to an unsubstituted or at least monosubstituted mono- or polycyclic ring system;
X is an aryl or heteroaryl radical which may be fused to an unsubstituted or at least monosubstituted mono- or polycyclic ring system and/or may be substituted by identical or different substituents R¹⁰ ;
Y is an aryl or heteroaryl radical which may be fused to an unsubstituted or at least monosubstituted mono- or polycyclic ring system and/or may be substituted by identical or different substituents R¹¹ ;
Z is a linear or branched, saturated or unsaturated, unsubstituted or at least monosubstituted aliphatic radical;
or is an aryl or heteroaryl radical which may be fused to an unsubstituted or at least monosubstituted mono- or polycyclic ring system and/or may be substituted by identical or different substituents R¹²;
R¹⁰, R¹¹ and R¹² are each independently
a radical selected from the group consisting of F, Cl, Br, I, -CN, -CF₃, -SF₅, -OH, -O-C₁₋₁₀-alkyl, -O-C₂₋₁₀-alkenyl, -NH₂, -O-CF₃, -S-CF₃, -SH, - S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-C₁₋₅-alkyl, -S(=O)₂-phenyl, -O-S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl; where the cyclic moiety of the pyridinyl, cyclopentyl, cyclohexyl, pyridazinyl, -S(=O)₂-phenyl, -O-S(=O)₂-phenyl, -O-phenyl, -O-benzyl, phenyl, -(CH₂)-benzo[b]furanyl and benzyl radicals may in each case be substituted;
is a -C(=O)-NR¹³-(CH₂)ₘ-NR¹⁴-R¹⁵ group where m is 0, 1, 2, 3, 4 or 5;
or is a -C(=O)-R¹⁶ group;
R¹³ and R¹⁴ are each independently
a hydrogen radical
or a linear or branched, saturated or unsaturated, unsubstituted or at least monosubstituted aliphatic radical;
R¹⁵ is an unsubstituted or at least monosubstituted aryl or heteroaryl radical which may be fused to an unsubstituted or at least monosubstituted mono- or polycyclic ring system;
and
R¹⁶ is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates;
for producing a medicament for prophylaxis and/or treatment of pain.

2. The use of a compound as claimed in claim 1, **characterized in that**
R¹ is an unsaturated or saturated, optionally substituted 3-, 4-, 5-, 6-, 7-, 8- or 9-membered cycloaliphatic radical which may be fused to a saturated or unsaturated, optionally substituted mono- or polycyclic ring system;
is an optionally substituted 6- or 10-membered aryl radical which may be fused to a saturated or unsaturated, optionally substituted mono- or polycyclic ring system;
is an NR³R⁴ group;
is an NR⁵-C(=O)-R⁶ group
or is an NR⁷-C(=O)-NR⁸R⁹ group;
R² is a unsaturated or saturated, optionally substituted 3-, 4-, 5-, 6-, 7-, 8- or 9-membered cycloaliphatic radical which may be fused to a saturated or unsaturated, optionally substituted mono- or polycyclic ring system;
is CH-U-X;
is CH-CH₂-V-Y, CH-CH(CH₃)-V-Y, CH-CHCI-V-Y, CH-CHBr-V-Y, CH-CHF-V-Y or CH-CH(OH)-V-Y;
or is CH-CH=C(CH₃)-W-Z, CH-CH=CH-W-Z, CH-C(CH₃)=CH-W-Z, CH-C(phenyl)=CH-W-Z, CH-CBr=CH-W-Z, CH-CCl=CH-W-Z, CH-CF=CH-W-Z, CH-C(OH)=CH-W-Z, CH-CH₂-CH₂-W-Z, CH-CH₂-CH(CH₃)-W-Z or CH-CH(CH₃)-CH₂-W-Z;
where U, V and W may in each case be absent or are in each case independently a radical selected from the group consisting of O, S, N(H), N(CH₃), N(C₂H₅) and N[CH(CH₃)₂];
R³ is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
is a radical selected from the group consisting of cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, thiomorpholinyl and azepanyl; where the radical may in each case optionally be substituted by 1, 2, 3, 4 or 5 substituents selected independently from the group consisting of methyl, ethyl, n-propyl, isopropyl, n-butyl, sec-butyl, isobutyl, tert-butyl, -N(CH₃)₂, -N(C_{2H5})₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)_{2]}-phenyl, -N(CH₃)-pyridinyl, -N(C₂H₅)-pyridinyl, -N[CH(CH₃)_{2]}-pyridinyl; where the cyclic moiety of the -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl and -N[CH(CH₃)₂]-pyridinyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or is a radical selected from the group consisting of phenyl, naphthyl, (1,3)-benzodioxolyl and (1,4)-benzodioxanyl; where the radical may be bonded via a -(CH₂)-, -(CH₂)-(CH₂) or -(CH₂)-(CH₂)-(CH₂) group and/or may in each case optionally be substituted by 1, 2, 3, 4 or 5 substituents selected independently from the group consisting of F, Cl, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ and -NH-C(CH₃)₃;
R⁴ is a hydrogen radical
or is a linear or branched, saturated or unsaturated, optionally substituted C₁₋₁₀ aliphatic radical optionally having 1, 2 or 3 heteroatoms selected independently from the group consisting of oxygen, nitrogen (NH) and sulfur;
R⁵, R⁷ and R⁸ are each independently
a hydrogen radical
or a linear or branched, saturated or unsaturated, optionally substituted C₁₋₁₀ aliphatic radical;
R⁶ and R⁹ are each independently
an optionally substituted 5- to 14-membered aryl or heteroaryl radical which may be fused to a saturated or unsaturated, optionally substituted mono- or polycyclic ring system;
X is a 5- to 14-membered aryl or heteroaryl radical which may be fused to a saturated or unsaturated, optionally substituted mono- or polycyclic ring system and/or may optionally be substituted by 1, 2, 3, 4 or 5 identical or different substituents R¹⁰;
Y or is an optionally substituted 5- to 14-membered aryl or heteroaryl radical which may be fused to a saturated or unsaturated, optionally substituted mono- or polycyclic ring system and/or may optionally be substituted by 1, 2, 3, 4 or 5 identical or different substituents R¹¹;
Z is a linear or branched, saturated or unsaturated, optionally substituted C₁₋₂₀ aliphatic radical;
or is an optionally substituted 5- to 14-membered aryl or heteroaryl radical which may be fused to a saturated or unsaturated, optionally substituted mono- or polycyclic system and/or may optionally be substituted by 1, 2, 3, 4 or 5 identical or different substituents R¹²;
R¹⁰, R¹¹ and R¹² are each independently
a radical selected from the group consisting of F, Cl, Br, I, -CN, -CF₃, -SF₅, -OH, -O-C₁₋₁₀-alkyl, -O-C₂₋₁₀-alkenyl, -NH₂, -O-CF₃, -S-CF₃, -SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-_{C1-5}-alkyl, -S(=O)₂-phenyl, -O-S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl; where the cyclic moiety of the pyridinyl, cyclopentyl, cyclohexyl, pyridazinyl, -S(=O)₂-phenyl, -O-S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl;
a -C(=O)-NR¹³-(CH2)ₘ-NR¹⁴-R¹⁵ group where m is 0, 1, 2, 3, 4 or 5;
or a -C(=O)-R¹⁶ group;
R¹³ and R¹⁴ are each independently
a hydrogen radical
or is a linear or branched, saturated or unsaturated, optionally substituted C₁₋₁₀ aliphatic radical;
R¹⁵ is a 5- to 14-membered aryl or heteroaryl radical which may be fused to a saturated or unsaturated, optionally substituted mono- or polycyclic ring system;
and
R¹⁶ is a unsaturated or saturated, optionally substituted 3-, 4-, 5-, 6-, 7-, 8- or 9-membered cycloaliphatic radical which may be fused to a saturated or unsaturated, optionally substituted mono- or polycyclic ring system;
where
the aforementioned cycloaliphatic radicals may each optionally be substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-phenyl, -NH-pyridinyl, -N(C₁₋₅-alkyl)-phenyl, -N(C₁₋₅-alkyl)-pyridinyl, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, [1,2,5]-thiadiazolyl, pyridazinyl, -(CH₂)-benzo[b]furanyl, -O-phenyl, -O-benzyl, phenyl and benzyl, where the cyclic moiety of the -NH-phenyl, -NH-pyridinyl, -N(C₁₋₅-alkyl)-phenyl, -N(C₁₋₅-alkyl)-pyridinyl, pyridinyl, cyclopentyl, [1,2,5]-thiadiazolyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the aforementioned cycloaliphatic radicals may each optionally have 1, 2, 3, 4 or 5 heteroatom(s) selected independently from the group consisting of oxygen, nitrogen and sulfur as ring member(s);
the rings of the aforementioned mono- or polycyclic ring systems may each optionally be substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the rings of the aforementioned mono- or polycyclic ring systems are each 5-, 6- or 7-membered and may each optionally have 1, 2, 3, 4 or 5 heteroatom(s) as ring member(s) which are selected independently from the group consisting of oxygen, nitrogen and sulfur;
the aforementioned C₁₋₁₀ aliphatic radicals or C₁₋₂₀ aliphatic radicals may each optionally be substituted by 1, 2, 3, 4, 5, 6, 7, 8 or 9 substituents selected independently from the group consisting of F, Cl, Br, I, -CN, -NO₂, -OH, -SH and -NH₂;
the aforementioned aryl or heteroaryl radicals may each optionally be substituted by 1, 2, 3, 4 or 5 substituents selected independently from the group consisting of F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the aforementioned heteroaryl radicals may each optionally have 1, 2, 3, 4 or 5 heteroatom(s) selected independently from the group consisting of oxygen, nitrogen and sulfur as ring member(s);
and the aforementioned C₁₋₅-alkylene groups may each optionally be substituted by 1, 2, 3, 4 or 5 substituents selected independently from the group consisting of F, Cl, Br, -OH, -SH, -NH₂, -CN and NO₂;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

3. The use of a compound as claimed in claim 1 or 2, **characterized in that**
R¹ is a radical selected from the group consisting of cyclopentyl cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, thiomorpholinyl, azepanyl, (2,3)-dihydro-1 H-isoindolyl, (2,3)-dihydroindolyl, (2,3,4,9)-tetrahydro-1H-β-carbolinyl, (2,3,4,9)-tetrahydro-1H-pyrido[2,3-b]indolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl and [3,4]-dihydro-2H-1,4-benzoxazinyl; where the radical may in each case optionally be substituted by 1, 2, 3, 4 or 5 substituents selected independently from the group consisting of oxo (=O), thioxo (=S), F, Cl, Br, I, -SF₅, -OH, -O-CH₃, -O-C₂H₅, -NH₂, -NO₂, - O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, - C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-CH(CH₃)₂, -NH-C(CH₃)₃, - NH-phenyl, -NH-pyridinyl, -N(CH₃)-phenyl, -N(C₂H₅)-phenyl, - N[CH(CH₃)₂]-phenyl, -N(CH₃)-pyridinyl, -N(C₂H₅)-pyridinyl, -N[CH(CH₃)₂]-pyridinyl, -NH-C(=O)-O-CH₃, -NH-C(=O)-O-C₂H₅, -NH-C(=O)-O-C(CH₃)₃, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, pyridinyl, pyridazinyl and phenyl; where the cyclic moiety of the -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl, pyridinyl, pyridazinyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
is a piperazinyl radical which may be substituted by 1, 2 or 3 substituents selected independently from the group consisting of methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -C(=O)-C₁₋₅-alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
is a radical selected from the group consisting of phenyl and naphthyl, where the radical may in each case optionally be substituted by 1, 2, 3, 4 or 5 substituents selected independently from the group consisting of F, Cl, Br, I, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ and -NH-C(CH₃)₃;
is an NR³R⁴ group;
is an NR⁵-C(=O)-R⁶ group
or is an NR⁷-C(=O)-NR⁸R⁹ group.

4. The use of a compound as claimed in one or more of claims 1 to 3, **characterized in that**
R² is a 1,3-dihydroindol-2-onyl or a 1,3-dihydroindol-2-thionyl radical;
is CH-X;
is CH-CH₂-Y, CH-CH(CH₃)-Y, CH-CHCl-Y, CH-CHBr-Y, CH-CHF-Y or CH-CH(OH)-Y;
or is CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(phenyl)=CH-Z, CH-CBr=CH-Z, CH-CCl=CH-Z, CH-CF=CH-Z, CH-C(OH)=CH-Z, CH-CH₂-CH₂-Z, CH-CH₂-CH(CH₃)-Z or CH-CH(CH₃)-CH₂-Z.

5. The use of a compound as claimed in one or more of claims 1 to 4, **characterized in that**
R⁴ is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂.

6. The use of a compound as claimed in one or more of claims 1 to 5, **characterized in that**
R⁵, R⁷ and R⁸ are each independently
a hyddrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂.

7. The use of a compound as claimed in one or more of claims 1 to 6, **characterized in that**
R⁶ and R⁹ are each independently
a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

8. The use of a compound as claimed in one or more of claims 1 to 7, **characterized in that**
X is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl, which may optionally be substituted by 1, 2, 3, 4 or 5 identical or different substituents R¹⁰.

9. The use of a compound as claimed in one or more of claims 1 to 8, **characterized in that**
Y is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl, which may optionally be substituted by 1, 2, 3, 4 or 5 identical or different substituents R¹¹.

10. The use of a compound as claimed in one or more of claims 1 to 9, **characterized in that**
Z is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl and n-eicosanyl, where the radical may in each case optionally be substituted by 1, 2, 3, 4 or 5 substituents selected independently from the group consisting of F, Cl, Br, I, -CN, -NO₂, -OH, -SH and -NH₂;
or is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl, which may optionally be substituted by 1, 2, 3, 4 or 5 identical or different substituents R¹².

11. The use of a compound as claimed in one or more of claims 1 to 10, **characterized in that**
R¹⁰, R¹¹ and R¹² are each independently
a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl, n-octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, - N(C₂H₅)₂, -NH-CH₃, -NH-C₂H₅, -NH-C(=O)-O-CH₃, -NH-C(=O)-O-C₂H₅, - NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-phenyl, -O-S(=O)₂-phenyl, cyclohexyl, cyclopentyl, pyridinyl, pyridazinyl, -(CH₂)-benzo[b]furanyl, -O-phenyl, -O-benzyl, phenyl and benzyl; where the cyclic moiety of the pyridinyl, cyclopentyl, cyclohexyl, pyridazinyl, -S(=O)₂-phenyl, -O-S(=O)₂-phenyl, - O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, - CN, -NO₂ methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a -C(=O)-NR¹³-(CH₂)ₘ-NR¹⁴-R¹⁵ group where m is 0, 1, 2 or 3;
or a -C(=O)-R¹⁶ group;
where
R¹³ and R¹⁴ are each independently
a hydrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
R¹⁵ is a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃;
and
R¹⁶ is a radical selected from the group consisting of piperazinyl, thiomorpholinyl, azepanyl, morpholinyl, 2,3-dihydro-1 H-isoindolyl, 2,3-dihydroindolyl, piperidinyl and pyrrolidinyl; where the radical may be substituted by 1, 2 or 3 substituents selected independently from the group consisting of -OH, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -C(=O)-C₁₋₅-alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

12. The use of a compound as claimed in one or more of claims 1 to 11, **characterized in that**
R¹ is one of the following radicals 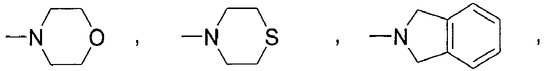 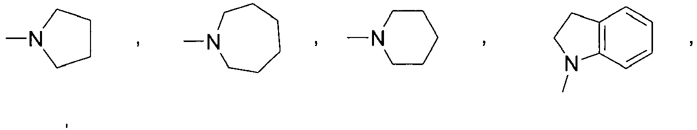 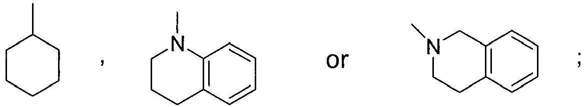 where the radical may be substituted by 1, 2 or 3 substituents selected independently from the group consisting of -OH, -O-CH₃, -O-C₂H₅, methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl and phenyl; where the cyclic moiety of the phenyl radical may in each case be substituted by 1, 2 or 3 substituents selected independently from the group consisting of F, Cl, Br and -CF₃;
is one of the following radicals 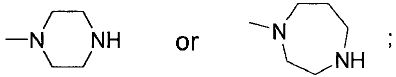 where the hydrogen atom of the -NH group may be replaced by a substituent selected from the group consisting of methyl, ethyl, n-propyl, isopropyl, n-butyl, sec-butyl, isobutyl, tert-butyl, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -0-C₂H₅, -O-CF₃ and -S-CF₃;
is a radical selected from the group consisting of phenyl and naphthyl, where the radical may in each case optionally be substituted by 1, 2, 3, 4 or 5 substituents selected independently from the group consisting of -OH, -O-CH₃ and -O-C₂H₅;
is an NR³R⁴ group
or is an NR⁷-C(=O)-NR⁸R⁹ group;
R² is a 1,3-dihydroindol-2-onyl or a 1,3-dihydroindol-2-thionyl radical;
is CH-X;
is CH-CH₂-Y, CH-CH(CH₃)-Y, CH-CHCl-Y, CH-CHBr-Y, CH-CHF-Y or CH-CH(OH)-Y;
or is CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(phenyl)=CH-Z, CH-CBr=CH-Z, CH-CCl=CH-Z, CH-CF=CH-Z, CH-C(OH)=CH-Z, CH-CH₂-CH₂-Z, CH-CH₂-CH(CH₃)-Z or CH-CH(CH₃)-CH₂-Z;
R³ is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl;
is a radical selected from the group consisting of cyclopentyl, cyclohexyl, cycloheptyl, cyclopentenyl, cyclohexenyl and cycloheptenyl; where the radical may in each case optionally be substituted by 1, 2, 3, 4 or 5 substituents selected independently from the group consisting of methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl;
or is a radical selected from the group consisting of phenyl, naphthyl, (1,3)-benzodioxolyl and (1,4)-benzodioxanyl; where the radical may be bonded via a -(CH₂)-, -(CH₂)-(CH₂) or-(CH₂)-(CH₂)-(CH₂) group and/or may in each case optionally be substituted by 1, 2, 3, 4 or 5 substituents selected independently from the group consisting of F, Cl, Br, -CF₃, -0-CF₃, -OCH₃, -OC₂H₅, methyl, ethyl, n-propyl, isopropyl, n-butyl and tert-butyl;
R⁴ is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl;
R⁷ and R⁸ are each independently
a hydrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
R⁹ is a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -O-CH₃, -O-C₂H₅, -O-CF₃, -S-CF₃, methyl, ethyl, n-propyl, isopropyl, n-butyl, sec-butyl, isobutyl and tert-butyl;
X is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl; which may optionally be substituted by 1, 2, 3, 4 or 5 identical or different substituents R¹⁰;
Y is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl; which may optionally be substituted by 1, 2, 3, 4 or 5 identical or different substituents R¹¹;
Z is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl and n-eicosanyl;
or is a radical selected from the group consisting of phenyl, naphthyl, phenanthrenyl, (1,3)-benzodioxolyl, (1,4)-benzodioxanyl, thiophenyl, furanyl, pyrrolyl, pyrazolyl, pyrazinyl, pyridinyl, pyridazinyl, pyrimidinyl, quinolinyl and isoquinolinyl; which may optionally be substituted by 1, 2, 3, 4 or 5 identical or different substituents R¹²;
R¹⁰ is a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl, n-octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-CH₃, -NH-C(=O)-C₂H₅, -NH-C(=O)-C(CH₃)₃, -O-S(=O)₂-phenyl, -O-phenyl, -O-benzyl, phenyl and benzyl;
is a -C(=O)-NR¹³-(CH₂)ₘ-N¹⁴-R¹⁵ group where m is 0, 1, 2 or 3;
or is a -C(=O)-R¹⁶ group;
R¹¹ is a radical selected from the group consisting of F, Cl, Br, -SF₅, methyl, ethyl, n-propyl, isopropyl, n-butyl, sec-butyl, tert-butyl, n-pentyl, n-hexyl, n-heptyl and n-octyl;
R¹² is a radical selected from the group consisting of F, Cl, Br, -SF₅, -CF₃, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, methyl, ethyl, n-propyl, isopropyl, n-butyl, sec-butyl, tert-butyl, n-pentyl, n-hexyl, n-heptyl and n-octyl;
are each a hydrogen radical;
R¹⁵ is a radical selected from the group consisting of phenyl and pyridinyl, where the radical may in each case optionally be substituted by 1, 2, 3, 4 or 5 substituents selected independently from the group consisting of F, Cl, Br, -CF₃, -O-CF₃, -S-CF₃, methyl, ethyl, n-propyl, isopropyl, n-butyl, sec-butyl, isobutyl and tert-butyl;
and
R¹⁶ is a radical selected from the group consisting of thiomorpholinyl, azepanyl, morpholinyl, (2,3)-dihydro-1H-isoindolyl, (2,3)-dihydroindolyl, piperidinyl and pyrrolidinyl; where the radical may be substituted by 1, 2 or 3 substituents selected independently from the group consisting of -OH, methyl, ethyl, n-propyl, isopropyl, n-butyl, sec-butyl, isobutyl, tert-butyl, benzyl and phenyl; where the cyclic moiety of the benzyl and phenyl radicals may in each case be substituted by 1, 2, 3, 4 or 5 substituents selected independently from the group consisting of F, Cl, Br and -CF₃;
or is one of the following radicals 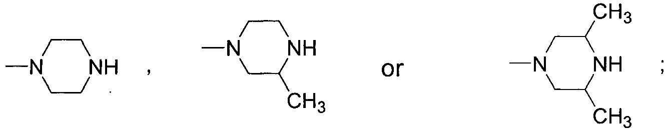 where the hydrogen atom of the -NH group may be replaced by a substituent selected from the group consisting of methyl, ethyl, n-propyl, isopropyl, n-butyl, sec-butyl, isobutyl, tert-butyl, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -O-C₂H₅, -O-CF₃ and -S-CF₃;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

13. The use of a compound as claimed in one or more of claims 1 to 12, **characterized in that**
R¹ is one of the following radicals 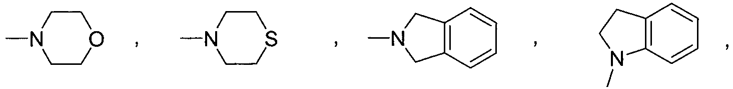 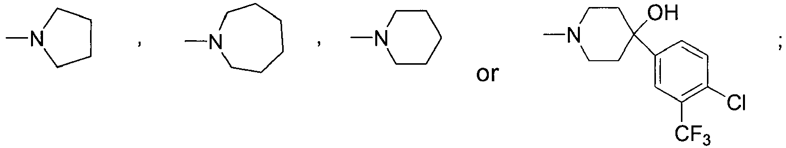 is the following radical 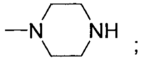 where the hydrogen atom of the -NH group may be replaced by a substituent selected from the group consisting of methyl, ethyl, n-propyl, isopropyl, n-butyl, sec-butyl, isobutyl, tert-butyl, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may be substituted in each case by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -O-C₂H₅, -O-CF₃ and -S-CF₃;
is a radical selected from the group consisting of phenyl and naphthyl, where the radical may in each case optionally be substituted by 1, 2, 3, 4 or 5 substituents selected independently from the group consisting of -OH, -O-CH₃ and -O-C₂H₅;
is an NR³R⁴ group
or is an NR⁷-C(=O)-NR⁸R⁹ group;
R² is a 1,3-dihydroindol-2-onyl or a 1,3-dihydroindol-2-thionyl radical;
is CH-X;
is CH-CH₂-Y, CH-CH(CH₃)-Y or CH-CH(OH)-Y;
or is CH-CH=C(CH₃)-Z, CH-CH=CH-Z, CH-CH=CH-S-Z, CH-C(CH₃)=CH-Z,
CH-C(phenyl)=CH-Z, CH-CBr=CH-Z, CH-CCl=CH-Z, CH-CF=CH-Z, CH-CH₂-CH₂-Z, CH-CH₂-CH(CH₃)-Z or CH-CH(CH₃)-CH₂-Z;
R³ is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl;
or is a radical selected from the group consisting of phenyl, naphthyl, benzyl, (1,3)-benzodioxolyl and (1,4)-benzodioxanyl; where the radical may in each case optionally be substituted by 1, 2, 3, 4 or 5 substituents selected independently from the group consisting of F, Cl, Br, -CF₃, -0-CF₃, -OCH₃, -OC₂H₅, methyl, ethyl, n-propyl, isopropyl, n-butyl and tert-butyl;
R⁴ is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl;
R⁷ and R⁸ are each a hydrogen radical;
R⁹ is a phenyl radical which may be substituted by 1, 2, 3, 4 or 5 substituents selected independently from the group consisting of F, Cl, Br and -CF₃;
X is a radical selected from the group consisting of phenyl, naphthyl, phenanthrenyl, (1,3)-benzodioxolyl, (1,4)-benzodioxanyl, thiophenyl, furanyl, pyridinyl, pyridazinyl, pyrazinyl, pyrimidinyl, quinolinyl and isoquinolinyl; which may be substituted by 1, 2, 3, 4 or 5 identical or different substituents R¹⁰;
Y is a phenyl or naphthyl radical which may be substituted by 1, 2, 3 4 or 5 identical or different substituents R¹¹;
Z is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl and n-octyl;
or is a phenyl or naphthyl radical which may be substituted by 1,2, 3, 4 or 5 identical or different substituents R¹²;
R¹⁰ is a radical selected from the group consisting of F, Cl, Br, I, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, methyl, ethyl, n-propyl, isopropyl, n-butyl, sec-butyl, tert-butyl, n-pentyl, n-hexyl, n-heptyl, n-octyl, -C(=O)-OH, -C(=O)-O-CH₃, -C(=O)-O-C₂H₅, -C(=O)-O-C(CH₃)₃, -N(CH₃)₂, -N(C₂H₅)₂, -NH-CH₃, -NH-C₂H₅, -NH-C(=O)-CH₃, -O-S(=O)₂-phenyl, -O-phenyl, -O-benzyl and phenyl;
is a -C(=O)-NR¹³-(CH₂)ₘ-NR¹⁴-R¹⁵ group where m is 0, 1, 2 or 3;
or is a -C(=O)-R¹⁶ group;
R¹¹ is a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl and n-octyl;
R¹² is a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl and n-octyl;
R¹³ and R¹⁴ are each a hydrogen radical;
R¹⁵ is a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -O-CF₃, -S-CF₃, methyl, ethyl, n-propyl, isopropyl, n-butyl,
sec-butyl, isobutyl and tert-butyl;
and
R¹⁶ is one of the following radicals 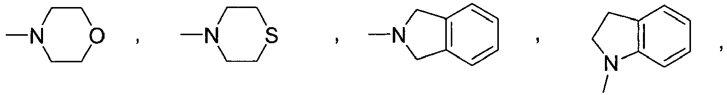 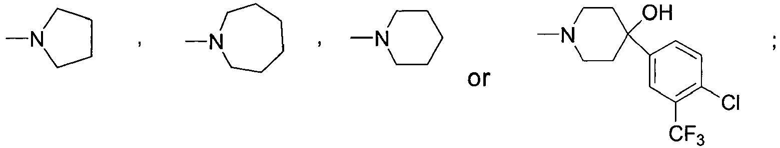 or is one of the following radicals 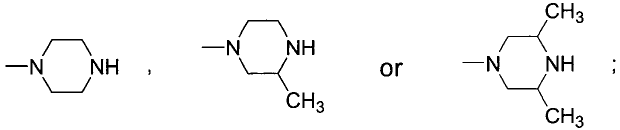 where the hydrogen atom of the -NH group may be replaced by a substituent selected from the group consisting of pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -O-C₂H₅, -O-CF₃ and -S-CF₃;
in each case, as appropriate, in the form of one of its pure stereoisomers, especially in enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

14. The use of a compound as claimed in one or more of claims 1 to 13, selected from the group consisting of
| | |
|---|---|
| 1 | 5-[3-(4-methoxyphenyl)but-2-enylidene]-2-morpholin-4-ylthiazol-4-one |
| 2 | 2-morpholin-4-yl-5-phenethylidenethiazol-4-one |
| 3 | 2-morpholin-4-yl-5-(2-o-tolylethylidene)thiazol-4-one |
| 4 | 2-morpholin-4-yl-5-(3-phenylallylidene)thiazol-4-one |
| 5 | 2-morpholin-4-yl-5-(3-phenylbutylidene)thiazol-4-one |
| 6 | 2-morpholin-4-yl-5-(3-phenylpropylidene)thiazol-4-one |
| 7 | 5-[3-(4-fluorophenyl)allylidene]-2-morpholin-4-ylthiazol-4-one |
| 8 | N-[4-(2-morpholin-4-yl-4-oxo-4H-thiazol-5-ylidenemethyl)phenyl]acetamide |
| 9 | 5-benzo[1,3]dioxol-5-ylmethylene-2-morpholin-4-ylthiazol-4-one |
| 10 | 2-morpholin-4-yl-5-(3-m-tolylallylidene)thiazol-4-one |
| 11 | 5-(3-iodo-4,5-dimethoxybenzylidene)-2-morpholin-4-ylthiazol-4-one |
| 12 | 2-morpholin-4-yl-5-(3-phenylsulfanylallylidene)thiazol-4-one |
| 13 | 5-(3-benzyloxy-4-methoxybenzylidene)-2-morpholin-4-ylthiazol-4-one |
| 14 | 5-(4-methylbenzylidene)-2-thiomorpholin-4-ylthiazol-4-one |
| 15 | N-[4-(2-morpholin-4-yl-4-oxo-4H-thiazol-5-ylidenemethyl)phenyl]amine |
| 16 | 5-(4-butoxybenzylidene)-2-morpholin-4-ylthiazol-4-one |
| 17 | 2-morpholin-4-yl-5-(4-phenoxybenzylidene)thiazol-4-one |
| 18 | 2-morpholin-4-yl-5-(3-phenoxybenzylidene)thiazol-4-one |
| 19 | 2-morpholin-4-yl-5-(3-p-tolylallylidene)thiazol-4-one |
| 20 | 5-(3-benzyloxybenzylidene)-2-morpholin-4-ylthiazol-4-one |
| 21 | 5-(4-benzyloxybenzylidene)-2-morpholin-4-ylthiazol-4-one |
| 22 | 2-thiomorpholin-4-yl-5-(2-trifluoromethylbenzylidene)thiazol-4-one |
| 23 | 5-(4-bromobenzylidene)-2-thiomorpholin-4-ylthiazol-4-one |
| 24 | 2-thiomorpholin-4-yl-5-(4-trifluoromethoxybenzylidene)thiazol-4-one |
| 25 | 5-(3-phenylallylidene)-2-thiomorpholin-4-ylthiazol-4-one |
| 26 | 2-morpholin-4-yl-5-octylidenethiazol-4-one |
| 27 | 5-(4-benzyloxy-3-methoxybenzylidene)-2-morpholin-4-ylthiazol-4-one |
| 28 | 5-(3,4-bis(benzyloxy)benzylidene)-2-morpholin-4-ylthiazol-4-one |
| 29 | 5-butylidene-2-morpholin-4-ylthiazol-4-one |
| 30 | 2-[4-(3-chloropyridin-2-yl)piperazin-1-yl]-5-(4-methylbenzylidene)thiazol-4-one |
| 31 | 2-[4-(3-chloropyridin-2-yl)piperazin-1-yl]-5-(3-phenylallylidene)thiazol-4-one |
| 32 | 5-hexylidene-2-morpholin-4-ylthiazol-4-one |
| 33 | 2-morpholin-4-yl-5-(3-p-tolylbut-2-enylidene)thiazol-4-one |
| 34 | 5-[3-(4-tert-butylphenyl)but-2-enylidene]-2-morpholin-4-ylthiazol-4-one |
| 35 | 5-(4-methoxynaphthalen-1-ylmethylene)-2-morpholin-4-ylthiazol-4-one |
| 36 | 2-morpholin-4-yl-5-(4-trifluoromethoxybenzylidene)thiazol-4-one |
| 37 | 2-[4-(3-methoxyphenyl)piperazin-1-yl]-5-(3-phenylallylidene)thiazol-4-one |
| 38 | 5-[3-(4-tert-butylphenyl)allylidene]-2-[4-(3-methoxyphenyl)piperazin-1-yl]-thiazol-4-one |
| 39 | 5-(4-hydroxy-3-methoxybenzylidene)-2-thiomorpholin-4-ylthiazol-4-one |
| 40 | 5-[3-(4-hydroxy-3-methoxyphenyl)allylidene]-2-thiomorpholin-4-ylthiazol-4-one |
| 41 | 5-naphthalen-1-ylmethylene-2-thiomorpholin-4-ylthiazol-4-one |
| 42 | 5-benzylidene-2-thiomorpholin-4-ylthiazol-4-one |
| 43 | 2-morpholin-4-yl-5-[3-(4-trifluoromethylphenyl)but-2-enylidene]thiazol-4-one |
| 44 | 2-morpholin-4-yl-5-[3-(3-trifluoromethylphenyl)but-2-enylidene]thiazol-4-one |
| 45 | 5-(3-methylbutylidene)-2-morpholin-4-ylthiazol-4-one |
| 46 | 2-[4-(3-chloropyridin-2-yl)piperazin-1-yl]-5-(2-trifluoromethyl-benzylidene)thiazol-4-one |
| 47 | 5-(4-bromobenzylidene)-2-[4-(3-chloropyridin-2-yl)piperazin-1-yl]-thiazol-4-one |
| 48 | 5-benzylidene-2-[4-(3-chloropyridin-2-yl)piperazin-1-yl]thiazol-4-one |
| 49 | 2-[4-(3-chloropyridin-2-yl)piperazin-1-yl]-5-naphthalen-2-ylmethylenethiazol-4-one |
| 50 | 5-(2,3-diphenylallylidene)-2-morpholin-4-ylthiazol-4-one |
| 51 | 2-morpholin-4-yl-5-phenanthren-9-ylmethylenethiazol-4-one |
| 52 | 2-morpholin-4-yl-5-quinolin-3-ylmethylenethiazol-4-one |
| 53 | 2-(4-ethylpiperazin-1-yl)-5-(4-methylbenzylidene)thiazol-4-one |
| 54 | 5-naphthalen-2-ylmethylene-2-thiomorpholin-4-ylthiazol-4-one |
| 55 | 2-[4-(4-methoxyphenyl)piperazin-1-yl]-5-(3-phenylallylidene)thiazol-4-one |
| 56 | 2-[4-(2-chlorophenyl)piperazin-1-yl]-5-(3-phenylallylidene)thiazol-4-one |
| 57 | 5-(3-phenylallylidene)-2-(4-phenylpiperazin-1-yl)thiazol-4-one |
| 58 | 5-decylidene-2-morpholin-4-ylthiazol-4-one |
| 59 | 5-[3-(4-tert-butylphenyl)allylidene]-2-morpholin-4-ylthiazol-4-one |
| 60 | 2-(2-morpholin-4-yl-4-oxo-4H-thiazol-5-ylidenemethyl)benzoic acid |
| 61 | 5-(2-bromo-3-phenylallylidene)-2-[4-(3-chloropyridin-2-yl)piperazin-1-yl]thiazol-4-one |
| 62 | 5-[3-(4-tert-butylphenyl)allylidene]-2-[4-(3-chloropyridin-2-yl)piperazin-1-yl]-thiazol-4-one |
| 63 | 2-(4-benzylpiperazin-1-yl)-5-(4-methylbenzylidene)thiazol-4-one |
| 64 | 2-(4-phenylpiperazin-1-yl)-5-quinolin-3-ylmethylenethiazol-4-one |
| 65 | 2-[4-(3-chloropyridin-2-yl)piperazin-1-yl]-5-(2-methyl-3-phenyl-allylidene)thiazol-4-one |
| 66 | 2-[4-(3-chloropyridin-2-yl)piperazin-1 -yl]-5-naphthalen-1 -ylmethylenethiazol-4-one |
| 67 | 5-(2-chloro-3-phenylallylidene)-2-[4-(3-chloropyridin-2-yl)piperazin-1-yl]thiazol-4-one |
| 68 | 5-(4-hydroxy-3-methoxybenzylidene)-2-(4-phenylpiperazin-1-yl)thiazol-4-one |
| 69 | 5-phenanthren-9-ylmethylene-2-(4-phenylpiperazin-1-yl)thiazol-4-one |
| 70 | 5-(6-methoxynaphthalen-2-ylmethylene)-2-(4-phenylpiperazin-1-yl)thiazol-4-one |
| 71 | 5-naphthalen-1-ylmethylene-2-(4-phenylpiperazin-1-yl)th iazol-4-one |
| 72 | 5-(4-bromobenzylidene)-2-(4-phenylpiperazin-1-yl)thiazol-4-one |
| 73 | 5-(4-methylbenzylidene)-2-(4-phenylpiperazin-1-yl)thiazol-4-one |
| 74 | 2-(4-phenylpiperazin-1-yl)-5-(2-trifluoromethylbenzylidene)thiazol-4-one |
| 75 | 3-(4-oxo-2-thiomorpholin-4-yl-4H-thiazol-5-ylidene)-1,3-dihydroindol-2-one |
| 76 | 3-(2-morpholin-4-yl-4-oxo-4H-thiazol-5-ylidene)-1,3-dihydroindol-2-one |
| 77 | 2-[4-(2-fluorophenyl)piperazin-1-yl]-5-(4-methylbenzylidene)thiazol-4-one |
| 78 | 5-(4-tert-butylbenzylidene)-2-[4-(2-fluorophenyl)piperazin-1 -yl]thiazol-4-one |
| 79 | 2-[4-(2-fluorophenyl)piperazin-1-yl]-5-(3-phenylallylidene)thiazol-4-one |
| 80 | 5-[3-(4-tert-butylphenyl)allylidene]-2-[4-(2-fluorophenyl)piperazin-1-yl]thiazol-4-one |
| 81 | 2-[4-(2-fluorophenyl)piperazin-1-yl]-5-(4-trifluoromethylbenzylidene)thiazol-4-one |
| 82 | 2-[4-(2-fluorophenyl)piperazin-1-yl]-5-(4-trifluoromethoxybenzylidene)thiazol-4-one ' |
| 83 | 5-(2-chloro-3-phenylallylidene)-2-[4-(2-fluorophenyl)piperazin-1-yl]thiazol-4-one |
| 84 | 2-[4-(2-fluorophenyl)piperazin-1-yl]-5-naphthalen-1-ylmethylenethiazol-4-one |
| 85 | 5-(2-chloro-3-phenylallylidene)-2-thiomorpholin-4-ylthiazol-4-one |
| 86 | 3-(4-oxo-2-thiomorpholin-4-yl-4H-thiazol-5-ylidenemethyl)benzoic acid |
| 87 | 4-(4-oxo-2-thiomorpholin-4-yl-4H-thiazol-5-ylidenemethyl)benzoic acid |
| 88 | 5-{3-[4-(3-chloropyridin-2-yl)piperazine-1-carbonyl]benzylidene}-2-thio-morpholin-4-ylthiazol-4-one |
| 89 | 5-{4-[4-(3-chloropyridin-2-yl)piperazine-1-carbonyl]benzylidene}-2-thio-morpholin-4-ylthiazol-4-one |
| 90 | 2-[4-(3-chloropyridin-2-yl)piperazin-1-yl]-5-quinolin-3-ylmethylenethiazol-4-one |
| 91 | 2-[4-(3-chloropyridin-2-yl)piperazin-1-yl]-5-(4-trifluoromethyl-benzylidene)thiazol-4-one |
| 92 | 2-(2,3-dihydroindol-1-yl)-5-(4-methylbenzylidene)thiazol-4-one |
| 93 | 2-(2,3-dihydroindol-1-yl)-5-(4-trifluoromethylbenzylidene)thiazol-4-one |
| 94 | 5-[4-(morpholine-4-carbonyl)benzylidene]-2-thiomorpholin-4-ylthiazol-4-one |
| 95 | 2-[4-(3-chloropyridin-2-yl)piperazin-1-yl]-5-(4-hydroxy-3-methoxy-benzylidene)thiazol-4-one |
| 96 | 1-[4-oxo-5-(4-trifluoromethylbenzylidene)-4,5-dihydrothiazol-2-yl]-3-(2-trifluoromethylphenyl)urea |
| 97 | 2-(4-benzylpiperazin-1-yl)-5-quinolin-3-ylmethylenethiazol-4-one |
| 98 | 2-(4-benzylpiperazin-1-yl)-5-naphthalen-2-ylmethylenethiazol-4-one |
| 99 | 2-(4-benzylpiperazin-1-yl)-5-(3-phenoxybenzytidene)thiazol-4-one |
| 100 | 2-(4-benzylpiperazin-1-yl)-5-(4-trifluoromethylbenzylidene)thiazol-4-one |
| 101 | 1-[5-(4-methylbenzylidene)-4-oxo-4,5-dihydrothiazol-2-yl]-3-(2-trifluoro-methylphenyl)urea |
| 102 | 2-(4-benzylpiperazin-1-yl)-5-naphthalen-1-ylmethylenethiazol-4-one |
| 103 | 2-(4-benzylpiperazin-1-yl)-5-(6-methoxynaphthalen-2-ylmethylene)thiazol-4-one |
| 104 | 2-(4-benzylpiperazin-1-yl)-5-(4-tert-butylbenzylidene)thiazol-4-one |
| 105 | 1-[4-oxo-5-(3-phenylallylidene)-4,5-dihydrothiazol-2-yl]-3-(2-trifluoro-methylphenyl)urea |
| 106 | 1-[4-oxo-5-(4-trifluoromethylbenzylidene)-4,5-dihydrothiazol-2-yl]-3-(4-trifluoromethylphenyl)urea |
| 107 | 1-[5-(4-methylbenzylidene)-4-oxo-4,5-dihydrothiazol-2-yl]-3-(4-trifluoro-methylphenyl)urea |
| 108 | 5-{3-[4-(6-chloropyridin-2-yl)piperazine-1-carbonyl]benzylidene}-2-thio-morpholin-4-ylthiazol-4-one |
| 109 | 2-(4-benzylpiperazin-1-yl)-5-(4-trifluoromethoxybenzylidene)thiazol-4-one |
| 110 | 5-{3-[4-(3-chloropyridin-2-yl)-3-methylpiperazine-1-carbonyl]benzylidene}-2-thiomorpholin-4-ylthiazol-4-one |
| 111 | N-[3-(3-chloro-5-trifluoromethylpyridin-2-ylamino)propyl]-3-(4-oxo-2-thiomorpholin-4-yl-4H-thiazol-5-ylidenemethyl)benzamide |
| 112 | 3-(4-oxo-2-thiomorpholin-4-yl-4H-thiazol-5-ylidenemethyl)benzoic acid N'-(2-chloro-4-trifluoromethylphenyl)hydrazide |
| 113 | 5-{3-[4-(3-chloro-5-trifluoromethylpyridin-2-yl)piperazine-1-carbonyl]-benzylidene}-2-thiomorpholin-4-ylthiazol-4-one |
| 114 | 3-(4-oxo-2-thiomorpholin-4-yl-4H-thiazol-5-ylidenemethyl)benzoic acid N'-(3-chloro-5-trifluoromethylpyridin-2-yl)hydrazide |
| 115 | 5-{3-[4-(4-chloro-[1,2,5]thiadiazol-3-yl)piperazine-1-carbonyl]benzylidene}-2-thiomorpholin-4-ylthiazol-4-one |
| 116 | 3-(4-oxo-2-thiomorpholin-4-yl-4H-thiazol-5-ylidenemethyl)benzoic acid N'-(2-chloro-5-trifluoromethylphenyl)hydrazide |
| 117 | 2-[4-(4-chloro-[1,2,5]thiadiazol-3-yl)piperazin-1-yl]-5-(4-methyl-benzylidene)thiazol-4-one |
| 118 | 2-[4-(4-chloro-[1,2,5]thiadiazol-3-yl)piperazin-1-yl]-5-(4-trifluoromethyl-benzylidene)thiazol-4-one |
| 119 | 2-[4-(4-chloro-[1,2,5]thiadiazol-3-yl)piperazin-1-yl]-5-(4-trifluoro-methoxybenzylidene)thiazol-4-one |
| 120 | 5-(4-tert-butylbenzylidene)-2-[4-(4-chloro-[1,2,5]thiadiazol-3-yl)piperazin-1-yl]thiazol-4-one |
| 121 | 2-[4-(4-chloro-[1,2,5]thiadiazol-3-yl)piperazin-1-yl]-5-(3-phenyl-allylidene)thiazol-4-one |
| 122 | 5-[3-(4-tert-butylphenyl)allylidene]-2-[4-(4-chloro-[1,2,5]thiadiazol-3-yl)-piperazin-1-yl]thiazol-4-one |
| 123 | 2-[4-(4-chloro-[1,2,5]thiadiazol-3-yl)piperazin-1-yl]-5-naphthalen-1-ylmethylenethiazol-4-one |
| 124 | 2-[4-(4-chloro-[1,2,5]thiadiazol-3-yl)piperazin-1-yl]-5-(3-phenoxy-benzylidene)thiazol-4-one |
| 125 | 2-[4-(4-chloro-[1,2,5]thiadiazol-3-yl)piperazin-1-yl]-5-(4-phenoxy-benzylidene)thiazol-4-one |
| 126 | 5-(3-benzyloxybenzylidene)-2-[4-(4-chloro-[1,2,5]thiadiazol-3-yl)piperazin-1-yl]thiazol-4-one |
| 127 | 2-[4-(4-chloro-[1,2,5]thiadiazol-3-yl)piperazin-1-yl]-5-(4-hydroxy-3-methoxybenzylidene)thiazol-4-one |
| 128 | 2-[4-(4-chloro-[1,2,5]thiadiazol-3-yl)piperazin-1-yl]-5-furan-2-yl-methylenethiazol-4-one |
| 129 | 2-[4-(4-chloro-[1,2,5]thiadiazol-3-yl)piperazin-1-yl]-5-naphthalen-2-yl-methylenethiazol-4-one |
| 130 | 3-[2-(4-benzylpiperazin-1-yl)-4-oxo-4H-thiazol-5-ylidenemethyl]benzoic acid |
| 131 | 5-naphthalen-1-ylmethylene-2-piperazin-1-ylthiazol-4-one |
| 132 | 2-piperazin-1-yl-5-(4-trifluoromethylbenzylidene)thiazol-4-one |
| 133 | 5-(4-methylbenzylidene)-2-piperazin-1-ylthiazol-4-one |
| 134 | 5-(4-hydroxy-3-methoxybenzylidene)-2-piperazin-1-ylthiazol-4-one |
| 135 | 5-(4-isopropylbenzylidene)-2-morpholin-4-ylthiazol-4-one |
| 136 | 5-(4-isopropylbenzylidene)-2-thiomorpholin-4-ylthiazol-4-one |
| 137 | 2-[4-(2-fluorophenyl)piperazin-1-yl]-5-(4-isopropylbenzylidene)thiazol-4-one |
| 138 | 5-(4-pentafluorosulfanyl)benzylidene)-2-thiomorpholin-4-ylthiazol-4-one |
| 139 | 2-[4-(3-chloropyridin-2-yl)piperazin-1-yl]-5-(4-isopropylbenzylidene)thiazol-4-one |
| 140 | 2-(4-benzylpiperazin-1-yl)-5-(4-butylbenzylidene)thiazol-4-one |
| 141 | 2-(4-benzylpiperazin-1-yl)-5-(4-pentylbenzylidene)thiazol-4-one |
| 142 | 2-(4-benzylpiperazin-1-yl)-5-(4-octylbenzylidene)thiazol-4-one |
| 143 | 2-[4-(6-chloropyridin-2-yl)piperazin-1-yl]-5-(4-methylbenzylidene)thiazol-4-one |
| 144 | 5-(4-tert-butylbenzylidene)-2-[4-(6-chloropyridin-2-yl)piperazin-1-yl]thiazol-4-one |
| 145 | 2-[4-(6-chloropyridin-2-yl)piperazin-1-yl]-5-(4-trifluoromethyl-benzylidene)thiazol-4-one |
| 146 | 2-[4-(6-chloropyridin-2-yl)piperazin-1-yl]-5-(3-phenylallylidene)thiazol-4-one |
| 147 | 5-[3-(4-tert-butylphenyl)allylidene]-2-[4-(6-chloropyridin-2-yl)piperazin-1-yl]thiazol-4-one |
| 148 | 2-[4-(6-chloropyridin-2-yl)piperazin-1-yl]-5-(4-hydroxy-3-methoxy-benzylidene)thiazol-4-one |
| 149 | 2-[4-(6-chloropyridin-2-yl)piperazin-1-yl]-5-(4-isopropylbenzylidene)thiazol-4-one |
| 150 | 2-[4-(6-chloropyridin-2-yl)piperazin-1-yl]-5-(4-trifluoromethoxy-benzylidene)thiazol-4-one |
| 151 | 2-[4-(6-chloropyridin-2-yl)piperazin-1-yl]-5-naphthalen-2-ylmethylenethiazol-4-one |
| 152 | 5-(4-methylbenzylidene)-2-[4-(6-methylpyridazin-3-yl)piperazin-1-yl]thiazol-4-one |
| 153 | 5-(4-isopropylbenzylidene)-2-[4-(6-methylpyridazin-3-yl)piperazin-1-yl]thiazol-4-one |
| 154 | 5-(4-tert-butylbenzylidene)-2-[4-(6-methylpyridazin-3-yl)piperazin-1-yl]thiazol-4-one |
| 155 | 5-[3-(4-tert-butylphenyl)allylidene]-2-[4-(6-methylpyridazin-3-yl)piperazin-1-yl]thiazol-4-one |
| 156 | 2-[4-(6-methylpyridazin-3-yl)piperazin-1-yl]-5-(4-trifluoromethoxy-benzylidene)thiazol-4-one |
| 157 | 5-(4-hydroxy-3-methoxybenzylidene)-2-[4-(6-methylpyridazin-3-yl)piperazin-1-yl]thiazol-4-one |
| 158 | 2-(4-benzylpiperazin-1-yl)-5-(4-pentafluorosulfanylbenzylidene)thiazol-4-one |
| 159 | 2-(4-benzylpiperazin-1-yl)-5-{3-[4-(4-chloro-[1,2,5]thiadiazol-3-yl)piperazine-1-carbonyl]benzylidene}thiazol-4-one |
| 160 | 2-[4-(6-methylpyridazin-3-yl)piperazin-1-yl]-5-(4-trifluoromethyl-benzylidene)thiazol-4-one |
| 161 | 2-[4-(6-methylpyridazin-3-yl)piperazin-1-yl]-5-(3-phenylallylidene)thiazol-4-one |
| 162 | 2-[4-(6-methylpyridazin-3-yl)piperazin-1-yl]-5-naphthalen-2-yl-methylenethiazol-4-one |
| 163 | 2-[4-(6-methylpyridazin-3-yl)piperazin-1-yl]-5-naphthalen-1-yl-methylenethiazol-4-one |
| 164 | 2-(4-benzylpiperazin-1-yl)-5-(4-isopropylbenzylidene)thiazol-4-one |
| 165 | 3-{2-[4-(4-chloro-[1,2,5]thiadiazol-3-yl)piperazin-1-yl]-4-oxo-4H-thiazol-5-ylidenemethyl}benzoic acid |
| 166 | 5-{3-[4-(4-chloro-3-trifluoromethylphenyl)-4-hydroxypiperidine-1-carbonyl]benzylidene}-2-thiomorpholin-4-ylthiazol-4-one |
| 167 | 5-{3-[4-(4-chloro-[1,2,5]thiadiazol-3-yl)piperazine-1-carbonyl]benzylidene}-2-[4-(4-chloro-[1,2,5]thiadiazol-3-yl)piperazin-1-yl]thiazol-4-one |
| 168 | 5-{3-[4-(3-chloropyridin-2-yl)piperazine-1-carbonyl]benzylidene}-2-[4-(4-chloro-[1,2,5]thiadiazol-3-yl)piperazin-1-yl]thiazol-4-one |
| 169 | 5-[3-(4-benzyipiperazine-1-carbonyl)benzylidene]-2-[4-(4-chloro-[1,2,5]thiadiazol-3-yl)piperazin-1-yl]thiazol-4-one |
| 170 | 2-[4-(4-chloro-[1,2,5]thiadiazol-3-yl)piperazin-1-yl]-5-{3-[4-(6-methoxy-pyridin-2-yl)piperazine-1-carbonyl]benzylidene}thiazol-4-one |
| 171 | 2-[4-(4-chloro-3-trifluoromethylphenyl)-4-hyd roxypiperid in-1-yl]-5-(4-methylbenzylidene)thiazol-4-one |
| 172 | 2-[4-(4-chloro-3-trifluoromethylphenyl)-4-hydroxypiperidin-1-yl]-5-(4-hydroxy-3-methoxybenzylidene)thiazol-4-one |
| 173 | 2-[4-(4-chloro-3-trifluoromethylphenyl)-4-hydroxypiperidin-1-yl]-5-naphthalen-1-ylmethylenethiazol-4-one |
| 174 | 2-[4-(4-chloro-3-trifluoromethylphenyl)-4-hydroxypiperidin-1-yl]-5-(3-methoxybenzylidene)thiazol-4-one |
| 175 | 5-benzylidene-2-morpholin-4-ylthiazol-4-one |
| 176 | 5-(4-methylbenzylidene)-2-morpholin-4-ylthiazol-4-one |
| 177 | 2-diethylamino-5-(4-methylbenzylidene)thiazol-4-one |
| 178 | 5-(4-chlorobenzylidene)-2-diethylaminothiazol-4-one |
| 179 | 5-(4-isopropylbenzylidene)-2-morpholin-4-ylthiazol-4-one |
| 180 | 5-benzylidene-2-diethylaminothiazol-4-one |
| 181 | 5-(4-chlorobenzylidene)-2-morpholin-4-ylthiazol-4-one |
| 182 | 5-(4-chlorobenzylidene)-2-piperidin-1-ylthiazol-4-one |
| 183 | 5-(4-chlorobenzylidene)-2-pyrrolidin-1-ylthiazol-4-one |
| 184 | 5-benzylidene-2-pyrrolidin-1-ylthiazol-4-one |
| 185 | 5-benzylidene-2-pyrrolidin-1-ylthiazol-4-one |
| 186 | 5-biphenyl-4-ylmethylene-2-piperidin-1-ylthiazol-4-one |
| 187 | 2-azepan-1-yl-5-biphenyl-4-ylmethylenethiazol-4-one |
| 188 | 5-biphenyl-4-ylmethylene-2-pyrrolidin-1-ylthiazol-4-one |
| 189 | 2-morpholin-4-yl-5-(3-phenylallylidene)thiazol-4-one |
| 190 | 5-(3-phenylallylidene)-2-thiomorpholin-4-ylthiazol-4-one |
| 191 | 5-(3-phenylallylidene)-2-piperidin-1-ylthiazol-4-one |
| 192 | 2-azepan-1-yl-5-(3-phenylallylidene)thiazol-4-one |
| 193 | 5-(3-phenylallylidene)-2-pyrrolidin-1-ylthiazol-4-one |
| 194 | 5-biphenyl-4-ylmethylene-2-morpholin-4-ylthiazol-4-one |
| 195 | 2-thiomorpholin-4-yl-5-(3-trifluoromethylbenzylidene)thiazol-4-one |
| 196 | 5-(4-chlorobenzylidene)-2-thiomorpholin-4-ylthiazol-4-one |
| 197 | 5-(4-tert-butylbenzylidene)-2-morpholin-4-ylthiazol-4-one |
| 198 | 5-(4-tert-butylbenzylidene)-2-thiomorpholin-4-ylthiazol-4-one |
| 199 | 5-(4-tert-butylbenzylidene)-2-pyrrolidin-1-ylthiazol-4-one |
| 200 | 2-azepan-1-yl-5-(4-tert-butylbenzylidene)thiazol-4-one |
| 201 | 5-(4-tert-butylbenzylidene)-2-piperidin-1-ylthiazol-4-one |
| 202 | 2-thiomorpholin-4-yl-5-(4-trifluoromethylbenzylidene)thiazol-4-one |
| 203 | 5-(4-tert-butylbenzylidene)-2-diethylaminothiazol-4-one |
| 204 | 2-morpholin-4-yl-5-(4-trifluoromethylbenzylidene)thiazol-4-one |
| 205 | 2-morpholin-4-yl-5-(3-trifluoromethylbenzylidene)thiazol-4-one |
| 206 | 2-morpholin-4-yl-5-(3-trifluoromethylbenzylidene)thiazol-4-one |
| 207 | 5-biphenyl-4-ylmethylene-2-diethylaminothiazol-4-one |
| 208 | 2-morpholin-4-yl-5-thiophen-2-ylmethylenethiazol-4-one |
| 209 | 2-morpholin-4-yl-5-naphthalen-1-ylmethylenethiazol-4-one |
| 210 | 2-morpholin-4-yl-5-pyridin-2-ylmethylenethiazol-4-one |
| 211 | 2-morpholin-4-yl-5-(4-trifluoromethylbenzylidene)thiazol-4-one |
| 212 | 2-morpholin-4-yl-5-pyridin-3-ylmethylenethiazol-4-one |
| 213 | 2-morpholin-4-yl-5-pyridin-4-ylmethylenethiazol-4-one |
| 214 | 2-diethylamino-5-(3-trifluoromethylbenzylidene)thiazol-4-one |
| 215 | 5-(4-methylbenzylidene)-2-pyrrolidin-1-ylthiazol-4-one |
| 216 | 5-(4-tert-butylbenzylidene)-2-(2-methoxyethylamino)thiazol-4-one |
| 217 | 2-morpholin-4-yl-5-(4-octylbenzylidene)thiazol-4-one |
| 218 | 5-(4-methylbenzylidene)-2-morpholin-4-ylthiazol-4-one |
| 219 | 5-(3,4-dimethoxybenzylidene)-2-morpholin-4-ylthiazol-4-one |
| 220 | 5-(2-hydroxybenzylidene)-2-morpholin-4-ylthiazol-4-one |
| 221 | 5-(2-hydroxy-3-methoxybenzylidene)-2-morpholin-4-ylthiazol-4-one |
| 222 | 5-(4-tert-butylbenzylidene)-2-morpholin-4-ylthiazol-4-one |
| 223 | 5-(4-diethylaminobenzylidene)-2-morpholin-4-ylthiazol-4-one |
| 224 | 5-(4-hydroxy-3-methoxybenzylidene)-2-morpholin-4-ylthiazol-4-one |
| 225 | 4-(2-morpholin-4-yl-4-oxo-4H-thiazol-5-ylidenemethyl)benzoic acid |
| 226 | 2-morpholin-4-yl-5-(2,3,4-trimethoxybenzylidene)thiazol-4-one |
| 227 | 2-morpholin-4-yl-5-(3,4,5-trimethoxybenzylidene)thiazol-4-one |
| 228 | 5-(4-methoxybenzylidene)-2-morpholin-4-ylthiazol-4-one |
| 229 | 5-(4-hydroxybenzylidene)-2-morpholin-4-ylthiazol-4-one |
| 230 | 5-(3-ethoxy-4-hydroxybenzylidene)-2-morpholin-4-ylthiazol-4-one |
| 231 | 5-(3-hydroxybenzylidene)-2-morpholin-4-ylthiazol-4-one |
| 232 | 5-(4-bromobenzylidene)-2-morpholin-4-ylthiazol-4-one |
| 233 | 2-morpholin-4-yl-5-(4-vinyloxybenzylidene)thiazol-4-one |
| 234 | benzenesulfonic acid 4-(2-morpholin-4-yl-4-oxo-4H-thiazol-5-ylidenemethyl)phenyl ester |
| 235 | 5-(4-dimethylaminobenzylidene)-2-morpholin-4-ylthiazol-4-one |
| 236 | 4-(2-morpholin-4-yl-4-oxo-4H-thiazol-5-ylidenemethyl)benzoic acid methyl ester |
| 237 | 5-(3-hydroxy-4-methoxybenzylidene)-2-morpholin-4-ylthiazol-4-one |
| 238 | 5-[4-(3,3-dimethylbutoxy)-3-methoxybenzylidene]-2-morpholin-4-ylthiazol-4-one |
| 239 | 5-(2-methoxybenzylidene)-2-morpholin-4-ylthiazol-4-one |
| 240 | 5-(4-hydroxybenzylidene)-2-morpholin-4-ylthiazol-4-one |
| 241 | 2-[(benzo[1,3]dioxol-5-ylmethyl)amino]-5-[3-(4-tert-butylphenyl)-allylidene]thiazol-4-one |
| 242 | 2-[(benzo[1,3]dioxol-5-ylmethyl)amino]-5-(4-trifluoromethyl-benzylidene)thiazol-4-one |
| 243 | 2-[(benzo[1,3]dioxol-5-ylmethyl)amino]-5-(4-trifluoromethoxy-benzylidene)thiazol-4-one |
| 244 | 2-[(benzo[1,3]dioxol-5-ylmethyl)amino]-5-naphthalen-1-ylmethylenethiazol-4-one |
| 245 | 2-[(benzo[1,3]dioxol-5-ylmethyl)amino]-5-(2-chloro-3-phenylallylidene)thiazol-4-one |
| 246 | 2-(4-methylbenzylamino)-5-naphthalen-1-ylmethylenethiazol-4-one |
| 247 | 2-(4-methylbenzylamino)-5-(3-phenoxybenzylidene)thiazol-4-one |
| 248 | 2-(4-methylbenzylamino)-5-(4-phenoxybenzylidene)thiazol-4-one |
| 249 | 5-(2-chloro-3-phenylallylidene)-2-(4-methylbenzylamino)thiazol-4-one |
| 250 | 2-(4-methylbenzylamino)-5-naphthalen-2-ylmethylenethiazol-4-one |
| 251 | 2-(3-methoxybenzylamino)-5-(4-methylbenzylidene)thiazol-4-one |
| 252 | 5-(4-tert-butylbenzylidene)-2-(3-methoxybenzylamino)thiazol-4-one |
| 253 | 2-(3-methoxybenzylamino)-5-(4-trifluoromethylbenzylidene)thiazol-4-one |
| 254 | 2-(3-methoxybenzylamino)-5-(4-trifluoromethoxybenzylidene)thiazol-4-one |
| 255 | 2-(3-methoxybenzylamino)-5-(3-phenylallylidene)thiazol-4-one |
| 256 | 5-naphthalen-1-ylmethylene-2-(4-trifluoromethylbenzylamino)thiazol-4-one |
| 257 | 5-(3-phenoxybenzylidene)-2-(4-trifluoromethylbenzylamino)thiazol-4-one |
| 258 | 5-(4-phenoxybenzylidene)-2-(4-trifluoromethylbenzylamino)thiazol-4-one |
| 259 | 5-(2-chloro-3-phenylallylidene)-2-(4-trifluoromethylbenzylamino)thiazol-4-one |
| 260 | 5-(3-benzyloxybenzylidene)-2-(4-trifluoromethylbenzylamino)thiazol-4-one |
| 261 | 5-naphthalen-2-ylmethylene-2-(4-trifluoromethylbenzylamino)thiazol-4-one |
| 262 | 2-(4-hydroxy-3-methoxyphenyl)-5-(4-methylbenzylidene)thiazol-4-one and |
| 263 | 2-(4-tert-butylphenylamino)-5-(4-methylbenzylidene)thiazol-4-one |
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

15. The use of at least one compound as claimed in one or more of claims 1 to 14 for producing a medicament for prophylaxis and/or treatment of pain selected from the group consisting of acute pain, chronic pain, visceral pain, joint pain and neuropathic pain.

16. The use of at least one compound as defined in one or more of claims 1 to 14 for producing a medicament for prophylaxis and/or treatment of one or more disorders selected from the group consisting of migraine; depression; neuropathy; neural injuries; neurodegenerative disorders; preferably selected from the group consisting of multiple sclerosis, Alzheimer's disease, Parkinson's disease and Huntington's disease; cognitive dysfunctions, preferably cognitive deficiencies, more preferably memory impairments; epilepsy; respiratory pathway disorders, preferably selected from the group consisting of asthma and lung inflammation; coughing; urine incontinence; an overactive bladder (OAB); stomach ulcers; irritable bowel syndrome; stroke; eye irritation; skin irritation; neurotic skin disorders; inflammation disorders, preferably inflammation of the bowel; diarrhea; pruritus; disorders of food uptake, preferably selected from the group consisting of bulimia, cachexia, anorexia and obesity; medicament dependence; medicament abuse; withdrawal symptoms in the case of medicament dependence; evolution of tolerance to medicaments, preferably to natural or synthetic opioids; drug dependence; drug abuse; withdrawal symptoms in the case of drug dependence; alcohol dependence; alcohol abuse and withdrawal symptoms in the case of alcohol dependence; for diuresis; for antinatriuresis; for influencing the cardiovascular system; for enhancing vigilance; for enhancing libido; for modulating movement activity; for anxiolysis; for local anesthesia and/or for inhibiting undesired side effects, preferably selected from the group consisting of hyperthermia, hypertension and narrowing of the bronchia, triggered by the administration of Vanilloid receptor 1 (VR1/TRPV1 receptors) agonists, preferably selected from the group consisting of capsaicin, resiniferatoxin, olvanil, arvanil, SDZ-249665, SDZ-249482, nuvanil and capsavanil (DA-5018).

17. A 2,5-disubstituted thiazol-4-one derivative of the general formula la, 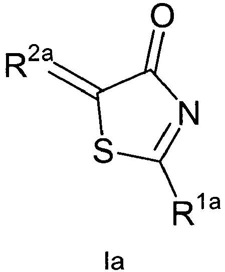 in which
R^{1a} is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
is an NR^{3a}R^{4a} group;
is an NR^{5a}-C(=O)-R^{6a} group;
or is an NR^{7a}-C(=O)-NR^{8a}R^{9a} group;
R^{2a} is CH-CH=C(CH₃)-W^{a}-Z^{a}, CH-CH=CH-W^{a}-Z^{a}, CH-C(CH₃)=CH-W^{a}-Z^{a}, CH-C(phenyl)=CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} or CH-CH(CH₃)-CH₂-W^{a}-Z^{a};
where W^{a} may in each case be absent or is in each case a radical selected from the group consisting of O, S, N(H), N(CH₃), N(C₂H₅) and N[CH(CH₃)₂];
R^{3a} is a linear or branched, saturated or unsaturated, unsubstituted or at least monosubstituted aliphatic radical optionally having a heteroatom as a chain member;
is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
or is an unsubstituted or at least monosubstituted aryl or heteroaryl radical which may be fused to an unsubstituted or at least monosubstituted mono- or polycyclic ring system and/or may be bonded via a linear or branched, unsubstituted or at least monosubstituted alkylene, alkenylene or alkynylene group;
R^{4a} is a hydrogen radical
or is a linear or branched, saturated or unsaturated, unsubstituted or at least monosubstituted aliphatic radical optionally having at least one heteroatom as a chain member;
R^{5a}, R^{7a} and R^{9a} are each independently
a hydrogen radical
or a linear or branched, saturated or unsaturated, unsubstituted or at least monosubstituted aliphatic radical;
R^{6a} and R^{8a} are each independently
an unsubstituted or at least monosubstituted aryl or heteroaryl radical which may be fused to an unsubstituted or at least monosubstituted mono- or polycyclic ring system;
Z^{a} is a linear or branched, saturated or unsaturated, unsubstituted or at least monosubstituted aliphatic radical;
is an aryl radical which may be fused to an unsubstituted or at least monosubstituted mono- or polycyclic ring system and/or may be substituted by identical or different substituents R^{12a};
or is an heteroaryl radical which may be fused to an unsubstituted or at least monosubstituted mono- or polycyclic ring system and/or may be substituted by identical or different substituents R^{12a};
R^{12a} is a radical selected from the group consisting of F, Cl, Br, I, -CN, -CF₃, -SF₅, -OH, -O-C₁₋₁₀-alkyl, -O-C₂₋₁₀-alkenyl, -NH₂, -O-CF₃, -S-CF₃, -SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-C₁₋₅-alkyl, -S(=O)₂-phenyl, -O-S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -0-benzyl, phenyl and benzyl; where the cyclic moiety of the pyridinyl, cyclopentyl, cyclohexyl, pyridazinyl, -S(=O)₂-phenyl, -O-S(=O)₂-phenyl, -O-phenyl, -O-benzyl, phenyl, -(CH₂)-benzo[b]furanyl and benzyl radicals may in each case be substituted;
is a -C(=O)-NR^{13a}-(CH₂)ₘₐ-NR^{14a}-R^{15a} group where m^{a} is 0, 1, 2, 3, 4 or 5;
or is a -C(=O)-R^{16a} group;
R^{13a} and R^{14a} are each independently
a hydrogen radical
or a linear or branched, saturated or unsaturated, unsubstituted or at least monosubstituted aliphatic radical;
R^{15a} is an unsubstituted or at least monosubstituted aryl or heteroaryl radical which may be fused to an unsubstituted or at least monosubstituted mono- or polycyclic ring system;
and
R^{16a} is an unsubstituted or at least monosubstituted, unsaturated or saturated cycloaliphatic radical which optionally has at least one heteroatom as a ring member and may be fused to an unsubstituted or at least monosubstituted mono- or polycyclic ring system;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

18. A compound as claimed in claim 17, **characterized in that**
R^{1a} is an unsaturated or saturated, optionally substituted 3-, 4-, 5-, 6-, 7-, 8-or 9-membered cycloaliphatic radical which may be fused to a saturated or unsaturated, optionally substituted mono- or polycyclic ring system;
is an NR^{3a}R^{4a} group;
is an NR^{5a}-C(=O)-R^{6a} group
or is an NR^{7a}-C(=O)-NR^{8a}R^{9a} group;
R^{2a} is CH-CH=C(CH₃)-W^{a}-Z^{a}, CH-CH=CH-W^{a}-Z^{a}, CH-C(CH₃)=CH-W^{a}-Z^{a}, CH-C(phenyl)=CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} or CH-CH(CH₃)-CH₂-W^{a}-Z^{a};
where W^{a} may in each case be absent or is in each case a radical selected from the group consisting of O, S, N(H), N(CH₃), N(C₂H₅) and N[CH(CH₃)₂];
R^{3a} is a linear or branched, saturated or unsaturated, optionally substituted C₁₋₁₀ aliphatic radical optionally having 1, 2 or 3 heteroatoms selected independently from the group consisting of oxygen, nitrogen (NH) and sulfur;
is an unsaturated or saturated, optionally substituted 3-, 4-, 5-, 6-, 7-, 8-or 9-membered cycloaliphatic radical which may be fused to a saturated or unsaturated, optionally substituted mono- or polycyclic ring system;
or is an optionally substituted 5- to 14-membered aryl or heteroaryl radical which may be fused to a saturated or unsaturated, optionally substituted mono- or polycyclic ring system and/or be bonded via a C₁₋₅-alkylene group;
R^{4a} is a hydrogen radical
or is a linear or branched, saturated or unsaturated, optionally substituted C₁₋₁₀ aliphatic radical optionally having 1, 2 or 3 heteroatoms selected independently from the group consisting of oxygen, nitrogen (NH) and sulfur;
R^{5a}, R^{7a} and R^{8a} are each independently
a hydrogen radical
or a linear or branched, saturated or unsaturated, optionally substituted C₁₋₁₀ aliphatic radical;
R^{6a} and R^{9a} are each independently
an optionally substituted 5- to 14-membered aryl or heteroaryl radical which may be fused to a saturated or unsaturated, optionally substituted mono- or polycyclic ring system;
Z^{a} is a linear or branched, saturated or unsaturated, optionally substituted C₁₋₂₀ aliphatic radical;
is a substituted 6- or 10-membered aryl radical which is fused to a saturated or unsaturated, optionally substituted mono- or polycyclic ring system and/or is substituted by 1, 2, 3, 4 or 5 identical or different substituents R^{12a};
or is an optionally substituted 5- to 14-membered heteroaryl radical which is fused to a saturated or unsaturated, optionally substituted mono- or polycyclic ring system and/or is substituted by 1, 2, 3, 4 or 5 identical or different substituents R^{12a};
R^{12a} is a radical selected from the group consisting of F, Cl, Br, I, -CN, -CF₃, -SF₅, -OH, -O-C₁₋₁₀-alkyl, -O-C₂₋₁₀-alkenyl, -NH₂, -O-CF₃, -S-CF₃,-SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-C₁₋₅-alkyl, -S(=O)₂-phenyl, -O-S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl; where the cyclic moiety of the pyridinyl, cyclopentyl, cyclohexyl, pyridazinyl, -S(=O)₂-phenyl, -O-S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -0-benzyl;
a -C(=O)-NR¹³-(CH₂)ₘₐ-NR¹⁴-R¹⁵ group where m^{a} is 0, 1, 2, 3, 4 or 5;
or a -C(=O)-R^{16a} group;
R^{13a} and R^{14a} are each independently
a hydrogen radical
or a linear or branched, saturated or unsaturated, optionally substituted C₁₋₁₀ aliphatic radical;
R^{15a} is a 5- to 14-membered aryl or heteroaryl radical which may be fused to a saturated or unsaturated, optionally substituted mono- or polycyclic ring system;
and
R^{16a} is an unsaturated or saturated, optionally substituted 3-, 4-, 5-, 6-, 7-, 8-or 9-membered cycloaliphatic radical which may be fused to a saturated or unsaturated, optionally substituted mono- or polycyclic ring system;
where
the aforementioned cycloaliphatic radicals may each optionally be substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-phenyl, -NH-pyridinyl, -N(C₁₋₅-alkyl)-phenyl, -N(C₁₋₅-alkyl)-pyridinyl, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, [1,2,5]-thiadiazolyl, pyridazinyl, -(CH₂)-benzo[b]furanyl, -O-phenyl, -O-benzyl, phenyl and benzyl, where the cyclic moiety of the -NH-phenyl, -NH-pyridinyl, -N(C₁₋₅-alkyl)-phenyl, -N(C₁₋₅-alkyl)-pyridinyl, pyridinyl, cyclopentyl, [1,2,5]-thiadiazolyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the aforementioned cycloaliphatic radicals may each optionally have 1, 2, 3, 4 or 5 heteroatom(s) selected independently from the group consisting of oxygen, nitrogen and sulfur as ring member(s);
the rings of the aforementioned mono- or polycyclic ring systems may each optionally be substituted by 1, 2, 3, 4 or 5 substituents selected independently from the group consisting of oxo (=O), thioxo (=S), F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₅-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -C(=O)-H, -C(=O)-C₁₋₅-alkyl, -C(=O)-NH₂, -C(=O)-NH-C₁₋₅-alkyl, C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the rings of the aforementioned mono- or polycyclic ring systems are each 5-, 6- or 7-membered and may each optionally have 1, 2, 3, 4 or 5 heteroatom(s) as ring member(s) which are selected independently from the group consisting of oxygen, nitrogen and sulfur;
the aforementioned C₁₋₁₀ aliphatic radicals or C₁₋₂₀ aliphatic radicals may each optionally be substituted by 1, 2, 3, 4, 5, 6, 7, 8 or 9 substituents selected independently from the group consisting of F, Cl, Br, I, -CN, -NO₂, -OH, -SH and -NH₂;
the aforementioned aryl or heteroaryl radicals may each optionally be substituted by 1, 2, 3, 4 or 5 substituents selected independently from the group consisting of F, Cl, Br, I, -CN, -CF₃, -SF₅, -OH, -O-C₁₋₅-alkyl, -NH₂, -NO₂, -O-CF₃, -S-CF₃, -SH, -S-C₁₋₅-alkyl, -C₁₋₁₀-alkyl, -C(=O)-OH, -C(=O)-O-C₁₋₅-alkyl, -O-C(=O)-C₁₋₅-alkyl, -NH-C₁₋₅-alkyl, -N(C₁₋₅-alkyl)₂, -NH-C(=O)-O-C₁₋₅-alkyl, -NH-C(=O)-C₁₋₅-alkyl, -C(=O)-H, -C(=O)-C₁₋₅-alkyl, -C(=O)-NH₂, -C(=O)-NH-C₁₋₅-alkyl, -C(=O)-N-(C₁₋₅-alkyl)₂, -S(=O)₂-C₁₋₅-alkyl, -S(=O)₂-phenyl, -NH-S(=O)₂-C₁₋₅-alkyl, -S(=O)₂-NH-C₁₋₅-alkyl, cyclohexyl, cyclopentyl, pyridinyl, pyridazinyl, -(CH₂)-benzo[b]furanyl, -O-phenyl, -O-benzyl, phenyl and benzyl, where the cyclic moiety of the pyridinyl, cyclopentyl, cyclohexyl, pyridazinyl, -S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, -C₁₋₅-alkyl, -O-C₁₋₅-alkyl, -O-CF₃, -S-CF₃, phenyl and -O-benzyl,
and the aforementioned heteroaryl radicals may each optionally have 1, 2, 3, 4 or 5 heteroatom(s) selected independently from the group consisting of oxygen, nitrogen and sulfur as ring member(s);
and the aforementioned C₁₋₅-alkylene groups may each optionally be substituted by 1, 2, 3, 4 or 5 substituents selected independently from the group consisting of F, Cl, Br, -OH, -SH, -NH₂, -CN and NO₂;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

19. A compound as claimed in claim 17 or 18, **characterized in that**
R^{1a} is a radical selected from the group consisting of cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, thiomorpholinyl, azepanyl, (2,3)-dihydro-1 H-isoindolyl, (2,3)-dihydroindolyl, (2,3,4,9)-tetrahydro-1H-β-carbolinyl, (2,3,4,9)-tetrahydro-1H-pyrido[2,3-b]indolyl, [1,2,3,4]-tetrahydronaphthyl, [1,2,3,4]-tetrahydroquinolinyl, [1,2,3,4]-tetrahydroisoquinolinyl, [1,2,3,4]-tetrahydroquinazolinyl and [3,4]-dihydro-2H-1,4-benzoxazinyl; where the radical may in each case optionally be substituted by 1, 2, 3, 4 or 5 substituents selected independently from the group consisting of oxo (=O), thioxo (=S), F, Cl, Br, I, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl, -NH-C(=O)-O-CH₃, -NH-C(=O)-O-C₂H₅, -NH-C(=O)-O-C(CH₃)₃, -C(=O)-CH₃, -C(=O)-C₂H₅, - C(=O)-NH-CH₃, -C(=O)-NH-C₂H₅, -C(=O)-N-(CH₃)₂, -C(=O)-N-(C₂H₅)₂, pyridinyl, pyridazinyl and phenyl; where the cyclic moiety of the -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl, pyridinyl, pyridazinyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
is a piperazinyl radical which may be substituted by 1, 2 or 3 substituents selected independently from the group consisting of methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -C(=O)-C₁₋₅-alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
is an NR^{3a}R^{4a} group;
is an NR^{5a}-C(=O)-R^{6a} group
or is an NR^{7a}-C(=O)-NR^{8a}R^{9a} group.

20. A compound as claimed in one or more of claims 17 to 19, **characterized in that**
R^{2a} is CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(phenyl)=CH-Z^{a}, CH-CBr=CH-Z^{a}, CH-CCl=CH-Z^{a}, CH-CF=CH-Z^{a}, CH-C(OH)=CH-Z^{a}, CH-CH₂-CH₂-Z^{a}, CH-CH₂-CH(CH₃)-Z^{a} or CH-CH(CH₃)-CH₂-Z^{a}.

21. A compound as claimed in one or more of claims 17 to 20, **characterized in that**
R^{3a} is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂;
is a radical selected from the group consisting of cyclopentyl, cyclohexyl, cycloheptyl, cyclopentenyl, cyclohexenyl, cycloheptenyl, imidazolidinyl, tetrahydrofuranyl, tetrahydrothiophenyl, pyrrolidinyl, piperidinyl, morpholinyl, thiomorpholinyl and azepanyl; where the radical may in each case optionally be substituted by 1, 2, 3, 4 or 5 substituents selected independently from the group consisting of methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl; where the cyclic moiety of the -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl and -N[CH(CH₃)₂]-pyridinyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
or is a radical selected from the group consisting of phenyl, naphthyl, (1,3)-benzodioxolyl and (1,4)-benzodioxanyl; where the radical may be bonded via a -(CH₂)-, -(CH₂)-(CH₂) or -(CH₂)-(CH₂)-(CH₂) group and/or may in each case optionally be substituted by 1, 2, 3, 4 or 5 substituents selected independently from the group consisting of F, Cl, Br, I, -SF₅, -OH, -O-CH₃, -O-C₂H₅, -NH₂, -NO₂, -CF₃, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ and -NH-C(CH₃)₃.

22. A compound as claimed in one or more of claims 17 to 21, **characterized in that**
R^{4a} is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂.

23. A compound as claimed in one or more of claims 17 to 22, **characterized in that**
R^{5a}, R^{7a} and R^{8a} are each independently
a hydrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl; where the radical may in each case optionally be substituted by 1, 2, 3, 4 or 5 substituents selected independently from the group consisting of F, Cl, Br, I, -CN, -NO₂, -OH, -SH and -NH₂.

24. A compound as claimed in one or more of claims 17 to 23, **characterized in that**
R^{6a} and R^{9a} are each independently
a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, - N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃.

25. A compound as claimed in one or more of claims 17 to 24, **characterized in that**
Z^{a} is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl and n-eicosanyl, where the radical may in each case optionally be substituted by 1, 2, 3, 4 or 5 substituents selected independently from the group consisting of F, Cl, Br, I, -CN, -NO₂, -OH, -SH and -NH₂;
is a radical selected from the group consisting of phenyl, naphthyl, phenanthrenyl, (1,3)-benzodioxolyl and (1,4)-benzodioxanyl, which is optionally substituted by 1, 2, 3, 4 or 5 identical or different substituents R^{12a};
or is a radical selected from the group consisting of thiophenyl, furanyl, pyrrolyl, pyrazolyl, pyrazinyl, pyridinyl, pyridazinyl, pyrimidinyl, quinolinyl and isoquinolinyl, which may optionally be substituted by 1, 2, 3, 4 or 5 identical or different substituents R^{12a}.

26. A compound as claimed in one or more of claims 17 to 25, **characterized in that**
R^{12a} a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl, n-octyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(-O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-phenyl, -O-S(=O)₂-phenyl, cyclohexyl, cyclopentyl, pyridinyl, pyridazinyl, -(CH₂)-benzo[b]furanyl, -O-phenyl, -0-benzyl, phenyl and benzyl; where the cyclic moiety of the pyridinyl, cyclopentyl, cyclohexyl, pyridazinyl, -S(=O)₂-phenyl, -O-S(=O)₂-phenyl, -O-phenyl, -O-benzyl, phenyl, -(CH₂)-benzo[b]furanyl and benzyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl;
a -C(=O)-NR^{13a}-(CH₂)ₘₐ-NR^{14a}-R^{15a} group where m^{a} is 0, 1, 2 or 3;
or a -C(=O)-R^{16a} group;
where
R^{13a} and R^{14a} are each independently
a hydrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
R^{15a} is a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, methyl, ethyl, n-propyl, isopropyl, n-butyl, sec-butyl, isobutyl, tert-butyl, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -C(=O)-H, -C(=O)-CH₃, -C(=O)-C₂H₅, -C(=O)-C(CH₃)₃;
and
R^{16a} is a radical selected from the group consisting of piperazinyl, thiomorpholinyl, azepanyl, morpholinyl, 2,3-dihydro-1H-isoindolyl, 2,3-dihydroindolyl, piperidinyl and pyrrolidinyl; where the radical may be substituted by 1, 2 or 3 substituents selected independently from the group consisting of -OH, methyl, ethyl, n-propyl, isopropyl, n-butyl, sec-butyl, isobutyl, tert-butyl, -C(=O)-O-CH₃, -C(=O)-O-C₂H₅, -C(=O)-O-C(CH₃)₃, -C(=O)-C₁₋₅-alkyl, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂H₅)₂, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2, 3, 4 or 5 substituents selected independently from the group consisting of F, Cl, Br, -OH, -CF₃, -SF₅, -CN, -NO₂, methyl, ethyl, n-propyl, isopropyl, n-butyl, sec-butyl, isobutyl, tert-butyl, -O-CH₃, -O-C₂H₅, -O-CF₃, -S-CF₃, phenyl and -O-benzyl.

27. A compound as claimed in one or more of claims 17 to 26, **characterized in that**
R^{1a} is one of the following radicals 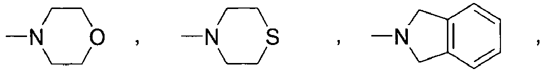 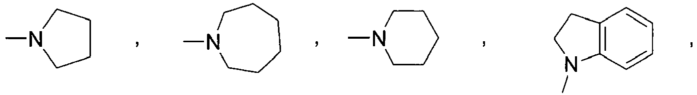 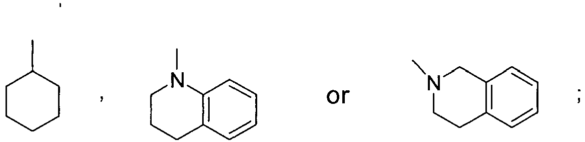 where the radical may be substituted by 1, 2 or 3 substituents selected independently from the group consisting of -OH, -O-CH₃, -O-C₂H₅, methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl and phenyl; where the cyclic moiety of the phenyl radical may in each case be substituted by 1, 2 or 3 substituents selected independently from the group consisting of F, Cl, Br and -CF₃;
is one of the following radicals 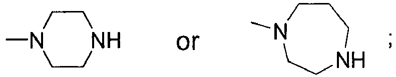 where the hydrogen atom of the -NH group may be replaced by a substituent selected from the group consisting of methyl, ethyl, n-propyl, isopropyl, n-butyl, sec-butyl, isobutyl, tert-butyl, pyridinyl, pyridazinyl, [1,2,5]-thiadiazolyl, benzyl and phenyl; where the cyclic moiety of the pyridinyl, pyridazinyl, [1 ,2,5]-thiadiazolyl, benzyl and phenyl radicals may in each case be substituted by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -0-C₂H₅, -O-CF₃ and -S-CF₃;
is an NR^{3a}R^{4a} group
or is an NR^{7a}-C(=O)-NR^{8a}R^{9a} group;
R^{2a} is CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(phenyl)=CH-Z^{a}, CH-CBr=CH-Z^{a}, CH-CCl=CH-Z^{a}, CH-CF=CH-Z^{a}, CH-C(OH)=CH-Z^{a}, CH-CH₂-CH₂-Z^{a}, CH-CH₂-CH(CH₃)-Z^{a} or CH-CH(CH₃)-CH₂-Z^{a};
R^{3a} is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl;
is a radical selected from the group consisting of cyclopentyl, cyclohexyl, cycloheptyl, cyclopentenyl, cyclohexenyl and cycloheptenyl; where the radical may in each case optionally be substituted by 1, 2, 3, 4 or 5 substituents selected independently from the group consisting of methyl, ethyl, n-propyl, isopropyl, n-butyl, sec-butyl, isobutyl, tert-butyl, -N(CH₃)₂, -N(C₂H₅)₂, -NH-CH₃, -NH-C₂H₅, -NH-CH(CH₃)₂, -NH-C(CH₃)₃, -NH-phenyl, -NH-pyridinyl, -N(CH₃)-phenyl, -N(C₂H₅)-phenyl, -N[CH(CH₃)₂]-phenyl, -N(CH₃)-pyridinyl, -N(C₂H₅)-pyridinyl, -N[CH(CH₃)₂]-pyridinyl;
or is a radical selected from the group consisting of phenyl, naphthyl, (1,3)-benzodioxolyl and (1,4)-benzodioxanyl; where the radical may be bonded via a -(CH₂)-, -(CH₂)-(CH₂) or -(CH₂)-(CH₂)-(CH₂) group and/or may in each case optionally be substituted by 1, 2, 3, 4 or 5 substituents selected independently from the group consisting of F, Cl, Br, -CF₃, -0-CF₃, -OCH₃, -OC₂H₅, methyl, ethyl, n-propyl, isopropyl, n-butyl and tert-butyl;
R^{4a} is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl;
R^{7a} and R^{8a} are each independently
a hydrogen radical
or a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl and n-heptyl;
R^{9a} is a radical selected from the group consisting of phenyl, naphthyl, thiophenyl, furanyl, pyridinyl, thiazolyl and oxazolyl, where the radical may in each case optionally be substituted by 1, 2, 3, 4 or 5 substituents selected independently from the group consisting of F, Cl, Br, I, -CN, -CF₃, -SF₅, -O-CH₃, -O-C₂H₅, -O-CF₃, -S-CF₃, methyl, ethyl, n-propyl, isopropyl, n-butyl, sec-butyl, isobutyl and tert-butyl;
Z^{a} is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl, n-octyl, n-nonyl, n-decyl, n-undecyl, n-dodecyl, n-tridecyl, n-tetradecyl, n-pentadecyl, n-hexadecyl, n-heptadecyl, n-octadecyl, n-nonadecyl and n-eicosanyl;
is a radical selected from the group consisting of phenyl, naphthyl, phenanthrenyl, (1,3)-benzodioxolyl and (1,4)-benzodioxanyl, which is optionally substituted by 1, 2, 3, 4 or 5 identical or different substituents R^{12a};
or is a radical selected from the group consisting of thiophenyl, furanyl, pyrrolyl, pyrazolyl, pyrazinyl, pyridinyl, pyridazinyl, pyrimidinyl, quinolinyl and isoquinolinyl, which may optionally be substituted by 1, 2, 3, 4 or 5 identical or different substituents R^{12a};
R^{12a} is a radical selected from the group consisting of F, Cl, Br, -SF⁵, -CF₃, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, methyl, ethyl, n-propyl, isopropyl, n-butyl, sec-butyl, tert-butyl, n-pentyl, n-hexyl, n-heptyl and n-octyl;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

28. A compound as claimed in one or more of claims 17 to 27, **characterized in that**
R^{1a} is one of the following radicals 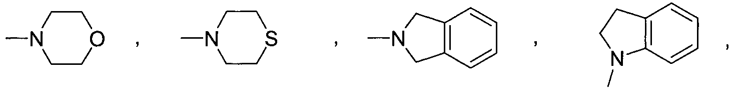 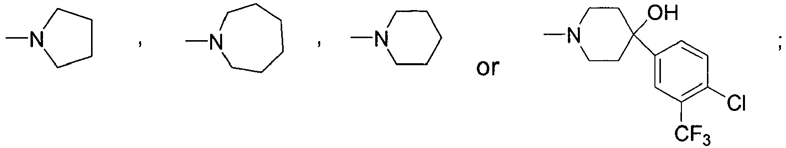 is the following radical 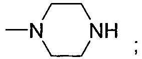 where the hydrogen atom of the -NH group may be replaced by a substituent selected from the group consisting of methyl, ethyl, n-propyl, isopropyl, n-butyl, sec-butyl, isobutyl, tert-butyl, pyridinyl, pyridazinyl, [1,2,5]-thiadiazolyl, benzyl and phenyl; where the cyclic moiety of the pyridinyl, pyridazinyl, [1,2,5]-thiadiazolyl, benzyl and phenyl radicals may in each case be substituted by 1, 2 or 3 substituents selected independently from the group consisting of F, Cl, Br, -CF₃, methyl, ethyl, n-propyl, isopropyl, n-butyl, sec-butyl, isobutyl, tert-butyl, -O-CH₃, -0-C₂H₅, -O-CF₃ and -S-CF₃;
is an NR^{3a}R^{4a} group
or is an NR^{7a}-C(=O)-NR^{8a}R^{9a} group;
R^{2a} is CH-CH=C(CH₃)-Z^{a}, CH-CH=CH-Z^{a}, CH-CH=CH-S-Z^{a}, CH-C(CH₃)=CH-Z^{a}, CH-C(phenyl)=CH-Z^{a}, CH-CBr=CH-Z^{a}, CH-CCl=CH-Z^{a}, CH-CF=CH-Z^{a}, CH-CH₂-CH₂-Z^{a}, CH-CH₂-CH(CH₃)-Z^{a} or CH-CH(CH₃)-CH₂-Z^{a};
R^{3a} is a radical selected from the group consisting of methyl, -CH₂-O-CH₃, -CH₂-S-CH₃, ethyl, -CH₂-CH₂-S-CH₃, -CH₂-CH₂-O-CH₃, -CH₂-CH₂-N(CH₃)-CH₃, n-propyl, -CH₂-CH₂-CH₂-S-CH₃, -CH₂-CH₂-CH₂-O-CH₃, -CH₂-CH₂-CH₂-N(CH₃)-CH₃, isopropyl, n-butyl, sec-butyl, isobutyl, tert-butyl, 1,1-dimethylpropyl, n-pentyl, sec-pentyl, n-hexyl and n-heptyl;
or is a radical selected from the group consisting of phenyl, naphthyl, benzyl, (1,3)-benzodioxolyl and (1,4)-benzodioxanyl; where the radical may in each case optionally be substituted by 1, 2, 3, 4 or 5 substituents selected independently from the group consisting of F, Cl, Br, -CF₃, -0-CF₃, -OCH₃, -OC₂H₅, methyl, ethyl, n-propyl, isopropyl, n-butyl and tert-butyl;
R^{4a} is a hydrogen radical
or is a radical selected from the group consisting of methyl, ethyl, n-propyl, isopropyl, n-butyl, sec-butyl, isobutyl, tert-butyl, 1,1-dimethyl-propyl, n-pentyl, sec-pentyl, n-hexyl and n-heptyl;
R^{7a} and R^{8a} are each a hydrogen radical;
R^{9a} is a phenyl radical which may be substituted by 1, 2, 3, 4 or 5 substituents selected independently from the group consisting of F, Cl, Br and -CF₃;
Z^{a} is a radical selected from the group consisting of methyl, ethyl, n-propyl, isopropyl, n-butyl, sec-butyl, isobutyl, tert-butyl, 1,1-dimethylpropyl, n-pentyl, sec-pentyl, n-hexyl, n-heptyl and n-octyl;
or is a phenyl or naphthyl radical which may be substituted by 1, 2, 3, 4 or 5 identical or different substituents R^{12a}.
R^{12a} is a radical selected from the group consisting of F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, methyl, ethyl, n-propyl, isopropyl, n-butyl, sec-butyl, tert-butyl, n-pentyl, n-hexyl, n-heptyl and n-octyl;
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

29. A compound as claimed in one or more of claims 17 to 28, selected from the group consisting of
1. 5-[3-(4-methoxyphenyl)but-2-enylidene]-2-morpholin-4-ylthiazol-4-one
2. 5-[3-(4-fluorophenyl)allylidene]-2-morpholin-4-ylthiazol-4-one
3. 2-morpholin-4-yl-5-(3-m-tolylallylidene)thiazol-4-one
4. 2-morpholin-4-yl-5-(3-p-tolylallylidene)thiazol-4-one
5. 2-morpholin-4-yl-5-octylidenethiazol-4-one
6. 5-butylidene-2-morpholin-4-ylthiazol-4-one
7. 5-hexylidene-2-morpholin-4-ylthiazol-4-one
8. 2-morpholin-4-yl-5-(3-p-tolylbut-2-enylidene)thiazol-4-one
9. 5-[3-(4-tert-butylphenyl)but-2-enylidene]-2-morpholin-4-ylthiazol-4-one
10. 5-[3-(4-tert-butylphenyl)allylidene]-2-[4-(3-methoxyphenyl)piperazin-1-yl]thiazol-4-one
11. 5-[3-(4-hydroxy-3-methoxyphenyl)allylidene]-2-thiomorpholin-4-ylthiazol-4-one
12. 2-morpholin-4-yl-5-[3-(4-trifluoromethylphenyl)but-2-enylidene]thiazol-4-one
13. 2-morpholin-4-yl-5-[3-(3-trifluoromethylphenyl)but-2-enylidene]thiazol-4-one
14. 5-decylidene-2-morpholin-4-ylthiazol-4-one
15. 5-[3-(4-tert-butylphenyl)allylidene]-2-morpholin-4-ylthiazol-4-one
16. 5-[3-(4-tert-butylphenyl)allylidene]-2-[4-(3-chloropyridin-2-yl)piperazin-1-yl]thiazol-4-one
17. 5-[3-(4-tert-butylphenyl)allylidene]-2-[4-(2-fluorophenyl)piperazin-1-yl]thiazol-4-one
18. 5-[3-(4-tert-butylphenyl)allylidene]-2-[4-(4-chloro-[1,2,5]thiadiazol-3-yl)-piperazin-1-yl]thiazol-4-one
19. 5-[3-(4-tert-butylphenyl)allylidene]-2-[4-(6-chloropyridin-2-yl)piperazin-1-yl]thiazol-4-one
20. 5-[3-(4-tert-butylphenyl)allylidene]-2-[4-(6-methylpyridazin-3-yl)piperazin-1-yl]thiazol-4-one
21. 2-[(benzo[1,3]dioxol-5-ylmethyl)amino]-5-[3-(4-tert-butylphenyl)-allylidene]thiazol-4-one
in each case, as appropriate, in the form of one of its pure stereoisomers, especially enantiomers or diastereomers, of its racemates or in the form of a mixture of stereoisomers, especially of the enantiomers and/or diastereomers, in any mixing ratio, or in each case in the form of corresponding salts, or in each case in the form of corresponding solvates.

30. A process for preparing 2,5-disubstituted thiazol-4-one derivatives of the general formula la as claimed in one or more of claims 18 to 30, **characterized in that** 2-aminothiazol-4-one is reacted in acetic acid in the presence of at least one salt selected from the group consisting of sodium acetate and potassium acetate or in a reaction medium selected from the group consisting of methanol, ethanol, isopropanol and n-butanol in the presence of at least one organic base selected from the group consisting of triethylamine, pyridine, diisopropylamine and N-methylmorpholine with at least one compound of the general formula R-C(=O)-H, in which R is -CH=C(CH₃)-W^{a}-Z^{a}, -CH=CH-W^{a}-Z^{a}, -C(CH₃)=CH-W^{a}-Z^{a}, -C(phenyl)=CH-W^{a}-Z^{a}, -CBr=CH-W^{a}-Z^{a}, -CCl=CH-W^{a}-Z^{a}, -CF=CH-W^{a}-Z^{a}_{,} -C(OH)=CH-W^{a}-Z^{a}, -CH²-CH₂-W^{a}-Z^{a}, -CH₂-CH(CH₃)-W^{a}-Z^{a} or -CH(CH₃)-CH₂-W^{a}-Z^{a}, where W^{a} and Z^{a} are each as defined in one or more of claims 18 to 30, to give at least one compound of the general formula IIa, 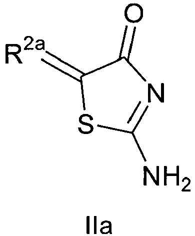 where R^{2a} is as defined in one or more of claims 17 to 29, and the latter is optionally purified and/or isolated, and at least one compound of the general formula IIa is reacted in a reaction medium in the presence of at least one base, preferably in the presence of at least one metal hydride salt or of a metal alkoxide salt, more preferably in the presence of a metal hydride salt or of a metal alkoxide salt selected from the group consisting of sodium hydride, potassium hydride, potassium tert-butoxide, sodium tert-butoxide, potassium methoxide, sodium methoxide, sodium ethoxide and potassium ethoxide, with at least one compound of the general formula LG-R^{3a} or of the general formula LG-R^{7a} or of the general formula LG-R^{5a}, in which LG is in each case a leaving group, preferably a halogen atom, more preferably a chlorine atom, and R^{3a}, R^{5a} and R^{7a} are each as defined in one or more of claims 18 to 30 to give at least one compound of the general formula Ia in which R^{2a} is as defined above and R^{1a} is an NHR^{3a}-, NHR^{5a}- or NHR^{7a} group, and the latter is optionally purified and/or isolated,
and optionally at least one compound of the general formula IIa in which R^{2a} is as defined above and R^{1a} is an NHR^{7a} group is reacted in a reaction medium with at least one isocyanate of the general formula R^{8a}-N=C=O, in which R^{8a} is as defined in one or more of claims 17 to 29, optionally in the presence of at least one base, preferably in the presence of at least one base selected from the group consisting of triethylamine, 4,4-dimethylaminopyridine and diisopropylethylamine, to give at least one compound of the general formula la in which R^{2a} is as defined above and R^{1a} is an NR^{7a}-C(=O)-NHR^{8a} group, and the latter is optionally purified and/or isolated;
and optionally at least one compound of the general formula la in which R^{2a} is as defined above and R^{1a} is an NR^{7a}-C(=O)-NHR^{8a} group is reacted in a reaction medium in the presence of at least one base, preferably in the presence of at least one metal hydride salt or of a metal alkoxide salt, more preferably in the presence of a metal hydride salt or of a metal alkoxide salt selected from the group consisting of sodium hydride, potassium hydride, potassium tert-butoxide, sodium tert-butoxide, potassium methoxide, sodium methoxide, sodium ethoxide and potassium ethoxide, with at least one compound of the general formula LG-R^{9a} in which LG is a leaving group, preferably a halogen atom, more preferably a chlorine atom, and R^{9a} is as defined in one or more of claims 18 to 30 to give at least one compound of the general formula la in which R^{2a} is as defined above and R^{1a} is an NR^{7a}-C(=O)-NR^{8a}R^{9a} group, and the latter is optionally purified and/or isolated;
or
optionally at least one compound of the general formula IIa in which R^{2a} is as defined above and R^{1a} is an NHR^{5a} group is reacted in a reaction medium, optionally in the presence of at least one base, with at least one compound of the general formula R^{6a}-C(=O)-LG in which R^{6a} is as defined in one or more of claims 18 to 30 and LG is a leaving group, preferably a halogen radical, or in a reaction medium in the presence of at least one coupling reagent, optionally in the presence of at least one base, with a compound of the general formula R^{6a}-C(=O)-OH in which R^{6a} is as defined in one or more of claims 18 to 30, to give at least one compound of the general formula la in which R^{2a} is as defined above and R^{1a} is an NR^{5a}-C(=O)-R^{6a} group, and the latter is optionally purified and/or isolated
or
optionally at least one compound of the general formula la in which R^{2a} is as defined above and R^{1a} is an NHR^{3a} group is reacted in a reaction medium in the presence of at least one base, preferably in the presence of at least one metal hydride salt or of a metal alkoxide salt, more preferably in the presence of a metal hydride salt or of a metal alkoxide salt selected from the group consisting of sodium hydride, potassium hydride, potassium tert-butoxide, sodium tert-butoxide, potassium methoxide, sodium methoxide, sodium ethoxide and potassium ethoxide, with at least one compound of the general formula LG-R^{4a} in which LG is a leaving group, preferably a halogen atom, more preferably a chlorine atom, and R^{4a} is as defined in one or more of claims 18 to 30 to give at least one compound of the general formula la in which R^{2a} is as defined above and R^{1a} is an NR^{3a}R^{4a} group, and the latter is optionally purified and/or isolated.

31. A process for preparing 2,5-disubstituted thiazol-4-one derivatives of the general formula la as claimed in one or more of claims 17 to 29, **characterized in that** at least one compound of the general formula R^{1a}-CN in which R^{1a} is as defined in one or more of claims 18 to 30 with the exception of an NR³aR^{4a} group, of an NR^{5a}-C(=O)-R^{6a} group and of an NR^{7a}-C(=O)-NR^{8a}R^{9a} group, is reacted in a reaction medium selected from the group consisting of methanol, ethanol, isopropanol and n-butanol, in the presence of at least one organic base selected from the group consisting of triethylamine, pyridine, diisopropylamine and N-methylmorpholine or in pyridine as a reaction medium, with thioglycolic acid to give at least one compound of the general formula IIIa, 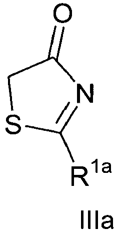 in which R^{1a} is as defined in one or more of claims 17 to 29 with the exception of an NR^{3a}R^{4a} group, of an NR^{5a}-C(=O)-R^{6a} group and of an NR^{7a}-C(=O)-NR^{8a}R^{9a} group, and the latter is optionally purified and/or isolated;
or at least one compound of the general formula H₂N-C(=S)-NHR^{1a} in which R^{1a} is as defined in one or more of claims 18 to 30 with the exception of an NR^{3a}R^{4a} group, of an NR^{5a}-C(=O)-R^{6a} group and of an NR^{7a}-C(=O)-NR^{8a}R^{9a} group, is reacted in a reaction medium selected from the group consisting of methanol, ethanol, isopropanol and n-butanol, optionally in the presence of at least one organic base selected from the group consisting of triethylamine, pyridine, diisopropylamine and N-methylmorpholine, with chloroacetic acid to give at least one compound of the general formula IIIa in which R^{1a} is as defined in one or more of claims 18 to 30 with the exception of an NR^{3a}R^{4a} group, of an NR^{5a}-C(=O)-R^{6a} group and of an NR^{7a}-C(=O)-NR^{8a}R^{9a} group, and the latter is optionally purified and/or isolated;
and at least one compound of the general formula IIIa is reacted in acetic acid in the presence of at least one salt selected from the group consisting of sodium acetate and potassium acetate or in a reaction medium selected from the group consisting of methanol, ethanol, isopropanol and n-butanol in the presence of at least one organic base selected from the group consisting of triethylamine, pyridine, diisopropylamine and N-methylmorpholine with at least one compound of the general formula R-C(=O)-H in which R is -CH=C(CH₃)-W^{a}-Z^{a}, -CH=CH-W^{a}-Z^{a}, -C(CH₃)=CH-W^{a}-Z^{a}, -C(phenyl)=CH-W^{a}-Z^{a}, -CBr=CH-W^{a}-Z^{a}, -CCl=CH-W^{a}-Z^{a}, -CF=CH-W^{a}-Z^{a}, -C(OH)=CH-W^{a}-Z^{a}, -CH₂-CH₂-W^{a}-Z^{a}, -CH₂-CH(CH₃)-W^{a}-Z^{a} or -CH(CH₃)-CH₂-W^{a}-Z^{a}, where W^{a} and Z^{a} are each as defined in one or more of claims 18 to 30, to give at least one compound of the general formula la in which R^{1a} is as defined in one or more of claims 18 to 30 with the exception of an NR^{3a}R^{4a} group, of an NR^{5a}-C(=O)-R^{6a} group and of an NR^{7a}-C(=O)-NR^{8a}R^{9a} group and R^{2a} is as defined in one or more of claims 17 to 29.

32. A medicament comprising at least one compound as claimed in one or more of claims 17 to 29 and optionally one or more physiologically compatible excipients.

33. The medicament as claimed in claim 32 for prophylaxis and/or treatment of pain, preferably of pain selected from the group consisting of acute pain, chronic pain, visceral pain, neuropathic pain and joint pain.

34. The medicament as claimed in claim 33 for prophylaxis and/or treatment of one or more disorders selected from the group consisting of joint pain; migraine; depression; neuropathy; neural injuries; neurodegenerative disorders; preferably selected from the group consisting of multiple sclerosis, Alzheimer's disease, Parkinson's disease and Huntington's disease; cognitive dysfunctions, preferably cognitive deficiencies, more preferably memory impairments; epilepsy; respiratory pathway disorders, preferably selected from the group consisting of asthma and lung inflammation; coughing; urine incontinence; an overactive bladder (OAB); stomach ulcers; irritable bowel syndrome; stroke; eye irritation; skin irritation; neurotic skin disorders; inflammation disorders, preferably inflammation of the bowel; diarrhea; pruritus; disorders of food uptake, preferably selected from the group consisting of bulimia, cachexia, anorexia and obesity; medicament dependence; medicament abuse; withdrawal symptoms in the case of medicament dependence; evolution of tolerance to medicaments, preferably to natural or synthetic opioids; drug dependence; drug abuse; withdrawal symptoms in the case of drug dependence; alcohol dependence; alcohol abuse and withdrawal symptoms in the case of alcohol dependence; for diuresis; for antinatriuresis; for influencing the cardiovascular system; for enhancing vigilance; for enhancing libido; for modulating movement activity; for anxiolysis; for local anesthesia and/or for inhibiting undesired side effects, preferably selected from the group consisting of hyperthermia, hypertension and narrowing of the bronchia, triggered by the administration of Vanilloid receptor 1 (VR1/TRPV1 receptors) agonists, preferably selected from the group consisting of capsaicin, resiniferatoxin, olvanil, arvanil, SDZ-249665, SDZ-249482, nuvanil and capsavanil (DA-5018).

## Revendications

1. Utilisation d'au moins un dérivé de thiazol-4-one substitué en position 2,5 de formule générale I, 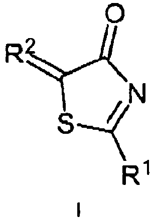 où
R¹ représente un radical cycloaliphatique non substitué ou au moins monosubstitué, insaturé ou saturé, présentant le cas échéant au moins un hétéroatome comme élément de cycle, qui peut être condensé avec un système cyclique monocyclique ou polycyclique non substitué ou au moins monosubstitué ;
un radical aryle non substitué ou au moins monosubstitué, qui peut être condensé avec un système cyclique monocyclique ou polycyclique non substitué ou au moins monosubstitué ;
un groupe NR³R⁴ _{;}
un groupe NR⁵-C(=O)-R⁶
ou un groupe NR⁷-C(=O)-NR⁸R⁹
R² représente un radical cycloaliphatique non substitué ou au moins monosubstitué, insaturé ou saturé, présentant le cas échéant au moins un hétéroatome comme élément de cycle, qui peut être condensé avec un système cyclique monocyclique ou polycyclique non substitué ou au moins monosubstitué ;
CH-U-X ;
CH-CH₂-V-Y, CH-CH(CH₃)-V-Y, CH-CHCl-V-Y, CH-CHBr-V-Y, CH-CHF-V-Y ou CH-CH(OH)-V-Y ;
ou CH-CH=C(CH₃)-W-Z, CH-CH=CH-W-Z, CH-C(CH3)=CH-W-Z, CH-C(phényl)=CH-N-Z, CH-CBr=CH-W-Z, CH-CCl=CH-W-Z, CH-CF=CH-W-Z, CH-C(OH)=CH-W-Z, CH-CH₂-CH₂-W-Z, CH-CH₂-CH(CH₃)-W-Z ou CH-CH(CH₃)-CH₂-W-Z ;
U, V et W pouvant être à chaque fois absents ou représentant à chaque fois indépendamment les uns des autres un radical choisi dans le groupe constitué par 0, S, N(H), N(CH₃), N(C₂H₅) et N[CH(CH3)2] ;
R³ représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N (CH₃) -CH₃, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par cyclopentyle cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, thiomorpholinyle et azépanyle ; le radical pouvant le cas échéant à chaque fois être substitué par 1, 2, 3, 4 ou 5 substituants choisis indépendamment les uns des autres dans le groupe constitué par méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH (CH₃)₂, -NH-C (CH₃)₃, -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle, -N[CH(CH₃)₂]-pyridinyle ; la partie cyclique des radicaux -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle et -N[CH(CH₃)₂]-pyridinyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou un radical choisi dans le groupe constitué par phényle, naphtyle, (1,3)-benzodioxolyle et (1,4)-benzodioxanyle ; le radical pouvant être lié via un groupe -(CH₂), -(CH₂)-(CH₂) ou -(CH₂)-(CH₂)-(CH₂) et/ou le cas échéant à chaque fois être substitué par 1, 2, 3, 4 ou 5 substituants choisis indépendamment les uns des autres dans le groupe constitué par F, Cl, Br, I, -SF₅, -OH, -O-CH₃, -0-C₂H₅, -NH₂, -NO₂, -CF₃, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -C(=O)-O-C(CH₃)₃, -OC(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ et -NH-C(CH₃)₃ ;
R⁴ représente un radical hydrogène
ou un radical aliphatique linéaire ou ramifié, saturé ou insaturé, non substitué ou au moins monosubstitué, présentant le cas échéant au moins un hétéroatome comme élément de chaîne ;
R⁵, R⁷ et R⁹ représentent indépendamment les uns des autres à chaque fois un radical hydrogène ou un radical aliphatique linéaire ou ramifié, saturé ou insaturé, non substitué ou au moins monosubstitué ;
R⁶ et R⁸ représentent, indépendamment l'un de l'autre, à chaque fois
un radical aryle ou hétéroaryle non substitué ou au moins monosubstitué, qui peut être condensé avec un système cyclique monocyclique ou polycyclique non substitué ou au moins monosubstitué ;
X représente un radical aryle ou hétéroaryle, qui peut être condensé avec un système cyclique monocyclique ou polycyclique non substitué ou au moins monosubstitué et/ou substitué par un ou plusieurs substituants R¹⁰ identiques ou différents ;
Y représente un radical aryle ou hétéroaryle, qui peut être condensé avec un système cyclique monocyclique ou polycyclique non substitué ou au moins monosubstitué et/ou substitué par un ou plusieurs substituants R¹¹ identiques ou différents ;
Z représente un radical aliphatique linéaire ou ramifié, saturé ou insaturé, non substitué ou au moins monosubstitué ;
ou un radical aryle ou hétéroaryle, qui peut être condensé avec un système cyclique monocyclique ou polycyclique non substitué ou au moins monosubstitué et/ou substitué par un ou plusieurs substituants R¹² identiques ou différents ;
R¹⁰, R¹¹ et R¹² représentent, indépendamment les uns des autres, à chaque fois
un radical choisi dans le groupe constitué par F, Cl, Br, I, -CN, -CF₃, -SF₅, -OH, -O-C₁₋₁₀-alkyle, -O-C₂₋₁₀-alcényle, -NH₂, -O-CF₃, -S-CF₃, -SH, -S-C₁₋₅-alkyle, -C₁₋₁₀-alkyle, -C(=O)-OH, -C(=O)-O-C₁₋₅-alkyle, -O-C (=O)-C₁₋₅-alkyle, -NH-C₁₋₅-alkyle, -N(C₁₋₅-alkyle)₂, -NH-C(=O)-O-C₁₋₅-alkyle, -C(=O)-H, -C (=O) -C₁₋₅-alkyle, -C (=O) -NH₂, -C(=O) -NH-C₁₋₅-alkyle, -C(=O)-N-(C₁₋₅-alkyle)₂, -S(=O)₂-C₁₋₅-alkyle, -S(=O)₂-phényle, -O-S(=O)₂-phényle, -NH-S(=O)₂-C₁₋₅-alkyle, -S (=O)₂-NH-C₁₋₅-alkyle, cyclohexyle, cyclopentyle, pyridinyle, pyridazinyle, -(CH₂)-benzo[b]furannyle, -0-phényle, -0-benzyle, phényle et benzyle ; la partie cyclique des radicaux pyridinyle, cyclopentyle, cyclohexyle,
pyridazinyle, -S(=O)₂-phényle, -O-S(=O)₂-phényle, -O-phényle, -0-benzyle, phényle, -(CH₂)-benzo[b]furannyle et benzyle pouvant à chaque fois être substituée ;
un groupe -C(=O)-NR¹³-(CH₂)ₘ-NR¹⁴-R¹⁵, m valant 0, 1, 2, 3, 4 ou 5 ;
ou un groupe -C(=O)-R¹⁶ ;
R¹³ et R¹⁴ représentent, indépendamment l'un de l'autre, à chaque fois
un radical hydrogène
ou un radical aliphatique linéaire ou ramifié, saturé ou insaturé, non substitué ou au moins monosubstitué ;
R¹⁵ représente un radical aryle ou hétéroaryle non substitué ou au moins monosubstitué, qui peut être condensé avec un système cyclique monocyclique ou polycyclique non substitué ou au moins monosubstitué ; et
R¹⁶ représente un radical cycloaliphatique non substitué ou au moins monosubstitué, insaturé ou saturé, présentant le cas échéant au moins un hétéroatome comme élément de cycle, qui peut être condensé avec un système cyclique monocyclique ou polycyclique non substitué ou au moins monosubstitué ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants ;
pour la préparation d'un médicament destiné à la prophylaxie et/ou au traitement de la douleur.

2. Utilisation d'un composé selon la revendication 1, **caractérisée en ce que**
R¹ représente un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ;
un radical aryle de 6 à 10 chaînons, le cas échéant substitué, qui peut être condensé avec un système cyclique monocyclique ou polycyclique, saturé ou insaturé, le cas échéant substitué ;
un groupe NR³R⁴ ;
un groupe NR⁵-C(=O)-R⁶
ou un groupe NR⁷-C(=O)-NR⁸R⁹ ;
R² représente un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ; représente CH-U-X ;
CH-CH₂-V-Y, CH-CH(CH₃)-V-Y, CH-CHCl-V-Y, CH-CHBr-V-Y, CH-CHF-V-Y ou CH-CH(OH)-V-Y ;
ou CH-CH=C(CH₃)-W-Z, CH-CH=CH-W-Z, CH-C(CH₃)=CH-W-Z, CH-C(phényl)=CH-W-Z, CH-CBr=CH-W-Z, CH-CCl=CH-W-Z, CH-CF=CH-W-Z, CH-C(OH)=CH-W-Z, CH-CH₂-CH₂-W-Z, CH-CH₂-CH(CH₃)-W-Z ou CH-CH(CH₃)-CH₂-W-Z ;
U, V et W pouvant être à chaque fois absents ou représentant à chaque fois indépendamment les uns des autres un radical choisi dans le groupe constitué par O, S, N(H), N(CH₃), N(C₂H₅) et N[CH(CH₃)₂] ;
R³ représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N(CH₃)-CH₃, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par cyclopentyle cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, thiomorpholinyle et azépanyle ; le radical pouvant le cas échéant à chaque fois être substitué par 1, 2, 3, 4 ou 5 substituants choisis indépendamment les uns des autres dans le groupe constitué par méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH(CH₃)₂ -NH-C(CH₃)₃, -NH-phényle, -NH-pyridinyle, -N(CH₃)-phényle, -N (C₂H₅) - phényle, -N[CH(CH₃)₂]-phényle, -N(CH₃)-pyridinyle, -N(C₂H₅)-pyridinyle, -N[CH(CH₃)₂]-pyridinyle; la partie cyclique des radicaux -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅) - pyridinyle et -N[CH(CH₃)₂]-pyridinyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
ou un radical choisi dans le groupe constitué par phényle, naphtyle, (1,3)-benzodioxolyle et (1,4)-benzodioxanyle ; le radical pouvant être lié via un groupe -(CH₂), -(CH₂) -(CH₂) ou -(CH₂) -(CH₂) -(CH₂) et/ou le cas échéant à chaque fois être substitué par 1, 2, 3, 4 ou 5 substituants choisis indépendamment les uns des autres dans le groupe constitué par F, Cl, Br, I, -SF₅, -OH, -O-CH₃, -0-C₂H₅, -NH₂, -NO₂, -CF₃, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH_{3,} -NH-C₂H₅, -NH-CH(CH₃)₂ et -NH-C(CH₃)₃ ;
R⁴ représente un radical hydrogène
ou un radical aliphatique en C₁-₁₀ linéaire ou ramifié, saturé ou insaturé, le cas échéant substitué, présentant le cas échéant 1, 2 ou 3 hétéroatomes choisis indépendamment les uns des autres dans le groupe constitué par oxygène, azote (NH) et soufre %
R⁵, R⁷ et R⁸ représentent, indépendamment les uns des autres, à chaque fois
un radical hydrogène
ou un radical aliphatique en C₁₋₁₀ linéaire ou ramifié, saturé ou insaturé, le cas échéant substitué ;
R⁶ et R⁹ représentent, indépendamment l'un de l'autre, à chaque fois
un radical aryle ou hétéroaryle de 5 à 14 chaînons, le cas échéant substitué, qui peut être condensé avec un système cyclique monocyclique ou polycyclique, saturé ou insaturé, le cas échéant substitué ;
X représente un radical aryle ou hétéroaryle de 5 à 14 chaînons, qui peut être condensé avec un système cyclique monocyclique ou polycyclique, saturé ou insaturé le cas échéant substitué et/ou le cas échéant substitué par 1, 2, 3, 4 ou 5 substituants R¹⁰ identiques ou différents ;
Y représente un radical aryle ou hétéroaryle de 5 à 14 chaînons, le cas échéant substitué, qui peut être condensé avec un système cyclique monocyclique ou polycyclique, saturé ou insaturé le cas échéant substitué et/ou le cas échéant substitué par 1, 2, 3, 4 ou 5 substituants R¹¹ identiques ou différents ;
Z représente un radical aliphatique en C₁₋₂₀ linéaire ou ramifié, saturé ou insaturé, le cas échéant substitué ;
ou un radical aryle ou hétéroaryle de 5 à 14 chaînons, le cas échéant substitué, qui peut être condensé avec un système cyclique monocyclique ou polycyclique, saturé ou insaturé le cas échéant substitué et/ou le cas échéant substitué par 1, 2, 3, 4 ou 5 substituants R¹² identiques ou différents ;
R¹⁰ R¹¹ et R¹² représentent, indépendamment les uns des autres, à chaque fois
un radical choisi dans le groupe constitué par F, Cl, Br, I, -CN, -CF₃, -SF₅, -OH, -O-C₁₋₁₀-alkyle, -O-C₂₋₁₀-alcényle, -NH₂, -O-CF₃, -S-CF₃, -SH, -S-C₁₋₅-alkyle, -C₁-₁₀-alkyle, -C(=O)-OH, -C(=O)-O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)C₁₋₅-alkyle, -C(=O)-NH₂, -C(=O)-NH-C₁₋₅-alkyle, -C (=O) -N- (C₁₋₅-alkyle)₂, -S (=O)₂-C₁₋₅-alkyle, -S (=O)₂-phényle, -O-S (=O)₂-phényle, -NH-S (=O)₂-C₁₋₅-alkyle, -S (=O)₂-NH-C₁₋₅-alkyle, cyclohexyle, cyclopentyle, pyridinyle, pyridazinyle, -(CH₂)-benzo[b]furannyle, -O-phényle, -O-benzyle, phényle et benzyle ; la partie cyclique des radicaux pyridinyle, cyclopentyle, cyclohexyle, pyridazinyle, -S(=O)₂-phényle, -O-S(=O)₂-phényle, -0-phényle, -0-benzyle, phényle, -(CH₂)-benzo[b]furannyle et benzyle pouvant être à chaque fois substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle ;
un groupe -C(=O)-NR¹³-(CH₂)ₘ-NR¹⁴-R¹⁵, m valant 0, 1, 2, 3, 4 ou 5 ;
ou un groupe -C(=O)-R¹⁶ ;
R¹³ et R¹⁴ représentent, indépendamment l'un de l'autre, à chaque fois
un radical hydrogène
ou un radical aliphatique en C₁₋₁₀ linéaire ou ramifié, saturé ou insaturé, le cas échéant substitué ;
R¹⁵ représente un radical aryle ou hétéroaryle de 5 à 14 chaînons, qui peut être condensé avec un système cyclique monocyclique ou polycyclique, saturé ou insaturé, le cas échéant substitué ; et
R¹⁶ représente un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ;
les radicaux cycloaliphatiques susmentionnés pouvant à chaque fois être le cas échéant substitués par 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-OH, -C (=O)-O-C₁₋₅-alkyle, -O-C (=O) -C₁₋₅-alkyle, -NH-C₁₋₅-alkyle, -N (C₁₋₅-alkyle)₂, -NH-phényle, -NH-pyridinyle, -N (C₁₋₅-alkyl) -phényle, -N(C₁₋₅-alkyl)-pyridinyle, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle, -NH-S(=O)₂-C₁₋₅-alkyle, -S(=O)₂-NH-C₁₋₅-alkyle, cyclohexyle, cyclopentyle, pyridinyle, [1,2,5]-thiadiazolyle, pyridazinyle, -(CH₂)-benzo[b]furannyle, -0-phényle, -0-benzyle, phényle et benzyle, la partie cyclique des radicaux -NH-phényle, -NH-pyridinyle, -N(C₁₋₅-alkyl)-phényle, -N(C₁₋₅-alkyl)-pyridinyle, pyridinyle, cyclopentyle, [1,2,5]-thiadiazolyle, cyclohexyle, pyridazinyle, -S(=O)₂-phényle, -O-phényle, -O-benzyle, phényle, -(CH₂)-benzo[b]furannyle et benzyle pouvant être à chaque fois substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -0-benzyle,
et les radicaux cycloaliphatiques susmentionnés pouvant présenter, le cas échéant à chaque fois 1, 2, 3, 4 ou 5 hétéroatome(s) comme élément(s) de cycle, choisi(s) indépendamment les uns des autres dans le groupe constitué par oxygène, azote et soufre ;
les cycles des systèmes cycliques monocycliques ou polycycliques susmentionnés pouvant le cas échéant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-OH, -C (=O) -O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C (=O) -N- (C₁₋₅-alkyle)₂, -S (=O)₂-C₁₋₅-alkyle, -S(=O)₂-phényle, -NH-S (=O)₂-C₁₋₅-alkyle, -S (=O)₂-NH-C₁₋₅-alkyle, cyclohexyle, cyclopentyle, pyridinyle, pyridazinyle, -(CH₂)-benzo[b]furannyle, -0-phényle, -0-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les cycles des systèmes cycliques monocycliques ou polycycliques susmentionnés pouvant présenter à chaque fois 5, 6 ou 7 chaînons et le cas échant à chaque fois 1, 2, 3, 4 ou 5 hétéroatome(s) comme élément(s) de cycle, choisi(s), indépendamment les uns des autres, dans le groupe constitué par oxygène, azote et soufre ; les radicaux aliphatiques en C₁₋₁₀ ou les radicaux aliphatiques en C₁₋₂₀ susmentionnés pouvant le cas échéant à chaque fois être substitués par 1, 2, 3, 4, 5, 6, 7, 8 ou 9 substituants, choisis indépendamment les uns des autres dans le groupe constitué par F, Cl, Br, I, -CN, -NO₂, -OH, -SH et -NH₂ ;
les radicaux aryle ou hétéroaryle susmentionnés pouvant le cas échéant à chaque fois être substitués par 1, 2, 3, 4 ou 5 substituants choisis indépendamment les uns des autres dans le groupe constitué par F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₁₀-alkyle, -C(=O)-OH, -C (=O) -O-C₁₋₅-alkyle, -O-C(=O)-C₁₋₅-alkyle, -NH-C₁₋₅-alkyle, -N (C₁₋₅-alkyle)₂, -NH-C(=O)-O-C₁₋₅-alkyle, -NH-C(=O)-C₁₋₅-alkyle, -C(=O)-H, -C(=O)-C₁₋₅-alkyle, -C(=O)-NH₂, -C(=O)-NH-C₁₋₅-alkyle, -C (=O)-N- (C₁₋₅-alkyle)₂, S (=O)₂-C₁₋₅-alkyle, -S (=O)₂-phényle, -NH-S (=O)₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -0-phényle, -0-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -0-benzyle,
et les radicaux hétéroaryle susmentionnés pouvant présenter, le cas échéant à chaque fois 1, 2, 3, 4 ou 5 hétéroatome(s) comme élément(s) de cycle, choisi(s) indépendamment les uns des autres dans le groupe constitué par oxygène, azote et soufre ;
les radicaux C₁₋₅-alkylène susmentionnés pouvant le cas échéant à chaque fois être substitués par 1, 2, 3, 4 ou 5 substituants, choisis indépendamment les uns des autres dans le groupe constitué par F, Cl, Br, -OH, -SH, -NH₂, -CN et NO₂ ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

3. Utilisation d'un composé selon la revendication 1 ou 2, **caractérisée en ce que**
R¹ représente un radical choisi dans le groupe constitué par cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, thiomorpholinyle, azépanyle, (2,3)-dihydro-1H-iso-indolyle, (2,3)-dihydro-indolyle, (2,3,4,9)-tétrahydro-1H-β-carbolinyle, (2,3,4,9)-tétrahydro-1H-pyrido[2,3-b]indolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle et [3,4]-dihydro-2H-1,4-benzoxazinyle ; le radical pouvant le cas échéant à chaque fois être substitué par 1, 2, 3, 4 ou 5 substituants choisis indépendamment les uns des autres dans le groupe constitué par oxo (=O), thioxo (=S), F, Cl, Br, I, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C (=0) -0-C₂H₅, -C (-O) -O-C (CH₃)₃, -O-C (=O) -CH₃, -O-C (=O) -C₂H₅, -O-C(=O)-C(CH₃)₃, -N(CH₃)₂, -N(C₂H₅)₂, -NH-CH₃, -NH-C₂H₅, -NH-CH(CH₃)₂, -NH-C(CH₃)₃, -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle, -N[CH(CH₃)₂]-pyridinyle, -NH-C(=O)-O-CH₃, -NH-C (=O)-O-C₂H₅, -NH-C (=O) -O-C (CH₃)₃, -C (=O) - CH₃, -C (=O) -C₂H₅, -C (=O) -NH-CH₃, -C (=O) -NH-C₂H₅, -C (=O) -N- (CH₃)₂, -C (=O) -N- (C₂H₅)₂, pyridinyle, pyridazinyle et phényle ; la partie cyclique des radicaux -NH-phényle, -NH-pyridinyle, -N(CH₃)-phényle, -N (C₂H₅) -phényle, -N [CH (CH₃)₂] -phényle, -N(CH₃)-pyridinyle, -N(C₂H₅) -pyridinyle, -N[CH(CH₃)₂]-pyridinyle, pyridinyle, pyridazinyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical pipérazinyle, qui peut être substitué par 1, 2 ou 3 substituants choisis indépendamment les uns des autres dans le groupe constitué par méthyle, éthyle, n-propyle, isopropyle, n-butyle, sec-butyle, isobutyle, tert-butyle, -C(=O)-O-CH₃, -C(=O)-O-C₂H₅, -C(=O)-O-C(CH₃)₃, -C(=O)-C₁₋₅-alkyle, -C(=O)-NH₂, -C(=O)-CH₃, -C(=O)-C₂H₅, -C(=O)-NH-CH₃, -C(=O)-NH-C₂H₅, -C (=O) -N- (CH₃)₂, -C (=O) -N- (C₂H₅)₂, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un radical choisi dans le groupe constitué par phényle et naphtyle, le radical pouvant le cas échéant à chaque fois être substitué par 1, 2, 3, 4 ou 5 substituants choisis indépendamment les uns des autres dans le groupe constitué par F, Cl, Br, I, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-OC₂H₅, -C (=O) -O-C (CH₃)₃, -O-C (-O)-CH₃, -O-C (=O) -C₂H₅, -O-C (=O) -C (CH₃)₃, -N (CH₃)₂, -N (C₂H₅)₂, -NH-CH₃, -NH-C₂H₅, -NH-CH(CH₃)₂ est -NH-C(CH₃)₃ ;
un groupe NR²R⁴ ;
un groupe NR⁵-C(=O)-R⁶
ou un groupe NR⁷-C(=O)-NR⁸R⁹.

4. Utilisation d'un composé selon l'une ou plusieurs des revendications 1 à 3, **caractérisée en ce que**
R² représente un radical 1,3-dihydro-indol-2-onyle ou 1,3-dihydro-indol-2-thionyle ;
CH-X ;
CH-CH₂-Y, CH-CH(CH₃)-Y, CH-CHCl-Y, CH-CHBr-Y, CH-CHF-Y ou CH-CH(OH)-Y ;
ou CH-CH=C(CH₃)-Z, CH-CH=CH-Z, CH-CH=CH-S-Z, CH-CH=CH-O-Z, CH-CH=CH-N(CH₃)-Z, CH-C(CH₃)=CH-Z, CH-C(phényl)=CH-Z, CH-CBr=CH-Z, CH-CCl=CH-Z, CH-CF=CH-Z, CH-C(OH)=CH-Z, CH-CH₂-CH₂-Z, CH-CH₂-CH(CH₃)-Z ou CH-CH(CH₃)-CH₂-Z.

5. Utilisation d'un composé selon l'une ou plusieurs des revendications 1 à 4, **caractérisée en ce que**
R⁴ représente un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂.

6. Utilisation d'un composé selon l'une ou plusieurs des revendications 1 à 5, **caractérisée en ce que**
R⁵ R⁷ et R⁸ représentent indépendamment les uns des autres à chaque fois un radical hydrogène ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂.

7. Utilisation d'un composé selon l'une ou plusieurs des revendications 1 à 6, **caractérisée en ce que**
R⁶ et R⁹ représentent, indépendamment l'un de l'autre, à chaque fois
un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -OC(=O)-C(CH₃)₃, -N(CH₃)₂, -N(C₂H₅)₂, -NH-CH₃, -NH-C₂H₅, -NH-C(=O)-O-CH₃, -NH-C (=O) -O-C₂H₅, -NH-C (=O) -0-C(CH₃)₃, -C(=O)-H, -C (=O) -CH₃, -C (=O) -C₂H₅, -C (=O)-C(CH₃)₃.

8. Utilisation d'un composé selon l'une ou plusieurs des revendications 1 à 7, **caractérisée en ce que**
X représente un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹⁰ identiques ou différents.

9. Utilisation d'un composé selon l'une ou plusieurs des revendications 1 à 8, **caractérisée en ce que**
Y représente un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹¹ identiques ou différents.

10. Utilisation d'un composé selon l'une ou plusieurs des revendications 1 à 9, **caractérisée en ce que**
Z représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle et n-eicosanyle, le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂ ;
ou un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹² identiques ou différents.

11. Utilisation d'un composé selon l'une ou plusieurs des revendications 1 à 10, **caractérisée en ce que**
R¹⁰, R¹¹ et R¹² représentent, indépendamment les uns des autres, à chaque fois
un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C (CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle, n-octyle, -C(=O)-OH, -C(=O)-O-CH₃, -C (=O) -O-C₂H₅, -C (=O)-O-C (CH₃)₃, -O-C (=O) -CH₃, -O-C (=O) -C₂H₅, -O-C (=O) -C (CH₃)₃, -N (CH₃)₂, -N (C₂H₅)₂, -NH-CH₃, -NH-C₂H₅, -NH-C (=O) -O-CH₃, -NH-C (=O) -OC₂H₅, -NH-C(=O)-O-C(CH₃)₃, -NH-C(=O)-CH₃, -NH-C(=O)-C₂H₅, -NH-C (=O) -C (CH₃)₃, -C(=O) -H, -C(=O) -CH₃, -C(=O)-C₂H₅, -C (=O) -C (CH₃)₃, -C (=O) -NH₂, -C (=O) -NH-CH₃, -C(=O)-NH-C₂H₅, -C(=O)-N-(CH₃)₂, -C(=O)-N-(C₂H₅)₂, -S (=O)₂-CH₃, -S (=O)₂-C₂H₅, -NH-S (=O)₂-CH₃, -NH-S(=O)₂-C₂H₅, -S(=O)₂-NH-CH₃, -S(=O)₂-phényle, -O-S(=O)₂-phényle, cyclohexyle, cyclopentyle, pyridinyle, pyridazinyle, -(CH₂)-benzo[b] furannyle, -O-phényle, -O-benzyle, phényle et benzyle ; la partie cyclique des radicaux pyridinyle, cyclopentyle, cyclohexyle, pyridazinyle, -S(-O)₂-phényle, -O-S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un groupe -C(=O)-NR¹³- (CH₂)ₘ-NR¹⁴-R¹⁵, m valant 0, 1, 2 ou 3 ;
ou un groupe -C(=O)-R¹⁶
R¹³ et R¹⁴ représentant indépendamment l'un de l'autre à chaque fois un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle,- n-hexyle et n-heptyle ;
R¹⁵ représentant un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C (=O) -O-C₂H₅, -C (=O) -O-C (CH₃)₃, -O-C(=O)-CH₃, -O-C (O) -C₂H₅, -OC(=O) -C(CH₃)₃, -N(CH₃)₂, -N (C₂H₅)₂, -NH-CH₃, -NH-C₂H₅, -NH-C(=O)-O-CH₃, -NH-C(=O)-O-C₂H₅, -NH-C(=O)-OC(CH₃)₃, -C(=O)-H, -C(=O)-CH₃, -C(=O)-C₂H₅, -C(=O)-C(CH₃)₃ ; et
R¹⁶ représentant un radical choisi dans le groupe constitué par pipérazinyle, thiomorpholinyle, azépanyle, morpholinyle, (2,3)-dihydro-1H-iso-indolyle, (2,3)-dihydro-indolyle, pipéridinyle et pyrrolidinyle ; le radical pouvant être substitué par 1, 2 ou 3 substituants choisis indépendamment les uns des autres dans le groupe constitué par -OH, méthyle, éthyle, n-propyle, isopropyle, n-butyle, sec-butyle, isobutyle, tert-butyle, -C(=O)-O-CH₃, -C(=O)-O-C₂H₅, -C (=O)-O-C (CH₃)₃, -C(=O)-C₁₋₅-alkyle, -C(=O)-NH₂, -C(=O)-CH₃, -C(=O) - C₂H₅, -C(=O)-NH-CH₃, -C(=O)-NH-C₂H₅, -C(=O)-N-(CH₃)₂, -C(=O)-N-(C₂₋₅)₂, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle.

12. Utilisation d'un composé selon l'une ou plusieurs des revendications 1 à 11, **caractérisée en ce que**
R¹ représente un des radicaux suivants 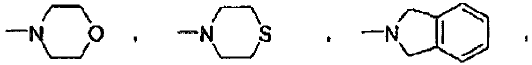 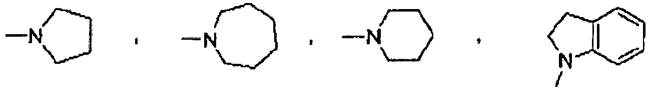 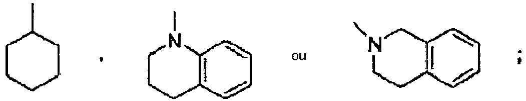 le radical pouvant être substitué par 1, 2 ou 3 substituants choisis indépendamment les uns des autres dans le groupe constitué par -OH, -O-CH₃, -O-C₂H₅, méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH (CH₃)₂, -NH-C(CH₃)₃, -NH-phényle, -NH-pyridinyle, -N(CH₃)-phényle, -N(C₂H₅) -phényle, -N [CH (CH₃)₂] -phényle, -N(CH₃)-pyridinyle, -N(C₂H₅)-pyridinyle, -N[CH(CH3)₂]-pyridinyle et phényle ; la partie cyclique du radical phényle pouvant à chaque fois être substituée par 1, 2 ou 3 substituants choisis indépendamment les uns des autres dans le groupe constitué par F, Cl, Br et -CF₃ ;
un des radicaux suivants 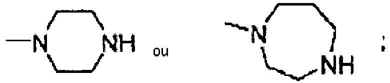 l'atome d'hydrogène du groupe -NH pouvant être substitué par un substituant choisi dans le groupe constitué par méthyle, éthyle, n-propyle, isopropyle, n-butyle, sec-butyle, isobutyle, tert-butyle, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
un radical choisi dans le groupe constitué par phényle et naphtyle, le radical pouvant le cas échéant à chaque fois être substitué par 1, 2, 3, 4 ou 5 substituants choisis indépendamment les uns des autres dans le groupe constitué par -OH, -O-CH₃ et -O-C₂H₅ ;
un groupe NR³R⁴
ou un groupe NR⁷-C(=O)-NR⁸R⁹ ;
R² représente un radical 1,3-dihydro-indol-2-onyle ou 1,3-dihydro-indol-2-thionyle ;
CH-X ;
CH-CH₂-Y, CH-CH(CH₃)-Y, CH-CHCl-Y, CH-CHBr-Y, CH-CHF-Y ou CH-CH(OH)-Y ;
ou CH-CH=C(CH₃)-Z, CH-CH=CH-Z, CH-CH=CH-S-Z, CH-CH=CH-O-Z , CH-CH=CH-N(CH₃)-Z, CH-C(CH₃)=CH-Z, CH-C(phényl)=CH-Z, CH-CBr=CH-Z, CH-CCl=CH-Z, CH-CF=CH-Z, CH-C(OH)=CH-Z, CH-CH₂-CH₂-Z, CH-CH₂-CH(CH₃)-Z ou CH-CH(CH₃)-CH₂-Z ;
R³ représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N (CH₃) -CH₃, isopropyle, n-butyle, sec-butyle, isobutyle, tert-butyle, (1,1)-diméthylpropyle, n-pentyle, sec-pentyle, n-hexyle et n-heptyle ;
un radical choisi dans le groupe constitué par cyclopentyle cyclohexyle, cycloheptyle, cyclopentényle, cyclohexényle et cycloheptényle ; le radical pouvant le cas échéant à chaque fois être substitué par 1, 2, 3, 4 ou 5 substituants choisis indépendamment les uns des autres dans le groupe constitué par méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH(CH₃)₂, -NH-C(CH₃)₃, -NH-phényle, -NH-pyridinyle, -N(CH₃)-phényle, -N (C₂H₅) -phényle, -N [CH (CH₃)₂]-phényle, -N(CH₃)-pyridinyle, -N(C₂H₅)-pyridinyle, -N[CH(CH₃)₂]-pyridinyle ;
ou un radical choisi dans le groupe constitué par phényle, naphtyle, (1,3)-benzodioxolyle et (1,4)-benzodioxanyle ; le radical pouvant être lié via un groupe -(CH₂)-, -(CH₂)-(CH₂) ou -(CH₂)-(CH₂)-(CH₂)- et/ou le cas échéant à chaque fois substitué par 1, 2, 3, 4 ou 5 substituants choisis indépendamment les uns des autres dans le groupe constitué par F, Cl, Br, -CF₃, -O-CF₃, -OCH₃, -OC₂H₅, méthyle, éthyle, n-propyle, isopropyle, n-butyle et tert-butyle ;
R⁴ représente un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R⁷ et R⁸ représentent, indépendamment l'un de l'autre à chaque fois
un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R⁹ représente un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SE₅, -O-CH₃, -O-C₂H₅, -O-CF₃, -S-CF₃, méthyle, éthyle, n-propyle, isopropyle, n-butyle, sec-butyle, isobutyle et tert-butyle ;
X représente un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹⁰ identiques ou différents.
Y représente un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹¹ identiques ou différents ;
Z représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle et n-eicosanyle ;
ou un radical choisi dans le groupe constitué par phényle, naphtyle, phénanthrényle, (1,3)-benzodioxolyle, (1,4)-benzodioxanyle, thiophényle, furannyle, pyrrolyle, pyrazolyle, pyrazinyle, pyridinyle, pyridazinyle, pyrimidinyle, quinoléinyle et isoquinoléinyle, qui peut le cas échéant être substitué par 1, 2, 3, 4 ou 5 substituants R¹² identiques ou différents ;
R¹⁰ représente un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle, n-octyle, -C(=O)-OH, -C(=O)-O-CH₃, -C(=O)-O-C₂H₅, -C(=O)-O-C(CH₃)₃, -OC(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O) -CH₃, -NH-C(=O)-C₂H₅, -NH-C(=O)-C(CH₃)₃, -O-S(=O)₂-phényle, -0-phényle, -O-benzyle, phényle et benzyle ;
un groupe -C (=O) -NR¹³- (CH₂) ₘ-NR¹⁴-R¹⁵, m valant 0, 1, 2 ou 3 ;
ou un groupe -C(=O)-R¹⁶ _{;}
R¹¹ représente un radical choisi dans le groupe constitué par F, Cl, Br, -SF₅, méthyle, éthyle, n-propyle, isopropyle, n-butyle, sec-butyle, tert-butyle, n-pentyle, n-hexyle, n-heptyle et n-octyle ;
R¹² représente un radical choisi dans le groupe constitué par F, Cl, Br, -SF₅, -CF₃, -O-CH₃, -OC₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, méthyle, éthyle, n-propyle, isopropyle, n-butyle, sec-butyle, tert-butyle, n-pentyle, n-hexyle, n-heptyle et n-octyle ;
R¹³ et R¹⁴ représentent à chaque fois un radical hydrogène ;
R¹⁵ représente un radical choisi dans le groupe constitué par phényle et pyridinyle, le radical pouvant le cas échéant à chaque fois être substitué par 1, 2, 3, 4 ou 5 substituants choisis indépendamment les uns des autres dans le groupe constitué par F, Cl, Br, -CF₃, -O-CF₃, -S-CF₃, méthyle, éthyle, n-propyle, isopropyle, n-butyle, sec-butyle, isobutyle et tert-butyle ; et
R¹⁶ représente un radical choisi dans le groupe constitué par thiomorpholinyle, azépanyle, morpholinyle, (2,3)-dihydro-1H-iso-indolyle, (2,3)-dihydro-indolyle, pipéridinyle et pyrrolidinyle ; le radical pouvant être substitué par 1, 2 ou 3 substituants choisis indépendamment les uns des autres dans le groupe constitué par -OH, méthyle, éthyle, n-propyle, isopropyle, n-butyle, sec-butyle, isobutyle, tert-butyle, benzyle et phényle ; la partie cyclique des radicaux benzyle et phényle pouvant à chaque fois être substituée par 1, 2, 3, 4 ou 5 substituants choisis indépendamment les uns des autres dans le groupe constitué par F, Cl, Br et -CF₃ ;
ou un des radicaux suivants 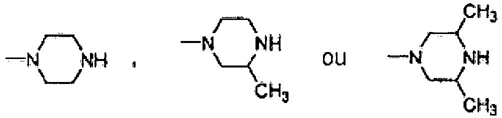 l'atome d'hydrogène du groupe -NH pouvant être substitué par un substituant choisi dans le groupe constitué par méthyle, éthyle, n-propyle, isopropyle, n-butyle, sec-butyle, isobutyle, tert-butyle, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

13. Utilisation d'un composé selon l'une ou plusieurs des revendications 1 à 12, **caractérisée en ce que**
R¹ représente un des radicaux suivants 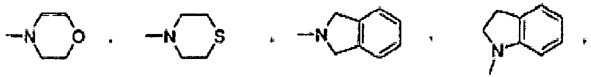 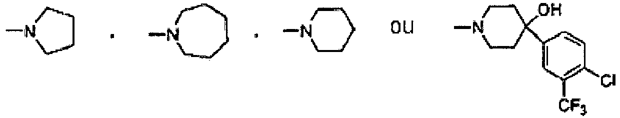 le radical suivant 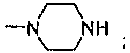 l'atome d'hydrogène du groupe -NH pouvant être substitué par un substituant choisi dans le groupe constitué par méthyle, éthyle, n-propyle, isopropyle, n-butyle, sec-butyle, isobutyle, tert-butyle, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
un radical choisi dans le groupe constitué par phényle et naphtyle, le radical pouvant le cas échéant à chaque fois être substitué par 1, 2, 3, 4 ou 5 substituants choisis indépendamment les uns des autres dans le groupe constitué par -OH, -O-CH₃ et -O-C₂H₅ ;
un groupe NR³R⁴
ou un groupe NR⁷-C(=O)-NR⁸R⁹ ;
R² représente un radical 1,3-dihydro-indol-2-onyle ou 1,3-dihydro-indol-2-thionyle ;
CH-X ;
CH-CH₂-Y, CH-CH(CH₃)-Y ou CH-CH(OH)-Y ;
ou CH-CH=C(CH₃₎-Z, CH-CH=CH-Z, CH-CH=CH-S-Z, CH-C(CH₃)=CH-Z, CH-C(phényl)=CH-Z, CH-CBr=CH-Z, CH-CCl=CH-Z, CH-CF=CH-Z, CH-CH₂-CH₂-Z, CH-CH₂-CH(CH₃)-Z ou CH-CH(CH₃)-CH₂-Z ;
R³ représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N (CH₃) -CH₃, isopropyle, n-butyle, sec-butyle, isobutyle, tert-butyle, (1,1)-diméthylpropyle, n-pentyle, sec-pentyle, n-hexyle et n-heptyle ;
ou un radical choisi dans le groupe constitué par phényle, naphtyle, benzyle, (1,3)-benzodioxolyle et (1,4)-benzodioxanyle ; le radical pouvant le cas échéant à chaque fois être substitué par 1, 2, 3, 4 ou 5 substituants choisis indépendamment les uns des autres dans le groupe constitué par F, Cl, Br, -CF₃, -O-CF₃, -OCH₃, -OC₂H₅, méthyle, éthyle, n-propyle, isopropyle, n-butyle et tert-butyle ;
R⁴ représente un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R⁷ et R⁸ représentent à chaque fois un radical hydrogène ;
R⁹ représente un radical phényle, qui peut être substitué par 1, 2, 3, 4 ou 5 substituants choisis indépendamment les uns des autres dans le groupe constitué par F, Cl, Br et -CF₃ ;
X représente un radical choisi dans le groupe constitué par phényle, naphtyle, phénanthrényle, (1,3)-benzodioxolyle, (1,4)-benzodioxanyle, thiophényle, furannyle, pyridinyle, pyridazinyle, pyrazinyle, pyrimidinyle, quinoléinyle et isoquinoléinyle, qui peut être substitué par 1, 2, 3, 4 ou 5 substituants R¹⁰ identiques ou différents ;
Y représente un radical phényle ou naphtyle qui peut être substitué par 1, 2, 3, 4 ou 5 substituants R¹¹ identiques ou différents ;
Z représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle et n-octyle ; ou un radical phényle ou naphtyle qui peut être substitué par 1, 2, 3, 4 ou 5 substituants R¹² identiques ou différents ;
R¹⁰ représente un radical choisi dans le groupe constitué par F, Cl, Br, I, -CF₃, -SF₅, -OH, -O-CH₃, -O-C₂H₅, -O-CH₂-CH₂-CH_{3,} -O-CH₂-CH₂-CH₂-CH₃, -OC(CH₃)₃, -O-CH₂-CH₂-CH₂-CH₂-CH₃, -O-CH₂-CH₂-C (CH₃)₃, -O-CH=CH₂-, -O-CH₂-CH=CH₂, -NH₂, -O-CF₃, -S-CF₃, méthyle, éthyle, n-propyle, isopropyle, n-butyle, sec-butyle, tert-butyle, n-pentyle, n-hexyle, n-heptyle, n-octyle, -C(=O)-OH, -C(=O)-O-CH₃, -C(=O)-O-C₂H₅, -C(=O) -O-C (CH₃)₃, -N(CH₃)₂, -N (C₂H₅)₂, -NH-CH₃, -NH-C₂H₅, -NH-C(=O)-CH₃, -O-S(=O)₂-phényle, -0- phényle, -0-benzyle et phényle ;
un groupe -C(=O)-NR¹³- (CH₂)ₘ-NR¹⁹-R¹⁵_{,} m valant 0, 1, 2 ou 3 ;
ou un groupe -C(=O)-R¹⁶;
R¹¹ représente un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH_{3,} -O-C(CH₃)₃, -O-CH₂-CH₂-CH₂-CH₂-CH₃, -O-CH-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle et n-octyle ;
R¹² représente un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH_{3,} -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle et n-octyle ;
R¹³ et R¹⁴ représentent à chaque fois un radical hydrogène ;
R¹⁵ représente un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -O-CF₃, -S-CF₃, méthyle, éthyle, n-propyle, isopropyle, n-butyle, sec-butyle, isobutyle et tert-butyle ; et
R¹⁶ représente un des radicaux suivants 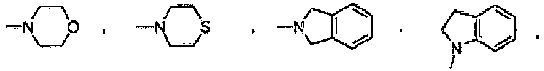 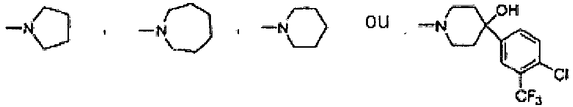 ou un des radicaux suivants 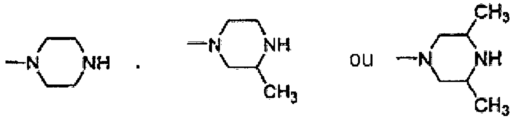 l'atome d'hydrogène du groupe -NH pouvant être substitué par un substituant choisi dans le groupe constitué par pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

14. Utilisation d'un composé selon l'une ou plusieurs des revendications 1 à 13 choisi dans le groupe constitué par :
1. la 5-[3-(4-méthoxyphényl)-but-2-énylidène]-2-morpholin-4-ylthiazol-4-one
2. la 2-morpholin-4-yl-5-phénéthylidènethiazol-4-one
3. la 2-morpholin-4-yl-5-(2-o-toluyléthylidène)-thiazol-4-one
4. la 2-morpholin-4-yl-5-(3-phénylallylidène)-thiazol-4-one
5. la 2-morpholin-4-yl-5-(3-phénylbutylidène)-thiazol-4-one
6. la 2-morpholin-4-yl-5-(3-phénylpropylidène)-thiazol-4-one
7. la 5-[3-(4-fluorophényl)-allylidène]-2-morpholin-4-ylthiazol-4-one
8. le N-[4-(2-morpholin-4-yl-4-oxo-4H-thiazol-5-ylidèneméthyl)-phényl]-acétamide
9. la 5-benzo[1,3]dioxol-5-ylméthylène-2-morpholin-4-ylthiazol-4-one
10. la 2-morpholin-4-yl-5-(3-m-toluylallylidène)-thiazol-4-one
11. la 5-(3-iodo-4,5-diméthoxybenzylidène)-2-morpholin-4-ylthiazol-4-one
12. la 2-morpholin-4-yl-5-(3-phénylsulfanylallylidène)-thiazol-4-one
13. la 5-(3-benzyloxy-4-méthoxybenzylidène)-2-morpholin-4-ylthiazol-4-one
14. la 5-(4-méthylbenzylidène)-2-thiomorpholin-4-ylthiazol-4-one
15. la N-[4-(2-morpholin-4-yl-4-oxo-4H-thiazol-5-ylidèneméthyl)-phényl]-amine
16. la 5-(4-butoxybenzylidène)-2-morpholin-4-yl-thiazol-4-one
17. la 2-morpholin-4-yl-5-(4-phénoxybenzylidène)-thiazol-4-one
18. la 2-morpholin-4-yl-5-(3-phénoxybenzylidène)-thiazol-4-one
19. la 2-morpholin-4-yl-5-(3-p-toluylallylidène)-thiazol-4-one
20. la 5-(3-benzyloxybenzylidène)-2-morpholin-4-ylthiazol-4-one
21. la 5-(4-benzyloxybenzylidène)-2-morpholin-4-ylthiazol-4-one
22. la 2-thiomorpholin-4-yl-5-(2-trifluorométhylbenzylidène)-thiazol-4-one
23. la 5-(4-bromobenzylidène)-2-thiomorpholin-4-ylthiazol-4-one
24. la 2-thiomorpholin-4-yl-5-(4-trifluorométhoxybenzylidène)-thiazol-4-one
25. la 5-(3-phénylallylidène)-2-thiomorpholin-4-ylthiazol-4-one
26. la 2-morpholin-4-yl-5-octylidènethiazol-4-one
27. la 5-(4-benzyloxy-3-méthoxybenzylidène)-2-morpholin-4-ylthiazol-4-one
28. la 5-(3,4-bis-benzyloxybenzylidène)-2-morpholin-4-ylthiazol-4-one
29. la 5-butylidène-2-morpholin-4-ylthiazol-4-one
30. la 2-[4-(3-chloropyridin-2-yl)-pipérazin-1-yl]-5-(4-méthylbenzylidène)-thiazol-4-one
31. la 2-[4-(3-chloropyridin-2-yl)-pipérazin-1-yl]-5-(3-phénylallylidène)-thiazol-4-one
32. la 5-hexylidène-2-morpholin-4-ylthiazol-4-one
33. la 2-morpholin-4-yl-5-(3-p-toluylbut-2-énylidène)-thiazol-4-one
34. la 5-[3-(4-tert-butylphényl)-but-2-énylidène]-2-morpholin-4-yl-thiazol-4-one
35. la 5-(4-méthoxynaphtalén-1-ylméthylène)-2-morpholin-4-ylthiazol-4-one
36. la 2-morpholin-4-yl-5-(4-trifluorométhoxybenzylidène)-thiazol-4-one
37. la 2-[4-(3-méthoxyphényl)-pipérazin-1-yl]-5-(3-phénylallylidène)-thiazol-4-one
38. la 5-[3-(4-tert-butylphényl)-allylidène]-2-[4-(3-méthoxyphényl)-pipérazin-1-yl]-thiazol-4-one
39. la 5-(4-hydroxy-3-méthoxybenzylidène)-2-thiomorpholin-4-ylthiazol-4-one
40. la 5-[3-(4-hydroxy-3-méthoxyphényl)-allylidène]-2-thiomorpholin-4-ylthiazol-4-one
41. la 5-naphtalén-1-ylméthylène-2-thiomorpholin-4-ylthiazol-4-one
42. la 5-benzylidène-2-thiomorpholin-4-ylthiazol-4-one
43. la 2-morpholin-4-yl-5-[3-(4-trifluorométhylphényl)-but-2-énylidène]-thiazol-4-one
44. la 2-morpholin-4-yl-5-[3-(3-trifluorométhylphényl)-but-2-énylidène]-thiazol-4-one
45. la 5-(3-méthylbutylidène)-2-morpholin-4-ylthiazol-4-one
46. la 2-[4-(3-chloropyridin-2-yl)-pipérazin-1-yl]-5-(2-trifluorométhylbenzylidène)-thiazol-4-one
47. la 5-(4-bromobenzylidène)-2-[4-(3-chloropyridin-2-yl)-pipérazin-1-yl)-thiazol-4-one
48. la 5-benzylidène-2-[4-(3-chloropyridin-2-yl)-pipérazin-1-yl]-thiazol-4-one
49. la 2-[4-(3-chloropyridin-2-yl)-pipérazin-1-yl]-5-naphtalén-2-ylméthylènethiazol-4-one
50. la 5-(2,3-diphénylallylidène)-2-morpholin-4-ylthiazol-4-one
51. la 2-morpholin-4-yl-5-phénanthrén-9-ylméthylènethiazol-4-one
52. la 2-morpholin-4-yl-5-quinoléin-3-ylméthylènethiazol-4-one
53. la 2-(4-éthylpipérazin-1-yl)-5-(4-méthylbenzylidène)-thiazol-4-one
54. la 5-naphtalén-2-ylméthylène-2-thiomorpholin-4-ylthiazol-4-one
55. la 2-[4-(4-méthoxyphényl)-pipérazin-1-yl]-5-(3-phénylallylidène)-thiazol-4-one
56. la 2-[4-(2-chlorophényl)-pipérazin-1-yl]-5-(3-phénylallylidène)-thiazol-4-one
57. la 5-(3-phénylallylidène)-2(4-phénylpipérazin-1-yl)-thiazol-4-one
58. la 5-décylidène-2-morpholin-4-ylthiazol-4-one
59. la 5-[3-(4-tert-butylphényl)-allylidène]-2-morpholin-4-ylthiazol-4-one
60. l'acide 2-(2-morpholin-4-yl-4-oxo-4H-thiazol-5-ylidèneméthyl)-benzoïque
61. la 5-(2-bromo-3-phénylallylidène)-2-[4-(3-chloropyridin-2-yl)-pipérazin-1-ylthiazol-4-one
62. la 5-[3-(4-tert-butylphényl)-allylidène]-2-[4-(3-chloropyridin-2-yl)-pipérazin-1-yl]-thiazol-4-one
63. la 2-(4-benzylpipérazin-1-yl)-5-(4-méthylbenzylidène)thiazol-4-one
64. la 2-(4-phénylpipérazin-1-yl)-5-quinoléin-3-ylméthylènethiazol-4-one
65. la 2-[4-(3-chloropyridin-2-yl)-pipérazin-1-yl]-5-(2-méthyl-3-phénylallylidène)-thiazol-4-one
66. la 2-[4-(3-chloropyridin-2-yl)-pipérazin-1-yl]-5-naphtalén-1-ylméthylènethiazol-4-one
67. la 5-[2-chloro-3-phénylallylidène)-2-[4-(3-chloropyridin-2-yl)-pipérazin-1-yl]-thiazol-4-one
68. la 5-(4-hydroxy-3-méthoxybenzylidène)-2-(4-phénylpipérazin-1-yl)-thiazol-4-one
69. la 5-phénanthrén-9-ylméthylène-2-(4-phénylpipérazin-1-yl)-thiazol-4-one
70. la 5-(6-méthoxynaphtalén-2-ylméthylène)-2-(4-phénylpipérazin-1-yl)-thiazol-4-one
71. la 5-naphtalén-1-ylméthylène-2-(4-phénylpipérazin-1-yl)-thiazol-4-one
72. la 5-(4-bromobenzylidène)-2-(4-phénylpipérazin-1-yl-thiazol-4-one
73. la 5-(4-méthylbenzylidène)-2-(4-phénylpipérazin-1-yl)-thiazol-4-one
74. la 2-(4-phénylpipérazin-1-yl)-5-(2-trifluorométhylbenzylidène)-thiazol-4-one
75. la 3-(4-oxo-2-thiomorpholin-4-yl-4H-thiazol-5-ylidène)-1,3-dihydro-indol-2-one
76. la 3-(2-morpholin-4-yl-4-oxo-4H-thiazol-5-ylidène)-1,3-dihydro-indol-2-one
77. la 2-[4-(2-fluorophényl)-pipérazin-1-yl]-5-(4-méthylbenzylidène)-thiazol-4-one
78. la 5-(4-tert-butyl-benzylidène)-2-[4-[2-fluorophényl)-pipérazin-1-yl]-thiazol-4-one
79. la 2-[4-(2-fluorophényl)-pipérazin-1-yl]-5-(3-phénylallylidène)-thiazol-4-one
80. la 5-[3-(4-tert-butylphényl)-allylidène]-2-[4-(2-fluorophényl)-pipérazin-1-yl]-thiazol-4-one
81. la 2-[4-(2-fluorophényl)-pipérazin-1-yl]-5-(4-trifluorométhylbenzylidène)-thiazol-4-one
82. la 2-[4-(2-fluorophényl)-pipérazin-1-yl]-5-(4-trifluorométhoxybenzylidène)-thiazol-4-one
83. la 5-(2-chloro-3-phénylallylidène)-2-[4-[2-fluorophényl)-pipérazin-1-yl]-thiazol-4-one
84. la 2-[4-(2-fluorophényl)-pipérazin-1-yl]-5-naphtalén-1-ylméthylènethiazol-4-one
85. la 5-(2-chloro-3-phénylallylidène)-2-thiomorpholin-4-ylthiazol-4-one
86. l'acide 3-(4-oxo-2-thiomorpholin-4-yl-4H-thiazol-5-ylidèneméthyl)benzoïque
87. l'acide 4-(4-oxo-2-thiomorpholin-4-yl-4H-thiazol-5-ylidèneméthyl)-benzoïque
88. la 5-(3-[4-(3-chloropyridin-2-yl)-pipérazin-1-carbonyl]-benzylidène)-2-thiomorpholin-4-yl-thiazol-4-one
89. la 5-{4-[4-(3-chloropyridin-2-yl)-pipérazin-1-carbonyl]-benzylidène}-2-thiomorpholin-4-ylthiazol-4-one
90. la 2-[4-(3-chloropyridin-2-yl)-pipérazin-1-yl]-5-quinoléin-3-ylméthylènethiazol-4-one
91. la 2-[4-(3-chloropyridin-2-yl)-pipérazin-1-yl]-5-(4-trifluorométhylbenzylidène)-thiazol-4-one
92. la 2-(2,3-dihydro-indol-1-yl)-5-(4-méthylbenzylidène)-thiazol-4-one
93. la 2-(2,3-dihydro-indol-1-yl)-5-(4-trifluorométhylbenzylidène)-thiazol-4-one
94. la 5-[4-(morpholin-4-carbonyl)-benzylidène]-2-thiomorpholin-4-ylthiazol-4-one
95. la 2-[4-(3-chloropyridin-2-yl)-pipérazin-1-yl]-5-(4-hydroxy-3-méthoxybenzylidène)-thiazol-4-one
96. la 1-[4-oxo-5-(4-trifluorométhylbenzylidène)-4,5-dihydrothiazol-2-yl]-3-(2-trifluorométhylphényl)-urée
97. la 2-(4-benzylpipérazin-1-yl)-5-quinoléin-3-ylméthylènethiazol-4-one
98. la 2-(4-benzylpipérazin-1-yl)-5-naphtalén-2-ylméthylènethiazol-4-one
99. la 2-(4-benzylpipérazin-1-yl)-5-(3-phénoxybenzylidène)-thiazol-4-one
100. la 2-(4-benzylpipérazin-1-yl)-5-(4-trifluorométhylbenzylidène)-thiazol-4-one
101. la 1-[5-(4-méthylbenzylidène)-4-oxo-4,5-dihydrothiazol-2-yl]-3-(2-trifluorométhylphényl)-urée
102. la 2-(4-benzylpipérazin-1-yl)-5-naphtalén-1-ylméthylènethiazol-4-one
103. la 2-(4-benzylpipérazin-1-yl)-5-(6-méthoxynaphtalén-2-ylméthylène)-thiazol-4-one
104. la 2-(4-benzylpipérazin-1-yl)-5-(4-tert-butylbenzylidène)-thiazol-4-one
105. la 1-[4-oxo-5-(3-phénylallylidène)-4,5-dihydrothiazol-2-yl]-3-(2-trifluorométhylphényl)-urée
106. la 1-[4-oxo-5-(4-trifluorométhylbenzylidène)-4,5-dihydrothiazol-2-yl]-3-(4-trifluorométhylphényl)-urée
107. la 1-[5-[4-méthylbenzylidène)-4-oxo-4,5-dihydrothiazol-2-yl]-3-(4-trifluorométhylphényl)-urée
108. la 5-{3-[4-(6-chloropyridin-2-yl)-pipérazin-1-carbonyl]-benzylidène}-2-thiomorpholin-4-ylthiazol-4-one
109. la 2-(4-benzylpipérazin-1-yl)-5-(4-trifluorométhoxybenzylidène)-thiazol-4-one
110. la 5-{3-[4-(3-chloropyridin-2-yl)-3-méthylpipérazine-1-carbonyl]-benzylidène}-2-thiomorpholin-4-ylthiazol-4-one
111. le N-[3-(3-chloro-5-trifluorométhylpyridin-2-ylamino)-propyl]-3-(4-oxo-2-thiomorpholin-4-yl-4H-thiazol-5-ylidèneméthyl)-benzamide
112. le N'-(2-chloro-4-trifluorométhylphényl)-hydrazide de l'acide 3-(4-oxo-2-thiomorpholin-4-yl-4H-thiazol-5-ylidèneméthyl)-benzoïque
113. la 5-{3-[4-(3-chloro-5-trifluorométhylpyridin-2-yl)-pipérazin-1-carbonyl]-benzylidène}-2-thiomorpholin-4-ylthiazol-4-one
114. le N'-(3-chloro-5-trifluorométhylpyridin-2-yl)-hydrazide de l'acide 3-(4-oxo-2-thiomorpholin-4-yl-4H-thiazol-5-ylidèneméthyl)-benzoïque
115. la 5-{3-[4-(4-chloro-[1,2,5]thiadiazol-3-yl)-pipérazin-1-carbonyl]-benzylidène}-2-thiomorpholin-4-ylthiazol-4-one
116. le N'-(2-chloro-5-trifluorométhylphényl)-hydrazide de l'acide 3-(4-oxo-2-thiomorpholin-4-yl-4H-thiazol-5-ylidèneméthyl)-benzoïque
117. la 2-[4-(4-chloro-[1,2,5]thiadiazol-3-yl)-pipérazin-1-yl]-5-(4-méthylbenzylidène)-thiazol-4-one
118. la 2-[4-(4-chloro-[1,2,5]thiadiazol-3-yl)-pipérazin-1-yl]-5-(4-trifluorométhylbenzylidène)-thiazol-4-one
119. la 2-[4-(4-chloro-[1,2,5]thiadiazol-3-yl)-pipérazin-1-yl]-5-(4-trifluorométhoxybenzylidène)-thiazol-4-one
120. la 5-(4-tert-butylbenzylidène)-2-[4-(4-chloro-[1,2,5]thiadiazol-3-yl)-pipérazin-1-yl]-thiazol-4-one
121. la 2-[4-(4-chloro-[1,2,5]thiadiazol-3-yl)-pipérazin-1-yl]-5-(3-phénylallylidène)-thiazol-4-one
122. la 5-[3-(4-tert-butylphényl)-allylidène]-2-[4-(4-chloro-[1,2,5]thiadiazol-3-yl)-pipérazin-1-yl]-thiazol-4-one
123. la 2-[4-(4-chloro-[1,2,5]thiadiazol-3-yl)-pipérazin-1-yl]-5-naphtalén-1-ylméthylènethiazol-4-one
124. la 2-[4-(4-chloro-[1,2,5]thiadiazol-3-yl)-pipérazin-1-yl]-5-(3-phénoxybenzylidène)-thiazol-4-one
125. la 2-[4-(4-chloro-[1,2,5]thiadiazol-3-yl)-pipérazin-1-yl]-5-(4-phénoxybenzylidène)-thiazol-4-one
126. la 5-(3-benzyloxybenzylidène)-2-[4-(4-chloro-[1,2,5]thiadiazol-3-yl)-pipérazin-1-yl]-thiazol-4-one
127. la 2-[4-(4-chloro-[1,2,5]thiadiazol-3-yl)-pipérazin-1-yl]-5-(4-hydroxy-3-méthoxybenzylidène)-thiazol-4-one
128. la 2-[4-(4-chloro-[1,2,5]thiadiazol-3-yl)-pipérazin-1-yl]-5-furann-2-ylméthylènethiazol-4-one
129. la 2-[4-(4-chloro-[1,2,5]thiadiazol-3-yl)-pipérazin-1-yl]-5-naphtalén-2-ylméthylènethiazol-4-one
130. l'acide 3-[2-(4-benzylpipérazin-1-yl)-4-oxo-4H-thiazol-5-ylidèneméthyl]-benzoïque
131. la 5-naphtalén-1-ylméthylène-2-pipérazin-1-ylthiazol-4-one
132. la 2-pipérazin-1-yl-5-(4-trifluorométhylbenzylidène)-thiazol-4-one
133. la 5-(4-méthylbenzylidène)-2-pipérazin-1-ylthiazol-4-one
134. la 5-(4-hydroxy-3-méthoxybenzylidène)-2-pipérazin-1-ylthiazol-4-one
135. la 5-(4-isopropylbenzylidène)-2-morpholin-4-ylthiazol-4-one
136. la 5-(4-isopropylbenzylidène)-2-thiomorpholin-4-ylthiazol-4-one
137. la 2-[4-(2-fluorophényl)-pipérazin-1-yl]-5-(4-isopropylbenzylidène)-thiazol-4-one
138. la 5-(4-pentafluorosulfanyl)-benzylidène)-2-thiomorpholin-4-ylthiazol-4-one
139. la 2-[4-(3-chloropyridin-2-yl)-pipérazin-1-yl]-5-(4-isopropylbenzylidène)-thiazol-4-one
140. la 2-(4-benzylpipérazin-1-yl)-5-(4-butylbenzylidène)-thiazol-4-one
141. la 2-(4-benzylpipérazin-1-yl)-5-(4-pentylbenzylidène)-thiazol-4-one
142. la 2-(4-benzylpipérazin-1-yl)-5-(4-octylbenzylidène)-thiazol-4-one
143. la 2-[4-(6-chloropyridin-2-yl)-pipérazin-1-yl]-5-(4-méthylbenzylidène)-thiazol-4-one
144. la 5-[4-tert-butylbenzylidène)-2-[4-(6-chloropyridin-2-yl)-pipérazin-1-yl]-thiazol-4-one
145. la 2-[4-(6-chloropyridin-2-yl)-pipérazin-1-yl]-5-(4-trifluorométhylbenzylidène)-thiazol-4-one
146. la 2-[4-(6-chloropyridin-2-yl)-pipérazin-1-yl]-5-(3-phénylallylidène)-thiazol-4-one
147. la 5-[3-(4-tert-butylphényl)-allylidène]-2-[4-(6-chloropyridin-2-yl)-pipérazin-1-yl]-thiazol-4-one
148. la 2-[4-(6-chloropyridin-2-yl)-pipérazin-l-yl]-5-(4-hydroxy-3-méthoxybenzylidène)-thiazol-4-one
149. la 2-[4-(6-chloropyridin-2-yl)-pipérazin-1-yl]-5-(4-isopropylbenzylidène)-thiazol-4-one
150. la 2-[4-(6-chloropyridin-2-yl)-pipérazin-1-yl]-5-(4-trifluorométhoxybenzylidène)-thiazol-4-one
151. la 2-[4-(6-chloropyridin-2-yl)-pipérazin-1-yl]-5-naphtalén-2-ylméthylènethiazol-4-one
152. la 5-(4-méthylbenzylidène)-2-[4-(6-méthylpyridazin-3-yl)-pipérazin-1-yl]-thiazol-4-one
153. la 5-(4-isopropylbenzylidène)-2-[4-(6-méthylpyridazin-3-yl)-pipérazin-1-yl]-thiazol-4-one
154. la 5-(4-tert-butylbenzylidène)-2-[4-(6-méthylpyridazin-3-yl)-pipérazin-1-yl]-thiazol-4-one
155. la 5-[3-(4-tert-butylphényl)-allylidène]-2-[4-(6-méthylpyridazin-3-yl)-pipérazin-1-yl]-thiazol-4-one
156. la 2-[4-(6-méthylpyridazin-3-yl)-pipérazin-1-yl]-5-(4-trifluorométhoxybenzylidène)-thiazol-4-one
157. la 5-(4-hydroxy-3-méthoxybenzylidène)-2-[4-(6-méthylpyridazin-3-yl)-pipérazin-1-yl]-thiazol-4-one
158. la 2-(4-benzylpipérazin-1-yl)-5-(4-pentafluorosulfanylbenzylidène)-thiazol-4-one
159. la 2-(4-benzylpipérazin-1-yl)-5-{3-[4-(4-chloro-[1,2,5]thiadiazol-3-yl)-pipérazin-1-carbonyl]-benzylidène}-thiazol-4-one
160. la 2-[4-(6-méthylpyridazin-3-yl)-pipérazin-1-yl]-5-(4-trifluorométhylbenzylidène)-thiazol-4-one
161. la 2-[4(6-méthylpyridazin-3-yl)-pipérazin-1-yl]-5-(3-phénylallylidène)-thiazol-4-one
162. la 2-[4-(6-méthylpyridazin-3-yl)-pipérazin-1-yl]-5-naphtalén-2-ylméthylènethiazol-4-one
163. la 2-[4-(6-méthylpyridazin-3-yl)-pipérazin-1-yl]-5-naphtalén-1-ylméthylènethiazol-4-one
164. la 2-(4-benzylpipérazin-1-yl)-5-(4-isopropylbenzylidène)-thiazol-4-one
165. l'acide 3-{2-[4-(4-chloro-[1,2,5]thiadiazol-3-yl)-pipérazin-1-yl]-4-oxo-4H-thiazol-5-ylidèneméthyl}-benzoïque
166. la 5-{3-[4-(4-chloro-3-trifluorométhylphényl)-4-hydroxypipéridin-1-carbonyl]-benzylidène}-2-thiomorpholin-4-ylthiazol-4-one
167. la 5-[3-[4-(4-chloro-[1,2,5]thiadiazol-3-yl)-pipérazin-1-carbonyl]-benzylidène}-2-[4-(4-chloro-[1,2,5]thiadiazol-3-yl)-pipérazin-1-yl]-thiazol-4-one
168. la 5-{3-[4-(3-chloropyridin-2-yl)-pipérazine-1-carbonyl]-benzylidène}-2-[4-(4-chloro-[1,2,5]thiadiazol-3-yl)-pipérazin-1-yl]-thiazol-4-one
169. la 5-[3-(4-benzylpipérazin-1-carbonyl)-benzylidène]-2-[4-(4-chloro-[1,2,5]thiadiazol-3-yl)-pipérazin-1-yl]-thiazol-4-one
170. la 2-[4-(4-chloro-[1,2,5]thiadiazol-3-yl)-pipérazin-1-yl]-5-{3-[4-(6-méthoxypyridin-2-yl)-pipérazin-1-carbonyl]-benzylidène}-thiazol-4-one
171. la 2-[4-(4-chloro-3-trifluorométhylphényl)-4-hydroxypipéridin-1-yl]-5-(4-méthylbenzylidène)-thiazol-4-one
172. la 2-[4-(4-chloro-3-trifluorométhylphényl)-4-hydroxypipéridin-1-yl]-5-(4-hydroxy-3-méthoxybenzylidène)-thiazol-4-one
173. la 2-[4-(4-chloro-3-trifluorométhylphényl)-4-hydroxypipéridin-1-yl]-5-naphtalén-1-ylméthylènethiazol-4-one
174. la 2-[4-(4-chloro-3-trifluorométhylphényl)-4-hydroxypipéridin-1-yl]-5-(3-méthoxybenzylidène)-thiazol-4-one
175. la 5-benzylidène-2-morpholin-4-ylthiazol-4-one
176. la 5-(4-méthylbenzylidène)-2-morpholin-4-ylthiazol-4-one
177. la 2-diéthylamino-5-(4-méthylbenzylidène)-thiazol-4-one
178. la 5-(4-chlorobenzylidène)-2-diéthylaminothiazol-4-one
179. la 5-(4-isopropylbenzylidène)-2-morpholin-4-ylthiazol-4-one
180. la 5-benzylidène-2-diéthylaminothiazol-4-one
181. la 5-(4-chlorobenzylidène)-2-morpholin-4-ylthiazol-4-one
182. la 5-(4-chlorobenzylidène)-2-pipéridin-1-ylthiazol-4-one
183. la 5-(4-chlorobenzylidène)-2-pyrrolidin-1-ylthiazol-4-one
184. la 5-benzylidène-2-pyrrolidin-1-ylthiazol-4-one
185. la 5-benzylidène-2-pyrrolidin-1-ylthiazol-4-one
186. la 5-biphényl-4-ylméthylène-2-pipéridin-1-ylthiazol-4-one
187. la 2-azépan-1-yl-5-biphényl-4-ylméthylènethiazol-4-one
188. la 5-biphényl-4-ylméthylène-2-pyrrolidin-1-ylthiazol-4-one
189. la 2-morpholin-4-yl-5-(3-phénylallylidène)-thiazol-4-one
190. la 5-(3-phénylallylidène)-2-thiomorpholin-4-ylthiazol-4-one
191. la 5-(3-phénylallylidène)-2-pipéridin-1-ylthiazol-4-one
192. la 2-azépan-1-yl-5-(3-phénylallylidène)-thiazol-4-one
193. la 5-(3-phénylallylidène)-2-pyrrolidin-1-ylthiazol-4-one
194. la 5-biphényl-4-ylméthylène-2-morpholin-4-ylthiazol-4-one
195. la 2-thiomorpholin-4-yl-5-(3-trifluorométhylbenzylidène)-thiazol-4-one
196. la 5-(4-chlorobenzylidène)-2-thiomorpholin-4-ylthiazol-4-one
197. la 5-(4-tert-butylbenzylidène)-2-morpholin-4-ylthiazol-4-one
198. la 5-(4-tert-butylbenzylidène)-2-thiomorpholin-4-ylthiazol-4-one
199. la 5-(4-tert-butylbenzylidène)-2-pyrrolidin-1-ylthiazol-4-one
200. la 2-azépan-1-yl-5-(4-tert-butylbenzylidène)-thiazol-4-one
201. la 5-(4-tert-butylbenzylidène)-2-pipéridin-1-ylthiazol-4-one
202. la 2-thiomorpholin-4-yl-5-(4-trifluorométhylbenzylidène)-thiazol-4-one
203. la 5-(4-tert-butylbenzylidène)-2-diéthylamino-thiazol-4-one
204. la 2-morpholin-4-yl-5-(4-trifluorométhylbenzylidène)-thiazol-4-one
205. la 2-morpholin-4-yl-5-(3-trifluorométhylbenzylidène)-thiazol-4-one
206. la 2-morpholin-4-yl-5-(3-trifluorométhylbenzylidène)-thiazol-4-one
207. la 5-biphényl-4-ylméthylène-2-diéthylaminothiazol-4-one
208. la 2-morpholin-4-yl-5-thiophén-2-ylméthylènethiazol-4-one
209. la 2-morpholin-4-yl-5-naphtalén-1-ylméthylènethiazol-4-one
210. la 2-morpholin-4-yl-5-pyridin-2-ylméthylènethiazol-4-one
211. la 2-morpholin-4-yl-5-(4-trifluorométhylbenzylidène)-thiazol-4-one
212. la 2-morpholin-4-yl-5-pyridin-3-ylméthylènethiazol-4-one
213. la 2-morpholin-4-yl-5-pyridin-4-ylméthylènethiazol-4-one
214. la 2-diéthylamino-5-(3-trifluorométhylbenzylidène)-thiazol-4-one
215. la 5-(4-méthylbenzylidène)-2-pyrrolidin-1-ylthiazol-4-one
216. la 5-(4-tert-butylbenzylidène)-2-(2-méthoxyéthylamino)-thiazol-4-one
217. la 2-morpholin-4-yl-5-(4-octylbenzylidène)-thiazol-4-one
218. la 5-(4-méthylbenzylidène)-2-morpholin-4-ylthiazol-4-one
219. la 5-(3,4-diméthoxybenzylidène)-2-morpholin-4-ylthiazol-4-one
220. la 5-(2-hydroxybenzylidène)-2-morpholin-4-ylthiazol-4-one
221. la 5-(2-hydroxy-3-méthoxybenzylidène)-2-morpholin-4-ylthiazol-4-one
222. la 5-(4-tert-butylbenzylidène)-2-morpholin-4-ylthiazol-4-one
223. la 5-(4-diéthylaminobenzylidène)-2-morpholin-4-ylthiazol-4-one
224. la 5-(4-hydroxy-3-méthoxybenzylidène)-2-morpholin-4-ylthiazol-4-one
225. l'acide 4-(2-morpholin-4-yl-4-oxo-4H-thiazol-5-ylidèneméthyl)benzoïque
226. la 2-morpholin-4-yl-5-(2,3,4-triméthoxybenzylidène)-thiazol-4-one
227. la 2-morpholin-4-yl-5-(3,4,5-triméthoxybenzylidène)-thiazol-4-one
228. la 5-(4-méthoxybenzylidène)-2-morpholin-4-ylthiazol-4-one
229. la 5-(4-hydroxybenzylidène)-2-morpholin-4-ylthiazol-4-one
230. la 5-(3-éthoxy-4-hydroxybenzylidène)-2-morpholin-4-ylthiazol-4-one
231. la 5-(3-hydroxybenzylidène)-2-morpholin-4-ylthiazol-4-one
232. la 5-(4-bromobenzylidène)-2-morpholin-4-ylthiazol-4-one
233. la 2-morpholin-4-yl-5-(4-vinyloxybenzylidène)-thiazol-4-one
234. l'ester 4-(2-morpholin-4-yl-4-oxo-4H-thiazol-5-ylidèneméthyl)-phénylique de l'acide benzènesulfonique
235. la 5-(4-diméthylaminobenzylidène)-2-morpholin-4-ylthiazol-4-one
236. l'ester méthylique de l'acide 4-(2-morpholin-4-yl-4-oxo-4H-thiazol-5-ylidèneméthyl)-benzoïque
237. la 5-(3-hydroxy-4-méthoxybenzylidène)-2-morpholin-4-ylthiazol-4-one
238. la 5-[4-(3,3-diméthylbutoxy)-3-méthoxybenzylidène]-2-morpholin-4-ylthiazol-4-one
239. la 5-(2-méthoxybenzylidène)-2-morpholin-4-ylthiazol-4-one
240. la 5-(4-hydroxybenzylidène)-2-morpholin-4-ylthiazol-4-one
241. la 2-[(benzo[1,3]dioxol-5-ylméthyl)-amino]-5-[3-(4-tert-butylphényl)-allylidène]-thiazol-4-one
242. la 2-[(benzo[1,3]dioxol-5-ylméthyl)-amino]-5-(4-trifluorométhylbenzylidène)-thiazol-4-one
243. la 2-[(benzo[1,3]dioxol-5-ylméthyl)-amino]-5-(4-trifluorométhoxybenzylidène)-thiazol-4-one
244. la 2-[(benzo[1,3]dioxol-5-ylméthyl)-amino]-5-naphtalén-1-ylméthylènethiazol-4-one
245. la 2-[(benzo[1,3]dioxol-5-ylméthyl)-amino]-5-(2-chloro-3-phénylallylidène)-thiazol-4-one
246. la 2-(4-méthylbenzylamino)-5-naphtalén-1-ylméthylènethiazol-4-one
247. la 2-(4-méthylbenzylamino)-5-(3-phénoxybenzylidène)-thiazol-4-one
248. la 2-(4-méthylbenzylamino)-5-(4-phénoxybenzylidène)-thiazol-4-one
249. la 5-(2-chloro-3-phénylallylidène)-2-(4-méthylbenzylamino)-thiazol-4-one
250. la 2-(4-méthylbenzylamino)-5-naphtalén-2-ylméthylènethiazol-4-one
251. la 2-(3-méthoxybenzylamino)-5-(4-méthylbenzylidène)-thiazol-4-one
252. la 5-(4-tert-butylbenzylidène)-2-(3-méthoxybenzylamino)-thiazol-4-one
253. la 2-(3-méthoxybenzylamino)-5-(4-trifluorométhylbenzylidène)-thiazol-4-one
254. la 2-(3-méthoxybenzylamino)-5-(4-trifluorométhoxybenzylidène)-thiazol-4-one
255. la 2-(3-méthoxybenzylamino)-5-(3-phénylallylidène)-thiazol-4-one
256. la 5-naphtalén-1-ylméthylène-2-(4-trifluorométhylbenzylamino)-thiazol-4-one
257. la 5-(3-phénoxybenzylidène)-2-(4-trifluorométhylbenzylamino)-thiazol-4-one
258. la 5-(4-phénoxybenzylidène)-2-(4-trifluorométhylbenzylamino)-thiazol-4-one
259. la 5-(2-chloro-3-phénylallylidène)-2-(4-trifluorométhylbenzylamino)-thiazol-4-one
260. la 5-(3-benzyloxybenzylidène)-2-(4-trifluorométhylbenzylamino)-thiazol-4-one
261. la 5-naphtalén-2-ylméthylène-2-(4-trifluorométhylbenzylamino)-thiazol-4-one
262. la 2-(4-hydroxy-3-méthoxyphényl)-5-(4-méthylbenzylidène)-thiazol-4-one et
263. la 2-(4-tert-butylphénylamino)-5-(4-méthylbenzylidène)-thiazol-4-one
le cas échéant, à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

15. Utilisation d'au moins un composé selon l'une ou plusieurs des revendications 1 à 14 pour la préparation d'un médicament destiné à la prophylaxie et/ou au traitement de la douleur, choisie dans le groupe constitué par la douleur aiguë, la douleur chronique, la douleur viscérale, la douleur articulaire et la douleur neuropathique.

16. Utilisation d'au moins un composé tel que défini dans l'une ou plusieurs des revendications 1 à 14 pour la préparation d'un médicament destiné à la prophylaxie et/ou au traitement d'une ou de plusieurs maladies, choisies dans le groupe constitué par les migraines ; les dépressions ; les névropathies ; les lésions nerveuses ; les maladies neurodégénératives, de préférence choisies dans le groupe constitué par la sclérose en plaques, la maladie d'Alzheimer, la maladie de Parkinson et la maladie d'Huntington ; les dysfonctionnements cognitifs, de préférence les états de déficience cognitive, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères peptiques ; le syndrome de l'intestin irritable ; les attaques ; les irritations oculaires ; les irritations de la peau ; les maladies neurotiques de la peau ; les maladies inflammatoires, de préférence les inflammations de l'intestin ; la diarrhée ; le prurit ; les troubles d'absorption des aliments, de préférence choisis dans le groupe constitué par la boulimie, la cachexie, l'anorexie et l'obésité ; la dépendance aux médicaments ; l'abus de médicaments ; les symptômes de sevrage lors de l'abus de médicaments ; le développement de la tolérance aux médicaments, de préférence aux opioïdes naturels ou synthétiques ; la dépendance aux drogues ; l'abus de drogues ; les symptômes de sevrage lors de la dépendances aux drogues ; la dépendance à l'alcool ; l'abus d'alcool et les symptômes de sevrage lors de la dépendance à l'alcool ; destiné à la diurèse ; à l'antinatriurèse ; à l'influence du système cardiovasculaire ; à l'augmentation de la vigilance ; à l'augmentation de la libido ; à la modulation de l'activité motrice ; à l'anxiolyse ; à l'anesthésie locale et/ou à l'inhibition d'effets secondaires non souhaités, de préférence choisis dans le groupe constitué par l'hyperthermie, l'hypertension et le rétrécissement des bronches, déclenchés par l'administration d'agonistes du récepteur 1 des vanilloïdes (récepteurs VR1/TRPV1), de préférence choisis dans le groupe constitué par la capsaïcine, la résinifératoxine, l'olvanil, l'arvanil, le SDZ-249665, le SDZ-249482, le nuvanil et le capsavanil (DA-5018).

17. Dérivés de thiazol-4-one substituées en
position 2,5 de formule générale Ia, 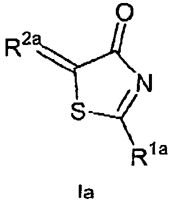 où
R^{1a} représente un radical cycloaliphatique non substitué ou au moins monosubstitué, insaturé ou
saturé, présentant le cas échéant au moins un hétéroatome comme élément de cycle, qui peut être condensé avec un système cyclique monocyclique ou polycyclique non substitué ou au moins monosubstitué ;
un groupe NR^{3a}R^{4a} ;
un groupe NR^{5a}-C(=O)-R^{6a}
ou un groupe NR^{7a}-C(=O)-NR^{8a}R^{9a} ;
R^{2a} représente CH-CH=C(CH₃) -W^{a}-Z^{a}, CH-CH=CH-W^{a}-Z^{a}, CH-C (CH₃) =CH-W^{a}-Z^{a}, CH-C (phényl) =CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH (CH₃)-W^{a}-Z^{a} ou CH-CH(CH₃)-CH₂-W^{a}-Z^{a} ;
W^{a} pouvant à chaque fois être absent ou représenter à chaque fois un radical choisi dans le groupe constitué par O, S, N(H), N(CH₃), N(C₂H₅) et N[CH(CH₃)₂] ;
R^{3a} représente un radical aliphatique linéaire ou ramifié, saturé ou insaturé, non substitué ou au moins monosubstitué, présentant le cas échéant au moins un hétéroatome comme élément chaîne ;
un radical cycloaliphatique non substitué ou au moins monosubstitué, insaturé ou saturé, présentant le cas échéant au moins un hétéroatome comme élément de cycle, qui peut être condensé avec un système cyclique monocyclique ou polycyclique non substitué ou au moins monosubstitué ;
ou un radical aryle ou hétéroaryle non substitué ou au moins monosubstitué, qui peut être condensé avec un système cyclique monocyclique ou polycyclique non substitué ou au moins monosubstitué et/ou lié via un groupe alkylène, alcénylène ou alcynylène linéaire ou ramifié, non substitué ou au moins monosubstitué ;
R^{4a} représente un radical hydrogène
ou un radical aliphatique linéaire ou ramifié, saturé ou insaturé, non substitué ou au moins monosubstitué, présentant le cas échéant au moins un hétéroatome comme élément chaîne ;
R^{5a}, R^{7a} et R^{9a} représentent, indépendamment l'un de l'autre, à chaque fois
un radical hydrogène
ou un radical aliphatique linéaire ou ramifié, saturé ou insaturé, non substitué ou au moins monosubstitué ;
R^{6a} et R^{8a} représentent, indépendamment l'un de l'autre, à chaque fois
un radical aryle ou hétéroaryle non substitué ou au moins monosubstitué, qui peut être condensé avec un système cyclique monocyclique ou polycyclique non substitué ou au moins monosubstitué ;
Z^{a} représente un radical aliphatique linéaire ou ramifié, saturé ou insaturé, non substitué ou au moins monosubstitué ;
un radical aryle, qui est condensé avec un système cyclique monocyclique ou polycyclique non substitué ou au moins monosubstitué et/ou substitué par des substituants R^{12a} identiques ou différents ;
ou un radical hétéroaryle, qui est condensé avec un système cyclique monocyclique ou polycyclique non substitué ou au moins monosubstitué et/ou substitué par des substituants R^{12a} identiques ou différents ;
R^{12a} représente un radical choisi dans le groupe constitué par F, Cl, Br, I, -CN, -CF₃, -SF₅, -OH, -O-C₁₋₁₀-alkyle, -O-C₂₋₁₀-alcényle, -NH₂, -O-CF₃, -S-CF₃, -SH, -S-C₁₋₅-alkyle, -C₁₋₁₀-alkyle, -C(=O)-OH, -C(=O)-O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, -C(=O) -N- (C₁₋₅-alkyle)₂, -S (=O)₂-C₁₋₅-alkyle, -S(=O)₂-phényle, -O-S(=O)₂-phényle, -NH-S(=O)₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -0-phényle, -0-benzyle, phényle et benzyle ; la partie cyclique des radicaux pyridinyle, cyclopentyle, cyclohexyle, pyridazinyle, -S(=O)₂-phényle, -OS(=O)₂-phényle, -O-phényle, -O-benzyle, phényle, -(CH₂)-benzo[b]furannyle et benzyle pouvant à chaque fois être substituée ;
un groupe -C(=O)-NR^{13a}-(CH₂)ₘₐ-NR^{14a}-R^{15a}, m^{a} valant 0, 1, 2, 3, 4 ou 5 ;
ou un groupe -C(=O)-R^{16a} ;
R^{13a} et R^{14a} représentent, indépendamment l'un de l'autre, à chaque fois
un radical hydrogène
ou un radical aliphatique linéaire ou ramifié, saturé ou insaturé, non substitué ou au moins monosubstitué ;
R^{15a} représente un radical aryle ou hétéroaryle non substitué ou au moins monosubstitué, qui peut être condensé avec un système cyclique monocyclique ou polycyclique non substitué ou au moins monosubstitué ; et
R^{16a} représente un radical cycloaliphatique non substitué ou au moins monosubstitué, insaturé ou saturé, présentant le cas échéant au moins un hétéroatome comme élément de cycle, qui peut être condensé avec un système cyclique monocyclique ou polycyclique non substitué ou au moins monosubstitué ;
le cas échéant à chaque fois sous forme d'un de leurs stéréo-isomères purs, en particulier leurs énantiomères ou diastéréo-isomères, leurs racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

18. Composé selon la revendication 17, **caractérisé en ce que**
R^{1a} représente un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ;
un groupe NR^{3a}R^{4a} ;
un groupe NR^{5a}-C (=O) -R^{6a}
ou un groupe NR^{7a}-C(=O)-NR^{8a}R^{9a} ;
R^{2a} représente CH-CH=C (CH₃) -W^{a}-Z^{a}, CH-CH=CH-W^{a}-Z^{a}, CH-C (CH₃)=CH-W^{a}-Z^{a}, CH-C(phényl)=CH-W^{a}-Z^{a}, CH-CBr=CH-W^{a}-Z^{a}, CH-CCl=CH-W^{a}-Z^{a}, CH-CF=CH-W^{a}-Z^{a}, CH-C(OH)=CH-W^{a}-Z^{a}, CH-CH₂-CH₂-W^{a}-Z^{a}, CH-CH₂-CH(CH₃)-W^{a}-Z^{a} ou CH-CH(CH₃)-CH₂-W^{a}-Z^{a} ;
W^{a} pouvant à chaque fois être absent ou représenter à chaque fois un radical choisi dans le groupe constitué par O, S, N(H), N(CH₃), N(C₂H₅) et N[CH(CH₃)₂] ;
R^{3a} représente un radical aliphatique en C₁₋₁₀ linéaire ou ramifié, saturé ou insaturé, le cas échéant substitué, présentant le cas échéant 1, 2 ou 3 hétéroatomes choisis indépendamment les uns des autres dans le groupe constitué par oxygène, azote (NH) et soufre ;
un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ;
ou un radical aryle ou hétéroaryle de 5 à 14 chaînons, le cas échéant substitué, qui peut être condensé avec un système monocyclique ou polycyclique, saturé ou insaturé, le cas échéant substitué et/ou lié via un groupe C₁₋₅-alkylène ;
R^{4a} représente un radical hydrogène
ou un radical aliphatique en C₁₋₁₀ linéaire ou ramifié, saturé ou insaturé, le cas échéant substitué, présentant le cas échéant 1, 2 ou 3 hétéroatomes choisis indépendamment les uns des autres dans le groupe constitué par oxygène, azote (NH) et soufre ;
R^{5a}, R^{7a} et R^{8a} représentent indépendamment les uns des autres à chaque fois un radical hydrogène ou un radical aliphatique en C₁₋₁₀ linéaire ou ramifié, saturé ou insaturé, le cas échéant substitué ;
R^{6a} et R^{9a} représentent, indépendamment l'un de l'autre, à chaque fois
un radical aryle ou hétéroaryle de 5 à 14 chaînons, le cas échéant substitué, qui peut être condensé avec un système cyclique monocyclique ou polycyclique, saturé ou insaturé, le cas échéant substitué ;
Z^{a} représente un radical aliphatique en C₁₋₂₀ linéaire ou ramifié, saturé ou insaturé, le cas échéant substitué ;
un radical aryle de 6 à 10 chaînons, substitué, qui est condensé avec un système cyclique monocyclique ou polycyclique, saturé ou insaturé le cas échéant substitué et/ou substitué par 1, 2, 3, 4 ou 5 substituants R^{12a} identiques ou différents ;
ou un radical hétéroaryle de 5 à 14 chaînons, le cas échéant substitué, qui est condensé avec un système cyclique monocyclique ou polycyclique, saturé ou insaturé le cas échéant substitué et/ou substitué par 1, 2, 3, 4 ou 5 substituants R^{12a} identiques ou différents ;
R^{12a} représente un radical choisi dans le groupe constitué par F, Cl, Br, I, -CN, -CF₃, -SF₅, -OH, -O-C₁₋₁₀-alkyle, -O-C₂₋₁₀-alcényle, -NH₂, -O-CF₃, -S-CF₃, -SH, -S-C₁₋₅-alkyle, -C₁₋₁₀-alkyle, -C(=O)-OH, -C(=O)-O-C₁₋₅-alkyle, -O-C(=O)-C₁₋₅-alkyle, -NH-C₁₋₅- alkyle, -N (C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, -C (=O) -N- (C₁₋₅-alkyle)₂, -S (=O)₂-C₁₋₅- alkyle, -S(=O)₂-phényle, -O-S(=O)₂-phényle, -NH-S(=O)₂-C₁₋₅-alkyle, -S(=O)₂NH-C₁₋₅-alkyle, cyclohexyle, cyclopentyle, pyridinyle, pyridazinyle, -(CH₂)-benzo[b]furannyle, -0-phényle, -0-benzyle, phényle et benzyle ; la partie cyclique des radicaux pyridinyle, cyclopentyle, cyclohexyle, pyridazinyle, -S(=O)₂-phényle, -OS(=O)₂-phényle, -O-phényle, -O-benzyle, phényle, -(CH₂)-benzo[b]furannyle et benzyle pouvant être à chaque fois substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅- alkyle, -O-CF₃, -S-CF₃, phényle et -O-benzyle ;
un groupe -C(=O)-NR^{13a}- (CH₂)ₘₐ-NR^{14a}-R^{15a}, m^{a} valant 0, 1, 2, 3, 4 ou 5 ;
ou un groupe -C(=O)-R^{16a} ;
R^{13a} et R^{14a}, représentent, indépendamment l'un de l'autre, à chaque fois
un radical hydrogène
ou un radical aliphatique en C₁₋₁₀ linéaire ou ramifié, saturé ou insaturé, le cas échéant substitué ;
R¹⁵ représente un radical aryle ou hétéroaryle de 5 à 14 chaînons, qui peut être condensé avec un système cyclique monocyclique ou polycyclique, saturé ou insaturé, le cas échéant substitué ; et
R^{16a} représente un radical cycloaliphatique de 3, 4, 5, 6, 7, 8 ou 9 chaînons, insaturé ou saturé, le cas échéant substitué, qui peut être condensé avec un système cyclique monocyclique ou polycyclique, saturé ou insaturé, le cas échéant substitué ;
les radicaux cycloaliphatiques susmentionnés pouvant à chaque fois être le cas échéant substitués par 1, 2, 3, 4 ou 5 substituants choisis indépendamment les uns des autres dans le groupe constitué par oxo (=0), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅- alkyle, -C(=O)-OH, -C(=O)-O-C₁₋₅-alkyle, -O-C(=O)-C₁₋₅-alkyle, -NH-C₁₋₅-alkyle, -N (C₁₋₅-alkyle)₂, -NH-phényle, -NH-pyridinyle, -N(C₁₋₅-alkyl)-phényle, -N(C₁₋₅-alkyl) - pyridinyle, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C(=O)-N-(C₁₋₅-alkyle)₂, -S(=O)₂-C₁₋₅-alkyle, -S(=O)₂-phényle_{,} -NH-S (=O)₂-C₁₋₅-alkyle, -S (=O)₂-NH-C₁₋₅-alkyle, cyclohexyle, cyclopentyle, pyridinyle, [1,2,5]-thiadiazolyle, pyridazinyle, -(CH₂)-benzo[b]furannyle, -0-phényle, -0-benzyle, phényle et benzyle, la partie cyclique des radicaux -NH-phényle, -NH-pyridinyle, -N(C₁₋₅₋alkyl)-phényle, -N(C₁₋₅-alkyl)-pyridinyle, pyridinyle, cyclopentyle, [1,2,5]-thiadiazolyle, cyclohexyle, pyridazinyle, -S(=O)₂-phényle, -O-phényle, -O-benzyle, phényle, -(CH₂)-benzo[b]furannyle et benzyle pouvant être à chaque fois substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -0-benzyle,
et les radicaux cycloaliphatiques susmentionnés pouvant présenter, le cas échéant à chaque fois 1, 2, 3, 4 ou 5 hétéroatome(s) comme élément(s) de cycle, choisi(s) indépendamment les uns des autres dans le groupe constitué par oxygène, azote et soufre ;
les cycles des systèmes cycliques monocycliques ou polycycliques susmentionnés pouvant le cas échéant à chaque fois être substitués par 1, 2, 3, 4 ou 5 substituants choisis indépendamment les uns des autres dans le groupe constitué par oxo (=O), thioxo (=S), F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₅-alkyle, -C(=O)-OH, -C(=O)-O-C₁₋₅-alkyle, -O-C(=O)-C₁₋₅-alkyle, -NH-C₁₋₅-alkyle, -N(C₁₋₅-alkyle)₂, -NH-C(=O)-O-C₁₋₅-alkyle, -C(=O)-H, -C(=O)-C₁₋₅-alkyle, -C(=O)-NH₂, -C(=O)-NH-C₁₋₅-alkyle, C (=O) -N- (C₁₋₅-alkyle)₂, -S (=O)₂-C₁₋₅-alkyle, -S (=O)₂-phényle, -NH-S (=O)₂-C₁₋₅-alkyle, -S (=O)₂-NH-C₁₋₅-alkyle, cyclohexyle, cyclopentyle, pyridinyle, pyridazinyle, -(CH₂)-benzo[b]furannyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les cycles des systèmes cycliques monocycliques ou polycycliques susmentionnés pouvant présenter à chaque fois 5, 6 ou 7 chaînons et le cas échant à chaque fois 1, 2, 3, 4 ou 5 hétéroatome(s) comme élément(s) de cycle, choisi(s), indépendamment les uns des autres, dans le groupe constitué par oxygène, azote et soufre ; les radicaux aliphatiques en C₁₋₁₀ ou les radicaux aliphatiques en C₁₋₂₀ susmentionnés pouvant le cas échéant à chaque fois être substitués par 1, 2, 3, 4, 5, 6, 7, 8 ou 9 substituants, choisis indépendamment les uns des autres dans le groupe constitué par F, Cl, Br, I, -CN, -NO₂, -OH, -SH et -NH ;
les radicaux aryle ou hétéroaryle susmentionnés pouvant le cas échéant à chaque fois être substitués par 1, 2, 3, 4 ou 5 substituants choisis indépendamment les uns des autres dans le groupe constitué par F, Cl, Br, I, -CN, -CF₃, -SF₅, -OH, -O-C₁₋₅-alkyle, -NH₂, -NO₂, -O-CF₃, -S-CF₃, -SH, -S-C₁₋₅-alkyle, -C₁₋₁₀-alkyle, -C(=O)-OH, -C(=O)-O-C₁₋₅-alkyle, -O-C(=O)-C₁₋₅-alkyle, -NH-C₁₋₅-alkyle, -N (C₁₋₅-alkyle)₂, -NH-C(=O)-O-C₁₋₅-alkyle, -NH-C(=O)-C₁₋₅-alkyle, -C(=O)-H, -C(=O)-C₁₋₅-alkyle, -C(=O)-NH₂, -C(=O)-NH-C₁₋₅-alkyle, -C (=O) -N- (C₁₋₅-alkyle)₂, -S (=O)₂-C₁₋₅-alkyle, -S(=O)₂-phényle, -NH-S (=O)₂-C₁₋₅-alkyle, -S(=O)₂-NH-C₁₋₅-alkyle, cyclohexyle, cyclopentyle, pyridinyle, pyridazinyle, -(CH₂)-benzo[b]furannyle, -O-phényle, -O-benzyle, phényle et benzyle, la partie cyclique des radicaux pyridinyle, cyclopentyle, cyclohexyle, pyridazinyle, -S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, -C₁₋₅-alkyle, -O-C₁₋₅-alkyle, -O-CF₃, -S-CF₃, phényle et -O-benzyle,
et les radicaux hétéroaryle susmentionnés pouvant présenter, le cas échéant à chaque fois 1, 2, 3, 4 ou 5 hétéroatome(s) comme élément(s) de cycle, choisi(s) indépendamment les uns des autres dans le groupe constitué par oxygène, azote et soufre ;
les radicaux C₁₋₅-alkylène susmentionnés pouvant le cas échéant à chaque fois être substitués par 1, 2, 3, 4 ou 5 substituants, choisis indépendamment les uns des autres dans le groupe constitué par F, Cl, Br, -OH, -SH, -NH₂, -CN et NO₂ ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

19. Composé selon la revendication 17 ou 18, **caractérisé en ce que**
R^{1a} représente un radical choisi dans le groupe constitué par cyclopentyle, cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, thiomorpholinyle, azépanyle, (2,3)-dihydro-1H-iso-indolyle, (2,3)-dihydro-indolyle, (2,3,4,9)-tétrahydro-1H-β-carbolinyle, (2,3,4,9)-tétrahydro-1H-pyrido[2,3-b]indolyle, [1,2,3,4]-tétrahydronaphtyle, [1,2,3,4]-tétrahydroquinoléinyle, [1,2,3,4]-tétrahydro-isoquinoléinyle, [1,2,3,4]-tétrahydroquinazolinyle et [3,4]-dihydro-2H-1,4-benzoxazinyle ; le radical pouvant le cas échéant à chaque fois être substitué par 1, 2, 3, 4 ou 5 substituants choisis indépendamment les uns des autres dans le groupe constitué par oxo (=O), thioxo (=S), F, Cl, Br, I, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-OC₂H₅, -C(-O)-O-C(CH₃)₃, -O-C(=O)-CH₃, -O-C(=O)-C₂H₅, -O-C(=O)-C(CH₃)_{3,} -N(CH₃)₂, -N(C₂H₅)₂, -NH-CH₃, -NH-C₂H₅, -NH-CH(CH₃)₂, -NH-C(CH₃)₃, -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle, -N[CH(CH₃)₂]-pyridinyle, -NH-C(=O)-O-CH₃, -NH-C (=O) -O-C₂H₅, -NH-C (=O) -O-C (CH₃)₃, -C (=O) - CH₃, -C(=O)-C₂H₅, -C(=O)-NH-CH₃, -C (=O) -NH-C₂H₅, -C (=O) -N- (CH₃) ₂, -C (=O) -N- (C₂H₅)₂, pyridinyle, pyridazinyle et phényle ; la partie cyclique des radicaux -NH-phényle, -NH-pyridinyle, -N(CH₃)-phényle, -N (C₂H₅) -phényle, -N [CH (CH₃)₂] -phényle, -N(CH₃)-pyridinyle, -N(C₂H₅) -pyridinyle, -N[CH(CH₃)₂]-pyridinyle, pyridinyle, pyridazinyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
un radical pipérazinyle, qui peut être substitué par 1, 2 ou 3 substituants choisis indépendamment les uns des autres dans le groupe constitué par méthyle, éthyle, n-propyle, isopropyle, n-butyle, sec-butyle, isobutyle, tert-butyle, -C(=O)-O-CH₃, -C(=O)-O-C₂H₅, -C(=O) -O-C (CH₃)₃, -C (=O) -C₁₋₅-alkyle, -C(=O)-NH₂, -C (=O) -CH₃, -C(=O)-C₂H₅, -C(=O)-NH-CH₃, -C (=O) -NH-C₂H₅, -C (=O) -N- (CH₃)₂, -C (=O) -N- (C₂H₅)₂, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -O-benzyle ;
un groupe NR^{3a}R^{4a} _{;}
un groupe NR^{5a}-C(=O)-R^{6a}
ou un groupe NR^{7a}-C(=O)-NR^{8a}R^{9a}.

20. Composé selon l'une ou plusieurs des revendications 17 à 19, **caractérisé en ce que**
R^{2a} représente CH-CH=C(CH₃)-Z^{a}, CH-CH=CH-Z^{a}, CH-CH=CH-S-Z^{a}, CH-CH=CH-O-Z^{a}, CH-CH=CH-N(CH₃)-Z^{a}, CH-C (CH₃) =CH-Z^{a}, CH-C (phényl) =CH-Z^{a}, CH-CBr=CH-Z^{a}, CH-CCl=CH-Z^{a}, CH-CF=CH-Z^{a}, CH-C (OH) =CH-Z^{a}, CH-CH₂-CH₂-Z^{a}, CH-CH₂-CH(CH₃)-Z^{a} ou CH-CH(CH₃)-CH₂-Z^{a}.

21. Composé selon l'une ou plusieurs des revendications 17 à 20, **caractérisé en ce que**
R^{3a} représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N (CH₃) -CH₃, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH ;
un radical choisi dans le groupe constitué par cyclopentyle cyclohexyle, cycloheptyle, cyclopentényle, cyclohexényle, cycloheptényle, imidazolidinyle, tétrahydrofurannyle, tétrahydrothiophényle, pyrrolidinyle, pipéridinyle, morpholinyle, thiomorpholinyle et azépanyle ; le radical pouvant le cas échéant à chaque fois être substitué par 1, 2, 3, 4 ou 5 substituants choisis indépendamment les uns des autres dans le groupe constitué par méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH(CH₃)₂, -NH-C (CH₃)₃, -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle, -N[CH(CH₃)₂]-pyridinyle; la partie cyclique des radicaux -NH-phényle, -NH-pyridinyle, -N(CH₃)-phényle, -N(C₂H₅)-phényle, -N[CH(CH₃)₂]-phényle, -N(CH₃)-pyridinyle, -N(C₂H₅)-pyridinyle et -N[CH(CH₃)₂]-pyridinyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle ;
ou un radical choisi dans le groupe constitué par phényle, naphtyle, (1,3)-benzodioxolyle et (1,4)-benzodioxanyle ; le radical pouvant être lié via un groupe -(CH₂), -(CH₂), -(CH₂) ou -(CH₂)-(CH₂)-(CH₂) et/ou le cas échéant à chaque fois être substitué par 1, 2, 3, 4 ou 5 substituants choisis indépendamment les uns des autres dans le groupe constitué par F, Cl, Br, I, -SF₅, -OH, -O-CH₃, -0-C₂H₅, -NH₂, -NO₂, -CF₃, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-O-CH₃, -C(=O)-O-C₂H₅, -C(=O)-O-C(CH₃)₃, -0-C(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-CH(CH₃)₂ et -NH-C(CH₃)₃.

22. Composé selon l'une ou plusieurs des revendications 17 à 21, **caractérisé en ce que**
R^{4a} représente un radical hydrogène ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂.

23. Composé selon l'une ou plusieurs des revendications 17 à 22, **caractérisé en ce que** R^{5a}
, R^{7a} et R^{8a} représentent, indépendamment les uns des autres, à chaque fois
un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ; le radical pouvant, le cas échéant, à chaque fois, être substitué par 1, 2, 3, 4 ou 5 substituants choisis indépendamment l'un de l'autre dans le groupe constitué par F, Cl, Br, I, -CN, -NO₂, -OH, -SH et -NH₂.

24. Composé selon l'une ou plusieurs des revendications 17 à 23, **caractérisé en ce que**
R^{6a} et R^{9a} représentent, indépendamment l'un de l'autre, à chaque fois
un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO₂, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -NH-C(=O)-O-CH₃, -NH-C (=O) -O-C₂H₅, -NH-C(=O)-OC(CH₃)₃, -C(=O)-H, -C(=O)-CH₃, -C(=O)-C₂H₅, -C(=O)-C(CH₃)₃.

25. Composé selon l'une ou plusieurs des revendications 17 à 24, **caractérisé en ce que**
Z^{a} représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle et n-eicosanyle, le radical pouvant le cas échéant à chaque fois être substitué par 1, 2, 3, 4 ou 5 substituants choisis indépendamment les uns des autres dans le groupe constitué par F, Cl, Br, I, -CN, -NO₂, -OH, -SH et -NH₂ ;
un radical choisi dans le groupe constitué par phényle, naphtyle, phénanthrényle, (1,3)-benzodioxolyle et (1,4)-benzodioxanyle, qui est substitué par 1, 2, 3, 4 ou 5 substituants R^{12a} identiques ou différents ;
ou un radical choisi dans le groupe constitué par thiophényle, furannyle, pyrrolyle, pyrazolyle, pyrazinyle, pyridinyle, pyridazinyle, pyrimidinyle, quinoléinyle et isoquinoléinyle, qui est substitué par 1, 2, 3, 4 ou 5 substituants R^{12a} identiques ou différents.

26. Composé selon l'une ou plusieurs des revendications 17 à 25, **caractérisé en ce que**
R^{12a} représente un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle, n-octyle, -C(=O)-OH, -C(=O)-O-CH₃, -C(=O)-O-C₂H₅, -C(=O)-O-C(CH₃)₃, -OC(=O)-CH₃, -O-C(=O)-C₂H₅, -O-C(=O)-C(CH₃)₃, -N(CH₃)₂, -N(C₂H₅)₂, -NH-CH₃, -NH-C₂H₅, -NH-C(=O)-O-CH₃, -NH-C(=O)-O-C₂H₅, -NH-C(=O)-O-C(CH₃)₃, -NH-C(=O)-CH₃, -NH-C(=O)-C₂H₅, -NH-C(=O)-C(CH₃)₃, -C(=O)-H, -C(=O)-CH₃, -C(=O)-C₂H₅, -C(=O)-C(CH₃)₃, -C(=O)-NH₂, -C(=O)-NH-CH₃, -C(=O)-NH-C₂H₅, -C(=O)-N-(CH₃)₂, -C(=O)-N-(C₂H₅)₂, -S(=O)₂-CH₃, -S(=O)₂-C₂H₅, -NH-S(=O)₂-CH₃, -NH-S(=O)₂-C₂H₅, -S(=O)₂-NH-CH₃, -S(=O)₂-phényle, -O-S(=O)₂-phényle, cyclohexyle, cyclopentyle, pyridinyle, pyridazinyle, -(CH₂)-benzo[b]furannyle, -O-phényle, -O-benzyle, phényle et benzyle ; la partie cyclique des radicaux pyridinyle, cyclopentyle, cyclohexyle, pyridazinyle, -S(-O)₂-phényle, -O-S(=O)₂-phényle, -O-phényle, -O-benzyle, phényle, -(CH₂)-benzo[b]furannyle et benzyle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -0-CH₃, -O-C₂H₅, -O-CF₃, -S-CF₃, phényle et -0-benzyle ;
un groupe -C(=O)-NR^{13a}- (CH₂)ₘₐ-NR^{14a}- R^{15a}, m valant 0, 1, 2 ou 3 ;
ou un groupe -C(=O)-R^{16a};
R^{13a} et R^{14a} représentant, indépendamment l'un de l'autre, à chaque fois
un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R^{15a} représentant un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -OH, -O-CH₃, -O-C₂H₅, -NH₂, -NO, -O-CF₃, -S-CF₃, -SH, -S-CH₃, -S-C₂H₅, méthyle, éthyle, n-propyle, isopropyle, n-butyle, sec-butyle, isobutyle, tert-butyle, -C(=O)-OH, -C(=O)-O-CH₃, -C(=O)-O-C₂H₅, -C(=O)-O-C(CH₃)₃, -O-C(=O)-CH₃, -O-C(=O)-C₂H₅, -0-C(=O)-C(CH₃)₃, -N(CH₃)₂, -N(C₂H₅)₂, -NH-CH₃, -NH-C₂H₅, -NH-C(=O)-O-CH₃, -NH-C (=O)-O-C₂H₅, -NH-C (=O)-OC(CH₃)₃, -C((=O)-H, -C(=O)-CH₃, -C(=O)-C₂H₅, -C(=O)-C(CH₃)₃ ; et
R^{16a} représentant un radical choisi dans le groupe constitué par pipérazinyle, thiomorpholinyle, azépanyle, morpholinyle, (2,3)-dihydro-1H-iso-indolyle, (2,3)-dihydro-indolyle, pipéridinyle et pyrrolidinyle ; le radical pouvant être substitué par 1, 2 ou 3 substituants choisis indépendamment les uns des autres dans le groupe constitué par -OH, méthyle, éthyle, n-propyle, isopropyle, n-butyle, sec-butyle, isobutyle, tert-butyle, -C(=O)-O-CH₃, -C(=O)-O-C₂H₅, -C (=O) -O-C (CH₃)₃, -C (=O) -C₁₋₅-alkyle, -C(=O)-NH₂, -C(=O)-CH₃, -C(=O)-C₂H₅, -C(=O)-NH-CH₃, -C(=O)-NH-C₂H₅, -C(=O)-N-(CH₃)₂, -C(=O)-N-(C₂₋₅)₂, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2, 3, 4 ou 5 substituants choisis indépendamment les uns des autres dans le groupe constitué par F, Cl, Br, -OH, -CF₃, -SF₅, -CN, -NO₂, méthyle, éthyle, n-propyle, isopropyle, n-butyle, sec-butyle, isobutyle, tert-butyle, -O-CH₃, -O-C₂H₅, -O-CF₃, -S-CF₃, phényle et -0-benzyle.

27. Composé selon l'une ou plusieurs des revendications 17 à 26, **caractérisé en ce que**
R^{1a} représente un des radicaux suivants 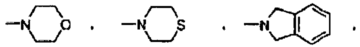 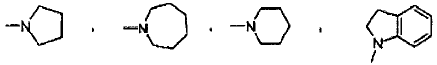 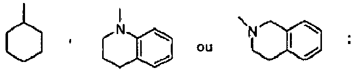 le radical pouvant être substitué par 1, 2 ou 3 substituants choisis indépendamment les uns des autres dans le groupe constitué par -OH, -O-CH₃, -O-C₂H₅, méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH(CH₃)₂, -NH-C(CH₃)₃, -NH-phényle, -NH-pyridinyle, -N(CH₃)-phényle, -N(C₂H₅) -phényle, -N[CH(CH₃)₂] -phényle, -N(CH₃)-pyridinyle, -N(C₂H₅)-pyridinyle, -N[CH(CH₃)₂]-pyridinyle et phényle ; la partie cyclique du radical phényle pouvant à chaque fois être substituée par 1, 2 ou 3 substituants choisis indépendamment les uns des autres dans le groupe constitué par F, Cl, Br et -CF₃ ;
un des radicaux suivants 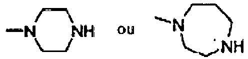 l'atome d'hydrogène du groupe -NH pouvant être substitué par un substituant choisi dans le groupe constitué par méthyle, éthyle, n-propyle, isopropyle, n-butyle, sec-butyle, isobutyle, tert-butyle, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
un groupe NR^{3a}R^{4a}
ou un groupe NR^{7a}-C(=O)-NR^{8a}R^{9a};
R^{2a} représente CH-CH=C (CH₃) -Z^{a}, CH-CH=CH-Z^{a}, CH-CH=CH-S-Z^{a}, CH-CH=CH-O-Z^{a}, CH-CH=CH-N(CH₃)-Z^{a}, CH-C(CH₃)=CH-Z^{a}, CH-C(phényl)=CH-Z^{a}, CH-CBr=CH-Z^{a}, CH-CCl=CH-Z^{a}, CH-CF=CH-Z^{a}, CH-C(OH)=CH-Z^{a}, CH-CH₂-CH₂-Z^{a}, CH-CH₂-CH(CH₃)-Z^{a} ou CH-CH(CH₃)-CH₂-Z^{a};
R^{3a} représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N (CH₃) -CH₃, isopropyle, n-butyle, sec-butyle, isobutyle, tert-butyle, (1,1)-diméthylpropyle, n-pentyle, sec-pentyle, n-hexyle et n-heptyle ;
un radical choisi dans le groupe constitué par cyclopentyle cyclohexyle, cycloheptyle, cyclopentényle, cyclohexényle et cycloheptényle ; le radical pouvant le cas échéant à chaque fois être substitué par 1, 2, 3, 4 ou 5 substituants choisis indépendamment les uns des autres dans le groupe constitué par méthyle, éthyle, n-propyle, isopropyle, n-butyle, sec-butyle, isobutyle, tert-butyle, -N(CH₃)₂, -N(C₂H₅)₂, -NH-CH₃, -NH-C₂H₅, -NH-CH(CH₃)₂, -NH-C(CH₃)₃, -NH-phényle, -NH-pyridinyle, -N (CH₃) -phényle, -N (C₂H₅)-phényle, -N [CH (CH₃)₂]-phényle, -N (CH₃) -pyridinyle, -N (C₂H₅)-pyridinyle, -N[CH(CH₃)₂]-pyridinyle ;
ou un radical choisi dans le groupe constitué par phényle, naphtyle, (1,3)-benzodioxolyle et (1,4)-benzodioxanyle ; le radical pouvant être lié via un groupe -(CH₂)-, -(CH₂)-(CH₂) ou -(CH₂)-(CH₂)-(CH₂)- et/ou le cas échéant à chaque fois substitué par 1, 2, 3, 4 ou 5 substituants choisis indépendamment les uns des autres dans le groupe constitué par F, Cl, Br, -CF₃, -O-CF₃, -OCH₃, -OC₂H₅, méthyle, éthyle, n-propyle, isopropyle, n-butyle et tert-butyle ;
R^{4a} représente un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R^{7a} et R^{8a} représentent, indépendamment l'un de l'autre à chaque fois
un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R^{9a} représente un radical choisi dans le groupe constitué par phényle, naphtyle, thiophényle, furannyle, pyridinyle, thiazolyle et oxazolyle, le radical pouvant le cas échéant à chaque fois être substitué par 1, 2, 3, 4 ou 5 substituants choisis indépendamment les uns des autres dans le groupe constitué par F, Cl, Br, I, -CN, -CF₃, -SF₅, -0-CH₃, -O-C₂H₅, -O-CF₃, -S-CF₃, méthyle, éthyle, n-propyle, isopropyle, n-butyle, sec-butyle, isobutyle et tert-butyle ;
Z^{a} représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle et n-eicosanyle ;
un radical choisi dans le groupe constitué par phényle, naphtyle, phénanthrényle, (1,3)-benzodioxolyle et (1,4)-benzodioxanyle, qui est substitué par 1, 2, 3, 4 ou 5 substituants R^{12a} identiques ou différents ;
ou un radical choisi dans le groupe constitué par thiophényle, furannyle, pyrrolyle, pyrazolyle, pyrazinyle, pyridinyle, pyridazinyle, pyrimidinyle, quinoléinyle et isoquinoléinyle, qui est substitué par 1, 2, 3, 4 ou 5 substituants R^{12a} identiques ou différents.
R^{12a} représente un radical choisi dans le groupe constitué par F, Cl, Br, -SF₅, -CF₃, -O-CH₃, -0-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, méthyle, éthyle, n-propyle, isopropyle, n-butyle, sec-butyle, tert-butyle, n-pentyle, n-hexyle, n-heptyle et n-octyle ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

28. Composé selon l'une ou plusieurs des revendications 17 à 27, **caractérisé en ce que**
R¹² représente un des radicaux suivants 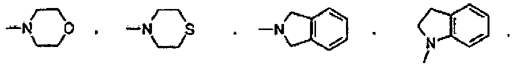 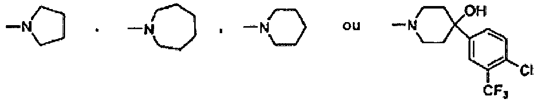 le radical suivant 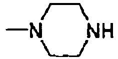 l'atome d'hydrogène du groupe -NH pouvant être substitué par un substituant choisi dans le groupe constitué par méthyle, éthyle, n-propyle, isopropyle, n-butyle, sec-butyle, isobutyle, tert-butyle, pyridinyle, pyridazinyle, [1,2,5]-thiadiazolyle, benzyle et phényle ; la partie cyclique des radicaux pyridinyle, pyridazinyle, [1,2,5]-thiadiazolyle, benzyle et phényle pouvant à chaque fois être substituée par 1, 2 ou 3 substituants choisis indépendamment les uns des autres dans le groupe constitué par F, Cl, Br, -CF₃, méthyle, éthyle, n-propyle, isopropyle, n-butyle, sec-butyle, isobutyle, tert-butyle, -O-CH₃, -O-C₂H₅, -O-CF₃ et -S-CF₃ ;
un groupe NR^{3a}R^{4a}
ou un groupe NR^{7a}-C(=O)-NR^{8a}R^{9a};
R^{2a} représente CH-CH=C (CH₃) -Z^{a}, CH-CH=CH-Z^{a}, CH-CH=CH-S-Z^{a}, CH-C(CH₃)=CH-Z^{a}, CH-C(phényl)=CH-Z^{a}, CH-CBr=CH-Z^{a}, CH-CCl=CH-Z^{a}, CH-CF=CH-Z^{a}, CH-CH₂-CH₂-Z^{a}, CH-CH₂-CH(CH₃)-Z^{a} ou CH-CH(CH₃)-CH₂-Z^{a};
R^{3a} représente un radical choisi dans le groupe constitué par méthyle, -CH₂-O-CH₃, -CH₂-S-CH₃, éthyle, -CH₂-CH₂-S-CH₃, -CH₂-CH₂-O-CH₃, -CH₂-CH₂-N(CH₃)-CH₃, n-propyle, -CH₂-CH₂-CH₂-S-CH₃, -CH₂-CH₂-CH₂-O-CH₃, -CH₂-CH₂-CH₂-N(CH₃)-CH₃, isopropyle, n-butyle, sec-butyle, isobutyle, tert-butyle, (1,1)-diméthylpropyle, n-pentyle, sec-pentyle, n-hexyle et n-heptyle ;
ou un radical choisi dans le groupe constitué par phényle, naphtyle, benzyle, (1,3)-benzodioxolyle et (1,4)-benzodioxanyle ; le radical pouvant le cas échéant à chaque fois être substitué par 1, 2, 3, 4 ou 5 substituants choisis indépendamment les uns des autres dans le groupe constitué par F, Cl, Br, -CF₃, -O-CF₃, -OCH₃, -OC₂H₅, méthyle, éthyle, n-propyle, isopropyle, n-butyle et tert-butyle ;
R^{4a} représente un radical hydrogène
ou un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle et n-heptyle ;
R^{7a} et R^{8a} représentent à chaque fois un radical hydrogène ;
R^{9a} représente un radical phényle, qui peut être substitué par 1, 2, 3, 4 ou 5 substituants choisis indépendamment les uns des autres dans le groupe constitué par F, Cl, Br et -CF₃ ;
Z^{a} représente un radical choisi dans le groupe constitué par méthyle, éthyle, n-propyle, isopropyle, n-butyle, sec-butyle, isobutyle, tert-butyle, (1,1)-diméthylpropyle, n-pentyle, sec-pentyle, n-hexyle, n-heptyle et n-octyle ;
ou un radical phényle ou naphtyle qui est substitué par 1, 2, 3, 4 ou 5 substituants R^{12a} identiques ou différents ;
R^{12a} représente un radical choisi dans le groupe constitué par F, Cl, Br, I, -CN, -CF₃, -SF₅, -OH, -O-CH₃, -O-C₂H₅, -O-CH₂-CH₂-CH₃, -O-CH₂-CH₂-CH₂-CH₃, -O-C(CH₃)₃, -O-CH₂-CH₂-CH₂-CH₂-CH₃, -O-CH₂-CH₂-C(CH₃)₃, -O-CH=CH₂, -O-CH₂-CH=CH₂, -NH₂, -O-CF₃, -S-CF₃, -SH, -S-CH₃, -S-C₂H₅, -S-CH₂-CH₂-CH₃, -S-CH₂-CH₂-CH₂-CH₃, -S-C(CH₃)₃, méthyle, éthyle, n-propyle, isopropyle, n-butyle, sec-butyle, tert-butyle, n-pentyle, n-hexyle, n-heptyle et n-octyle ;
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

29. Utilisation d'un composé selon l'une ou plusieurs des revendications 17 à 28 choisi dans le groupe constitué par :
1. la 5-[3-(4-méthoxyphényl)-but-2-énylidène]-2-morpholin-4-ylthiazol-4-one
2. la 5-[3-(4-fluorophényl)-allylidène]-2-morpholin-4-ylthiazol-4-one
3. la 2-morpholin-4-yl-5-(3-m-toluylallylidène)-thiazol-4-one
4. la 2-morpholin-4-yl-5-(3-p-toluylallylidène)-thiazol-4-one
5. la 2-morpholin-4-yl-5-octylidénéthiazol-4-one
6. la 5-butylidène-2-morpholin-4-ylthiazol-4-one
7. la 5-hexylidène-2-morpholin-4-ylthiazol-4-one
8. la 2-morpholin-4-yl-5-(3-p-toluylbut-2-énylidène)-thiazol-4-one
9. la 5-[3-(4-tert-butylphényl)-but-2-énylidène]-2-morpholin-4-ylthiazol-4-one
10. la 5-[3-(4-tert-butylphényl)-allylidène]-2-[4-(3-méthoxyphényl)-pipérazin-1-yl]-thiazol-4-one
11. la 5-[3-(4-hydroxy-3-méthoxyphényl)-allylidène]-2-thiomorpholin-4-ylthiazol-4-one
12. la 2-morpholin-4-yl-5-[3-(4-trifluorométhylphényl)-but-2-énylidène]-thiazol-4-one
13. la 2-morpholin-4-yl-5-[3-(3-trifluorométhylphényl)-but-2-énylidène]-thiazol-4-one
14. la 5-décylidène-2-morpholin-4-ylthiazol-4-one
15. la 5-[3-(4-tert-butylphényl)-allylidène]-2-morpholin-4-ylthiazol-4-one
16. la 5-[3-(4-tert-butylphényl)-allylidène]-2-[4-(3-chloropyridin-2-yl)-pipérazin-1-yl]thiazol-4-one
17. la 5-[3-(4-tert-butylphényl)-allylidène]-2-[4-(2-fluorophényl)-pipérazin-1-yl]-thiazol-4-one
18. la 5-[3-(4-tert-butylphényl)-allylidène]-2-[4-(4-chloro-[1,2,5]thiadiazol-3-yl)-pipérazin-1-yl]-thiazol-4-one
19. la 5-[3-(4-tert-butylphényl)-allylidène]-2-[4-(6-chloropyridin-2-yl)-pipérazin-1-yl]-thiazol-4-one
20. la 5-[3-(4-tert-butylphényl)-allylidène]-2-[4-(6-méthylpyridazin-3-yl)-pipérazin-1-yl]-thiazol-4-one
21. la 2-[(benzo[1,3]dioxol-5-ylméthyl)-amino]-5-[3-(4-tert-butyl-phényl)-allylidène]-thiazol-4-one
le cas échéant à chaque fois sous forme d'un de ses stéréo-isomères purs, en particulier ses énantiomères ou diastéréo-isomères, ses racémates ou sous forme d'un mélange de stéréo-isomères, en particulier des énantiomères et/ou des diastéréo-isomères, dans un rapport de mélange quelconque ou à chaque fois sous forme de sels correspondants ou à chaque fois sous forme de solvates correspondants.

30. Procédé pour la préparation de dérivés de thiazol-4-one substitués en position 2,5 de formule générale Ia selon l'une ou plusieurs des revendications 17 à 29, **caractérisé en ce qu'**on transforme de la 2-aminothiazol-4-one dans de l'acide acétique en présence d'au moins un sel choisi dans le groupe constitué par l'acétate de sodium et l'acétate de potassium ou dans un milieu réactionnel choisi dans le groupe constitué par le méthanol, l'éthanol, l'isopropanol et la n-butanol en présence d'au moins une base organique, choisie dans le groupe constitué par la triéthylamine, la pyridine, la diisopropylamine et la N-méthylmorpholine avec au moins un composé de formule générale R-C(=O)-H, dans laquelle R représente -CH=C (CH₃) -W^{a}-Z^{a}, -CH=CH-W^{a}-Z^{a}, -C (CH₃) =CH-W^{a}-Z^{a}, -C (phényl) =CH-W^{a}-Z^{a}, -CBr=CH-W^{a}-Z^{a}, -CCl=CH-W^{a}-Z^{a}, -CF=CH-W^{a}-Z^{a}, -C (OH) =CH-W^{a}-Z^{a}, -CH₂-CH₂-W^{a}-Z^{a}, -CH₂-CH(CH₃)-W^{a}-Z^{a} ou -CH(CH₃)-CH₂-W^{a}-Z^{a}, W^{a} et Z^{a} ayant la signification selon l'une ou plusieurs des revendications 17 à 29, en au moins un composé de formule générale IIa, 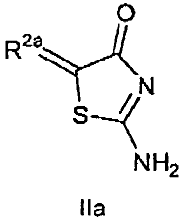 dans laquelle R^{2a} a la signification selon l'une ou plusieurs des revendications 17 à 29, et celui-ci est le cas échéant purifié et/ou isolé et on transforme au moins un composé de formule générale IIa dans un milieu réactionnel en présence d'au moins une base, de préférence en présence d'au moins un sel d'hydrure métallique ou d'un sel d'alcoolate métallique, de manière particulièrement préférée en présence d'un sel d'hydrure métallique ou d'un sel d'alcoolate métallique, choisi dans le groupe constitué par l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R^{3a} ou de formule générale LG-R^{7a} ou de formule générale LG-R^{3a}, dans lesquelles LG représente à chaque fois un groupe partant, de préférence un atome d'halogène, de manière particulièrement préférée un atome de chlore et R^{3a}, R^{5a} et R^{7a} présentent à chaque fois la signification selon l'une ou plusieurs des revendications 17 à 29, en au moins un composé de formule générale Ia, dans laquelle R^{2a} présente la signification susmentionnée et R^{1a} représente un groupe NHR^{3a}, NHR^{5a} ou NHR^{7a}, et celui-ci est le cas échéant purifié et/ou isolé,
et on transforme le cas échéant au moins un composé de formule générale IIa, dans laquelle R^{2a} présente la signification susmentionnée et R^{1a} représente un groupe NHR^{7a}, dans un milieu réactionnel avec au moins un isocyanate de formule générale R^{8a}-N=C=O, dans laquelle R^{8a} a la signification selon l'une ou plusieurs des revendications 17 à 29, le cas échéant en présence d'au moins une base, de préférence en présence d'au moins une base choisie dans le groupe constitué par la triéthylamine, la 4,4-diméthylaminopyridine et la diisopropyléthylamine, en au moins un composé de formule générale Ia, dans laquelle R^{2a} a la signification susmentionnée et R^{1a} représente un groupe NR^{7a}-C(=O)-NHR^{8a}, et celui-ci est le cas échéant purifié et/ou isolé ;
et on transforme le cas échéant au moins un composé de formule générale Ia, dans laquelle R^{2a} a la signification susmentionnée et R^{1a} représente un groupe NR^{7a}-C(=O)-NHR^{8a}, dans un mélange réactionnel en présence d'au moins une base, de préférence en présence d'au moins un sel d'hydrure métallique ou d'un sel d'alcoolate métallique, de manière particulièrement préférée en présence d'un sel d'hydrure métallique ou d'un sel d'alcoolate métallique choisi dans le groupe constitué par l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R^{9a}, dans laquelle LG représente un groupe partant, de préférence un atome d'halogène, de manière particulièrement préférée un atome de chlore et R^{9a} a la signification selon l'une ou plusieurs des revendications 17 à 29, en au moins un composé de formule générale Ia, dans laquelle R^{2a} a la signification susmentionnée et R^{1a} représente un groupe NR^{7a}-C(=O)-NR^{8a}R^{9a} et celui-ci est le cas échéant purifié et/ou isolé ; ou
on transforme le cas échéant au moins un composé de formule générale IIa, dans laquelle R^{2a} a la signification susmentionnée et R^{1a} représente un groupe NHR^{5a}, dans un milieu réactionnel, le cas échéant en présence d'au moins une base avec au moins un composé de formule générale R^{6a}-C(=O)-LG, dans laquelle R^{6a} a la signification selon l'une ou plusieurs des revendications 17 à 29 et LG représente un groupe partant, de préférence un radical halogène, ou dans un mélange réactionnel en présence d'au moins un réactif de couplage, le cas échéant en présence d'au moins une base avec un composé de formule générale R^{6a}-C(=O)-OH, dans laquelle R^{6a} a la signification selon l'une ou plusieurs des revendications 17 à 29, en au moins un composé de formule générale Ia, dans laquelle R^{2a} a la signification susmentionnée et R^{1a} représente un groupe NR^{5a}-C(=O)-R^{6a}, et celui-ci est le cas échéant purifié et/ou isolé ou
on transforme le cas échéant au moins un composé de formule générale Ia, dans laquelle R^{2a} a la signification susmentionnée et R^{1a} représente un groupe NHR^{3a}, dans un milieu réactionnel en présence d'au moins une base, de préférence en présence d'au moins un sel d'hydrure métallique ou d'un sel d'alcoolate métallique, de manière particulièrement préférée en présence d'un sel d'hydrure métallique ou d'un sel d'alcoolate métallique choisi dans le groupe constitué par l'hydrure de sodium, l'hydrure de potassium, le tert-butanolate de potassium, le tert-butanolate de sodium, le méthanolate de potassium, le méthanolate de sodium, l'éthanolate de sodium et l'éthanolate de potassium, avec au moins un composé de formule générale LG-R^{4a}, dans laquelle LG représente un groupe partant, de préférence un atome d'halogène, de manière particulièrement préférée un atome de chlore et R^{4a} a la signification selon l'une ou plusieurs des revendications 17 à 29, en au moins un composé de formule générale Ia, dans laquelle R^{2a} a la signification susmentionnée et R^{1a} représente un groupe NR^{3a}R^{4a} et celui-ci est le cas échéant purifié et/ou isolé.

31. Procédé pour la préparation de dérivés de thiazol-4-one substitués en position 2,5 de formule générale Ia selon l'une ou plusieurs des revendications 17 à 29, **caractérisé en ce qu'**on transforme au moins un composé de formule générale R^{1a}-CN, dans laquelle R^{1a} a la signification selon l'une ou plusieurs des revendications 17 à 29, à l'exception d'un groupe NR^{3a}R^{4a}, d'un groupe NR^{5a}-C(=O)-R^{6a} et d'un groupe NR^{7a}-C(=O)-NR^{8a}R^{9a}, dans un milieu réactionnel choisi dans le groupe constitué par le méthanol, l'éthanol, l'isopropanol et le n-butanol, en présence d'au moins une base organique choisie dans le groupe constitué par la triéthylamine, la pyridine, la diisopropylamine et la N-méthylmorpholine ou dans de la pyridine comme milieu réactionnel avec de l'acide thioglycolique en au moins un composé de formule générale IIIa, 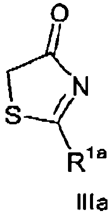 dans laquelle R^{1a} a la signification selon l'une ou plusieurs des revendications 17 à 29 à l'exception d'un groupe NR^{3a}R^{4a}, d'un groupe NR^{5a}-C(=O)-R^{6a} et d'un groupe NR^{8a}-C(=O)-NR^{8a}R^{9a} et celui-ci est le cas échéant purifié et/ou isolé ;
ou on transforme au moins un composé de formule générale H₂N-C (=8)-NHR^{1a}, dans laquelle R^{1a} a la signification selon l'une ou plusieurs des revendications 17 à 29 à l'exception d'un groupe NR^{3a}R^{4a}, d'un groupe NR^{5a}-C(=O)-R^{6a} et d'un groupe NR^{7a}-C(=O)-NR^{6a}R^{9a}, dans un milieu réactionnel choisi dans le groupe constitué par le méthanol, l'éthanol, l'isopropanol et le n-butanol, le cas échéant en présence d'au moins une base organique choisie dans le groupe constitué par la triéthylamine, la pyridine, la diisopropylamine et la N-méthylmorpholine, avec de l'acide chloroacétique en au moins un composé de formule générale IIIa, dans laquelle R^{1a} a la signification selon l'une ou plusieurs des revendications 17 à 29 à l'exception d'un groupe NR^{3a}R^{4a}, d'un groupe NR^{5a}-C(=O)-R^{6a} et d'un groupe NR^{7a}-C(=O)-NR^{8a}R^{9a} et celui-ci est le cas échéant purifié et/ou isolé ;
et on transforme au moins un composé de formule générale IIIa dans de l'acide acétique en présence d'au moins un sel choisi dans le groupe constitué par l'acétate de sodium et l'acétate de potassium ou dans un milieu réactionnel choisi dans le groupe constitué par le méthanol, l'éthanol, l'isopropanol et le n-butanol en présence d'au moins une base organique choisie dans le groupe constitué par la triéthylamine, la pyridine, la diisopropylamine et la N-méthylmorpholine avec au moins un composé de formule générale R-C(=O)-H, dans laquelle R représente -CH=C(CH₃)-W^{a}-Z^{a}, -CH=CH-W^{a}-Z^{a}, -C(CH₃)=CH-W^{a}-Z^{a}, -C (phényl)=CH-W^{a}-Z^{a}, -CBr=CH-W^{a}-Z^{a}, -CCl=CH-W^{a}-Z^{a}, -CF=CH-W^{a}-Z^{a}, -C(OH)=CH-W^{a}-Z^{a}, -CH₂-CH₂-W^{a}-Z^{a}, -CH₂-CH(CH₃)-W^{a}-Z^{a} ou -CH(CH₃)-CH₂-W^{a}-Z^{a}, W^{a} et Z^{a} ayant la signification selon l'une ou plusieurs des revendications 17 à 29, en au moins un composé de formule générale Ia, dans laquelle R^{1a} a la signification selon l'une ou plusieurs des revendications 17 à 29 à l'exception d'un groupe NR^{3a}R^{4a}, d'un groupe NR^{5a}-C(=O)-R^{6a} et d'un groupe NR^{7a}-C(=O)-NR^{8a}R^{9a} et R^{2a} a la signification selon l'une ou plusieurs des revendications 17 à 29.

32. Médicament contenant au moins un composé selon l'une ou plusieurs des revendications 17 à 29 et le cas échéant un ou plusieurs adjuvants physiologiquement acceptables.

33. Médicament selon la revendication 32 destiné à la prophylaxie et/ou au traitement de la douleur, de préférence de la douleur choisie dans le groupe constitué par la douleur aiguë, la douleur chronique, la douleur viscérale, la douleur neuropathique et la douleur articulaire.

34. Médicament selon la revendication 32 destiné à la prophylaxie et/ou au traitement d'une ou de plusieurs maladies, choisies dans le groupe constitué par la douleur articulaire ; les migraines ; les dépressions ; les névropathies ; les lésions nerveuses ; les maladies de neurodégénérescence, de préférence choisies dans le groupe constitué par la sclérose en plaques, la maladie d'Alzheimer, la maladie de Parkinson et la maladie d'Huntington ; les dysfonctionnement cognitifs, de préférence les états de déficience cognitive, de manière particulièrement préférée les troubles de la mémoire ; l'épilepsie ; les maladies des voies respiratoires, de préférence choisies dans le groupe constitué par l'asthme et l'inflammation pulmonaire ; la toux ; l'incontinence urinaire ; une vessie hyperactive (overactive bladder, OAB) ; les ulcères peptiques ; le syndrome de l'intestin irritable ; les attaques ; les irritations oculaires ; les irritations de la peau ; les maladies neurotiques de la peau ; les maladies inflammatoires, de préférence les inflammations de l'intestin ; la diarrhée ; le prurit ; les troubles d'absorption des aliments, de préférence choisis dans le groupe constitué par la boulimie, la cachexie, l'anorexie et l'obésité ; la dépendance aux médicaments ; l'abus de médicaments ; les symptômes de sevrage lors de l'abus de médicaments ; le développement de la tolérance aux médicaments, de préférence aux opioïdes naturels ou synthétiques ; la dépendance aux drogues ; l'abus de drogues ; les symptômes de sevrage lors de la dépendances aux drogues ; la dépendance à l'alcool ; l'abus d'alcool et les symptômes de sevrages lors de la dépendance à l'alcool ; destiné à la diurèse ; à l'antinatriurèse ; à l'influence du système cardiovasculaire ; à l'augmentation de la vigilance ; à l'augmentation de la libido ; à la modulation de l'activité motrice ; à l'anxiolyse ; à l'anesthésie locale et/ou à l'inhibition d'effets secondaires non souhaités, de préférence choisis dans le groupe constitué par l'hyperthermie, l'hypertension et le rétrécissement des bronches, déclenchés par l'administration d'agonistes du récepteur 1 des vanilloïdes (récepteurs VR1/TRPV1), de préférence choisis dans le groupe constitué par la capsaïcine, la résinifératoxine, l'olvanil, l'arvanil, le SDZ-249665, le SDZ-249482, le nuvanil et le capsavanil (DA-5018).
